# EUROPEAN PATENT APPLICATION

(11) **EP 3 868 742 A1**
(43) Date of publication of application: **25.08.2021**
(21) Application number: 19871449.5
(22) Date of filing: 10.10.2019
(51) Int. Cl.: C07D 241/18, C07D 498/10, C07D 471/10, A61K 31/495, A61P 35/00

(54) **REGULATOR OF NITROGEN-CONTAINING HETEROAROMATIC DERIVATIVES, PREPARATION METHOD THEREFOR AND USE THEREOF**

(30) Priority: 10.10.2018 CN 201811178487; 16.01.2019 CN 201910040837; 22.04.2019 CN 201910325374; 18.06.2019 CN 201910528210; 28.08.2019 CN 201910804612
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN)
(72) Inventor: LIU, Shiqiang, Lianyungang, Jiangsu 222047 (CN); YUAN, Yida, Lianyungang, Jiangsu 222047 (CN); BAO, Meng, Lianyungang, Jiangsu 222047 (CN); HUANG, Shengai, Lianyungang, Jiangsu 222047 (CN); BAO, Rudi, Lianyungang, Jiangsu 222047 (CN)
(74) Representative: Viganò, Elena
(86) International application number: PCT/CN2019/110314
(87) International publication number: WO 2020/073949

(57) **Abstract**

The present invention relates to a regulator of nitrogen-containing heteroaromatic derivatives, a preparation method therefor and the use thereof. In particular, the present invention relates to a compound as shown in the general formula (I), a preparation method therefor and a pharmaceutical composition containing the compound, and the use thereof as a protein tyrosine phosphatase-2C (SHP2) inhibitor in the treatment of diseases or conditions such as leukemia, neuroblastoma, melanoma, breast cancer, lung cancer and colorectal cancer, wherein the definition of each substituent in the general formula (I) is the same as that in the description.

## Description

### FIELD OF THE INVENTION

The present invention belongs to the field of drug synthesis, and specifically relates to a nitrogen-containing heteroaromatic derivative inhibitor, a method for preparing the same, and a use thereof.

### BACKGROUND OF THE INVENTION

Src homology-2 domain-containing phosphatase 2 (SHP-2), also known as tyrosine-protein phosphatase non-receptor type 11 (PTPN11), is encoded by PTPN11 gene, and belongs to the protein tyrosine phosphatase (PTP) family. As a downstream signal molecule of cytokines, growth factors and other extracellular stimulating factors, SHP-2 is widely expressed in various tissues and cells of the body, participates in cell signal transduction, and regulates cell growth, differentiation, migration, metabolism, gene transcription, immune response and the like.

SHP-2 has three main structural parts: SH-2 domain (N-SH2 and C-SH2), PTP active domain, and C-terminal (having a tyrosine phosphorylation site). The SH2 domain is highly conserved, which is a phosphotyrosine binding site and mediates the binding of the PTP domain to its ligand.

SHP-2 has two main states *in vivo*: inactivated state and activated state. In the inactivated state, N-SH2 in SHP-2 binds to the PTP domain, because the PTP domain is occupied, SHP-2 is inactivated. When N-SH2 specifically binds to the phosphorylated tyrosine residue ligand, the PTP domain is re-exposed and SHP-2 resumes its activity. Latest studies show that SHP-2 can also form dimers *in vivo,* which can also lead to SHP-2 inactivation.

SHP-2 mainly functions by regulating signal pathways such as ERK/MAPK, JAK-STAT, PI3K/AKT, Hippo, Wnt/β-catenin, so as to maintain biological development and homeostasis. Specific studies show that SHP-2 participates in the activation of the ERK/MAPK pathway by directly binding to receptor tyrosine kinase (RTK) or scaffold. In addition, activated SHP-2 can also recruit GRB2/SOS, and indirectly promote the activation of the RAS signaling pathway. Moreover, SHP-2 is also involved in signal transduction that inhibits immune response. For example, SHP-2 and SHP-1 can bind to and activate immunosuppressive receptors (such as PD-1), and block T cell activation.

As an important cell signaling factor, SHP-2 mutations are closely related to many diseases. Studies show that SHP-2 mutations are found in neuroblastoma, acute myeloid leukemia (AML, 4%), breast cancer, non-small cell lung cancer (NSCLC, 10%), lung adenocarcinoma (30%), esophageal cancer, head and neck tumor, melanoma and gastric cancer.

The mutation sites of SHP-2 mostly occur in N-SH2 and PTP active regions. The mutations reduce the mutual inhibition of N-CH2/PTP domains, and lead to highly active SHP-2, for example, Cys459Ser mutant, E76K mutant and the like will affect the activity of SHP-2. Studies show that highly active SHP-2 is closely related to inflammation, liver cirrhosis, the toxin CagA secreted by *Helicobacter pylori* and the like. Highly active SHP-2 can lead to tumor regeneration and development, and is equivalent to a proto-oncogene. With the deepening of the understanding of SHP-2, SHP-2 has been used as a tumor treatment target for drug development.

At present, several SHP-2 allosteric inhibitors have entered the clinical research phase. For example, TNO-155 developed by Novartis, RMC-4630 developed by Revolution Medicine, and JAB-3068 developed by Beijing Jacobio have all entered the phase I clinical research phase. However, there is no SHP-2 inhibitor on the market for the treatment of Noonan syndrome, leopard syndrome, leukemia, neuroblastoma, melanoma, breast cancer, esophageal cancer, head and neck tumor, lung cancer and colon cancer. Therefore, there is an urgent need to develop a class of SHP-2 inhibitor drugs with good pharmaceutical properties.

### SUMMARY OF THE INVENTION

The objective of the present invention is to provide a compound of formula (I), a stereoisomer thereof or a pharmaceutically acceptable salt thereof, wherein the structure of the compound of formula (I) is as follows: 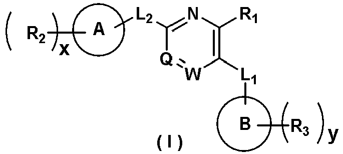 wherein:
W is selected from the group consisting of CR₄ and N;
Q is selected from the group consisting of CR₅ and N;
L₁ is selected from the group consisting of a bond, oxygen, sulfur, alkylene, alkenyl, alkynyl, cycloalkyl, heterocyclyl and -NRₐₐ-;
L₂ is selected from the group consisting of a bond, oxygen and sulfur;
ring A is selected from the group consisting of cycloalkyl, heterocyclyl, aryl and heteroaryl, wherein the cycloalkyl, heterocyclyl, aryl and heteroaryl are each optionally further substituted by one or more substituent(s) selected from the group consisting of deuterium, alkyl, haloalkyl, halogen, amino, oxo, nitro, cyano, hydroxy, alkenyl, alkynyl, alkoxy, haloalkoxy, hydroxyalkyl, cycloalkyl, heterocyclyl, aryl, heteroaryl, -(CH₂)ₙ₁-, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)_{b1}C(O)NRₐₐR_{bb}, -(CH₂)_{b1}NRₐₐC(O)R_{bb} and -(CH₂)ₙ₁NRₐₐS(O)ₘ₁R_{bb};
ring B is selected from the group consisting of cycloalkyl, heterocyclyl, aryl and heteroaryl, wherein the cycloalkyl, heterocyclyl, aryl and heteroaryl are each optionally further substituted by one or more substituent(s) selected from the group consisting of deuterium, alkyl, haloalkyl, halogen, amino, oxo, nitro, cyano, hydroxy, alkenyl, alkynyl, alkoxy, haloalkoxy, hydroxyalkyl, cycloalkyl, heterocyclyl, aryl, heteroaryl, -(CH₂)ₙ₁-, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)_{b1}C(O)NRₐₐR_{bb}, -(CH₂)_{b1}NRₐₐC(O)R_{bb} and -(CH₂)ₙ₁NRₐₐS(O)ₘ₁R_{bb};
R₁ is selected from the group consisting of hydrogen, deuterium, alkyl, deuterated alkyl, haloalkyl, alkoxy, haloalkoxy, hydroxyalkyl, halogen, amino, nitro, hydroxy, cyano, alkenyl, alkynyl, cycloalkyl, heterocyclyl, oxoheterocyclyl, thioxoheterocyclyl, aryl, heteroaryl, -(CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, -N=S=O(RₐₐP_{bb}), -P(O)RₐₐR_{bb}, -(CH₂)ₙ₁NRₐₐC(O)R_{bb} and -(CH₂)ₙ₁NRₐₐS(O)ₘ₁R_{bb}, wherein the alkyl, deuterated alkyl, haloalkyl, hydroxyalkyl, alkoxy, haloalkoxy, oxo, amino, alkenyl, alkynyl, cycloalkyl, heterocyclyl, aryl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
R₂ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐP_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)_{b1}NRₐₐS(O)ₘ₁R_{bb};
or, two R₂ on the same carbon atom or different carbon atoms are bonded to form a cycloalkyl, heterocyclyl, aryl or heteroaryl, wherein the cycloalkyl, heterocyclyl, aryl and heteroaryl are each optionally further substituted by one or more substituent(s) selected from the group consisting of deuterium, substituted or unsubstituted alkyl, substituted or unsubstituted cycloalkyl, substituted or unsubstituted haloalkyl, halogen, substituted or unsubstituted amino, oxo, thioxo, nitro, cyano, hydroxy, alkoxycarbonyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted heterocyclyl, substituted or unsubstituted aryl, substituted or unsubstituted heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)_{b1}C(O)NRₐₐR_{bb}, -(CH₂)ₙ₁C(O)NHRₐₐ, -(CH₂)ₙ₁NRₐₐC(O)R_{bb} and -(CH₂)_{b1}NRₐₐS(O)ₘ₁R_{bb};
R₃ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb}, wherein the alkyl, deuterated alkyl, haloalkyl, alkoxy, hydroxyalkyl, haloalkoxy, amino, alkenyl, alkynyl, cycloalkyl, heterocyclyl, aryl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
or, two R₃ on the same carbon atom or different carbon atoms are bonded to form a cycloalkyl, heterocyclyl, aryl or heteroaryl, wherein the cycloalkyl, heterocyclyl, aryl and heteroaryl are each optionally further substituted by one or more substituent(s) selected from the group consisting of deuterium, substituted or unsubstituted alkyl, substituted or unsubstituted cycloalkyl, substituted or unsubstituted haloalkyl, halogen, substituted or unsubstituted amino, oxo, thioxo, nitro, cyano, hydroxy, alkoxycarbonyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted heterocyclyl, substituted or unsubstituted aryl, substituted or unsubstituted heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)_{b1}NRₐₐC(O)R_{bb} and -(CH₂)ₙ₁NRₐₐS(O)ₘ₁R_{bb};
R₄ and R₅ are identical or different and are each independently selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ -(CH₂)ₙ₁NRₐₐR_{bb}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb};
Rₐₐ, R_{bb}, R_{cc} and R_{dd} are identical or different and are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are each optionally further substituted by one or more substituent(s) selected from the group consisting of deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

In a preferred embodiment of the present invention, the compound of formula (I), the stereoisomer thereof or the pharmaceutically acceptable salt thereof is a compound of formula (II), a stereoisomer thereof or a pharmaceutically acceptable salt thereof: 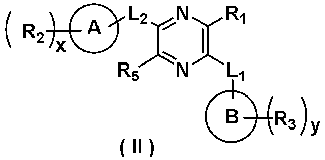 wherein:
L₁ is selected from the group consisting of a bond, oxygen, alkenyl, alkynyl, heterocyclyl and -NRₐₐ-;
L₂ is selected from the group consisting of a bond and sulfur;
ring A is selected from the group consisting of aryl and heteroaryl;
ring B is selected from the group consisting of cycloalkyl, heterocyclyl, aryl and heteroaryl;
R₁ is selected from the group consisting of hydrogen, halogen, alkyl, hydroxyalkyl, heterocyclyl, heteroaryl, hydroxyalkyl and -(CH₂)ₙ₁ORₐₐ, wherein the alkyl, hydroxyalkyl, heterocyclyl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₅ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are identical or different and are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

In a preferred embodiment of the present invention, the compound of formula (I), the stereoisomer thereof or the pharmaceutically acceptable salt thereof is a compound of formula (III), a stereoisomer thereof or a pharmaceutically acceptable salt thereof: 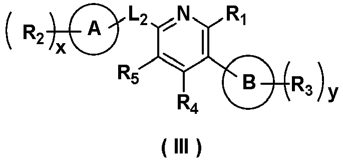 wherein:
L₂ is selected from the group consisting of a bond and sulfur;
ring A is selected from the group consisting of aryl and heteroaryl;
ring B is selected from the group consisting of heterocyclyl, aryl and heteroaryl;
R₁ is selected from the group consisting of hydrogen, alkyl, hydroxyalkyl, heterocyclyl, heteroaryl and -(CH₂)ₙ₁ORₐₐ;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₄ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
R₅ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are identical or different and are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

In a preferred embodiment of the present invention, the compound of formula (I), the stereoisomer thereof or the pharmaceutically acceptable salt thereof is a compound of formula (IV), a stereoisomer thereof or a pharmaceutically acceptable salt thereof: 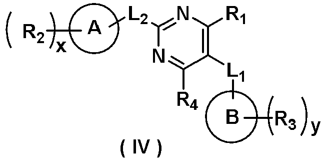 wherein:
L₁ is selected from the group consisting of a bond, oxygen, alkenyl, alkynyl, heterocyclyl and -NRₐₐ-;
L₂ is selected from the group consisting of a bond and sulfur;
ring A is selected from the group consisting of aryl and heteroaryl;
ring B is selected from the group consisting of cycloalkyl, heterocyclyl, aryl and heteroaryl;
R₁ is selected from the group consisting of hydrogen, alkyl, hydroxyalkyl, heterocyclyl, heteroaryl and -(CH₂)ₙ₁ORₐₐ;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₄ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are identical or different and are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

In a preferred embodiment of the present invention, the compound of formula (I), the stereoisomer thereof or the pharmaceutically acceptable salt thereof is a compound of formula (V), a stereoisomer thereof or a pharmaceutically acceptable salt thereof: 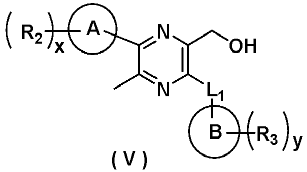 wherein:
ring A, ring B, L₁, R₂, R₃, x and y are as defined in formula (II).

In a preferred embodiment of the present invention, the compound of formula (I), the stereoisomer thereof or the pharmaceutically acceptable salt thereof is a compound of formula (VI), a stereoisomer thereof or a pharmaceutically acceptable salt thereof: 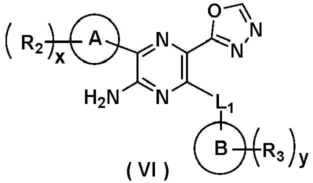 wherein:
ring A, ring B, L₁, R₂, R₃, x and y are as defined in formula (II).

In a preferred embodiment of the present invention, the compound of formula (I), the stereoisomer thereof or the pharmaceutically acceptable salt thereof is a compound of formula (VII), a stereoisomer thereof or a pharmaceutically acceptable salt thereof: 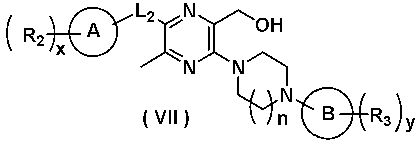 wherein:
n is an integer of 0, 1, 2 or 3; and
ring A, ring B, L₂, R₂, R₃, x and y are as defined in formula (II).

In a preferred embodiment of the present invention, the compound of formula (I), the stereoisomer thereof or the pharmaceutically acceptable salt thereof is a compound of formula (IIA) or (IIB), a stereoisomer thereof or a pharmaceutically acceptable salt thereof: 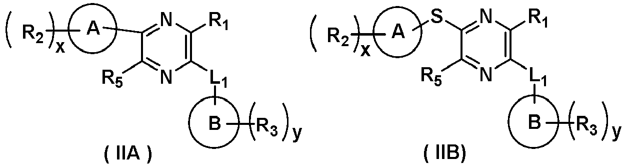 wherein:
ring A, ring B, L₁, R₁ to R₃, R₅, x and y are as defined in formula (II).

In a preferred embodiment of the present invention, the compound of formula (I), the stereoisomer thereof or the pharmaceutically acceptable salt thereof is a compound of formula (IIIA) or (IIIB), a stereoisomer thereof or a pharmaceutically acceptable salt thereof: 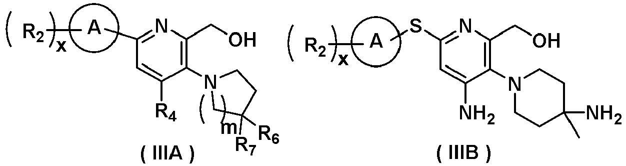 wherein:
m is an integer of 0, 1, 2 or 3;
R₆ and R₇ are identical or different and are each independently selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb} and -(CH₂)ₙ₁NRₐₐC(O)R_{bb};
or, R₆ and R₇ are bonded to form a cycloalkyl, heterocyclyl, aryl or heteroaryl, wherein the cycloalkyl, heterocyclyl, aryl and heteroaryl are each further substituted by one or more substituent(s) selected from the group consisting of deuterium, alkyl, cycloalkyl, haloalkyl, halogen, amino, oxo, thioxo, nitro, cyano, hydroxy, alkenyl, alkynyl, alkoxy, haloalkoxy, hydroxyalkyl, heterocyclyl, aryl and heteroaryl; and
ring A, R₂, R₄, Rₐₐ, R_{bb}, R_{cc}, R_{dd} and x are as defined in formula (III).

In a preferred embodiment of the present invention, the compound of formula (I), the stereoisomer thereof or the pharmaceutically acceptable salt thereof is a compound of formula (IVA) or (IVB), a stereoisomer thereof or a pharmaceutically acceptable salt thereof: 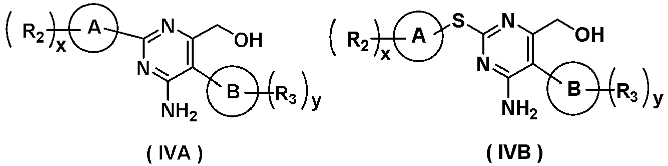 wherein:
ring A, ring B, R₂, R₃, x and y are as defined in formula (IV).

In a preferred embodiment of the present invention, the compound of formula (I), the stereoisomer thereof or the pharmaceutically acceptable salt thereof is a compound of formula (VA), a stereoisomer thereof or a pharmaceutically acceptable salt thereof: 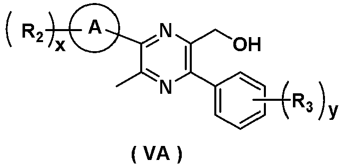 wherein:
ring A, R₂, R₃, x and y are as defined in formula (II).

In a preferred embodiment of the present invention, the compound of formula (I), the stereoisomer thereof or the pharmaceutically acceptable salt thereof is a compound of formula (VIII), a stereoisomer thereof or a pharmaceutically acceptable salt thereof: 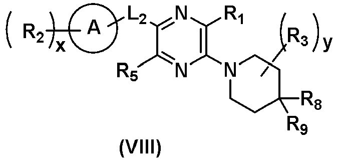 wherein:
L₂ is selected from the group consisting of a bond and sulfur;
ring A is selected from the group consisting of C₆₋₁₂ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₃ is selected from the group consisting of hydrogen, amino, halogen, oxo, hydroxy, C₁₋₆ alkyl and C₃₋₈ cycloalkyl;
R₅ is selected from the group consisting of hydrogen, deuterium, halogen, amino, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl and C₃₋₈ cycloalkyl;
R₈ and R₉ are identical or different and are each independently selected from the group consisting of hydrogen, deuterium, halogen, amino, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl and 5 to 12 membered heteroaryl;
or, R₈ and R₉ are bonded to form a C₃₋₁₂ cycloalkyl or 3 to 12 membered heterocyclyl, wherein the C₃₋₁₂ cycloalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
n₁ is an integer of 0, 1, 2, 3, 4 or 5;
x is an integer of 0, 1, 2, 3, 4 or 5; and
y is an integer of 0, 1, 2, 3, 4 or 5.

In a preferred embodiment of the present invention, the compound of formula (I), the stereoisomer thereof or the pharmaceutically acceptable salt thereof is a compound of formula (IX), a stereoisomer thereof or a pharmaceutically acceptable salt thereof: 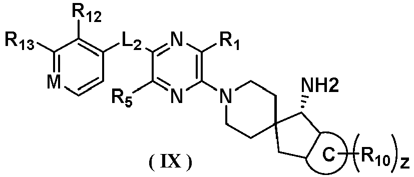 wherein:
M is selected from the group consisting of CR₁₁ and N;
L₂ is selected from the group consisting of a bond and sulfur;
ring C is selected from the group consisting of C₃₋₈ cycloalkyl, 3 to 12 membered heterocyclyl, C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of hydrogen, amino and C₁₋₆ alkyl;
R₁₀ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₁ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₃ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and z is an integer of 0, 1, 2 or 3.

In a preferred embodiment of the present invention, the compound of formula (I), the stereoisomer thereof or the pharmaceutically acceptable salt thereof is a compound of formula (X), a stereoisomer thereof or a pharmaceutically acceptable salt thereof: 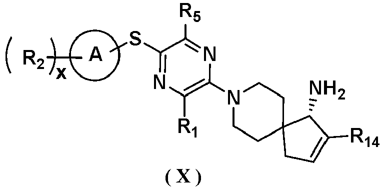 wherein:
ring A is selected from the group consisting of C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of hydrogen, amino and C₁₋₆ alkyl;
R₁₄ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and x is an integer of 0, 1, 2, 3, 4 or 5.

In a preferred embodiment of the present invention, the compound of formula (I), the stereoisomer thereof or the pharmaceutically acceptable salt thereof is a compound of formula (XI), a stereoisomer thereof or a pharmaceutically acceptable salt thereof: 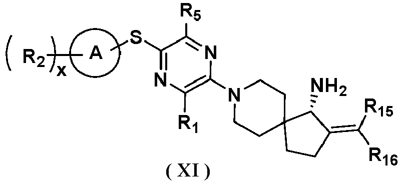 wherein:
ring A is selected from the group consisting of C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of amino and C₁₋₆ alkyl;
R₁₅ and R₁₆ are identical or different and are each independently selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and
x is an integer of 0, 1, 2, 3, 4 or 5.

In a preferred embodiment of the present invention, the compound of formula (I), the stereoisomer thereof or the pharmaceutically acceptable salt thereof is characterized in that the specific structure thereof is as shown in formula (X-A): 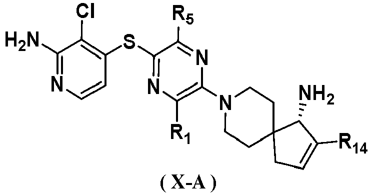 wherein:
R₁, R₅ and R₁₄ are as defined in formula (X).

In a preferred embodiment of the present invention, the compound of formula (I), the stereoisomer thereof or the pharmaceutically acceptable salt thereof is characterized in that the specific structure thereof is as shown in formula (XII): 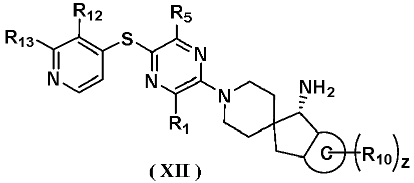 wherein:
ring C is selected from the group consisting of C₃₋₈ cycloalkyl, 3 to 12 membered heterocyclyl, C₆₋₁₀ aryl and 5 to 12 membered heteroaryl, and preferably cyclopropyl, phenyl, pyridyl, pyrazolyl, imidazolyl, oxazolyl or thiazolyl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁ is preferably hydrogen, halogen, C₁₋₃ alkyl, C₁₋₃ hydroxyalkyl or C₃₋₆ cycloalkyl, wherein the C₁₋₃ alkyl, C₁₋₃ hydroxyalkyl or C₃₋₆ cycloalkyl is optionally further substituted by one or more substituent(s) selected from the group consisting of hydrogen, halogen and hydroxy;
R₁ is more preferably hydrogen, fluorine, chlorine, bromine, methyl, hydroxyethyl or cyclopropyl substituted by hydroxy;
R₅ is selected from the group consisting of hydrogen, amino and C₁₋₆ alkyl, and preferably hydrogen, amino or methyl;
R₁₀ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₀ is preferably hydrogen, halogen, C₁₋₃ alkyl or C₃₋₆ cycloalkyl;
R₁₀ is more preferably hydrogen, chlorine or methyl;
R₁₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₂ is preferably halogen, C₁₋₃ alkyl or C₃₋₆ cycloalkyl;
R₁₂ is more preferably fluorine, chlorine, cyclopropyl or methyl;
R₁₃ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₃ is preferably hydrogen, fluorine, chlorine, amino, methyl, cyclopropyl, pyrrolidinyl, azetidinyl, fluorine-substituted azetidinyl, azetidinonyl, morpholinyl or pyrrolidonyl;
z is 0, 1, 2 or 3; and
when R₅ is amino or hydrogen, R₁₂ is chlorine and R₁₃ is amino, dimethylamino, fluorine or chlorine, then R₁ is not hydrogen.

In a preferred embodiment of the present invention, the compound of formula (I), the stereoisomer thereof or the pharmaceutically acceptable salt thereof is characterized in that the specific structure thereof is as shown in formula (XII-A): 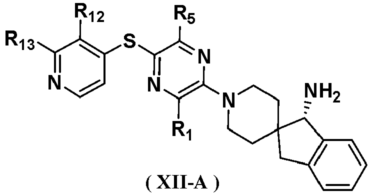 wherein:
R₁, R₅ and R₁₂ to R₁₃ are as defined in formula (XII).

In a preferred embodiment of the present invention, the compound of formula (I), the stereoisomer thereof or the pharmaceutically acceptable salt thereof is characterized in that the specific structure thereof is as shown in formula (XII-B): 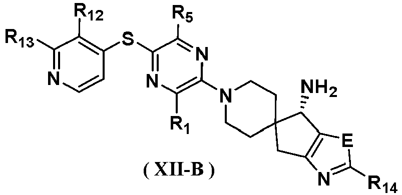 wherein:
E is selected from the group consisting of O, S and NR₁₅;
R₁₄ is selected from the group consisting of hydrogen, halogen and C₁₋₆ alkyl;
R₁₅ is selected from the group consisting of hydrogen and C₁₋₆ alkyl;
R₁, R₅ and R₁₂ to R₁₃ are as defined in formula (XII).

In a preferred embodiment of the present invention, the compound of any formula, the stereoisomer thereof or the pharmaceutically acceptable salt thereof is characterized in that:
ring A is selected from the group consisting of: 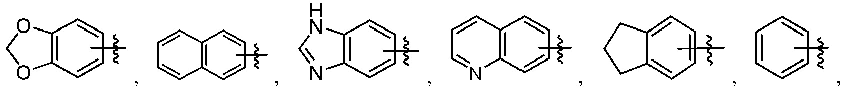 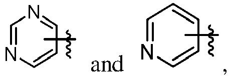
ring A is more preferably selected from the group consisting of:

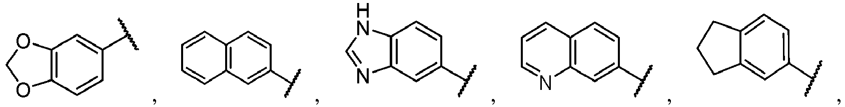

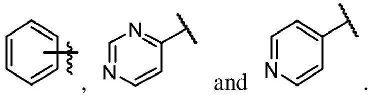

In a preferred embodiment of the present invention, the compound of any formula, the stereoisomer thereof or the pharmaceutically acceptable salt thereof is characterized in that:
ring B is selected from the group consisting of:

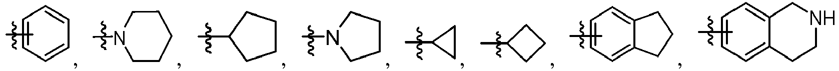

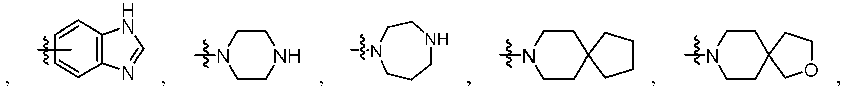

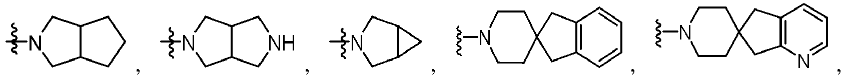

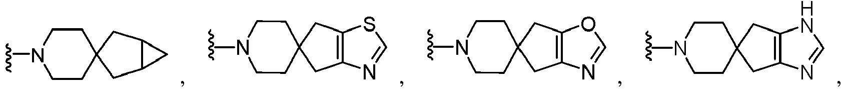

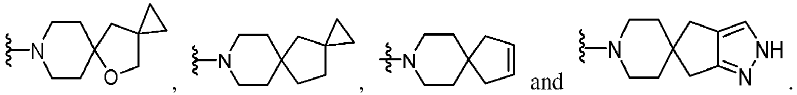

In a preferred embodiment of the present invention, the compound of any formula, the stereoisomer thereof or the pharmaceutically acceptable salt thereof is characterized in that:
ring C is selected from the group consisting of:

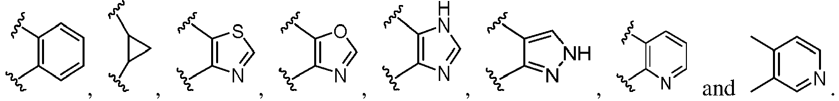

In a preferred embodiment of the present invention, the compound of any formula, the stereoisomer thereof or the pharmaceutically acceptable salt thereof is characterized in that,
R₁ is selected from the group consisting of hydrogen, halogen, C₁₋₆ alkyl, C₃₋₈ cycloalkyl, C₁₋₆ hydroxyalkyl, 3 to 8 membered heterocyclyl, 5 to 8 membered heteroaryl and -(CH₂)ₙ₁ORₐₐ, wherein the C₁₋₆ alkyl, C₃₋₈ cycloalkyl, C₁₋₆ hydroxyalkyl, 3 to 8 membered heterocyclyl and 5 to 8 membered heteroaryl are each optionally further substituted by one or more substituent(s) selected from the group consisting of hydrogen, deuterium, halogen, amino, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, 3 to 8 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl and 3 to 8 membered heterocyclyl are each optionally further substituted by one or more substituent(s) selected from the group consisting of hydrogen, deuterium, halogen, amino, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₃ is selected from the group consisting of hydrogen, amino, C₁₋₆ alkyl, C₃₋₈ cycloalkyl, 3 to 8 membered heterocyclyl, 5 to 12 membered heteroaryl,
-(CH₂)ₙ₁NRₐₐR_{bb} and -(CRₐₐR_{bb})ₙ₁NR_{cc}R_{dd};
R₄ is selected from the group consisting of hydrogen, halogen, amino, C₁₋₆ alkyl and C₁₋₆ hydroxyalkyl;
R₅ is selected from the group consisting of hydrogen, C₁₋₆ alkyl and amino;
R₁₄ is selected from the group consisting of hydrogen, C₁₋₆ alkyl, C₃₋₈ cycloalkyl, 3 to 8 membered heterocyclyl and 5 to 12 membered heteroaryl;
R₁₅ and R₁₆ are identical or different and are each independently selected from the group consisting of hydrogen and C₁₋₆ alkyl;
Rₐₐ and R_{bb} are identical or different and are each independently selected from the group consisting of hydrogen, C₁₋₆ alkyl, C₃₋₆ cycloalkyl, C₁₋₆ haloalkyl and C₁₋₆ alkoxy; and
R_{cc} and R_{dd} are identical or different and are each independently selected from the group consisting of hydrogen, C₁₋₆ alkyl, C₃₋₆ cycloalkyl, C₁₋₆ haloalkyl and C₁₋₆ alkoxy.
In a preferred embodiment of the present invention, the compound of any formula, the stereoisomer thereof or the pharmaceutically acceptable salt thereof is characterized in that,
R₁ is selected from the group consisting of hydrogen, halogen, C₁₋₃ alkyl, C₃₋₆ cycloalkyl, C₁₋₃ hydroxyalkyl, C₁₋₃ alkoxy, 3 to 6 membered heterocyclyl and 5 to 6 membered heteroaryl, wherein the C₁₋₃ alkyl, C₁₋₃ hydroxyalkyl, C₁₋₃ alkoxy, C₃₋₆ cycloalkyl, 3 to 6 membered heterocyclyl and 5 to 6 membered heteroaryl are each optionally further substituted by one or more substituent(s) selected from the group consisting of hydrogen, deuterium, halogen, amino, hydroxy, cyano, oxo, C₁₋₃ alkyl, C₁₋₃ deuterated alkyl, C₁₋₃ haloalkyl, C₁₋₃ alkoxy, C₁₋₃ hydroxyalkyl, C₁₋₃ haloalkoxy, C₂₋₄ alkenyl, C₂₋₄ alkynyl, C₃₋₆ cycloalkyl, 3 to 16 membered heterocyclyl, C₆₋₁₂ aryl and 5 to 12 membered heteroaryl;
R₅ is selected from the group consisting of hydrogen, amino and C₁₋₃ alkyl;
preferably, neither R₁ nor R₅ is hydrogen;
R₁₂ and R₁₃ are each independently selected from the group consisting of hydrogen, halogen, amino, C₃₋₆ cycloalkylamino, cyano, C₁₋₃ alkyl, C₁₋₃ haloalkyl, C₃₋₆ cycloalkyl and 3 to 6 membered heterocyclyl containing 1 to 2 nitrogen or oxygen atom(s), optionally further substituted by one or more substituent(s) selected from the group consisting of deuterium, halogen, amino, hydroxy, cyano, oxo, C₁₋₃ alkyl, C₁₋₃ deuterated alkyl, C₁₋₃ haloalkyl, C₁₋₃ alkoxy, C₁₋₃ hydroxyalkyl, C₁₋₃ haloalkoxy, C₂₋₄ alkenyl, C₂₋₄ alkynyl, C₃₋₆ cycloalkyl, 3 to 6 membered heterocyclyl, C₆₋₁₂ aryl and 5 to 12 membered heteroaryl; preferably, the heterocyclyl is selected from the group consisting of

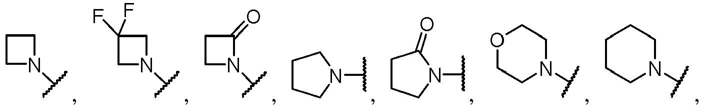

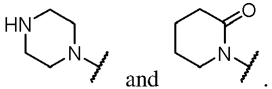

In a preferred embodiment of the present invention, any compound of formula (I), the stereoisomer thereof or the pharmaceutically acceptable salt thereof is characterized by being selected from the group consisting of: 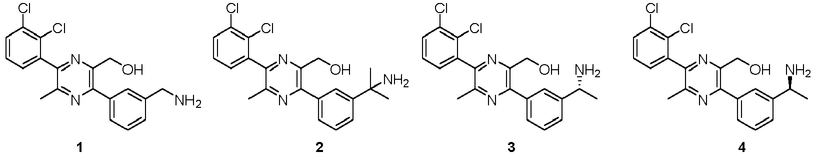 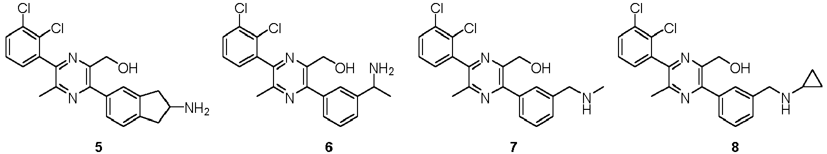 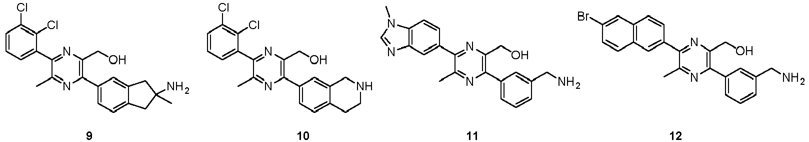 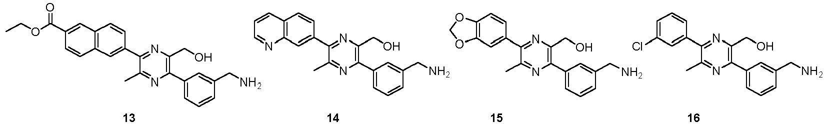 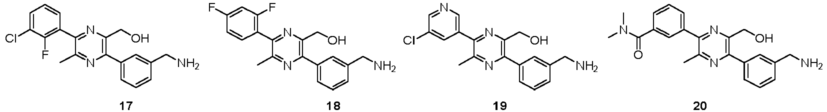 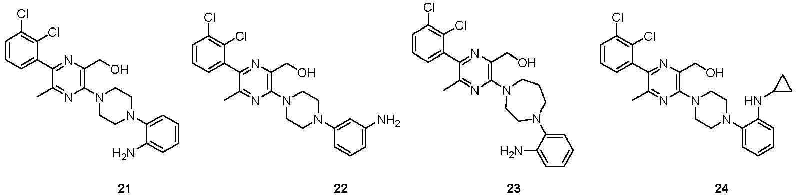 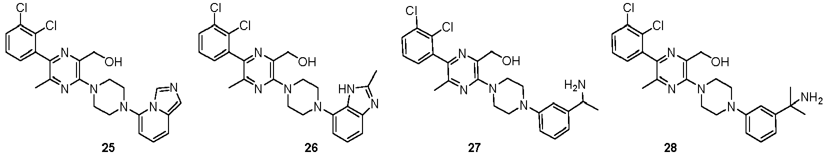 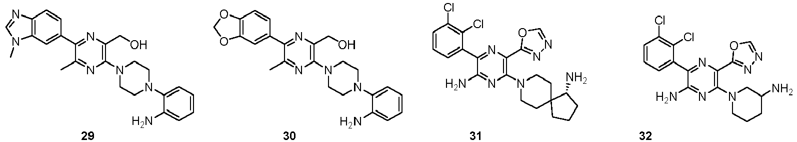 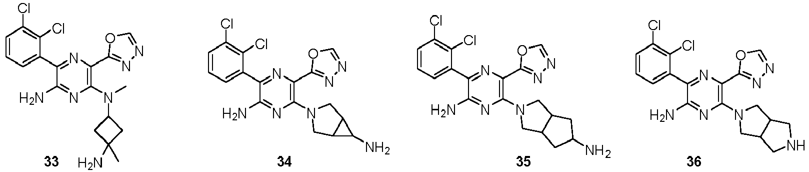 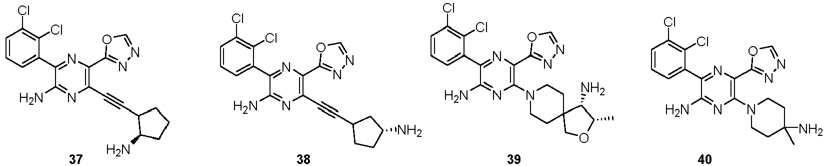 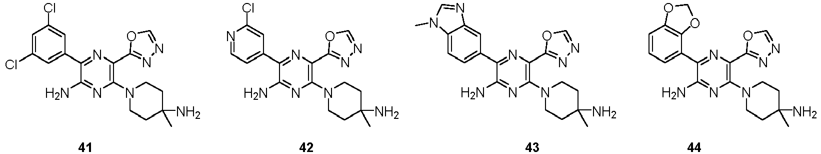 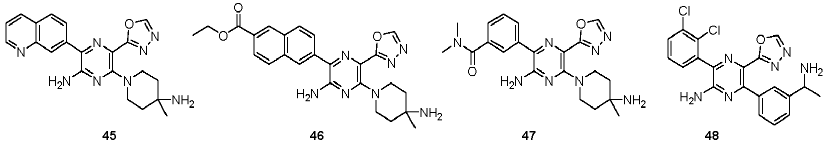 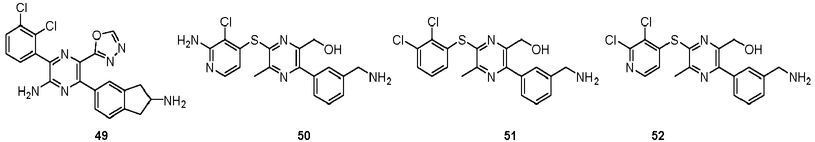 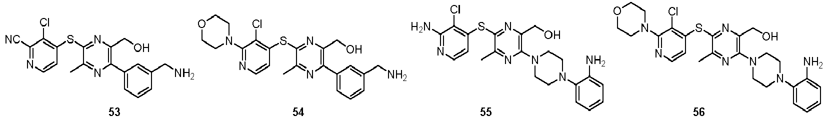 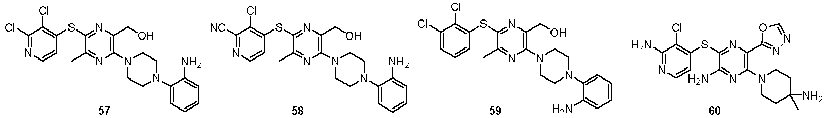 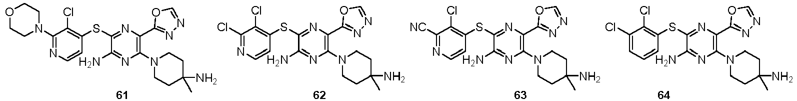 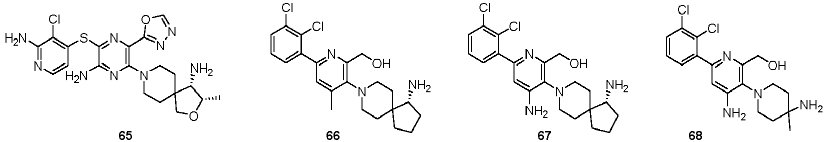 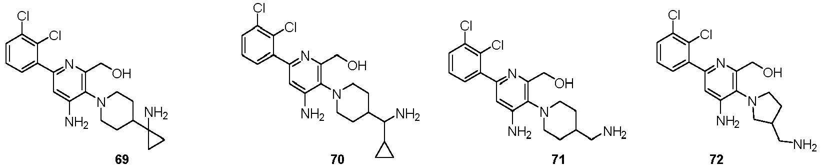 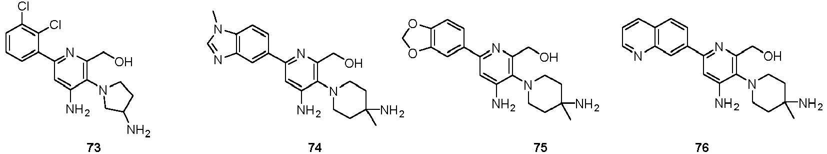 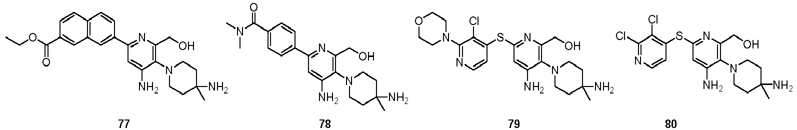 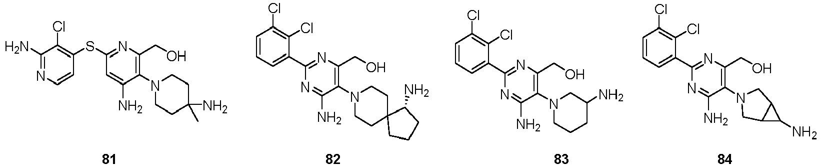 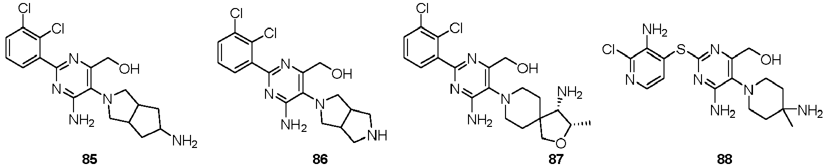 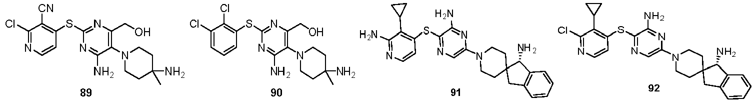 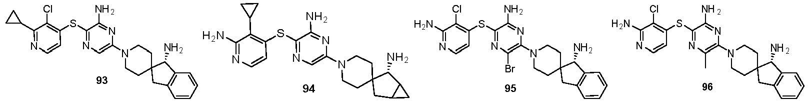 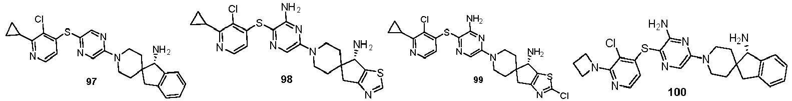 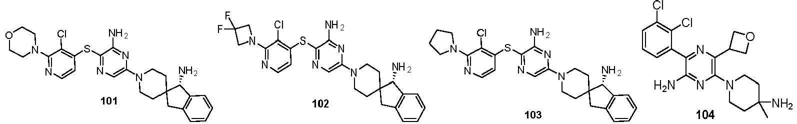 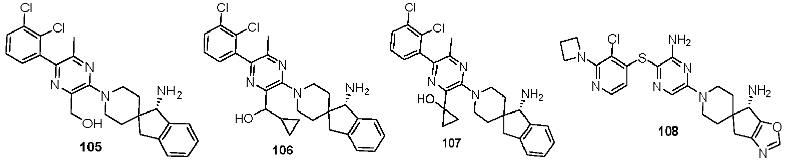 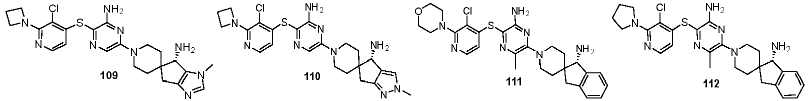 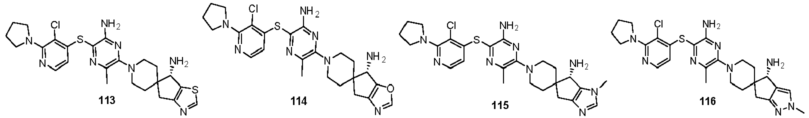 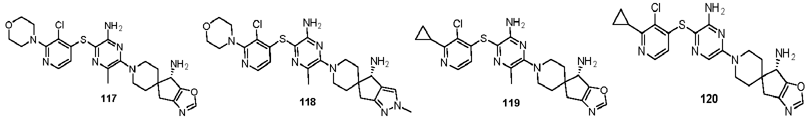 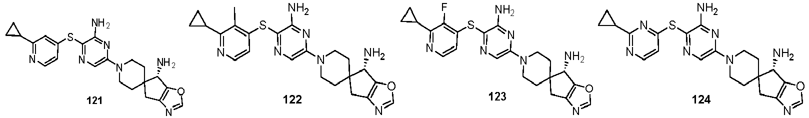 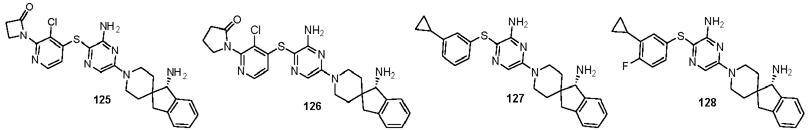 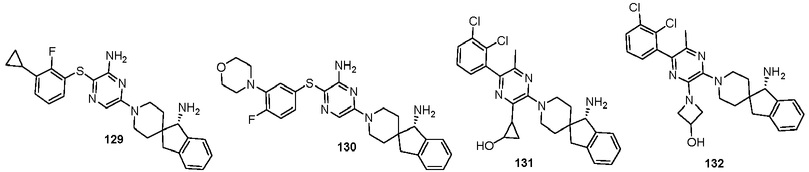 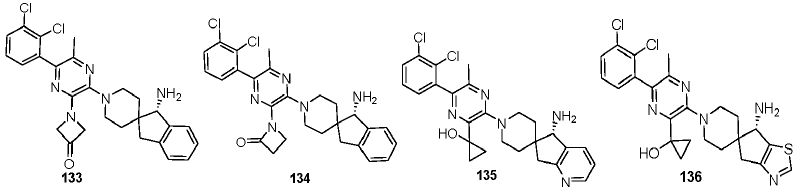 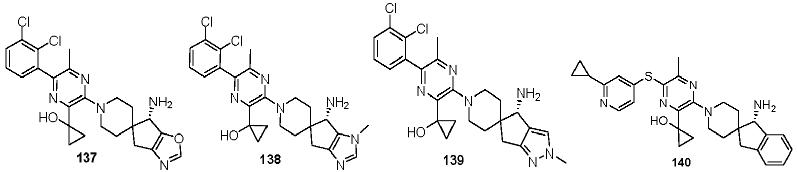 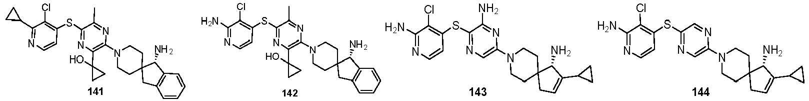 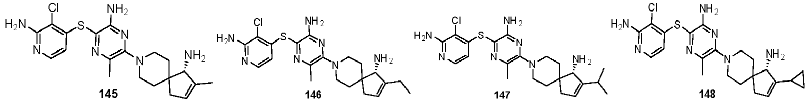 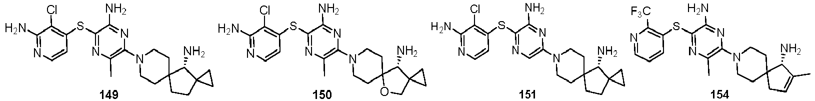 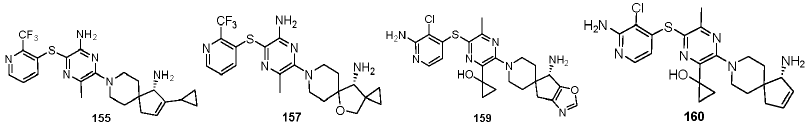 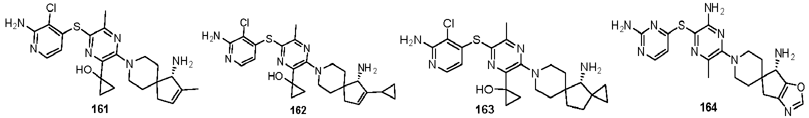 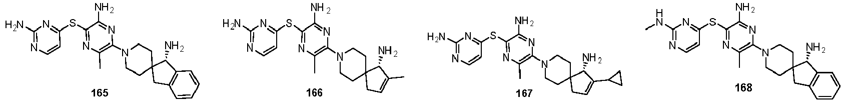 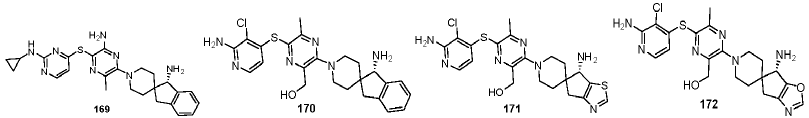 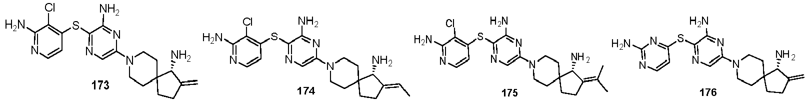 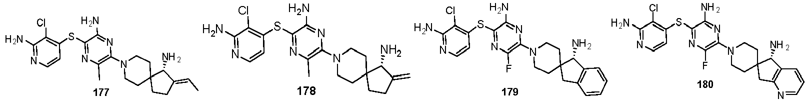 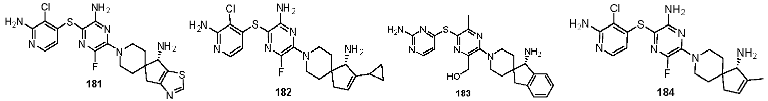 and

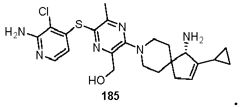

The present invention also relates to a method for preparing the compound of formula (XII), the stereoisomer thereof or the pharmaceutically acceptable salt thereof, comprising the following step of: 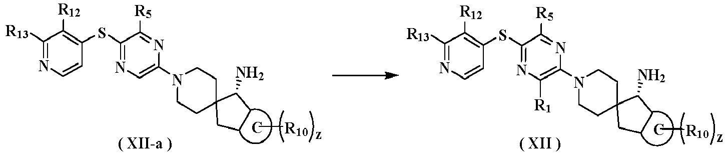 subjecting a compound of formula (XII-a) to a substitution reaction to obtain the compound of formula (XII), the stereoisomer thereof or the pharmaceutically acceptable salt thereof.

Further, the method comprises the following step of: 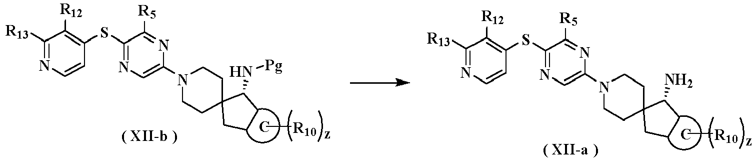 deprotecting a compound of formula (XII-b) to obtain the compound of formula (XII-a), the stereoisomer thereof or the pharmaceutically acceptable salt thereof;
wherein:
Pg is an amino protecting group selected from the group consisting of *tert*-butylsulfinyl, benzyloxycarbonyl, *tert*-butoxycarbonyl, 9-fluorenylmethoxycarbonyl, benzyl, *p*-methoxybenzyl, allyloxycarbonyl, trityl and phthaloyl, and preferably *tert*-butylsulfinyl.

Further, the method comprises the following step of: 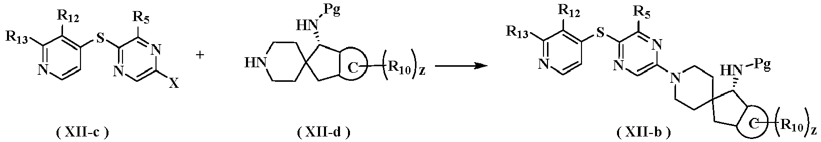 reacting a compound of formula (XII-c) with a compound of formula (XII-d) to obtain the compound of formula (XII-b), the stereoisomer thereof or the pharmaceutically acceptable salt thereof; wherein:
X is a halogen, preferably fluorine, chlorine, bromine or iodine, and more preferably chlorine.

The present invention also relates to a method for preparing the compound of formula (XII), the stereoisomer thereof or the pharmaceutically acceptable salt thereof, comprising the following steps of:

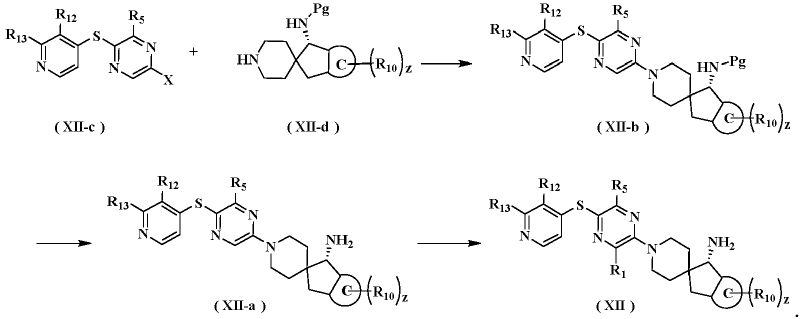

The present invention also relates to a method for preparing the compound of formula (XII-A), the stereoisomer thereof or the pharmaceutically acceptable salt thereof, comprising the following steps of:

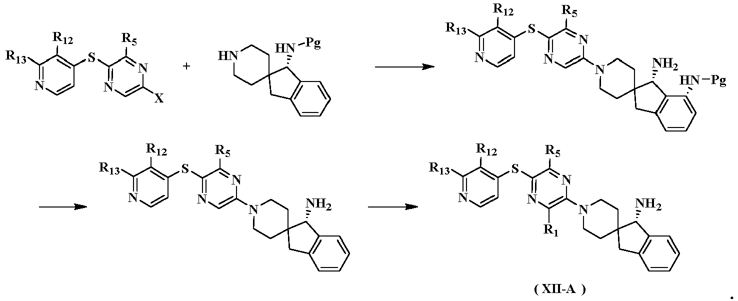

The present invention also relates to a method for preparing the compound of formula (XII-A), the stereoisomer thereof or the pharmaceutically acceptable salt thereof, further comprising the following steps of: 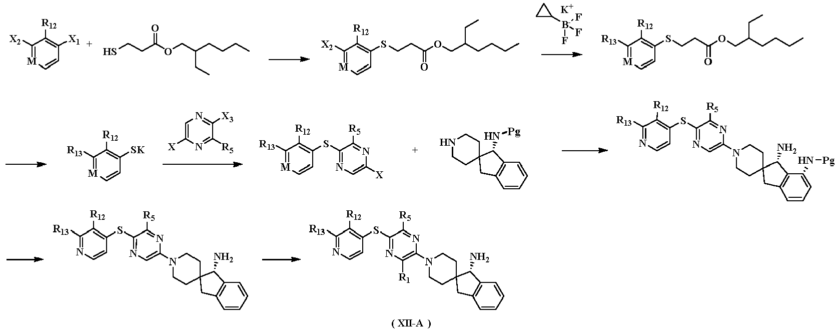 wherein:
X₁ is halogen, preferably fluorine, chlorine, bromine or iodine, and more preferably iodine;
X₂ is halogen, preferably fluorine, chlorine, bromine or iodine, and more preferably chlorine; and
X₃ is halogen, preferably fluorine, chlorine, bromine or iodine, and more preferably bromine.

The present invention further relates to a use of any one of the compound of formula (I), the stereoisomer thereof, or the pharmaceutically acceptable salt thereof or the pharmaceutical composition comprising the same in the preparation of a SHP-2 inhibitor medicament.

The present invention also relates to a method for preventing and/or treating a disease mediated by SHP-2 inhibitor, comprising administering to a patient a therapeutically effective amount of the compound of formula (I), the stereoisomer thereof, or the pharmaceutically acceptable salt thereof, or the pharmaceutical composition comprising the same.

In some embodiments, the present invention also relates to a use of the compound and composition of the present invention in the preparation for treating a disease or condition such as Noonan syndrome, leopard syndrome, leukemia, neuroblastoma, melanoma, breast cancer, esophageal cancer, head and neck tumor, stomach cancer, lung cancer and colon cancer.

The present invention also relates to the compound and composition of the present invention for use in treating a disease or condition such as Noonan syndrome, leopard syndrome, leukemia, neuroblastoma, melanoma, breast cancer, esophageal cancer, head and neck tumor, lung cancer and colon cancer.

In some embodiments, the present invention provides a method for treating a cancer, comprising administering to a patient suffering from the cancer the compound or composition of the present invention.

In some embodiments, the cancer treated by the compound or composition of the present invention is Noonan syndrome, leopard syndrome, leukemia, neuroblastoma, melanoma, breast cancer, esophageal cancer, head and neck tumor, stomach cancer, lung cancer and colon cancer, preferably non-small cell lung cancer, esophageal cancer and head and neck tumor, and more preferably the leukemia is AML and the lung cancer is NSCLC.

### DESCRIPTION OF THE DRAWINGS

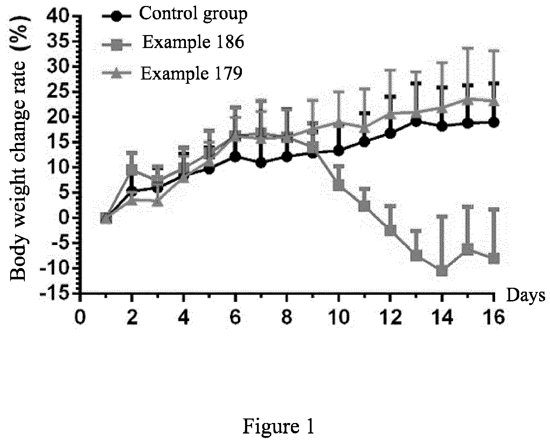

Figure 1 shows the body weight change rate of male mice within 16 days after the administration.

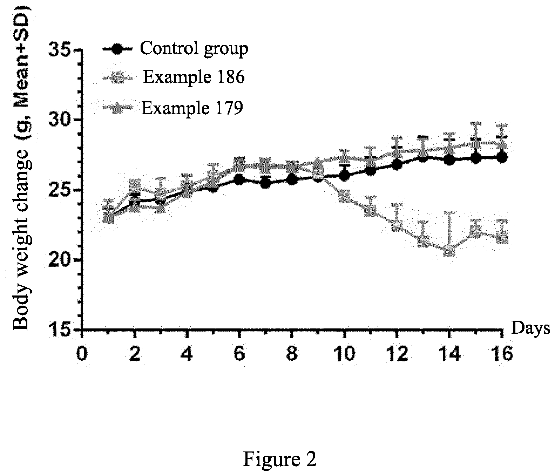

Figure 2 shows the body weight change of male mice within 16 days after the administration.

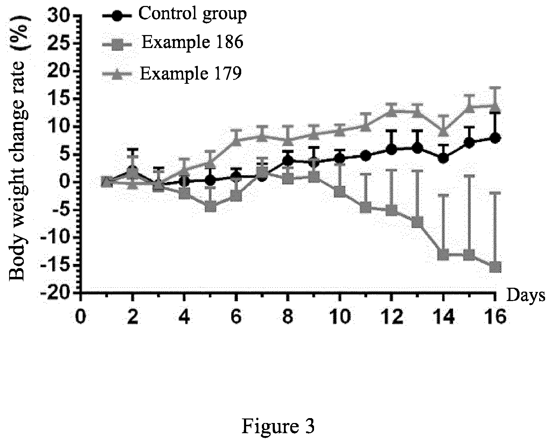

Figure 3 shows the body weight change rate of female mice within 16 days after the administration.

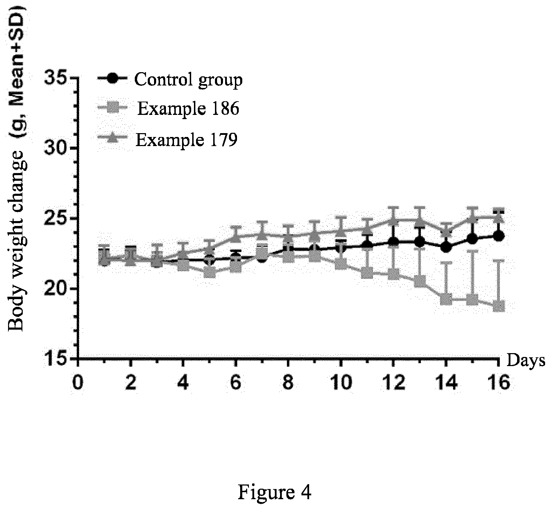

Figure 4 shows the body weight change of female mice within 16 days after the administration.

### DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec-*butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl.

The term "alkylene" refers to an alkyl of which a hydrogen atom is further substituted, for example, "methylene" refers to -CH₂-, "ethylene" refers to -(CH₂)₂-, "propylene" refers to -(CH₂)₃-, "butylene" refers to -(CH₂)₄- and the like. The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl group can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 8 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl or cycloheptyl, and more preferably cyclopropyl, cyclobutyl or cyclopentyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated *π*-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include: 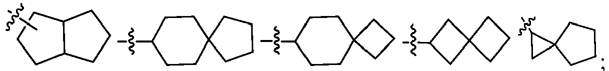 and also include spiro cycloalkyl in which a cycloalkyl and a heterocyclyl are connected through one spiro atom, non-limiting examples thereof include:

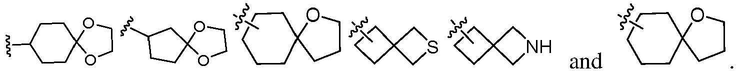

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated *π*-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 4-membered/4-membered, 5-membered/5-membered, or 5-membered/6-membered bicyclic fused cycloalkyl. Non-limiting examples of fused cycloalkyl include:

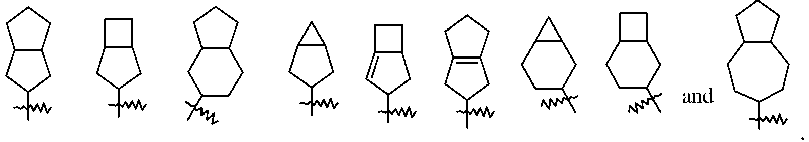

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated *π*-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

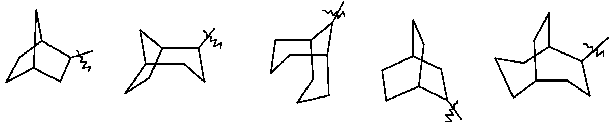

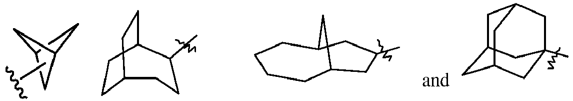

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of nitrogen, oxygen, boron, phosphorus, S(O)ₘ (wherein m is an integer of 0 to 2) and P(O)ₙ (wherein n is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include oxetanyl, azacycloheptanyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably oxetanyl, azacycloheptanyl, tetrahydrofuranyl, pyrazolidinyl, morpholinyl, piperazinyl and pyranyl, and more preferably azacycloheptanyl, piperazinyl, tetrahydropiperidinyl and pyrrolidinyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

The term "spiro heterocyclyl" refers to a 3 to 20 membered polycyclic heterocyclyl group with individual rings connected through one shared atom (called a spiro atom), wherein one or more ring atoms are heteroatoms selected from the group consisting of nitrogen, oxygen, boron, phosphorus, S(O)ₘ (wherein m is an integer of 0 to 2) and P(O)ₙ (wherein n is an integer of 0 to 2), with the remaining ring atoms being carbon atoms, and the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 3-membered/5-membered, 4-membered/5-membered, 4-membered/6-membered, 5-membered/5-membered, or 5-membered/6-membered mono-spiro heterocyclyl. Non-limiting examples of spiro heterocyclyl include: 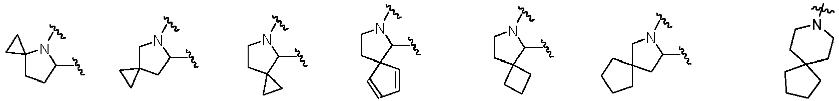 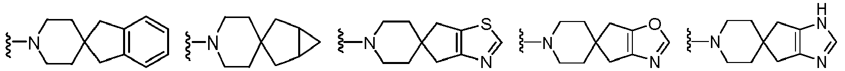 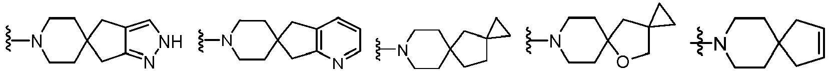 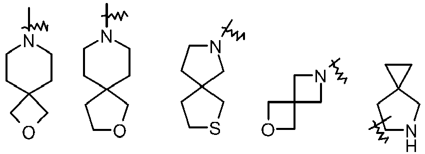 and the like.

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of nitrogen, oxygen and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 3-membered/5-membered, 4-membered/5-membered or 5-membered/6-membered bicyclic fused heterocyclyl. Non-limiting examples of fused heterocyclyl include: 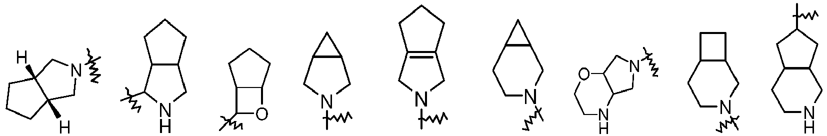 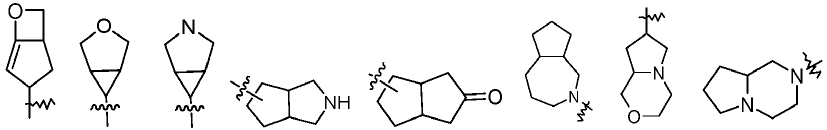 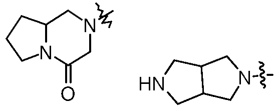 and the like.

The term "bridged heterocyclyl" refers to a 5 to 14 membered polycyclic heterocyclyl group, wherein every two rings in the system share two disconnected atoms, wherein the rings can have one or more double bond(s), but none of the rings has a completely conjugated *π*-electron system, and one or more ring atoms are heteroatoms selected from the group consisting of nitrogen, oxygen and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

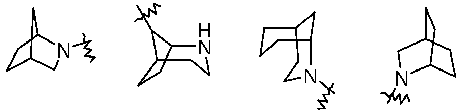

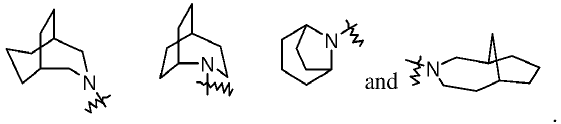

The heterocyclyl ring can be fused to the ring of aryl, heteroaryl or cycloalkyl, wherein the ring bound to the parent structure is heterocyclyl. Non-limiting examples thereof include: 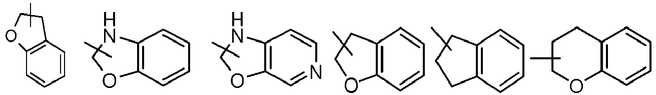 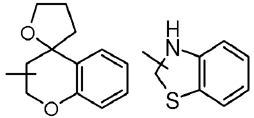 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include: 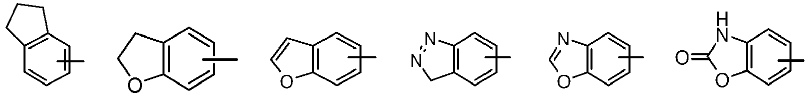 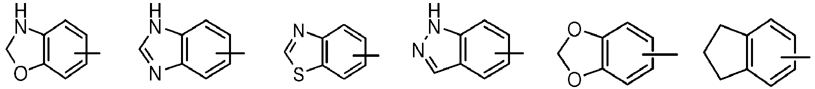 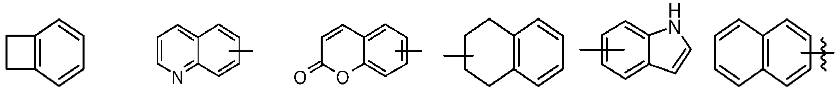 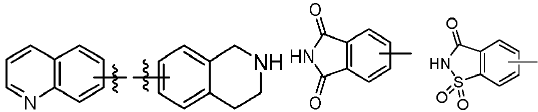 and the like.

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of oxygen, sulfur and nitrogen. The heteroaryl is preferably a 5 to 10 membered heteroaryl, and more preferably a 5 or 6 membered heteroaryl, for example imidazolyl, furyl, thienyl, thiazolyl, pyrazolyl, oxazolyl, pyrrolyl, triazolyl, tetrazolyl, pyridyl, pyrimidinyl, thiadiazolyl, oxadiazolyl, pyrazinyl and the like, preferably oxazolyl, oxadiazolyl, tetrazolyl, triazolyl, thienyl, imidazolyl, pyridyl, pyrazolyl, pyrimidinyl and thiazolyl, and more preferably oxazolyl, oxadiazolyl, tetrazolyl, triazolyl, thienyl, pyridyl, thiazolyl and pyrimidinyl. The heteroaryl ring can be fused to the ring of aryl, heterocyclyl or cycloalkyl, wherein the ring bound to the parent structure is heteroaryl ring. Non-limiting examples thereof include:

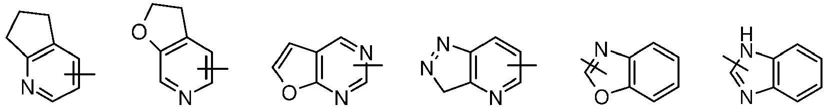

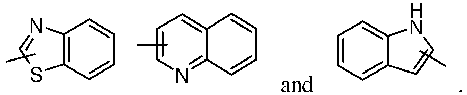

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl group substituted by one or more halogen(s), wherein the alkyl is as defined above.

"Haloalkoxy" refers to an alkoxy group substituted by one or more halogen(s), wherein the alkoxy is as defined above.

"Hydroxyalkyl" refers to an alkyl group substituted by hydroxy(s), wherein the alkyl is as defined above.

"Alkenyl" refers to a chain alkenyl, also known as alkene group. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Alkynyl" refers to (CH≡C- or -CH≡C-). The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Hydroxy" refers to an -OH group.

"Halogen" refers to fluorine, chlorine, bromine or iodine.

"Amino" refers to a -NH₂ group.

"Cyano" refers to a -CN group.

"Nitro" refers to a -NO₂ group.

"Carboxy" refers to a -C(O)OH group.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"Et₂O" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"Pd₂(dba)₃" refers to tris(dibenzylideneacetone)dipalladium.

"Dppf' refers to 1,1'-bisdiphenylphosphinoferrocene.

"HATU" refers to 2-(7-azabenzotriazol-1-yl)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to n-butyl lithium.

"NaBH(OAc)₃" refers to sodium triacetoxyborohydride.

Different expressions such as "X is selected from the group consisting of A, B or C", "X is selected from the group consisting of A, B and C", "X is A, B or C", "X is A, B and C" and the like, express the same meaning, that is, X can be any one or more of A, B and C.

The hydrogen atom of the present invention can be substituted by its isotope deuterium. Any of the hydrogen atoms in the compounds of the examples of the present invention can also be substituted by deuterium atom.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

### PREFERRED EMBODIMENTS

The present invention will be further described with reference to the following examples, but the examples should not be considered as limiting the scope of the present invention.

### EXAMPLES

The structures of the compounds of the present invention were identified by nuclear magnetic resonance (NMR) and/or liquid chromatography-mass spectrometry (LC-MS). NMR shifts (δ) are given in parts per million (ppm). NMR was determined by a Bruker AVANCE-400 machine. The solvents for determination were deuterated-dimethyl sulfoxide (DMSO-*d₆*), deuterated-methanol (CD₃OD) and deuterated-chloroform (CDCl₃), and the internal standard was tetramethylsilane (TMS).

Liquid chromatography-mass spectrometry (LC-MS) was determined on an Agilent 1200 Infinity Series mass spectrometer. High performance liquid chromatography (HPLC) was determined on an Agilent 1200DAD high pressure liquid chromatograph (Sunfire C18 150×4.6 mm chromatographic column), and a Waters 2695-2996 high pressure liquid chromatograph (Gimini C₁₈ 150×4.6 mm chromatographic column).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm. Yantai Huanghai 200 to 300 mesh silica gel was generally used as a carrier for column chromatography.

The raw materials used in the examples of the present invention are known and commercially available, or can be synthesized by adopting or according to known methods in the art.

Unless otherwise stated, all reactions of the present invention were carried out under continuous magnetic stirring under a dry nitrogen or argon atmosphere, the solvent was dry, and the reaction temperature was in degrees celsius.

### Example 1

### Preparation of

### (3-(4-(aminomethyl)phenyl)-6-(2,3-dichlorophenyl)-5-methylpyrazin-2-yl)methanol

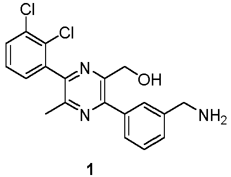

### Step 1: Preparation of ethyl 3-hydroxy-5-methylpyrazine-2-carboxylate

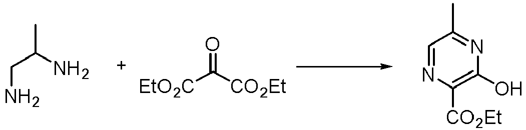

1,2-Propane diamine (7.4 g, 0.10 mol) and diethyl acetone dicarboxylate (17.4 g, 0.10 mol) in 150 mL of ethanol were refluxed and stirred for 24 hours. The reaction solution was concentrated to dryness, and the resulting residue was purified by column chromatography (CH₂Cl₂/MeOH=10:1) to obtain the target product ethyl 3-hydroxy-5-methylpyrazine-2-carboxylate (5.3 g, yield: 29%).

MS m/z (ESI): 183.1 [M+H]⁺.

### Step 2: Preparation of ethyl 6-bromo-3-hydroxy-5-methylpyrazine-2-carboxylate

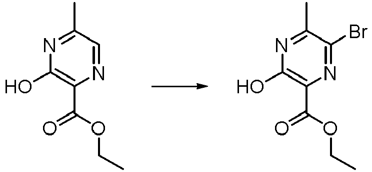

Ethyl 3-hydroxy-5-methylpyrazine-2-carboxylate (5.3 g, 29.09 mmol) was dissolved in 100 mL of DMF, and the solution was cooled in an ice water bath. NBS (5.4 g, 30.56 mmol) was added, and the reaction solution was stirred for 30 minutes. The reaction solution was warmed up to room temperature and stirred for for 2 hours. Water was added, and the solution was extracted with ethyl acetate. The organic phase was washed with brine, dried over anhydrous sodium sulfate, and concentrated to obtain the product ethyl 6-bromo-3-hydroxy-5-methylpyrazine-2-carboxylate (5.5 g, yield: 72%).

MS m/z (ESI): 261.0 [M+H]⁺, 262.9 [M+2+H]⁺.

### Step 3: Preparation of ethyl 6-(2,3-dichlorophenyl)-3-hydroxy-5-methylpyrazine-2-carboxylate

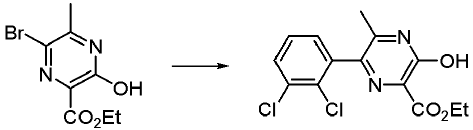

Ethyl 6-bromo-3-hydroxy-5-methylpyrazine-2-carboxylate (2.5 g, 9.58 mmol), 2,3-dichlorophenylboronic acid (2.7 g, 14.15 mmol), Pd(dppf)Cl₂ (690 mg, 0.94 mmol) and potassium carbonate (2.6 g, 18.81 mmol) were dissolved in anhydrous dioxane (50 mL)/water (10 mL). The reaction solution was purged with nitrogen, heated to 80°C and stirred overnight. The reaction solution was cooled to room temperature, and then water was added. The solution was extracted with dichloromethane, and the organic phase was dried over anhydrous sodium sulfate and filtered. The filtrate was concentrated to obtain a crude product, which was purified by column chromatography to obtain the product ethyl 6-(2,3-dichlorophenyl)-3-hydroxy-5-methylpyrazine-2-carboxylate (2.0 g, yield: 64%).

MS m/z (ESI): 327.0 [M+H]⁺, 329.0[M+2+H]⁺.

### Step 4: Preparation of ethyl 3-chloro-6-(2,3-dichlorophenyl)-5-methylpyrazine-2-carboxylate

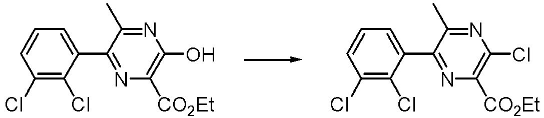

Ethyl 6-(2,3-dichlorophenyl)-3-hydroxy-5-methylpyrazine-2-carboxylate (2.0 g, 6.11 mmol) and phosphorus oxychloride (10 mL) were stirred at 120°C overnight. The reaction solution was cooled to room temperature, and then ice water was added dropwise. The solution was extracted with dichloromethane. The organic phase was dried over anhydrous sodium sulfate, and concentrated to dryness to obtain a crude product, which was purified by column chromatography to obtain the product ethyl 3-chloro-6-(2,3-dichlorophenyl)-5-methylpyrazine-2-carboxylate (1.2 g, yield: 57%).

MS m/z (ESI): 345.0 [M+H]⁺, 347.0[M+2+H]⁺.

### Step 5: Preparation of ethyl 3-(4-(aminomethyl)phenyl)-6-(2,3-dichlorophenyl)-5-methylpyrazine-2-carboxylate

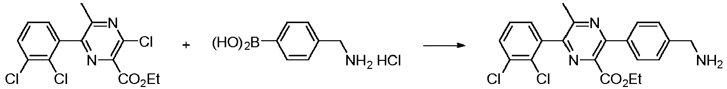

Ethyl 3-chloro-6-(2,3-dichlorophenyl)-5-methylpyrazine-2-carboxylate (100 mg, 0.29 mmol), 4-(aminomethyl)phenylborate hydrochloride (54 mg, 0.29 mmol), Pd(dppf)Cl₂ (42 mg, 0.057 mmol) and potassium carbonate (80 mg, 0.58 mmol) were dissolved in anhydrous dioxane (10 mL)/water (2 mL). The reaction solution was purged with nitrogen, heated to 100°C and stirred overnight. The reaction solution was cooled to room temperature, and then water was added. The solution was extracted with dichloromethane. The organic phase was dried over anhydrous sodium sulfate, and concentrated to dryness to obtain a crude product, which was purified by column chromatography to obtain the product ethyl 3-(4-(aminomethyl)phenyl)-6-(2,3-dichlorophenyl)-5-methylpyrazine-2-carboxylate (57 mg, yield: 47%).

MS m/z (ESI): 416.0 [M+H]⁺, 418.0[M+2+H]⁺.

### Step 6: Preparation of (3-(4-(aminomethyl)phenyl)-6-(2,3-dichlorophenyl)-5-methylpyrazin-2-yl)methanol

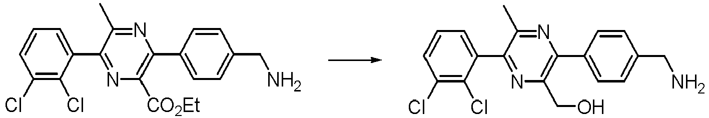

Ethyl 3-(4-(aminomethyl)phenyl)-6-(2,3-dichlorophenyl)-5-methylpyrazine-2-carboxylate (57 mg, 0.13 mmol) was dissolved in THF (5 mL), and the solution was cooled in an ice water bath. 2M solution of lithium borohydride in THF (0.14 mL, 0.28 mmol) was added dropwise, and the reaction solution was stirred for 1 hour. Water was added, and the solution was extracted with dichloromethane. The organic phases were combimed, dried over anhydrous sodium sulfate, and concentrated to dryness to obtain a crude product, which was purified by column chromatography to obtain the product (3-(4-(aminomethyl)phenyl)-6-(2,3-dichlorophenyl)-5-methylpyrazin-2-yl)methanol (20 mg, yield: 39%).

¹H NMR (400 MHz, MeOD) δ: 7.76 (d, *J* = 8.0 Hz, 2H), 7.70 (d, *J* = 7.8 Hz, 1H), 7.54 (d, *J* = 8.0 Hz, 2H), 7.47 (d, *J* = 7.8 Hz, 1H), 7.43 (d, *J* = 6.2 Hz, 1H), 4.70 (s, 2H), 3.95 (s, 2H), 2.43 (s, 3H).

MS m/z (ESI): 374.1 [M+H]⁺, 376.1 [M+2+H]⁺.

The compounds of Examples 2 to 20 and 50 to 54 were prepared by referring to the experimental scheme of Example 1.

### Example 2

### Preparation of

### (3-(3-(2-aminopropan-2-yl)phenyl)-6-(2,3-dichlorophenyl)-5-methylpyrazin-2-yl)metha nol

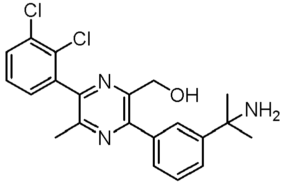

The compound of Example 2 was prepared by referring to the experimental scheme of Example 1.

MS m/z (ESI): 402.1 [M+H]⁺, 404.1 [M+2+H]⁺.

### Example 3

### Preparation of

### (R)-(3-(3-(1-aminoethyl)phenyl)-6-(2,3-dichlorophenyl)-5-methylpyrazin-2-yl)methano 1

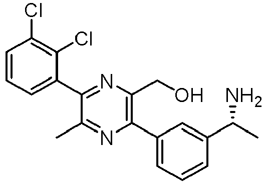

The compound of Example 3 was prepared by referring to the experimental scheme of Example 1.

MS m/z (ESI): 388.1 [M+H]⁺, 390.1 [M+2+H]⁺.

### Example 4

### Preparation of

### (S)-(3-(3-(1-aminoethyl)phenyl)-6-(2,3-dichlorophenyl)-5-methylpyrazin-2-yl)methanol

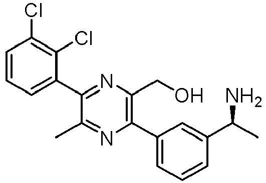

The compound of Example 4 was prepared by referring to the experimental scheme of Example 1.

MS m/z (ESI): 388.1 [M+H]⁺, 390.1 [M+2+H]⁺.

### Example 5

### Preparation of

### (3-(2-amino-2,3-dihydro-1H-inden-5-yl)-6-(2,3-dichlorophenyl)-5-methylpyrazin-2-yl) methanol

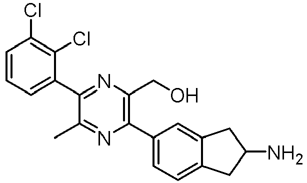

The compound of Example 5 was prepared by referring to the experimental scheme of Example 1.

MS m/z (ESI): 400.1 [M+H]⁺, 402.1 [M+2+H]⁺.

### Example 6

### Preparation of

### (3-(3-(1-aminoethyl)phenyl)-6-(2,3-dichlorophenyl)-5-methylpyrazin-2-yl)methanol

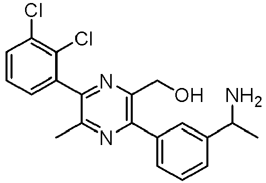

The compound of Example 6 was prepared by referring to the experimental scheme of Example 1.

MS m/z (ESI): 388.1 [M+H]⁺, 340.1 [M+2+H]⁺.

### Example 7

### Preparation of

### (6-(2,3-dichlorophenyl)-5-methyl-3-(3-((methylamino)methyl)phenyl)pyrazin-2-yl)met hanol

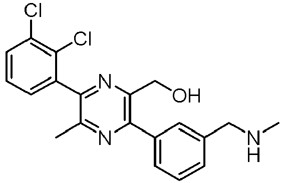

The compound of Example 7 was prepared by referring to the experimental scheme of Example 1.

MS m/z (ESI): 388.1 [M+H]⁺, 340.1 [M+2+H]⁺.

### Example 8

### Preparation of

### (3-(3-((cyclopropylamino)methyl)phenyl)-6-(2,3-dichlorophenyl)-5-methylpyrazin-2-yl )methanol

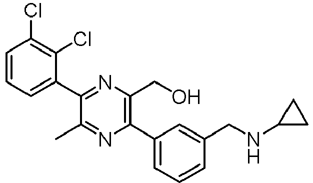

The compound of Example 8 was prepared by referring to the experimental scheme of Example 1.

MS m/z (ESI): 414.1 [M+H]⁺, 416.1 [M+2+H]⁺.

### Example 9

### Preparation of

### (3-(2-amino-2-methyl-2,3-dihydro-1H-inden-5-yl)-6-(2,3-dichlorophenyl)-5-methylpyra zin-2-yl)methanol

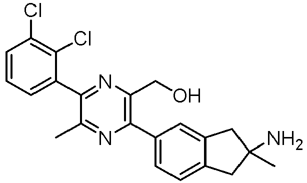

The compound of Example 9 was prepared by referring to the experimental scheme of Example 1.

MS m/z (ESI): 414.1 [M+H]⁺, 416.1 [M+2+H]⁺.

### Example 10

### Preparation of

### (6-(2,3-dichlorophenyl)-5-methyl-3-(1,2,3,4-tetrahydroisoquinolin-7-yl)pyrazin-2-yl)me thanol

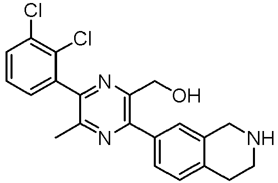

The compound of Example 10 was prepared by referring to the experimental scheme of Example 1.

MS m/z (ESI): 400.1 [M+H]⁺, 402.1 [M+2+H]⁺.

### Example 11

### Preparation of

### (3-(3-(aminomethyl)phenyl)-5-methyl-6-(1-methyl-1H-benzo[d]imidazol-5-yl)pyrazin-2 -yl)methanol

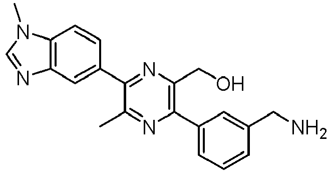

The compound of Example 11 was prepared by referring to the experimental scheme of Example 1.

MS m/z (ESI): 360.1 [M+H]⁺.

### Example 12

### Preparation of

### (3-(3-(aminomethyl)phenyl)-6-(6-bromonaphthalen-2-yl)-5-methylpyrazin-2-yl)methan ol

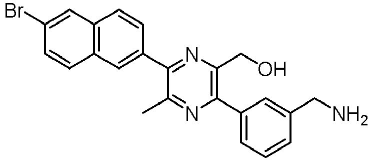

The compound of Example 12 was prepared by referring to the experimental scheme of Example 1.

MS m/z (ESI): 434.1 [M+H]⁺, 436.1 [M+2+H]⁺.

### Example 13

### Preparation of ethyl

### 6-(5-(3-(aminomethyl)phenyl)-6-(hydroxymethyl)-3-methylpyrazin-2-yl)-2-naphthoate

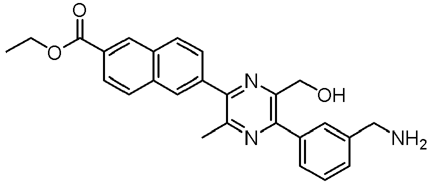

The compound of Example 13 was prepared by referring to the experimental scheme of Example 1.

MS m/z (ESI): 428.1 [M+H]⁺.

### Example 14

### Preparation of

### (3-(3-(aminomethyl)phenyl)-5-methyl-6-(quinolin-7-yl)pyrazin-2-yl)methanol

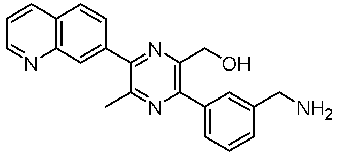

The compound of Example 14 was prepared by referring to the experimental scheme of Example 1.

MS m/z (ESI): 357.1 [M+H]⁺.

### Example 15

### Preparation of

### (3-(3-(aminomethyl)phenyl)-6-(benzo[d][1,3]dioxol-5-yl)-5-methylpyrazin-2-yl)methan ol

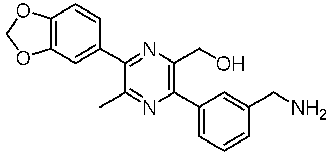

The compound of Example 15 was prepared by referring to the experimental scheme of Example 1.

MS m/z (ESI): 350.1 [M+H]⁺.

### Example 16

### Preparation of

### (3-(3-(aminomethyl)phenyl)-6-(3-chlorophenyl)-5-methylpyrazin-2-yl)methanol

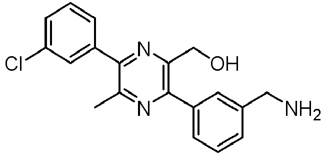

The compound of Example 16 was prepared by referring to the experimental scheme of Example 1.

MS m/z (ESI): 340.1 [M+H]⁺, 342.1 [M+2+H]⁺.

### Example 17

### Preparation of

### (3-(3-(aminomethyl)phenyl)-6-(3-chloro-2-fluorophenyl)-5-methylpyrazin-2-yl)methan ol

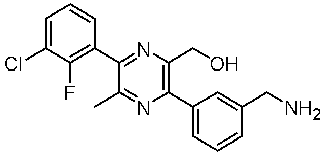

The compound of Example 17 was prepared by referring to the experimental scheme of Example 1.

MS m/z (ESI): 358.1 [M+H]⁺, 360.1 [M+2+H]⁺.

### Example 18

### Preparation of

### (3-(3-(aminomethyl)phenyl)-6-(2,4-difluorophenyl)-5-methylpyrazin-2-yl)methanol

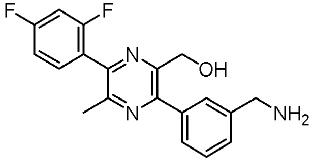

The compound of Example 18 was prepared by referring to the experimental scheme of Example 1.

MS m/z (ESI): 342.1 [M+H]⁺.

### Example 19

### Preparation of

### (3-(3-(aminomethyl)phenyl)-6-(5-chloropyridin-3-yl)-5-methylpyrazin-2-yl)methanol

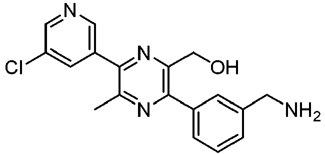

The compound of Example 19 was prepared by referring to the experimental scheme of Example 1.

MS m/z (ESI): 341.1 [M+H]⁺, 343.1 [M+2+H]⁺.

### Example 20

### Preparation of

### 3-(5-(3-(aminomethyl)phenyl)-6-(hydroxymethyl)-3-methylpyrazin-2-yl)-N,N-dimethyl benzamide

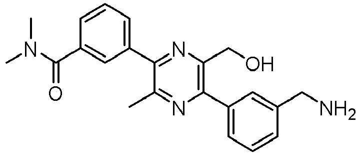

The compound of Example 20 was prepared by referring to the experimental scheme of Example 1.

MS m/z (ESI): 377.1 [M+H]⁺.

### Example 21

### Preparation of

### (3-(4-(2-aminophenyl)piperazin-1-yl)-6-(2,3-dichlorophenyl)-5-methylpyrazin-2-yl)met hanol

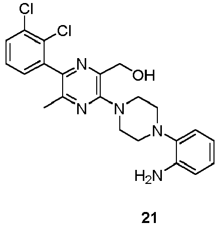

Step 1: Preparation of 1-(2-nitrophenyl)piperazine

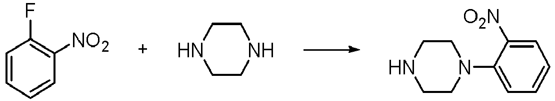

*O*-fluoronitrobenzene (5.0 g, 35.44 mmol) and anhydrous piperazine (12.2 g, 0.14 mol) were dissolved in 30 mL of ethanol, and the reaction solution was stirred at room temperature for 2 hours. The reaction solution was concentrated to dryness, and dichloromethane (80 mL) was added to dissolve the resulting residue. The solution was washed with water, dried and concentrated to dryness to obtain an oil, which was used directly in the next step.

MS m/z (ESI): 208.1 [M+H]⁺.

### Step 2: Preparation of 2-(piperazin-1-yl)aniline

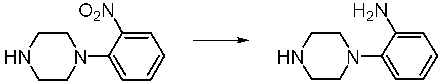

The oil of the above step 1-(2-nitrophenyl)piperazine was dissolved in 50 mL of ethanol, followed by the addition of 10% Pd/C (200 mg). The reaction solution was purged with hydrogen, and reacted in the presence of a hydrogen balloon at room temperature for 12 hours. The reaction solution was filtered, and the filter cake was washed with ethanol. The filtrate was concentrated to dryness to obtain the crude product (7.1 g) as a brown oil.

MS m/z (ESI): 178.2 [M+H]⁺.

### Step 3: Preparation of ethyl 3-(4-(2-aminophenyl)piperazin-1-yl)-6-(2,3-dichlorophenyl)-5-methylpyrazine-2-carbo xylate

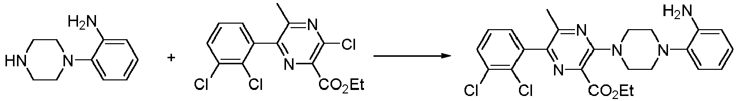

Ethyl 3-chloro-6-(2,3-dichlorophenyl)-5-methylpyrazine-2-carboxylate (100 mg, 0.29 mmol), 2-(piperazin-1-yl)aniline (103 mg, 0.58 mmol) and diisopropylethylamine (1 mL) were dissolved in DMF (5 mL). The reaction solution was purged with nitrogen, heated to 80°C and stirred overnight. The reaction solution was cooled to room temperature, and then water was added. The solution was extracted with dichloromethane, and the organic phase was dried over anhydrous sodium sulfate and filtered. The filtrate was concentrated to obtain a crude product, which was purified by column chromatography to obtain the product ethyl 3-(4-(2-aminophenyl)piperazin-1-yl)-6-(2,3-dichlorophenyl)-5-methylpyrazine-2-carbo xylate (100 mg, yield: 71%).

MS m/z (ESI): 486.1 [M+H]⁺, 488.1 [M+2+H]⁺.

### Step 4: Preparation of (3-(4-(2-aminophenyl)piperazin-1-yl)-6-(2,3-dichlorophenyl)-5-methylpyrazin-2-yl)met hanol

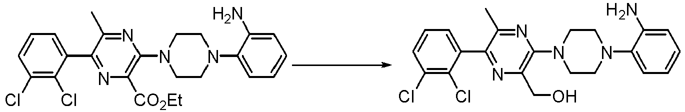

Ethyl 3-(4-(2-aminophenyl)piperazin-1-yl)-6-(2,3-dichlorophenyl)-5-methylpyrazine-2-carbo xylate (100 mg, 0.21 mmol) was dissolved in THF (5 mL), and the solution was cooled in an ice water bath. 2M solution of lithium borohydride in THF (0.6 mL, 1.2 mmol) was added dropwise, and the reaction solution was stirred for 1 hour. Water was added, and the solution was extracted with dichloromethane. The organic phase was dried over anhydrous sodium sulfate, and concentrated to dryness to obtain a crude product, which was purified by column chromatography to obtain the product (3-(4-(2-aminophenyl)piperazin-1-yl)-6-(2,3-dichlorophenyl)-5-methylpyrazin-2-yl)met hanol (60 mg, yield: 66%).

¹H NMR (400 MHz, CDCl₃) δ 7.55 (dd, *J* = 7.9, 1.6 Hz, 1H), 7.31 (t, *J* = 7.8 Hz, 1H), 7.28 - 7.23 (m, 1H), 7.07 (d, *J* = 7.8 Hz, 1H), 7.02 - 6.93 (m, 1H), 6.78 (t, *J* = 7.5 Hz, 2H), 4.75 (s, 2H), 4.40 - 3.68 (m, 3H), 3.47 (s, 4H), 3.22 - 2.99 (m, 4H), 2.33 (s, 3H).

MS m/z (ESI): 444.1 [M+H]⁺, 446.1 [M+2+H]⁺.

The compounds of Examples 22 to 30 and 55 to 59 were prepared by referring to the experimental scheme of Example 21.

### Example 22

### Preparation of

### (3-(4-(3-aminophenyl)piperazin-1-yl)-6-(2,3-dichlorophenyl)-5-methylpyrazin-2-yl)met hanol

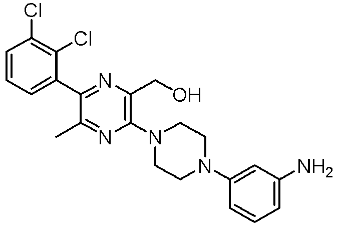

The compound of Example 22 was prepared by referring to the experimental scheme of Example 21.

MS m/z (ESI): 444.1 [M+H]⁺, 446.1 [M+2+H]⁺.

### Example 23

### Preparation of

### (3-(4-(2-aminophenyl)-1,4-diazepan-1-yl)-6-(2,3-dichlorophenyl)-5-methylpyrazin-2-yl )methanol

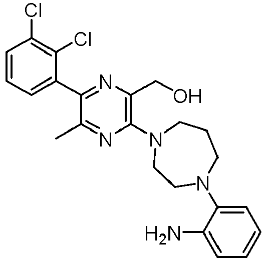

The compound of Example 23 was prepared by referring to the experimental scheme of Example 21.

MS m/z (ESI): 458.1 [M+H]⁺, 460.1 [M+2+H]⁺.

### Example 24

### Preparation of

### (3-(4-(2-(cyclopropylamino)phenyl)piperazin-1-yl)-6-(2,3-dichlorophenyl)-5-methylpyr azin-2-yl)methanol

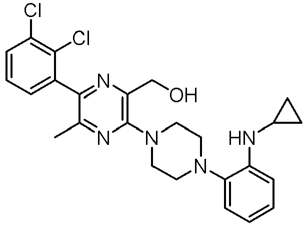

The compound of Example 24 was prepared by referring to the experimental scheme of Example 21.

MS m/z (ESI): 484.1 [M+H]⁺, 486.1 [M+2+H]⁺.

### Example 25

### Preparation of

### (6-(2,3-dichlorophenyl)-3-(4-(imidazo[1,5-a]pyridin-5-yl)piperazin-1-yl)-5-methylpyraz in-2-yl)methanol

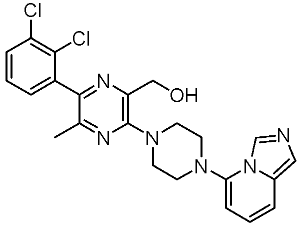

The compound of Example 25 was prepared by referring to the experimental scheme of Example 21.

MS m/z (ESI): 469.1 [M+H]⁺, 471.1 [M+2+H]⁺.

### Example 26

### Preparation of

### (6-(2,3-dichlorophenyl)-5-methyl-3-(4-(2-methyl-1H-benzo[d]imidazol-7-yl)piperazin-1-yl)pyrazin-2-yl)methanol

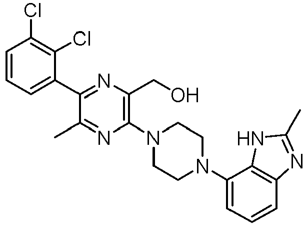

The compound of Example 26 was prepared by referring to the experimental scheme of Example 21.

MS m/z (ESI): 483.1 [M+H]⁺, 485.1 [M+2+H]⁺.

### Example 27

### Preparation of

### (3-(4-(3-(1-aminoethyl)phenyl)piperazin-1-yl)-6-(2,3-dichlorophenyl)-5-methylpyrazin-2-yl)methanol

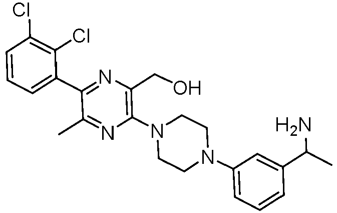

The compound of Example 27 was prepared by referring to the experimental scheme of Example 21.

MS m/z (ESI): 472.1 [M+H]⁺, 474.1 [M+2+H]⁺.

### Example 28

### Preparation of

### (3-(4-(3-(2-aminopropan-2-yl)phenyl)piperazin-1-yl)-6-(2,3-dichlorophenyl)-5-methylp yrazin-2-yl)methanol

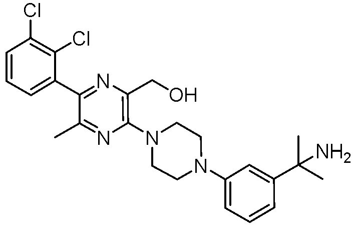

The compound of Example 28 was prepared by referring to the experimental scheme of Example 21.

MS m/z (ESI): 486.1 [M+H]⁺, 488.1 [M+2+H]⁺.

### Example 29

### Preparation of

### (3-(4-(2-aminophenyl)piperazin-1-yl)-5-methyl-6-(1-methyl-1H-benzo[d]imidazol-6-yl) pyrazin-2-yl)methanol

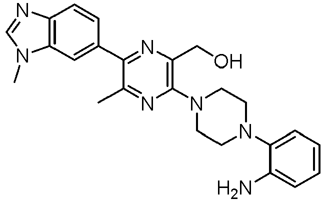

The compound of Example 29 was prepared by referring to the experimental scheme of Example 21.

MS m/z (ESI): 430.1 [M+H]⁺.

### Example 30

### Preparation of

### (3-(4-(2-aminophenyl)piperazin-1-yl)-6-(benzo[d][1,3]dioxol-5-yl)-5-methylpyrazin-2-yl)methanol

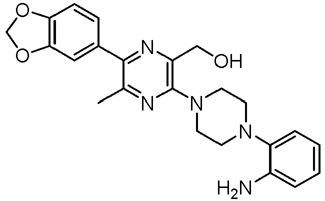

The compound of Example 30 was prepared by referring to the experimental scheme of Example 21.

MS m/z (ESI): 420.1 [M+H]⁺.

### Example 31

### Preparation of

### (R)-8-(6-amino-5-(2,3-dichlorophenyl)-3-(1,3,4-oxadiazol-2-yl)pyrazin-2-yl)-8-azaspiro [4.5]decan-1-amine

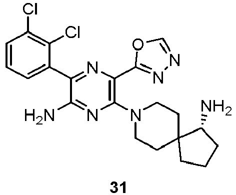

### Step 1: Preparation of ethyl 5-amino-6-(2,3-dichlorophenyl)pyrazine-2-carboxylate

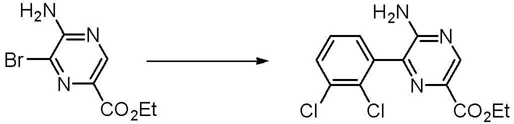

Ethyl 5-amino-6-bromopyrazine-2-carboxylate (1.0 g, 4.08 mmol), 2,3-dichlorobenzeneboronic acid (930 mg, 4.90 mmol) and potassium carbonate (1.7 g, 12.24 mmol) were dissolved in 50 ml of tetrahydrofuran and 5 mL of water. Pd(dppf)Cl₂ (599 mg, 0.82 mmol) was added, and the reaction solution was purged with nitrogen. The reaction solution was reacted under microwave at 90°C for 3 hours. The reaction solution was concentrated to remove the solvent, and extracted with ethyl acetate. The organic phase was dried over anhydrous sodium sulfate and filtered. The filtrate was concentrated to obtain a crude product, which was purified by column chromatography to obtain the target product ethyl 5-amino-6-(2,3-dichlorophenyl)pyrazine-2-carboxylate (680 mg, yield: 54%).

MS m/z (ESI): 312.1 [M+H]⁺, 314.1 [M+2+H]⁺.

### Step 2: Preparation of ethyl 5 -amino-3 -chloro-6-(2,3 -dichlorophenyl)pyrazine-2-carboxylate

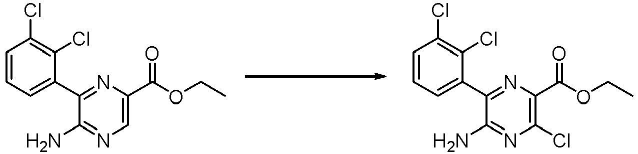

Ethyl 5-amino-6-(2,3-dichlorophenyl)pyrazine-2-carboxylate (650 mg, 2.09 mmol) was dissolved in 50 mL of DMF. NCS (335 mg, 2.51 mmol) and KOH (234 mg, 4.18 mmol) were added, and the reaction solution was stirred at room temperature for 3 hours. 200 mL of water was added, and the solution was stirred for 30 minutes and filtered. The filter cake was washed with water, and dried to obtain a solid crude product. The filtrate was extracted with dichloromethane, dried over anhydrous sodium sulfate, and concentrated to obtain a crude product. The combined crude products were purified by column chromatography to obtain the product ethyl 5-amino-3-chloro-6-(2,3-dichlorophenyl)pyrazine-2-carboxylate (650 mg, yield: 90%).

MS m/z (ESI): 346.1 [M+H]⁺, 348.1 [M+2+H]⁺.

### Step 3: Preparation of ethyl (R)-5-amino-3-(1-amino-8-azaspiro[4.5]decan-8-yl)-6-(2,3-dichlorophenyl)pyrazine-2-c arboxylate

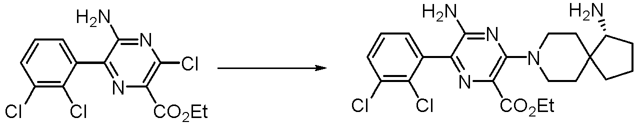

Ethyl 5-amino-3-chloro-6-(2,3-dichlorophenyl)pyrazine-2-carboxylate (600 mg, 1.74 mmol), (R)-8-azaspiro[4.5]decane-1-amine (402 mg, 2.61 mmol) and DIPEA (673 mg, 5.22 mmol) were dissolved in DMF (20 mL). The reaction solution was heated to 80°C and reacted overnight. The reaction solution was cooled to room temperature, and then water (50 mL) was added. The solution was extract with ethyl acetate (30 mL) three times, and the organic phase was dried over anhydrous sodium sulfate and filtered. The filtrate was concentrated to obtain a crude product, which was purified by column chromatography to obtain the product ethyl (R)-5-amino-3-(1-amino-8-azaspiro[4.5]decan-8-yl)-6-(2,3-dichlorophenyl)pyrazine-2-c arboxylate (640 mg, yield: 79%).

MS m/z (ESI): 464.1 [M+H]⁺, 466.1 [M+2+H]⁺.

### Step 4: Preparation of (R)-5-amino-3-(1-amino-8-azaspiro[4.5]decan-8-yl)-6-(2,3-dichlorophenyl)pyrazine-2-c arbohydrazide

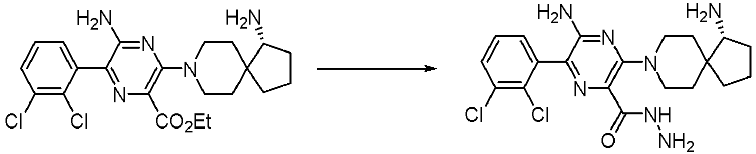

Ethyl (R)-5-amino-3-(1-amino-8-azaspiro[4.5]decan-8-yl)-6-(2,3-dichlorophenyl)pyrazine-2-c arboxylate (200 mg, 0.43 mmol) was dissolved in methanol (30 mL). Hydrazine hydrate (215 mg, 4.3 mmol) was added dropwise at room temperature, and the reaction solution was stirred overnight at room temperature after completion of the addition. Ethyl acetate (100 mL) was added, and the organic phase was washed with water and saturated brine, dried over anhydrous sodium sulfate and filtered. The filtrate was concentrated to obtain the product (R)-5-amino-3-(1-amino-8-azaspiro[4.5]decan-8-yl)-6-(2,3-dichlorophenyl)pyrazine-2-c arbohydrazide (183 mg, yield: 95%).

MS m/z (ESI): 450.1 [M+H]⁺, 452.1 [M+2+H]⁺.

### Step 5: Preparation of (R)-8-(6-amino-5-(2,3-dichlorophenyl)-3-(1,3,4-oxadiazol-2-yl)pyrazin-2-yl)-8-azaspiro [4.5]decan-1-amine

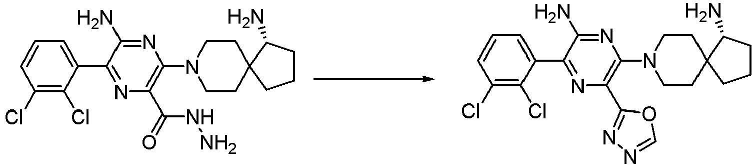

(R)-5-Amino-3-(1-amino-8-azaspiro[4.5]decan-8-yl)-6-(2,3-dichlorophenyl)pyrazi ne-2-carbohydrazide (180 mg, 0.4 mmol) was added to triethyl orthoformate (20 mL), and the reaction solution was heated to reflux for 24 hours. The reaction solution was cooled to room temperature, and then ethyl acetate (100 mL) was added. The organic phase was washed with water and saturated brine, dried over anhydrous sodium sulfate and filtered. The filtrate was concentrated to obtain a crude product, which was purified by column chromatography to obtain the product (R)-8-(6-amino-5-(2,3-dichlorophenyl)-3-(1,3,4-oxadiazol-2-yl)pyrazin-2-yl)-8-azaspiro [4.5]decan-1-amine (35 mg, yield: 19%).

¹H NMR (400 MHz, MeOD) δ: 8.05 (d, *J* = 7.2 Hz, 1H), 7.88 (d, *J* = 7.6 Hz, 1H), 7.54 (t, *J* = 7.2 Hz, 1H), 5.54 (s, 1H), 3.24 (m, 4H), 2.55 (m, 1H), 1.78-1.54 (m, 2H), 1.51-1.34 (m, 6H), 1.28-1.14 (m, 2H).

MS m/z (ESI): 460.1 [M+H]⁺, 462.1 [M+2+H]⁺.

The compounds of Examples 32 to 49 and 60 to 65 were prepared by referring to the experimental scheme of Example 31.

### Example 32

### Preparation of

### 6-(3-aminopiperidin-1-yl)-3-(2,3-dichlorophenyl)-5-(1,3,4-oxadiazol-2-yl)pyrazin-2-am ine

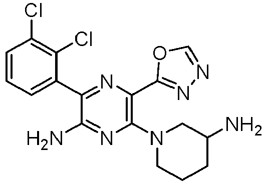

The compound of Example 32 was prepared by referring to the experimental scheme of Example 31.

MS m/z (ESI): 406.1 [M+H]⁺, 408.1 [M+2+H]⁺.

### Example 33

### Preparation of

### N2-(3-amino-3-methylcyclobutyl)-5-(2,3-dichlorophenyl)-N2-methyl-3-(1,3,4-oxadiazo 1-2-yl)pyrazine-2,6-diamine

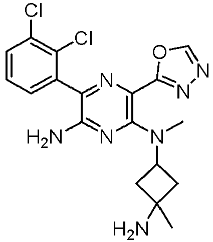

The compound of Example 33 was prepared by referring to the experimental scheme of Example 31.

MS m/z (ESI): 420.1 [M+H]⁺, 422.1 [M+2+H]⁺.

### Example 34

### Preparation of

### 3-(6-amino-5-(2,3-dichlorophenyl)-3-(1,3,4-oxadiazol-2-yl)pyrazin-2-yl)-3-azabicyclo[ 3.1.0]hexan-6-amine

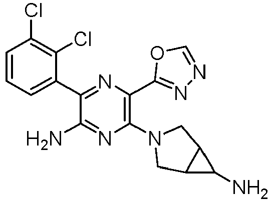

The compound of Example 34 was prepared by referring to the experimental scheme of Example 31.

MS m/z (ESI): 404.1 [M+H]⁺, 406.1 [M+2+H]⁺.

### Example 35

### Preparation of

### 2-(6-amino-5-(2,3-dichlorophenyl)-3-(1,3,4-oxadiazol-2-yl)pyrazin-2-yl)octahydrocyclo penta[c]pyrrol-5-amine

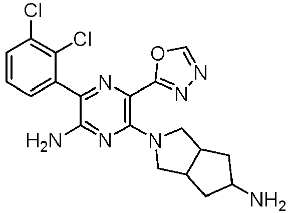

The compound of Example 35 was prepared by referring to the experimental scheme of Example 31.

MS m/z (ESI): 432.1 [M+H]⁺, 434.1 [M+2+H]⁺.

### Example 36

Preparation of

### 3-(2,3-dichlorophenyl)-6-(hexahydropyrrolo[3,4-c]pyrrol-2(1H)-yl)-5-(1,3,4-oxadiazol-2-yl)pyrazin-2-amine

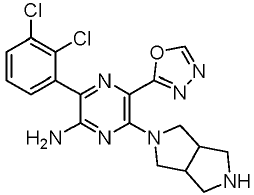

The compound of Example 36 was prepared by referring to the experimental scheme of Example 31.

MS m/z (ESI): 418.1 [M+H]⁺, 420.1 [M+2+H]⁺.

### Example 37

### Preparation of

### 6-(((2R)-2-aminocyclopentyl)ethynyl)-3-(2,3-dichlorophenyl)-5-(1,3,4-oxadiazol-2-yl)p yrazin-2-amine

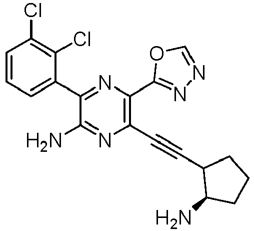

The compound of Example 37 was prepared by referring to the experimental scheme of Example 31.

MS m/z (ESI): 415.1 [M+H]⁺, 417.1 [M+2+H]⁺.

### Example 38

### Preparation of

### 6-(((3R)-3-aminocyclopentyl)ethynyl)-3-(2,3-dichlorophenyl)-5-(1,3,4-oxadiazol-2-yl)p yrazin-2-amine

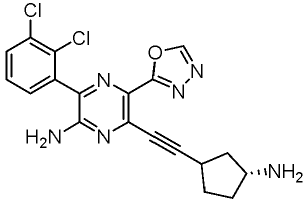

The compound of Example 38 was prepared by referring to the experimental scheme of Example 31.

MS m/z (ESI): 415.1 [M+H]⁺, 417.1 [M+2+H]⁺.

### Example 39

### Preparation of

### (3S,4S)-8-(6-amino-5-(2,3-dichlorophenyl)-3-(1,3,4-oxadiazol-2-yl)pyrazin-2-yl)-3-met hyl-2-oxa-8-azaspiro[4.5]decan-4-amine

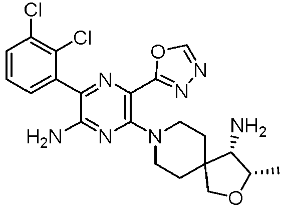

The compound of Example 39 was prepared by referring to the experimental scheme of Example 31.

MS m/z (ESI): 476.1 [M+H]⁺, 478.1 [M+2+H]⁺.

### Example 40

### Preparation of

### 6-(4-amino-4-methylpiperidin-1-yl)-3-(2,3-dichlorophenyl)-5-(1,3,4-oxadiazol-2-yl)pyr azin-2-amine

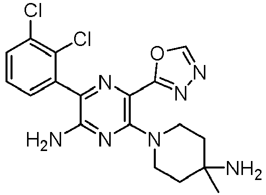

The compound of Example 40 was prepared by referring to the experimental scheme of Example 31.

MS m/z (ESI): 420.1 [M+H]⁺, 422.1 [M+2+H]⁺.

### Example 41

### Preparation of

### 6-(4-amino-4-methylpiperidin-1-yl)-3-(3,5-dichlorophenyl)-5-(I,3,4-oxadiazol-2-yl)pyr azin-2-amine

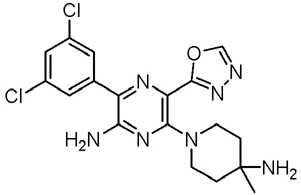

The compound of Example 41 was prepared by referring to the experimental scheme of Example 31.

MS m/z (ESI): 420.1 [M+H]⁺, 422.1 [M+2+H]⁺.

### Example 42

### Preparation of

### 6-(4-amino-4-methylpiperidin-1-yl)-3-(2-chloropyridin-4-yl)-5-(1,3,4-oxadiazol-2-yl)py razin-2-amine

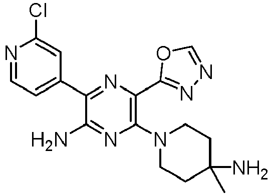

The compound of Example 42 was prepared by referring to the experimental scheme of Example 31.

MS m/z (ESI): 387.1 [M+H]⁺, 389.1 [M+2+H]⁺.

### Example 43

### Preparation of

### 6-(4-amino-4-methylpiperidin-1-yl)-3-(1-methyl-1H-benzo[d]imidazol-5-yl)-5-(1,3,4-o xadiazol-2-yl)pyrazin-2-amine

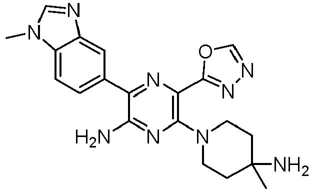

The compound of Example 43 was prepared by referring to the experimental scheme of Example 31.

MS m/z (ESI): 406.1 [M+H]⁺.

### Example 44

### Preparation of

### 6-(4-amino-4-methylpiperidin-1-yl)-3-(benzo[d][1,3]dioxol-4-yl)-5-(1,3,4-oxadiazol-2-yl)pyrazin-2-amine

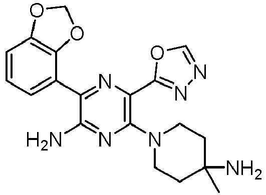

The compound of Example 44 was prepared by referring to the experimental scheme of Example 31.

MS m/z (ESI): 396.1 [M+H]⁺.

### Example 45

### Preparation of

### 6-(4-amino-4-methylpiperidin-1-yl)-5-(1,3,4-oxadiazol-2-yl)-3-(quinolin-7-yl)pyrazin-2 -amine

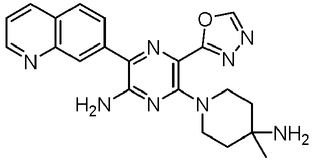

The compound of Example 45 was prepared by referring to the experimental scheme of Example 31.

MS m/z (ESI): 403.1 [M+H]⁺.

### Example 46

### Preparation of ethyl

### 6-(3-amino-5-(4-amino-4-methylpiperidin-1-yl)-6-(1,3,4-oxadiazol-2-yl)pyrazin-2-yl)-2 -naphthoate

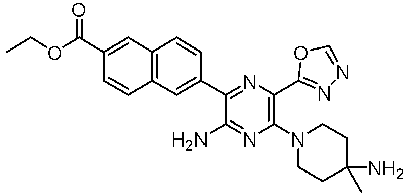

The compound of Example 46 was prepared by referring to the experimental scheme of Example 31.

MS m/z (ESI): 474.1 [M+H]⁺.

### Example 47

### Preparation of

### 3-(3-amino-5-(4-amino-4-methylpiperidin-1-yl)-6-(1,3,4-oxadiazol-2-yl)pyrazin-2-yl)-N,N-dimethylbenzamide

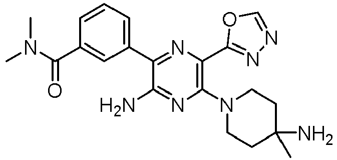

The compound of Example 47 was prepared by referring to the experimental scheme of Example 31.

MS m/z (ESI): 423.1 [M+H]⁺.

### Example 48

### Preparation of

### 6-(3-(1-aminoethyl)phenyl)-3-(2,3-dichlorophenyl)-5-(1,3,4-oxadiazol-2-yl)pyrazin-2-a mine

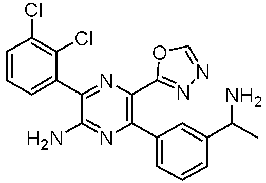

The compound of Example 48 was prepared by referring to the experimental scheme of Example 31.

MS m/z (ESI): 427.1 [M+H]⁺, 429.1 [M+2+H]⁺.

### Example 49

### Preparation of

### 6-(2-amino-2,3-dihydro-1H-inden-5-yl)-3-(2,3-dichlorophenyl)-5-(1,3,4-oxadiazol-2-yl) pyrazin-2-amine

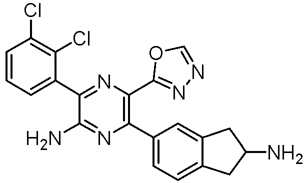

The compound of Example 49 was prepared by referring to the experimental scheme of Example 31.

MS m/z (ESI): 439.1 [M+H]⁺, 441.1 [M+2+H]⁺.

### Example 50

### Preparation of

### (6-((2-amino-3-chloropyridin-4-yl)thio)-3-(3-(aminomethyl)phenyl)-5-methylpyrazin-2-yl)methanol

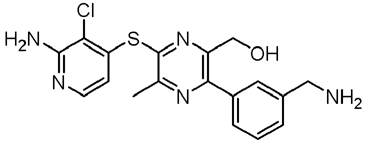

The compound of Example 50 was prepared by referring to the experimental scheme of Example 1.

MS m/z (ESI): 388.1 [M+H]⁺, 390.1 [M+2+H]⁺.

### Example 51

### Preparation of

### (3-(3-(aminomethyl)phenyl)-6-((2,3-dichlorophenyl)thio)-5-methylpyrazin-2-yl)methan ol

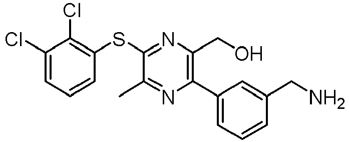

The compound of Example 51 was prepared by referring to the experimental scheme of Example 1.

MS m/z (ESI): 406.1 [M+H]⁺, 408.1 [M+2+H]⁺.

### Example 52

### Preparation of

### (3-(3-(aminomethyl)phenyl)-6-((2,3-dichloropyridin-4-yl)thio)-5-methylpyrazin-2-yl)m ethanol

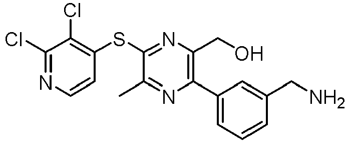

The compound of Example 52 was prepared by referring to the experimental scheme of Example 1.

MS m/z (ESI): 407.1 [M+H]⁺, 409.1 [M+2+H]⁺.

### Example 53

### Preparation of

### 4-((5-(3-(aminomethyl)phenyl)-6-(hydroxymethyl)-3-methylpyrazin-2-yl)thio)-3-chloro picolinonitrile

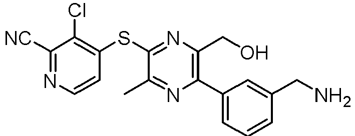

The compound of Example 53 was prepared by referring to the experimental scheme of Example 1.

MS m/z (ESI): 398.1 [M+H]⁺, 400.1 [M+2+H]⁺.

### Example 54

### Preparation of

### (3-(3-(aminomethyl)phenyl)-6-((3-chloro-2-morpholinopyridin-4-yl)thio)-5-methylpyra zin-2-yl)methanol

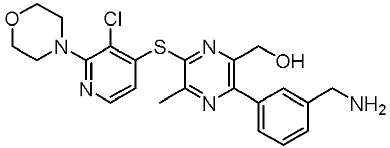

The compound of Example 54 was prepared by referring to the experimental scheme of Example 1.

MS m/z (ESI): 458.1 [M+H]⁺, 460.1 [M+2+H]⁺.

### Example 55

### Preparation of

### (6-((2-amino-3-chloropyridin-4-yl)thio)-3-(4-(2-aminophenyl)piperazin-1-yl)-5-methylp yrazin-2-yl)methanol

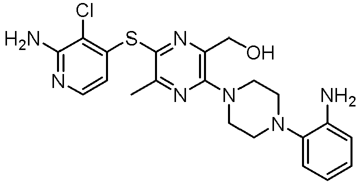

The compound of Example 55 was prepared by referring to the experimental scheme of Example 21.

MS m/z (ESI): 458.1 [M+H]⁺, 460.1 [M+2+H]⁺.

### Example 56

### Preparation of

### (3-(4-(2-aminophenyl)piperazin-1-yl)-6-((3-chloro-2-morpholinopyridin-4-yl)thio)-5-m ethylpyrazin-2-yl)methanol

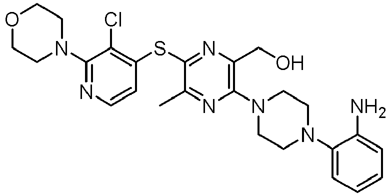

The compound of Example 56 was prepared by referring to the experimental scheme of Example 21.

MS m/z (ESI): 528.1 [M+H]⁺, 530.1 [M+2+H]⁺.

### Example 57

### Preparation of

### (3-(4-(2-aminophenyl)piperazin-1-yl)-6-((2,3-dichloropyridin-4-yl)thio)-5-methylpyrazi n-2-yl)methanol

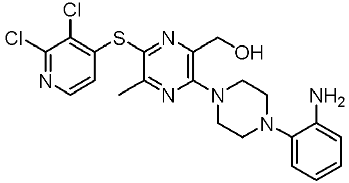

The compound of Example 57 was prepared by referring to the experimental scheme of Example 21.

MS m/z (ESI): 477.1 [M+H]⁺, 479.1 [M+2+H]⁺.

### Example 58

### Preparation of

### 4-((5-(4-(2-aminophenyl)piperazin-1-yl)-6-(hydroxymethyl)-3-methylpyrazin-2-yl)thio) -3-chloropicolinonitrile

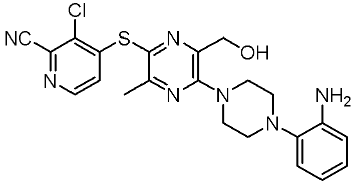

The compound of Example 58 was prepared by referring to the experimental scheme of Example 21.

MS m/z (ESI): 468.1 [M+H]⁺, 470.1 [M+2+H]⁺.

### Example 59

### Preparation of

### (3-(4-(2-aminophenyl)piperazin-1-yl)-6-((2,3-dichlorophenyl)thio)-5-methylpyrazin-2-y l)methanol

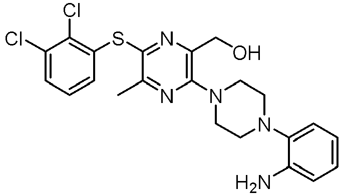

The compound of Example 59 was prepared by referring to the experimental scheme of Example 21.

MS m/z (ESI): 476.1 [M+H]⁺, 478.1 [M+2+H]⁺.

### Example 60

### Preparation of

### 3-((2-amino-3-chloropyridin-4-yl)thio)-6-(4-amino-4-methylpiperidin-1-yl)-5-(1,3,4-ox adiazol-2-yl)pyrazin-2-amine

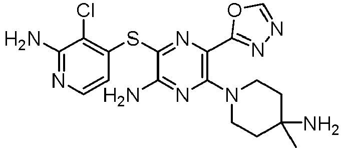

The compound of Example 60 was prepared by referring to the experimental scheme of Example 31.

MS m/z (ESI): 434.1 [M+H]⁺, 436.1 [M+2+H]⁺.

### Example 61

### Preparation of

### 6-(4-amino-4-methylpiperidin-1-yl)-3-((3-chloro-2-morpholinopyridin-4-yl)thio)-5-(1,3 ,4-oxadiazol-2-yl)pyrazin-2-amine

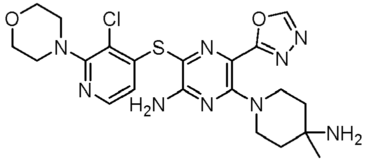

The compound of Example 61 was prepared by referring to the experimental scheme of Example 31.

MS m/z (ESI): 504.1 [M+H]⁺, 506.1 [M+2+H]⁺.

### Example 62

### Preparation of

### 6-(4-amino-4-methylpiperidin-1-yl)-3-((2,3-dichloropyridin-4-yl)thio)-5-(1,3,4-oxadiaz ol-2-yl)pyrazin-2-amine

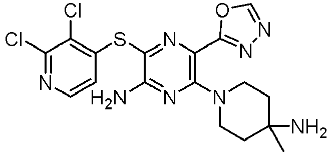

The compound of Example 62 was prepared by referring to the experimental scheme of Example 31.

MS m/z (ESI): 453.1 [M+H]⁺, 455.1 [M+2+H]⁺.

### Example 63

### Preparation of

### 4-((3-amino-5-(4-amino-4-methylpiperidin-1-yl)-6-(1,3,4-oxadiazol-2-yl)pyrazin-2-yl)t hio)-3-chloropicolinonitrile

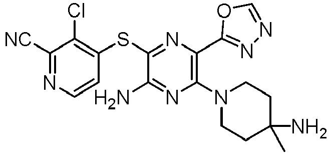

The compound of Example 63 was prepared by referring to the experimental scheme of Example 31.

MS m/z (ESI): 444.1 [M+H]⁺, 446.1 [M+2+H]⁺.

### Example 64

### Preparation of

### 6-(4-amino-4-methylpiperidin-1-yl)-3-((2,3-dichlorophenyl)thio)-5-(1,3,4-oxadiazol-2-y l)pyrazin-2-amine

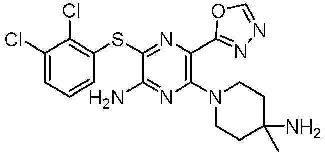

The compound of Example 64 was prepared by referring to the experimental scheme of Example 31.

MS m/z (ESI): 452.1 [M+H]⁺, 454.1 [M+2+H]⁺.

### Example 65

### Preparation of

### (3S,4S)-8-(6-amino-5-((2-amino-3-chloropyridin-4-yl)thio)-3-(1,3,4-oxadiazol-2-yl)pyr azin-2-yl)-3-methyl-2-oxa-8-azaspiro[4.5]decan-4-amine

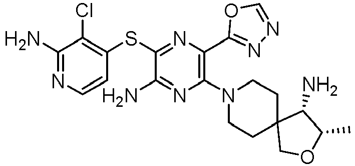

The compound of Example 65 was prepared by referring to the experimental scheme of Example 31.

MS m/z (ESI): 490.1 [M+H]⁺, 492.1 [M+2+H]⁺.

### Example 66

### Preparation of

### (R)-(3-(1-amino-8-azaspiro[4.5]decan-8-yl)-6-(2,3-dichlorophenyl)-4-methylpyridin-2-yl)methanol

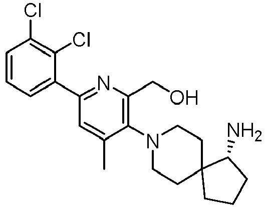

The compound of Example 66 was prepared by referring to the experimental scheme of Example 1.

MS m/z (ESI): 420.1 [M+H]⁺, 422.1 [M+2+H]⁺.

### Example 67

### Preparation of

### (R)-(4-amino-3-(1-amino-8-azaspiro[4.5]decan-8-yl)-6-(2,3-dichlorophenyl)pyridin-2-y l)methanol

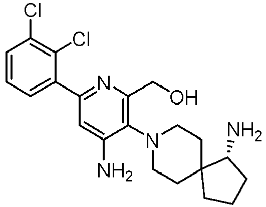

The compound of Example 67 was prepared by referring to the experimental scheme of Example 1.

MS m/z (ESI): 421.1 [M+H]⁺, 423.1 [M+2+H]⁺.

### Example 68

### Preparation of

### (4-amino-3-(4-amino-4-methylpiperidin-1-yl)-6-(2,3-dichlorophenyl)pyridin-2-yl)metha nol

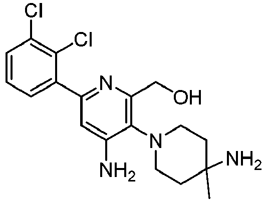

The compound of Example 68 was prepared by referring to the experimental scheme of Example 1.

MS m/z (ESI): 381.1 [M+H]⁺, 383.1[M+2+H]⁺.

### Example 69

### Preparation of

### (4-amino-3-(4-(1-aminocyclopropyl)piperidin-1-yl)-6-(2,3-dichlorophenyl)pyridin-2-yl) methanol

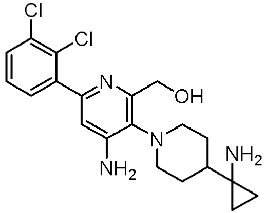

The compound of Example 69 was prepared by referring to the experimental scheme of Example 1.

MS m/z (ESI): 407.1 [M+H]⁺, 409.1 [M+2+H]⁺.

### Example 70

### Preparation of

### (4-amino-3-(4-(amino(cyclopropyl)methyl)piperidin-1-yl)-6-(2,3-dichlorophenyl)pyridi n-2-yl)methanol

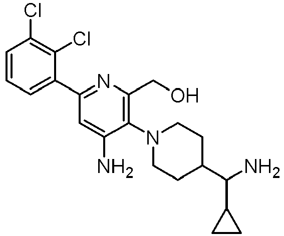

The compound of Example 70 was prepared by referring to the experimental scheme of Example 1.

MS m/z (ESI): 421.1 [M+H]⁺, 423.1 [M+2+H]⁺.

### Example 71

### Preparation of

### (4-amino-3-(4-(aminomethyl)piperidin-1-yl)-6-(2,3-dichlorophenyl)pyridin-2-yl)methan ol

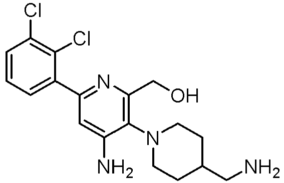

The compound of Example 71 was prepared by referring to the experimental scheme of Example 1.

MS m/z (ESI): 381.1 [M+H]⁺, 383.1[M+2+H]⁺.

### Example 72

### Preparation of

### (4-amino-3-(3-(aminomethyl)pyrrolidin-1-yl)-6-(2,3-dichlorophenyl)pyridin-2-yl)metha nol

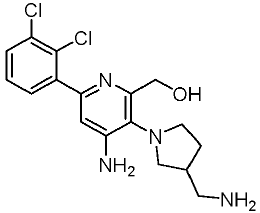

The compound of Example 72 was prepared by referring to the experimental scheme of Example 1.

MS m/z (ESI): 367.1 [M+H]⁺, 369.1 [M+2+H]⁺.

### Example 73

### Preparation of

### (4-amino-3-(3-aminopyrrolidin-1-yl)-6-(2,3-dichlorophenyl)pyridin-2-yl)methanol

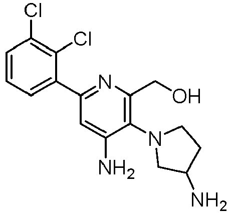

The compound of Example 73 was prepared by referring to the experimental scheme of Example 1.

MS m/z (ESI): 353.1 [M+H]⁺, 355.1 [M+2+H]⁺.

### Example 74

### Preparation of

### (4-amino-3-(4-amino-4-methylpiperidin-1-yl)-6-(1-methyl-1H-benzo[d]imidazol-5-yl)p yridin-2-yl)methanol

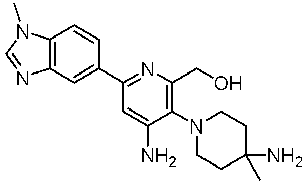

The compound of Example 74 was prepared by referring to the experimental scheme of Example 1.

MS m/z (ESI): 367.1 [M+H]⁺.

### Example 75

### Preparation of

### (4-amino-3-(4-amino-4-methylpiperidin-1-yl)-6-(benzo[d][1,3]dioxol-5-yl)pyridin-2-yl) methanol

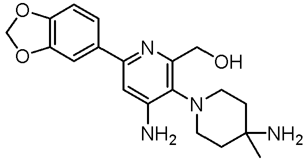

The compound of Example 75 was prepared by referring to the experimental scheme of Example 1.

MS m/z (ESI): 357.1 [M+H]⁺.

### Example 76

### Preparation of

### (4-amino-3-(4-amino-4-methylpiperidin-1-yl)-6-(quinolin-7-yl)pyridin-2-yl)methanol

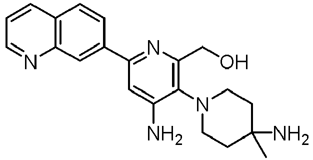

The compound of Example 76 was prepared by referring to the experimental scheme of Example 1.

MS m/z (ESI): 364.1 [M+H]⁺.

### Example 77

### Preparation of ethyl

### 7-(4-amino-5-(4-amino-4-methylpiperidin-1-yl)-6-(hydroxymethyl)pyridin-2-yl)-2-naph thoate

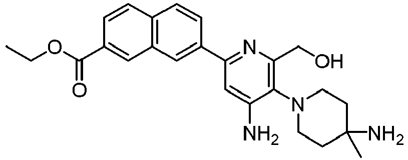

The compound of Example 77 was prepared by referring to the experimental scheme of Example 1.

MS m/z (ESI): 435.1 [M+H]⁺.

### Example 78

### Preparation of

### 4-(4-amino-5-(4-amino-4-methylpiperidin-1-yl)-6-(hydroxymethyl)pyridin-2-yl)-N,N-di methylbenzamide

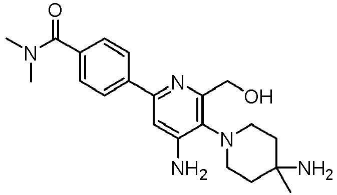

The compound of Example 78 was prepared by referring to the experimental scheme of Example 1.

MS m/z (ESI): 384.1 [M+H]⁺.

### Example 79

### Preparation of

### (4-amino-3-(4-amino-4-methylpiperidin-1-yl)-6-((3-chloro-2-morpholinopyridin-4-yl)th io)pyridin-2-yl)methanol

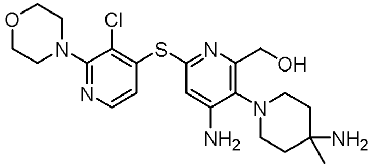

The compound of Example 79 was prepared by referring to the experimental scheme of Example 1.

MS m/z (ESI): 465.1 [M+H]⁺, 467.1 [M+2+H]⁺.

### Example 80

### Preparation of

### (4-amino-3-(4-amino-4-methylpiperidin-1-yl)-6-((2,3-dichloropyridin-4-yl)thio)pyridin-2-yl)methanol

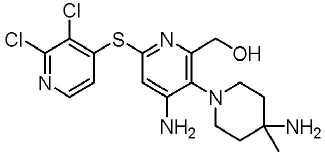

The compound of Example 80 was prepared by referring to the experimental scheme of Example 1.

MS m/z (ESI): 414.1 [M+H]⁺, 416.1 [M+2+H]⁺.

### Example 81

### Preparation of

### (4-amino-6-((2-amino-3-chloropyridin-4-yl)thio)-3-(4-amino-4-methylpiperidin-1-yl)py ridin-2-yl)methanol

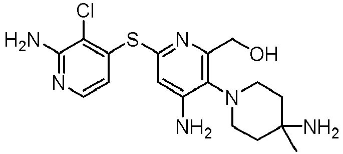

The compound of Example 81 was prepared by referring to the experimental scheme of Example 1.

MS m/z (ESI): 395.1 [M+H]⁺, 397.1 [M+2+H]⁺.

### Example 82

### Preparation of

### (R)-(6-amino-5-(1-amino-8-azaspiro[4.5]decan-8-yl)-2-(2,3-dichlorophenyl)pyrimidin-4 -yl)methanol

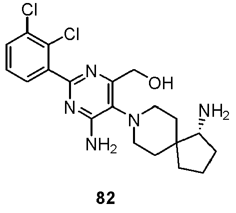

### Step 1: Preparation of methyl 6-amino-2-(2,3-dichlorophenyl)pyrimidine-4-carboxylate

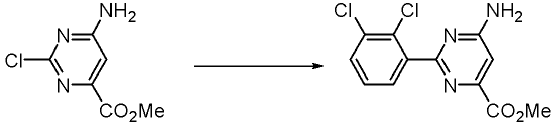

Methyl 6-amino-2-chloropyrimidine-4-carboxylate (1.0 g, 5.35 mmol), 2,3-dichlorobenzeneboronic acid (1.2 g, 6.42 mmol) and potassium carbonate (2.2 g, 16.05 mmol) were dissolved in 30 ml of tetrahydrofuran and 5 mL of water. Pd(dppf)Cl₂ (782 mg, 1.07 mmol) was added, and the reaction solution was purged with nitrogen to remove air. The reaction solution was reacted under microwave at 90°C for 120 minutes. The reaction solution was concentrated to remove the solvent, and extracted with ethyl acetate. The organic phase was dried over anhydrous sodium sulfate and filtered. The filtrate was concentrated to obtain a crude product, which was purified by column chromatography to obtain the target product methyl 6-amino-2-(2,3-dichlorophenyl)pyrimidine-4-carboxylate (1.1 g, yield: 69%).

MS m/z (ESI): 298.1 [M+H]⁺, 300.1 [M+2+H]⁺.

### Step 2: Preparation of methyl 6-amino-5-bromo-2-(2,3-dichlorophenyl)pyrimidine-4-carboxylate

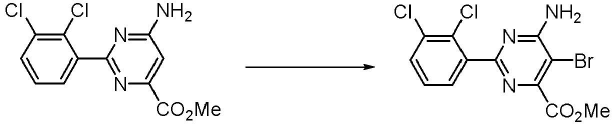

Methyl 6-amino-2-(2,3-dichlorophenyl)pyrimidine-4-carboxylate (1.0 g, 3.37 mmol) was dissolved in 50 mL of DMF. NBS (719 mg, 4.04 mmol) and KOH (377 mg, 6.74 mmol) were added, and the reaction solution was stirred at room temperature for 3 hours. 200 mL of water was added, and the solution was stirred for 30 minutes and filtered. The filter cake was washed with water, and dried to obtain a solid crude product. The filtrate was extracted with dichloromethane, dried over anhydrous sodium sulfate and concentrated. The resulting solid and the dried solid were extracted with ethyl acetate (30 mL) three times. The organic phase was dried over anhydrous sodium sulfate and filtered. The filtrate was concentrated to obtain a crude product, which was purified by column chromatography to obtain the product methyl 6-amino-5-bromo-2-(2,3-dichlorophenyl)pyrimidine-4-carboxylate (890 mg, yield: 70%).

MS m/z (ESI): 376.1 [M+H]⁺, 378.1 [M+2+H]⁺.

### Step 3: Preparation of methyl 6-amino-5-((3S,4S)-4-amino-3-methyl-2-oxa-8-azaspiro[4.5]decan-8-yl)-2-(2,3-dichlor ophenyl)pyrimidine-4-carboxylate

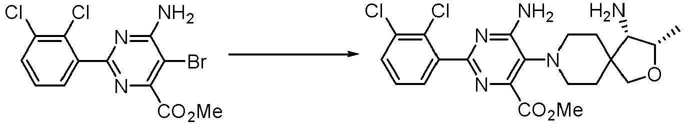

Methyl 6-amino-5-bromo-2-(2,3-dichlorophenyl)pyrimidine-4-carboxylate (800 mg, 2.13 mmol), (3S,4S)-3-methyl-2-oxa-8-azaspiro[4.5]decane-4-amine (400 mg, 2.35 mmol), Pd₂(dba)₃ (394 mg, 0.43 mmol), XantPhos (295 mg, 0.51 mmol) and cesium carbonate (2.1 g, 6.39 mmol) were dissolved in anhydrous dioxane (40 mL). The reaction solution was purged with nitrogen, heated to 100°C and reacted overnight. The reaction solution was cooled to room temperature, and concentrated to remove the solvent. The solution was extract with ethyl acetate (30 mL) three times, and the organic phase was dried over anhydrous sodium sulfate and filtered. The filtrate was concentrated to obtain a crude product, which was purified by column chromatography to obtain the product methyl 6-amino-5-((3S,4S)-4-amino-3-methyl-2-oxa-8-azaspiro[4.5]decan-8-yl)-2-(2,3-dichlor ophenyl)pyrimidine-4-carboxylate (340 mg, yield: 34%).

MS m/z (ESI): 466.1 [M+H]⁺, 468.1 [M+2+H]⁺.

### Step 4: Preparation of (R)-(6-amino-5-(1-amino-8-azaspiro[4.5]decan-8-yl)-2-(2,3-dichlorophenyl)pyrimidin-4 -yl)methanol

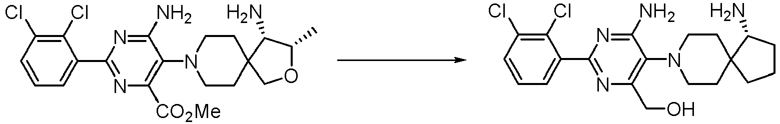

Methyl 6-amino-5-((3S,4S)-4-amino-3-methyl-2-oxa-8-azaspiro[4.5]decan-8-yl)-2-(2,3-dichlor ophenyl)pyrimidine-4-carboxylate (160 mg, 0.56 mmol) and wet palladium on carbon (50 mg) were added to tetrahydrofuran (30 mL), and stirred in the presence of a hydrogen balloon at room temperature for 2 hours. The reaction solution was filtered, and the filtrate was concentrated to obtain the product (R)-(6-amino-5-(1-amino-8-azaspiro[4.5]decan-8-yl)-2-(2,3-dichlorophenyl)pyrimidin-4 -yl)methanol (130 mg, yield: 90%).

¹H NMR (400 MHz, MeOD) δ: 8.13 (d, *J* = 7.6 Hz, 1H), 7.89 (d, *J* = 8 Hz, 1H), 7.45 (t, *J* = 7.6 Hz, 1H), 4.24 (m, 2H), 3.54 (m, 4H), 2.48 (m, 1H), 1.87-1.65 (m, 2H), 1.55-1.33 (m, 6H), 1.27-1.20 (m, 2H).

MS m/z (ESI): 422.1 [M+H]⁺, 424.1 [M+2+H]⁺.

The compounds of Examples 83 to 90 were prepared by referring to the experimental scheme of Example 82.

### Example 83

### Preparation of

### (6-amino-5-(3-aminopiperidin-1-yl)-2-(2,3-dichlorophenyl)pyrimidin-4-yl)methanol

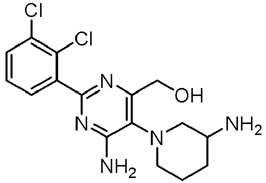

The compound of Example 83 was prepared by referring to the experimental scheme of Example 82.

MS m/z (ESI): 368.1 [M+H]⁺, 370.1 [M+2+H]⁺.

### Example 84

### Preparation of

### (6-amino-5-(6-amino-3-azabicyclo[3.1.0]hexan-3-yl)-2-(2,3-dichlorophenyl)pyrimidin-4-yl)methanol

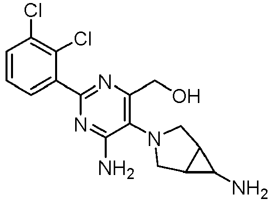

The compound of Example 84 was prepared by referring to the experimental scheme of Example 82.

MS m/z (ESI): 366.1 [M+H]⁺, 368.1 [M+2+H]⁺.

### Example 85

### Preparation of

### (6-amino-5-(5-aminohexahydrocyclopenta[c]pyrrol-2(1H)-yl)-2-(2,3-dichlorophenyl)py rimidin-4-yl)methanol

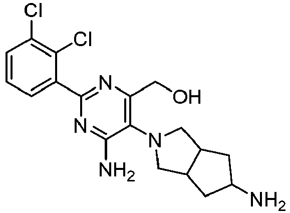

The compound of Example 85 was prepared by referring to the experimental scheme of Example 82.

MS m/z (ESI): 394.1 [M+H]⁺, 396.1 [M+2+H]⁺.

### Example 86

### Preparation of

### (6-amino-2-(2,3-dichlorophenyl)-5-(hexahydropyrrolo[3,4-c]pyrrol-2(1H)-yl)pyrimidin-4-yl)methanol

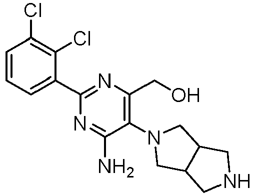

The compound of Example 86 was prepared by referring to the experimental scheme of Example 82.

MS m/z (ESI): 380.1 [M+H]⁺, 382.1 [M+2+H]⁺.

### Example 87

### Preparation of

### (6-amino-5-((3S,4S)-4-amino-3-methyl-2-oxa-8-azaspiro[4.5]decan-8-yl)-2-(2,3-dichlor ophenyl)pyrimidin-4-yl)methanol

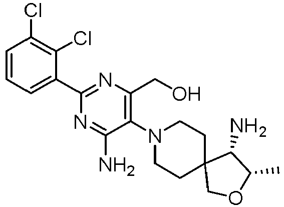

The compound of Example 87 was prepared by referring to the experimental scheme of Example 82.

MS m/z (ESI): 438.1 [M+H]⁺, 440.1 [M+2+H]⁺.

### Example 88

### Preparation of

### (6-amino-2-((3-amino-2-chloropyridin-4-yl)thio)-5-(4-amino-4-methylpiperidin-1-yl)py rimidin-4-yl)methanol

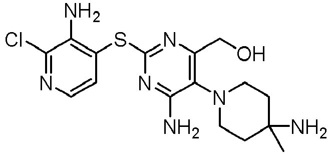

The compound of Example 88 was prepared by referring to the experimental scheme of Example 82.

MS m/z (ESI): 396.1 [M+H]⁺, 398.1 [M+2+H]⁺.

### Example 89

### Preparation of

### 4-((4-amino-5-(4-amino-4-methylpiperidin-1-yl)-6-(hydroxymethyl)pyrimidin-2-yl)thio )-2-chloronicotinonitrile

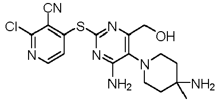

The compound of Example 89 was prepared by referring to the experimental scheme of Example 82.

MS m/z (ESI): 406.1 [M+H]⁺, 408.1 [M+2+H]⁺.

### Example 90

### Preparation of

### (6-amino-5-(4-amino-4-methylpiperidin-l-yl)-2-((2,3-dichlorophenyl)thio)pyrimidin-4-yl)methanol

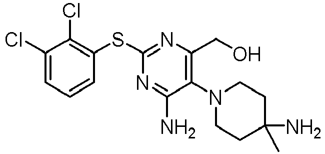

The compound of Example 90 was prepared by referring to the experimental scheme of Example 82.

MS m/z (ESI): 414.1 [M+H]⁺, 416.1 [M+2+H]⁺.

### Example 91

### Preparation of

### (S)-l'-(6-amino-5-((2-amino-3-cyclopropylpyridin-4-yl)thio)pyrazin-2-yl)-1,3-dihydros piro[indene-2,4'-piperidin]-1-amine

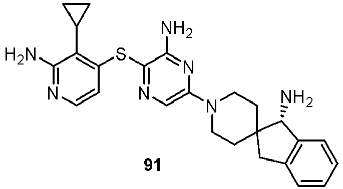

### Step 1: Preparation of 2-ethylhexyl 3-((2-amino-3-chloropyridin-4-yl)thio)propanoate

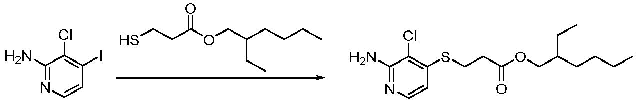

In a microwave reaction tube, 3-chloro-4-iodopyridin-2-amine (800 mg, 3.15 mmol) and 2-ethylhexyl 3-mercaptopropanoate (687 mg, 3.15 mmol) were dissolved in 1,4-dioxane (7 mL), followed by the addition of DIPEA (813 mg, 6.3 mmol), palladium acetate (35 mg, 0.16 mmol) and Xantphos (109 mg, 0.19 mmol). The reaction solution was bubbled with nitrogen for 3 minutes, heated under microwave to 100°C and reacted for 1 hour. The reaction solution was cooled, diluted with 20 mL of ethyl acetate, and filtered through diatomaceous earth to remove insoluble substance. The filtrate was concentrated to dryness by rotary evaporation, and the resulting residue was purified by column chromatography (15∼25% ethyl acetate/petroleum ether) to obtain the product (1.03 g, yield: 95 %) as a brown solid.

MS m/z (ESI): 345.1 [M+H]⁺.

### Step 2: Preparation of 2-ethylhexyl 3-((2-amino-3-cyclopropylpyridin-4-yl)thio)propanoate

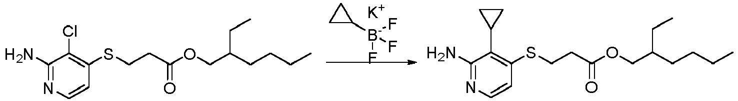

In a sealed tube, 2-ethylhexyl 3-((2-amino-3-chloropyridin-4-yl)thio)propanoate (500 mg, 1.45 mmol) was dissolved in a mixed solvent of toluene and water (toluene/water = 10 mL/1 mL), followed by the addition of potassium cyclopropyl trifluoroborate (280 mg, 1.89 mmol), *n*-butylbis(1-adamantyl)phosphine (52 mg, 0.145 mmol), palladium acetate (16 mg, 0.073 mmol) and cesium carbonate (1.41 g, 4.35 mmol). The reaction solution was bubbled with nitrogen for 3 minutes, heated to 100°C and reacted for 5 hours. The reaction solution was cooled to room temperature, and then 20 mL of saturated NH₄Cl solution was added. The reaction solution was extracted with ethyl acetate (20 mL×3). The ethyl acetate layer was washed with saturated sodium chloride solution, dried over anhydrous sodium sulfate, and purified by column chromatography (50% ethyl acetate/petroleum ether) to obtain the product (220 mg, yield: 43%) as a brown oil.

MS m/z (ESI): 350.1 [M+H]⁺.

### Step 3: Preparation of potassium 2-amino-3-cyclopropylpyridine-4-thiolate

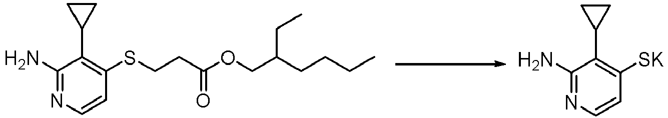

2-Ethylhexyl 3-((2-amino-3-cyclopropylpyridin-4-yl)thio)propanoate (220 mg, 0.63 mmol) was dissolved in 10 mL of ethanol. Potassium *tert*-butoxide (74 mg, 0.66 mmol) was added, and the reaction solution was stirred at room temperature for 1 hour. The reaction solution was concentrated to dryness, and the resulting residue was used directly in the next step.

MS m/z (ESI): 165.1 [M-H]⁻.

### Step 4: Preparation of 3-((2-amino-3-cyclopropylpyridin-4-yl)thio)-6-chloropyrazin-2-amine

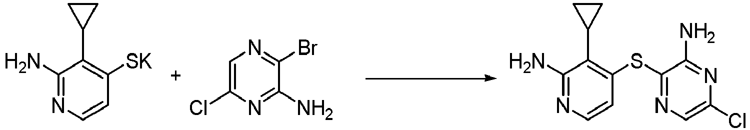

In a microwave reaction tube, potassium 2-amino-3-cyclopropylpyridine-4-thiolate (128 mg, 0.63 mmol) and 2-amino-3-bromo-6-chloropyrazine (149 mg, 0.72 mmol) were dissolved in 7 mL of 1,4-dioxane, followed by the addition of tris(dibenzylideneacetone)dipalladium (33 mg, 0.036 mmol), Xantphos (42 mg, 0.072 mmol) and DIPEA (279 mg, 2.16 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (50∼70% ethyl acetate/petroleum ether) to obtain the product (50 mg, yield: 24%) as a brown solid.

MS m/z (ESI): 294.1 [M+H]⁺.

### Step 5: Preparation of (R)-N-((S)-1'-(6-amino-5-((2-amino-3-cyclopropylpyridin-4-yl)thio)pyrazin-2-yl)-1,3-di hydrospiro[indene-2,4'-piperidin]-1-yl)-2-methylpropane-2-sulfinamide

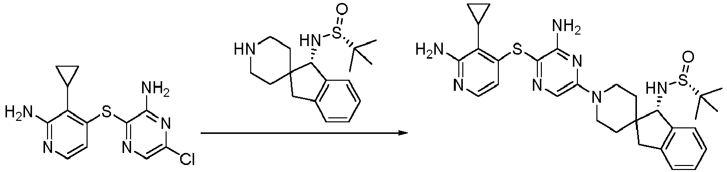

*Tert-butyl* (S)-1-(((R)-*tert*-butylsulfinyl)amino)-1,3-dihydrospiro[indene-2,4'-piperidine]-1'-carbox ylate (69 mg, 0.17 mmol) was dissolved in 3 mL of dichloromethane. 1 mL of trifluoroacetic acid was added, and the reaction solution was reacted at room temperature for 1 hour. The reaction solution was concentrated to dryness, and dissolved in 5 mL of DMF. Potassium carbonate (352 mg, 2.55 mmol) and 3-((2-amino-3-cyclopropylpyridin-4-yl)thio)-6-chloropyrazin-2-amine (50 mg, 0.17 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 mL×3), dried over anhydrous sodium sulfate, and purified by column chromatography (75∼80% ethyl acetate/petroleum ether) to obtain the product (20 mg, yield: 21%) as an oil.

MS m/z (ESI): 564.1 [M+H]⁺.

### Step 6: Preparation of (S)-1'-(6-amino-5-((2-amino-3-cyclopropylpyridin-4-yl)thio)pyrazin-2-yl)-1,3-dihydros piro[indene-2,4'-piperidin]-1-amine

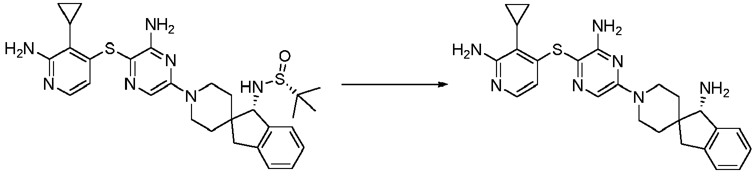

(R)-N-((S)-1'-(6-Amino-5-((2-amino-3-cyclopropylpyridin-4-yl)thio)pyrazin-2-yl)-1,3-dihydrospiro[indene-2,4'-piperidin]-1-yl)-2-methylpropane-2-sulfinamide (20 mg, 0.035 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M NH₃ in methanol. The solution was concentrated to dryness by rotary evaporation, and the resulting residue was purified by column chromatography (0 ∼ 10 % MeOH in DCM). The resulting crude product was purified by thin layer chromatography (dichloromethane: methanol = 10:1) to obtain the product (9.0 mg, yield: 56%) as a brown solid.

¹H NMR (400 MHz, Methanol-*d*₄) δ 7.90 (d, *J* = 5.4 Hz, 1H), 7.61 (s, 1H), 7.40 - 7.33 (m, 1H), 7.27 -7.16 (m, 3H), 6.57 (d, *J* = 5.4 Hz, 1H), 4.28 (d, *J* = 13.6 Hz, 2H), 3.96 (s, 1H), 3.30 - 3.19 (m, 2H), 3.15 (d, *J* = 15.7 Hz, 1H), 2.81 (d, *J* = 15.7 Hz, 1H), 1.90 - 1.78 (m, 2H), 1.82 - 1.70 (m, 1H), 1.58 (d, *J* = 13.5 Hz, 1H), 1.43 (d, *J* = 13.1 Hz, 1H), 1.26 - 1.15 (m, 2H), 0.94 - 0.85 (m, 2H).

MS m/z (ESI): 460.1 [M+H]⁺.

### Example 92

### Preparation of

### (S)-1'-(6-amino-5-((2-chloro-3-cyclopropylpyridin-4-yl)thio)pyrazin-2-yl)-1,3-dihydros piro[indene-2,4'-piperidin]-1-amine

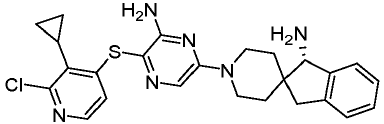

¹H NMR (400 MHz, Methanol-*d*₄) δ 7.88 (d, *J* = 5.5 Hz, 1H), 7.60 (s, 1H), 7.38 - 7.30 (m, 1H), 7.25 -7.15 (m, 3H), 6.54 (d, *J* = 5.5 Hz, 1H), 4.26 (d, *J* = 13.7 Hz, 2H), 3.95 (s, 1H), 3.28 - 3.17 (m, 2H), 3.13 (d, *J* = 15.6 Hz, 1H), 2.79(d, *J* = 15.6 Hz, 1H), 1.88 - 1.74 (m, 2H), 1.80 - 1.70 (m, 1H), 1.56 (d, *J* = 13.7 Hz, 1H), 1.43 (d, *J* = 13.1 Hz, 1H), 1.24 - 1.13 (m, 2H), 0.93 - 0.83 (m, 2H).

MS m/z (ESI): 479.1 [M+H]⁺, 481.1 [M+2+H]⁺.

The compound of Example 92 was prepared by referring to the experimental scheme of Example 93.

### Example 93

### Preparation of

### (S)-1'-(6-amino-5-((3-chloro-2-cyclopropylpyridin-4-yl)thio)pyrazin-2-yl)-1,3-dihydros piro[indene-2,4'-piperidin]-1-amine

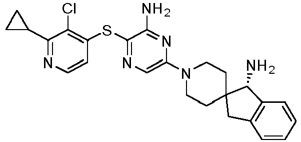

### Step 1: Preparation of 2-ethylhexyl 3-((2,3-dichloropyridin-4-yl)thio)propanoate

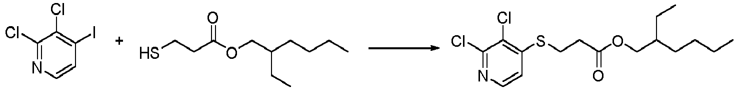

In a microwave reaction tube, 2,3-dichloro-4-iodopyridine (1.0 g, 3.65 mmol) and 2-ethylhexyl 3-mercaptopropanoate (0.88 g, 4.03 mmol) were dissolved in 1,4-dioxane (7 mL), followed by the addition of DIPEA (0.95 g, 7.34 mmol), palladium acetate (41 mg, 0.18 mmol) and Xantphos (127 mg, 0.22 mmol). The reaction solution was bubbled with nitrogen for 3 minutes, heated under microwave to 100°C and reacted for 1 hour. The reaction solution was cooled, diluted with 20 mL of ethyl acetate, and filtered through diatomaceous earth to remove insoluble substance. The filtrate was concentrated to dryness by rotary evaporation, and the resulting residue was purified by column chromatography (10∼15% ethyl acetate/petroleum ether) to obtain a brown oil (1.29 g, yield: 97%).

MS m/z (ESI): 364.1 [M+H]⁺.

### Step 2: Preparation of 2-ethylhexyl 3-((3-chloro-2-cyclopropylpyridin-4-yl)thio)propanoate

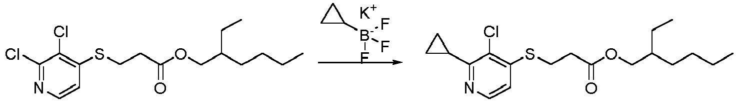

In a sealed tube, 2-ethylhexyl 3-((2,3-dichloropyridin-4-yl)thio)propanoate (1.29 g, 3.54 mmol) was dissolved in a mixed solvent of toluene and water (toluene/water = 20 mL/2 mL), followed by the addition of potassium cyclopropyl trifluoroborate (624 mg, 4.25 mmol), *n*-butylbis(1-adamantyl)phosphine (95 mg, 0.267 mmol), palladium acetate (40 mg, 0.178 mmol) and cesium carbonate (3.43 g, 3.54 mmol). The reaction solution was bubbled with nitrogen for 3 minutes, heated to 100°C and reacted for 5 hours. The reaction solution was cooled to room temperature, and then 20 mL of saturated NH₄Cl solution was added. The reaction solution was extracted with ethyl acetate (20 mL×3). The ethyl acetate layer was washed with saturated sodium chloride solution, dried over anhydrous sodium sulfate, and purified by column chromatography (5∼8% ethyl acetate/petroleum ether) to obtain a yellow oil (600 mg, yield: 45%).

MS m/z (ESI): 370.1 [M+H]⁺.

### Step 3: Preparation of potassium 3-chloro-2-cyclopropylpyridine-4-thiolate

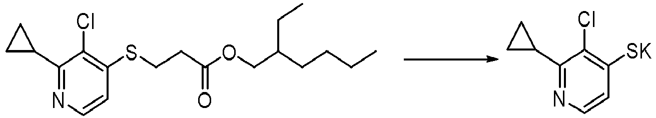

2-Ethylhexyl 3-((3-chloro-2-cyclopropylpyridin-4-yl)thio)propanoate (600 mg, 1.62 mmol) was dissolved in 15 mL of ethanol. Potassium *tert*-butoxide (190 mg, 1.70 mmol) was added, and the reaction solution was stirred at room temperature for 1 hour. The reaction solution was concentrated to dryness, and the resulting residue was used directly in the next step.

MS m/z (ESI): 186.1 [M+H]⁺.

### Step 4: Preparation of 6-chloro-3-((3-chloro-2-cyclopropylpyridin-4-yl)thio)pyrazin-2-amine

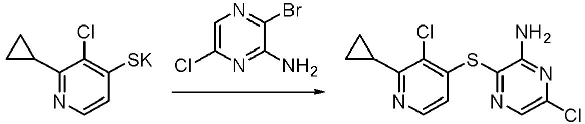

In a microwave reaction tube, potassium 3-chloro-2-cyclopropylpyridine-4-thiolate (120 mg, 0.54 mmol) and 2-amino-3-bromo-6-chloropyrazine (112 mg, 0.54 mmol) were dissolved in 5 mL of 1,4-dioxane, followed by the addition of tris(dibenzylideneacetone)dipalladium (25 mg, 0.027 mmol), Xantphos (31 mg, 0.054 mmol) and DIPEA (209 mg, 1.62 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (10∼20% ethyl acetate/petroleum ether) to obtain the product (135 mg, yield: 80%) as an off-white solid.

MS m/z (ESI): 313.1 [M+H]⁺.

### Step 5: Preparation of tert-butyl 1-oxo-1,3-dihydrospiro[indene-2,4'-piperidine]-1'-carboxylate

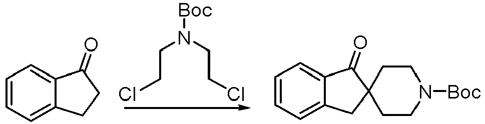

1-Indenone (5.14 g, 38.9 mmol) and *tert*-butyl N,N-bis(2-chloroethyl)carbamate (9.42 g, 38.9 mmol) were dissolved in 100 mL of DMF. 60% sodium hydride (3.89 g, 97.3 mmol) was added in batches in an ice bath. The reaction solution was heated to 60°C in an oil bath under a nitrogen atmosphere and reacted overnight. The reaction solution was cooled, and then 250 mL of saturated sodium chloride solution was added. The reaction solution was extracted with ethyl acetate (150 mL×2). The ethyl acetate layer was washed with saturated sodium chloride solution (100 mL×3), dried over anhydrous MgSO₄, and purified by column chromatography (10∼20% ethyl acetate/petroleum ether) to obtain the crude product (2.80 g, yield: 23%) as a brown oil.

MS m/z (ESI): 202.1 [M-Boc+H]⁺.

### Step 6: Preparation of tert-butyl (R,E)-1-((tert-butylsulfinyl)imino)-1,3-dihydrospiro[indene-2,4'-piperidine]-1'-carboxyl ate

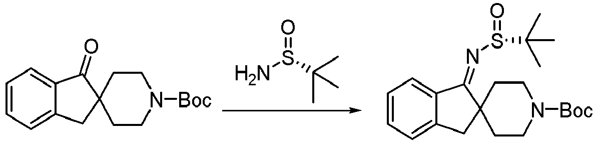

Tetraethyl titanate (40 mL) was heated to 90°C. Tert-butyl 1-oxo-1,3-dihydrospiro[indene-2,4'-piperidine]-1'-carboxylate (2.80 g, 9.27 mmol) and (R)-(+)-*tert*-butylsulfinamide (3.36 g, 27.8 mmol) were added, and the reaction solution was reacted for 24 hours at 90°C under a nitrogen atmosphere. After completion of the reaction, the reaction solution was poured into 400 mL of ethyl acetate. 400 mL of saturated sodium chloride solution was slowly added under stirring, and the reaction solution was stirred at room temperature for 20 minutes. The reaction solution was filtered through diatomaceous earth to remove the precipitated solid. After the resulting filtrate was separated into two layers, the ethyl acetate layer was dried over anhydrous magnesium sulfate, and purified by column chromatography (20∼30% ethyl acetate/petroleum ether) to obtain the product (2.20 g, yield: 59%) as a brown oil.

MS m/z (ESI): 405.1 [M+H]⁺.

### Step 7: Preparation of tert-butyl (S)-1-(((R)-tert-butylsulfinyl)amino)-1,3-dihydrospiro[indene-2,4'-piperidine]-1'-carbox ylate

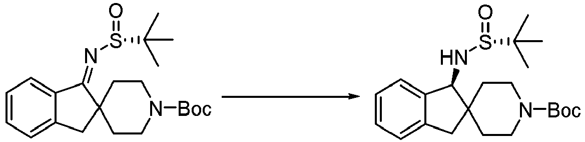

*Tert*-butyl (R,E)-1-((*tert*-butylsulfinyl)imino)-1,3-dihydrospiro[indene-2,4'-piperidine]-1'-carboxyl ate (2.20 g, 5.44 mmol) was dissolved in 30 mL of tetrahydrofuran. The solution was cooled to -78°C, and sodium borohydride (308 mg, 8.16 mmol) was added in batches at this temperature. The reaction solution was gradually warmed up to room temperature under a nitrogen atmosphere and stirred overnight. 200 mL of saturated sodium chloride solution was added, and the reaction solution was extracted with ethyl acetate (100 mL×2). The ethyl acetate layer was washed with saturated sodium chloride solution (100 mL), dried over anhydrous sodium sulfate, and purified by column chromatography (20∼35% ethyl acetate/petroleum ether) to obtain a brown foamy solid (1.21 g, yield: 54%).

MS m/z (ESI): 407.1 [M+H]⁺.

### Step 8: Preparation of (R)-N-((S)-1'-(6-amino-5-((3-chloro-2-cyclopropylpyridin-4-yl)thio)pyrazin-2-yl)-1,3-d ihydrospiro[indene-2,4'-piperidin]-1-yl)-2-methylpropane-2-sulfinamide

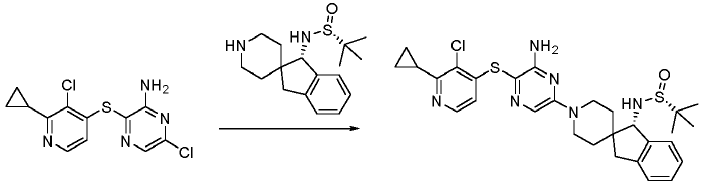

*Tert-*butyl (S)-1-(((R)-*tert*-butylsulfinyl)amino)-1,3-dihydrospiro[indene-2,4'-piperidine]-1'-carbox ylate (100 mg, 0.25 mmol) was dissolved in 3 mL of dichloromethane. 1 mL of trifluoroacetic acid was added, and the reaction solution was reacted at room temperature for 1 hour. The reaction solution was concentrated to dryness, and dissolved in 5 mL of DMF. Potassium carbonate (517 mg, 3.75 mmol) and 6-chloro-3-((3-chloro-2-cyclopropylpyridin-4-yl)thio)pyrazin-2-amine (78 mg, 0.25 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 mL×3), dried over anhydrous sodium sulfate, and purified by column chromatography (50% ethyl acetate/petroleum ether) to obtain the product (26 mg, yield: 18%) as an oil.

MS m/z (ESI): 583.1 [M+H]⁺.

### Step 9: Preparation of (S)-1'-(6-amino-5-((3-chloro-2-cyclopropylpyridin-4-yl)thio)pyrazin-2-yl)-1,3-dihydros piro[indene-2,4'-piperidin]-1-amine

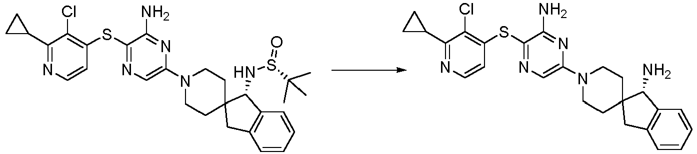

(R)-N-((S)-1'-(6-Amino-5-((3-chloro-2-cyclopropylpyridin-4-yl)thio)pyrazin-2-yl)-1,3-dihydrospiro[indene-2,4'-piperidin]-1-yl)-2-methylpropane-2-sulfinamide (26 mg, 0.044 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M NH₃ in methanol. The reaction solution was concentrated to dryness by rotary evaporation, and the resulting residue was purified by column chromatography (5∼8% MeOH in DCM) to obtain the product (12 mg, yield: 57%) as a light yellow solid.

¹H NMR (400 MHz, Chloroform-d) δ 8.03 (d, *J* = 5.2 Hz, 1H), 7.67 (s, 1H), 7.44 - 7.37 (m, 1H), 7.24 (dd, *J* = 6.4, 3.9 Hz, 3H), 6.39 (d, *J* = 5.2 Hz, 1H), 4.85 (s, 2H), 4.21 - 4.13 (m, 2H), 4.07 (s, 1H), 3.23 - 3.21 (m, 2H), 3.13 (d, / = 15.8 Hz, 1H), 2.79 (d, *J* = 15.7 Hz, 1H), 2.57 - 2.48 (m, 1H), 1.88 - 1.73 (m, 2H), 1.63 - 1.56 (m, 1H), 1.50 - 1.42 (m, 1H), 1.09 - 1.00 (m, 4H).

MS m/z (ESI): 479.1 [M+H]⁺.

### Example 94

### Preparation of

### (2R)-1'-(6-amino-5-((2-amino-3-cyclopropylpyridin-4-yl)thio)pyrazin-2-yl)spiro[bicycl o[3.1.0]hexane-3,4'-piperidin]-2-amine

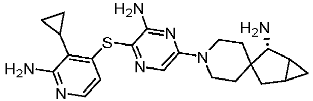

¹H NMR (400 MHz, Methanol-*d*₄) δ 7.73 - 7.67 (m, 1H), 7.65 - 7.60 (m, 1H), 6.08 - 6.01 (m, 1H), 4.53 - 4.41 (m, 2H), 3.63 - 3.51 (m, 2H), 3.14 (p, *J* = 9.6 Hz, 1H), 2.41 - 2.33 (m, 1H), 1.56 - 1.40 (m, 3H), 1.44 - 1.35 (m, 1H), 1.39 - 1.19 (m, 4H), 0.89 - 0.81 (m, 2H), 0.76 - 0.60 (m, 2H), 0.44 - 0.28 (m, 2H).

MS m/z (ESI): 424.1 [M+H]⁺.

The compound of Example 94 was prepared by referring to the experimental scheme of Example 91.

### Example 95

### Preparation of

### (S)-1'-(6-amino-5-((2-amino-3-chloropyridin-4-yl)thio)-3-bromopyrazin-2-yl)-1,3-dihyd rospiro[indene-2,4'-piperidin]-1-amine

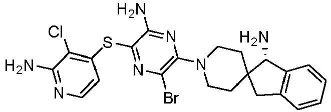

### Step 1: Preparation of 2-ethylhexyl 3-((3-amino-5-chloropyrazin-2-yl)thio)propanoate

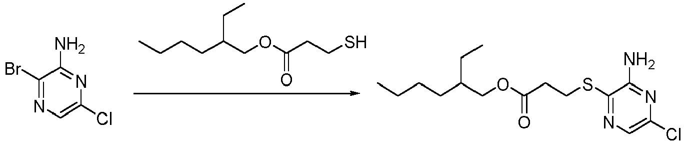

3-Bromo-6-chloropyrazin-2-amine (4 g, 20 mmol), 2-ethylhexyl 3-mercaptopropanoate (5.2 g, 24 mmol), tris(dibenzylideneacetone)dipalladium (916 mg, 1 mmol), 4,5-bis(diphenylphosphino)-9,9-dimethylxanthene (1.16 g, 2 mmol) and N,N-diisopropylethylamine (5.12 g, 40 mmol) were stirred in dioxane (35 mL) at 100°C for 18 hours. The reaction solution was filtered, and the filter cake was washed with ethyl acetate (30 mL) twice. The filtrate was concentrated, and purified by column chromatography [eluent: petroleum ether ∼ petroleum ether/ethyl acetate (90:10)] to obtain 2-ethylhexyl 3-((3-amino-5-chloropyrazin-2-yl)thio)propanoate (5.5 g, yield: 82%) as a brown oil.

MS m/z (ESI): 146.1 [M+H]⁺, 148.1 [M+2+H]⁺.

### Step 2: Preparation of 3-amino-5-chloropyrazine-2-thiol

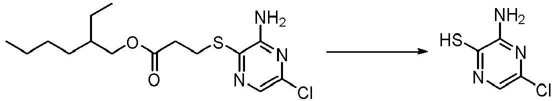

Potassium *tert*-butoxide (2.7 g, 23.9 mmol) was added to a solution of 2-ethylhexyl 3-((3-amino-5-chloropyrazin-2-yl)thio)propanoate (5.5 g, 15.9 mmol) in ethanol (100 mL). After completion of the addition, the reaction solution was stirred at room temperature for 3 hours. The reaction solution was concentrated by rotary evaporation to remove about 50 mL of ethanol. The remaining reaction solution was poured into aqueous ammonium chloride solution (100 mL), and extracted with ethyl acetate (100 mL) twice and dichloromethane (100 mL) twice. The organic phase was concentrated, and purified by column chromatography [eluent: dichloromethane ∼ dichloromethane/methanol (95:5)] to obtain 3-amino-5-chloropyrazine-2-thiol (1.8 g, yield: 70%) as a dark green solid.

MS m/z (ESI): 162.0[M+H]⁺, 164.0 [M+2+H]⁺.

### Step 3: Preparation of 3-((2-amino-3-chloropyridin-4-yl)thio)-6-chloropyrazin-2-amine

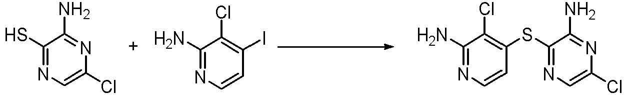

3-Amino-5-chloropyrazine-2-thiol (500 mg, 3.1 mmol), 3-chloro-4-iodopyridin-2-amine (789 mg, 3.1 mmol), tris(dibenzylideneacetone)dipalladium (142 mg, 0.16 mmol), 4,5-bis(diphenylphosphino)-9,9-dimethylxanthene (179 mg, 0.31 mmol) and N,N-diisopropylethylamine (1.2 g, 9.3 mmol) were stirred in dioxane (10 mL) at 130°C under microwave for 1 hour. The reaction solution was concentrated, and purified by column chromatography [eluent: dichloromethane ∼ dichloromethane/methanol (99:1)] to obtain 1 g of a crude product. The crude product was pulped in ethanol (5 mL), and filtered to obtain 3-((2-amino-3-chloropyridin-4-yl)thio)-6-chloropyrazin-2-amine (580 mg, yield: 65%) as a grey solid.

MS m/z (ESI): 288.0 [M+H]⁺, 290.0 [M+2+H]⁺.

### Step 4: Preparation of N-((S)-1,3-dihydrospiro[indene-2,4'-piperidin]-1-yl)-2-methylpropane-2-sulfinamide

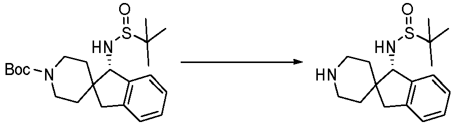

Trifluoroacetic acid (1 mL) was added to a solution of *tert*-butyl (1S)-1-((*tert*-butylsulfinyl<sulfenyl>)amino)-1,3-dihydrospiro[indene-2,4'-piperidine]-1' -carboxylate (150 mg, 0.37 mmol) in dichloromethane (3 mL). After completion of the addition, the reaction solution was stirred at room temperature for 2 hours. The reaction solution was concentrated to obtain N-((S)-1,3-dihydrospiro[indene-2,4'-piperidin]-1-yl)-2-methylpropane-2-sulfinamide hydrochloride (150 mg, yield: 100%) as a light yellow solid.

MS m/z (ESI): 307.2 [M+H]⁺.
[α]²⁰_{D}=1.773.

### Step 5: Preparation of N-((S)-1'-(6-amino-5-((2-amino-3-chloropyridin-4-yl)thio)pyrazin-2-yl)-1,3-dihydrospir o[indene-2,4'-piperidin]-1-yl)-2-methylpropane-2-sulfinamide

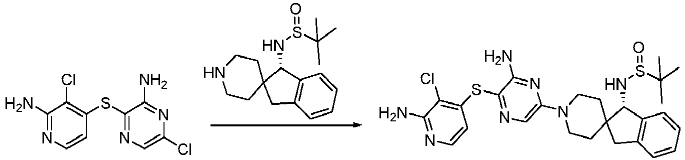

N-((S)-1,3-Dihydrospiro[indene-2,4'-piperidin]-1-yl)-2-methylpropane-2-sulfinami de hydrochloride (150 mg, 0.37 mmol), 3-((2-amino-3-chloropyridin-4-yl)thio)-6-chloropyrazin-2-amine (100 mg, 0.35 mmol) and potassium carbonate (335 mg, 2.43 mmol) were stirred in N,N-dimethylformamide (4 mL) at 100°C for 18 hours. The reaction solution was concentrated, and purified by column chromatography [eluent: dichloromethane ∼ dichloromethane/methanol (97:3)] to obtain N-((S)-1'-(6-amino-5-((2-amino-3-chloropyridin-4-yl)thio)pyrazin-2-yl)-1,3-dihydrospir o[indene-2,4'-piperidin]-1-yl)-2-methylpropane-2-sulfinamide (100 mg, yield: 52%) as a purple solid.

MS m/z (ESI): 558.1 [M+H]⁺, 560.2 [M+2+H]⁺.

### Step 6: Preparation of (S)-1'-(6-amino-5-((2-amino-3-chloropyridin-4-yl)thio)-3-bromopyrazin-2-yl)-1,3-dihyd rospiro[indene-2,4'-piperidin]-1-amine

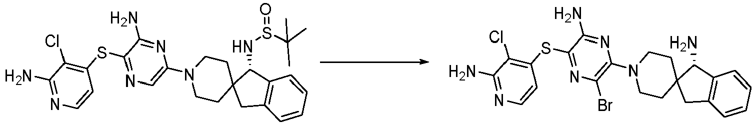

N-((S)-1'-(6-Amino-5-((2-amino-3-chloropyridin-4-yl)thio)pyrazin-2-yl)-1,3-dihyd rospiro[indene-2,4'-piperidin]-1-yl)-2-methylpropane-2-sulfinamide (100 mg, 0.18 mmol) and N-bromosuccinimide (64 mg, 0.36 mmol) were stirred in N,N-dimethylformamide (1 mL) at room temperature for 18 hours. The reaction solution was concentrated to obtain a crude product, which was purified by column chromatography [eluent: dichloromethane ∼ dichloromethane/methanol (95:5)] to obtain (S)-1'-(6-amino-5-((2-amino-3-chloropyridin-4-yl)thio)-3-bromopyrazin-2-yl)-1,3-dihyd rospiro[indene-2,4'-piperidin]-1-amine (80 mg, yield: 84%) as a khaki solid.

¹H NMR (400 MHz, DMSO-d6) δ 7.72-7.64 (m, 1H), 7.36 (d, *J* = 4 Hz, 1H), 7.27-7.16 (m, 3H), 6.58 (s, 2H), 6.33 (s, 2H), 5.80 (d, *J* = 4 Hz, 1H), 3.99 (s, 1H), 3.94-3.83 (m, 2H), 3.17 (d, *J* = 4 Hz, 1H), 3.14-2.98 (m, 3H), 2.77-2.64 (m, 1H), 1.97-1.74 (m, 2H), 1.55 (d, *J* = 12 Hz, 1H).

MS m/z (ESI): 532.0 [M+H]⁺, 534.0 [M+2+H]⁺.

### Example 96

### Preparation of

### (S)-1'-(6-amino-5-((2-amino-3-chloropyridin-4-yl)thio)-3-methylpyrazin-2-yl)-1,3-dihy drospiro[indene-2,4'-piperidin]-1-amine

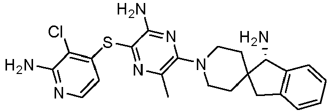

### Step 1: Preparation of (S)-1'-(6-amino-5-((2-amino-3-chloropyridin-4-yl)thio)-3-methylpyrazin-2-yl)-1,3-dihy drospiro[indene-2,4'-piperidin]-1-amine

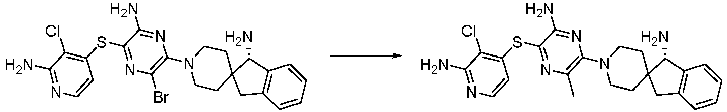

(S)-1'-(6-Amino-5-((2-amino-3-chloropyridin-4-yl)thio)-3-bromopyrazin-2-yl)-1,3-dihydrospiro[indene-2,4'-piperidin]-1-amine (30 mg, 0.056 mmol), trimethylboroxine (789 mg, 3.1 mmol), [1,1'-bis(diphenylphosphino)ferrocene]dichloropalladium(II) (4 mg, 0.0056 mmol) and potassium carbonate (15 mg, 0.11 mmol) were stirred in N,N-dimethylformamide (10 mL) at 130°C under microwave for 1 hour. The reaction solution was concentrated and purified by high performance liquid chromatography to obtain

### (S)-1'-(6-amino-5-((2-amino-3-chloropyridin-4-yl)thio)-3-methylpyrazin-2-yl)-1,3-dihy drospiro[indene-2,4'-piperidin]-1-amine (0.7 mg, yield: 3%) as a grey solid.

¹H NMR (400 MHz, DMSO) δ 7.73-7.62 (m, 1H), 7.35 (d, *J* = 4 Hz, 1H), 7.28-7.16 (m, 3H), 6.57 (s, 2H), 6.32 (s, 2H), 5.78 (d, *J* = 4 Hz, 1H), 3.97 (s, 1H), 3.94-3.81 (m, 2H), 3.15 (d, *J* = 4 Hz, 1H), 3.12-2.95 (m, 3H), 2.75-2.64 (m, 1H), 2.41 (s, 3H), 1.99-1.75 (m, 2H), 1.58 (d, *J* = 12 Hz, 1H).

MS m/z (ESI): 468.1 [M+H]⁺, 470.1 [M+2+H]⁺.

### Example 97

### Preparation of

### (S)-1'-(5-((3-chloro-2-cyclopropylpyridin-4-yl)thio)pyrazin-2-yl)-1,3-dihydrospiro[inde ne-2,4'-piperidin]-1-amine

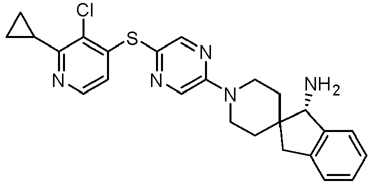

### Step 1: Preparation of 2-chloro-5-((3-chloro-2-cyclopropylpyridin-4-yl)thio)pyrazine

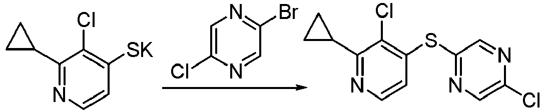

In a microwave reaction tube, potassium 3-chloro-2-cyclopropylpyridine-4-thiolate (120 mg, 0.54 mmol) and 2-bromo-5-chloropyrazine (104 mg, 0.54 mmol) were dissolved in 5 mL of 1,4-dioxane, followed by the addition of tris(dibenzylideneacetone)dipalladium (25 mg, 0.027 mmol), Xantphos (31 mg, 0.054 mmol) and DIPEA (209 mg, 1.62 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (10∼20% ethyl acetate/petroleum ether) to obtain a light yellow oil (120 mg, yield: 74%).

MS m/z (ESI): 298.0 [M+H]⁺.

### Step 2: Preparation of (R)-N-((S)-1'-(5-((3-chloro-2-cyclopropylpyridin-4-yl)thio)pyrazin-2-yl)-1,3-dihydrospi ro[indene-2,4'-piperidin]-1-yl)-2-methylpropane-2-sulfinamide

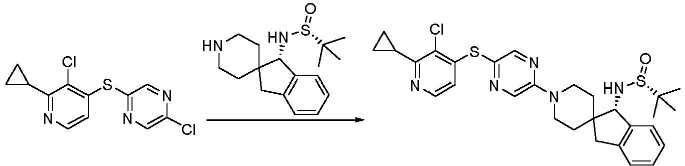

Tert-butyl (S)-1-(((R)-*tert*-butylsulfinyl)amino)-1,3-dihydrospiro[indene-2,4'-piperidine]-1'-carbox ylate (81 mg, 0.20 mmol) was dissolved in 3 mL of dichloromethane. 1 mL of trifluoroacetic acid was added, and the reaction solution was reacted at room temperature for 1 hour. The reaction solution was concentrated to dryness, and dissolved in 5 mL of DMF. Potassium carbonate (373 mg, 2.70 mmol) and 2-chloro-5-((3-chloro-2-cyclopropylpyridin-4-yl)thio)pyrazine (55 mg, 0.18 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 mL×3), dried over anhydrous sodium sulfate, and purified by column chromatography (50% ethyl acetate/petroleum ether) to obtain the product (48 mg, yield: 47%) as an oil.

MS m/z (ESI): 568.1 [M+H]⁺.

### Step 3: Preparation of (S)-1'-(5-((3-chloro-2-cyclopropylpyridin-4-yl)thio)pyrazin-2-yl)-1,3-dihydrospiro[inde ne-2,4'-piperidin]-1-amine

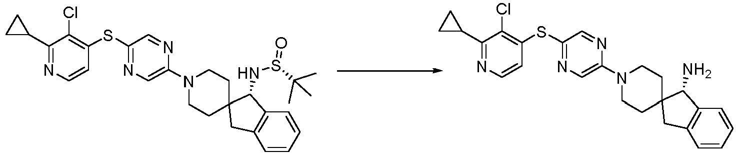

(R)-N-((S)-1'-(5-((3-Chloro-2-cyclopropylpyridin-4-yl)thio)pyrazin-2-yl)-1,3-dihy drospiro[indene-2,4'-piperidin]-1-yl)-2-methylpropane-2-sulfinamide (48 mg, 0.085 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M NH₃ in methanol. The reaction solution was concentrated to dryness by rotary evaporation, and the resulting residue was purified by column chromatography (0∼10% MeOH in DCM) to obtain the product (23 mg, yield: 58%) as a light yellow solid.

¹H NMR (400 MHz, Methanol-*d*₄) δ 8.36 (s, 1H), 8.30 (s, 1H), 7.98 (d, *J* = 5.3 Hz, 1H), 7.42 (d, *J* = 6.8 Hz, 1H), 7.32 - 7.21 (m, 3H), 6.43 (d, *J* = 5.3 Hz, 1H), 4.44 - 4.29 (m, 2H), 4.14 (s, 1H), 3.41 - 3.30 (m, 2H), 3.19 (d, *J* = 15.9 Hz, 1H), 2.96 (d, *J* = 15.9 Hz, 1H), 2.60 - 2.49 (m, 1H), 1.90 - 1.76 (m, 2H), 1.67 - 1.54 (m, 2H), 1.09 - 0.99 (m, 4H).

MS m/z (ESI): 464.1 [M+H]⁺.

### Example 98

### Preparation of

### (S)-1'-(6-amino-5-((3-chloro-2-cyclopropylpyridin-4-yl)thio)pyrazin-2-yl)-4,6-dihydros piro[cyclopenta[d]thiazole-5,4'-piperidin]-6-amine

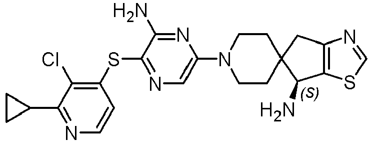

### Step 1: Preparation of tert-butyl (5-bromo-1,3,4-thiadiazol-2-yl)carbamate

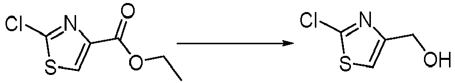

Ethyl 2-chlorothiazole-4-carboxylate (9.0 g, 46.97 mmol) was dissolved in 150 mL of methanol and stirred at room temperature. NaBH₄ (7.1 g, added in three batches) was added, and the reaction solution was stirred until the starting materials disappeared. The reaction solution was slowly added to brine under stirring to quench the reaction. The reaction solution was concentrated to remove the solvent, and extracted with ethyl acetate (200 mL) three times. The organic layers were combined, dried over anhydrous sodium sulfate, and concentrated to obtain a crude product, which was purified by column chromatography (ethyl acetate/petroleum ether = 3: 1) to obtain the target product (2-chlorothiazol-4-yl)methanol (4.2 g, yield: 60%) as a colorless oily liquid.

¹H NMR (400 MHz, CDCl₃) δ 7.11 (s, 1H), 4.71 (s, 2H), 2.22 (s, 1H).

MS m/z (ESI): 150.0 [M+H]⁺, 152.0 [M+2+H]⁺.

### Step 2: Preparation of (2-chlorothiazol-4-yl)methyl methanesulfonate

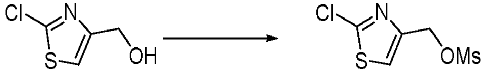

(2-Chlorothiazol-4-yl)methanol (2.6 g, 17.4 mmol) was dissolved in 40 mL of dichloromethane. Methanesulfonyl chloride (1.6 mL, 20.88 mmol, d=1.48 g/mL) was added dropwise under a nitrogen atmosphere in an ice water bath, and the reaction solution was stirred in the ice water bath for 30 minutes. After completion of the reaction, the reaction solution was added dropwise to saturated sodium chloride solution (100 mL), and extracted with dichloromethane (100 mL) three times. The organic layers were combined, dried over anhydrous sodium sulfate, and concentrated to obtain the target product (2-chlorothiazol-4-yl)methyl methanesulfonate (3.9 g, yield: 97%) as a white solid.

¹H NMR (400 MHz, CDCl₃) δ 7.35 (s, 1H), 5.25 (s, 2H), 3.07 (s, 3H). Step 3: Preparation of 1-(*tert*-butyl) 4-ethyl 4-((2-chlorothiazol-4-yl)methyl)piperidine-1,4-dicarboxylate 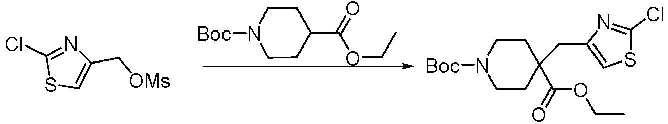 1-(*Tert*-butyl) 4-ethyl piperidine-1,4-dicarboxylate (4.8 g, 18.59 mmol) was dissolved in THF (40 mL). The solution was cooled to -60°C under a nitrogen atmosphere, and then lithium diisopropylamide (12.7 mL, 25.35 mmol) was added dropwise. After completion of the addition, the reaction solution was stirred at -60°C to -50°C for 30 minutes. A solution of (2-chlorothiazol-4-yl)methyl methanesulfonate in THF (15 mL) was added dropwise. After completion of the addition, the reaction solution was stirred at -60°C for 30 minutes, then slowly warmed up to room temperature and stirred for 2 hours. After completion of the reaction, the reaction solution was added dropwise to saturated brine (150 mL). The solution was extracted with ethyl acetate (100 mL) three times. The organic layers were combined, dried over anhydrous sodium sulfate, and concentrated to obtain a crude product, which was purified by column chromatography (ethyl acetate/petroleum ether = 3: 1) to obtain the target product 1-(*tert*-butyl) 4-ethyl 4-((2-chlorothiazol-4-yl)methyl)piperidine-1,4-dicarboxylate (3.7 g, yield: 56%) as a yellow liquid.

¹H NMR (400 MHz, CDCl₃) δ 6.79 (s, 1H), 4.14 (dd, *J* = 7.1, 3.1 Hz, 2H), 3.91 - 3.85 (m, 2H), 2.92 (s, 2H), 2.86 (dd, *J* = 16.3, 13.6 Hz, 2H), 2.10 (d, *J* = 13.5 Hz, 2H), 1.53 (s, 2H), 1.44 (s, 9H), 1.25 - 1.22 (m, 3H).

MS m/z (ESI): 289 [M-100+H]⁺.

### Step 4: Preparation of tert-butyl 2-chloro-6-oxo-4,6-dihydrospiro[cyclopenta[d]thiazole-5,4'-piperidine]-1'-carboxylate

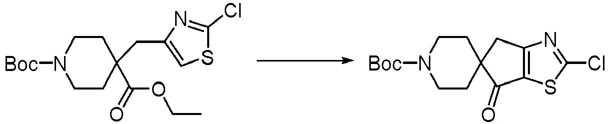

1-(*Tert*-butyl) 4-ethyl 4-((2-chlorothiazol-4-yl)methyl)piperidine-1,4-dicarboxylate (4.1 g, 10.6 mmol) was dissolved in THF (40 mL). The solution was cooled to -70°C under a nitrogen atmosphere, and then lithium diisopropylamide (13.4 mL, 26.5 mmol) was added dropwise. After completion of the addition, the reaction solution was stirred at -70°C for 30 minutes. After completion of the reaction, the reaction solution was added dropwise to saturated brine (150 mL), and extracted with ethyl acetate (100 mL) three times. The organic layers were combined, dried over anhydrous sodium sulfate, and concentrated to obtain a crude product, which was purified by column chromatography (ethyl acetate/petroleum ether = 3: 1) to obtain the target product *tert*-butyl 2-chloro-6-oxo-4,6-dihydrospiro[cyclopenta[d]thiazole-5,4'-piperidine]-1'-carboxylate (1.2 g, yield: 33%) as a yellow solid.

¹H NMR (400 MHz, CDCl₃) δ 4.15 (s, 4H), 3.05 (s, 2H), 2.06 - 1.93 (m, 2H), 1.60 (d, *J* = 12.7 Hz, 2H), 1.48 (s, 9H).

MS m/z (ESI): 243 [M-100+H]⁺.

### Step 5: Preparation of tert-butyl (R,Z)-6-((tert-butylsulfinyl<sulfenyl>)imino)-2-chloro-4,6-dihydrospiro[cyclopenta[d]t hiazole-5,4'-piperidine]-1'-carboxylate

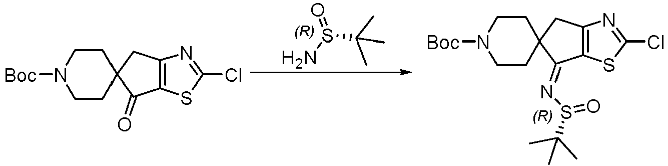

*Tert-butyl* 2-chloro-6-oxo-4,6-dihydrospiro[cyclopenta[d]thiazole-5,4'-piperidine]-1'-carboxylate (1.2 g, 3.5 mmol) was dissolved in THF (10 mL). Ti(OEt)₄ (10 mL) was added, and the reaction solution was heated to 95°C under a nitrogen atmosphere and stirred for 10 hours. The reaction solution was cooled to room temperature, and diluted with ethyl acetate (200 mL). Water (20 mL) was added under stirring, and the solution was stirred until cloudy. Anhydrous sodium sulfate was added until the solid in the mixture was sandy. The mixture was filtered, and the filtrate was concentrated to obtain a crude product, which was purified by column chromatography (PE/EA=5:1) to obtain the target product *tert*-butyl (R,Z)-6-((*tert*-butylsulfinyl<sulfenyl>)imino)-2-chloro-4,6-dihydrospiro[cyclopenta[d]t hiazole-5,4'-piperidine]-1'-carboxylate (1.1 g, yield: 75%) as a yellow solid.

¹H NMR (400 MHz, CDCl₃) δ 4.13 (dd, *J* = 22.7, 15.6 Hz, 4H), 2.92 (d, *J* = 2.9 Hz, 2H), 2.05 (s, 2H), 1.57 (s, 2H), 1.48 (s, 9H), 1.29 - 1.22 (m, 9H).

MS m/z (ESI): 346 [M-100+H]⁺.

### Step 6: Preparation of tert-butyl (S)-6-(((R)-tert-butylsulfinyl<sulfenyl>)amino)-4,6-dihydrospiro[cyclopenta[d]thiazole-5,4'-piperidine]-1'-carboxylate and tert-butyl (S)-6-(((R)-tert-butylsulfinyl<sulfenyl>)amino)-2-chloro-4,6-dihydrospiro[cyclopenta[d ]thiazole-5,4'-piperidine]-1'-carboxylate

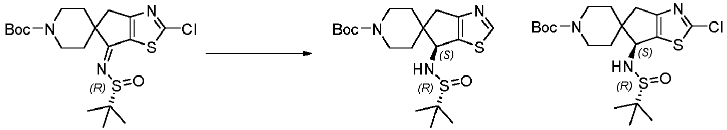

Tert-butyl (R,Z)-6-((*tert*-butylsulfinyl<sulfenyl>)imino)-2-chloro-4,6-dihydrospiro[cyclopenta[d]t hiazole-5,4'-piperidine]-1'-carboxylate (600 mg, 1.35 mmol) was dissolved in anhydrous THF (10 mL) in a 50 mL three-neck flask. The solution was cooled to -70°C under a nitrogen atmosphere, and then BH₃/THF (4.0 mL, D=1 M) was added dropwise. After completion of the addition, the reaction solution was slowly warmed to room temperature and stirred for 10 hours. The reaction solution was added dropwise to water, and saturated sodium bicarbonate solution was added to adjust the pH to 9. The reaction solution was extracted with ethyl acetate (100 mL) three times. The organic layers were combined, dried over anhydrous sodium sulfate, and concentrated to obtain a crude product, which was purified by column chromatography (PE/EA=1:1) to obtain the target product *tert*-butyl (S)-6-(((R)-*tert*-butylsulfinyl<sulfenyl>)amino)-4,6-dihydrospiro[cyclopenta[d]thiazole-5,4'-piperidine]-1'-carboxylate (112 mg, yield: 20%) as a yellow liquid and the product *tert*-butyl

(S)-6-(((R)-*tert*-butylsulfinyl<sulfenyl>)amino)-2-chloro-4,6-dihydrospiro[cyclopenta[d ]thiazole-5,4'-piperidine]-1'-carboxylate (56 mg, yield: 9 %) as a yellow liquid.

MS m/z (ESI): 414.0 [M+H]⁺;

MS m/z (ESI): 448.1 [M+H]⁺, 450.1 [M+H+2]⁺.

### Step 7: Preparation of (R)-N-((S)-4,6-dihydrospiro[cyclopenta[d]thiazole-5,4'-piperidin]-6-yl)-2-methylpropan e-2-sulfinamide

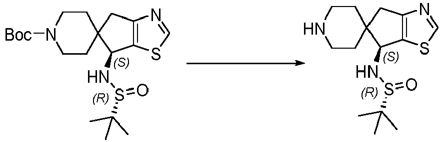

*Tert-*butyl (S)-6-(((R)-*tert*-butylsulfinyl<sulfenyl>)amino)-4,6-dihydrospiro[cyclopenta[d]thiazole-5,4'-piperidine]-1'-carboxylate (112 mg, 0.27 mmol) was dissolved in anhydrous CH₂Cl₂ (5 mL). Trifluoroacetic acid (1.0 mL) was added, and the reaction solution was stirred at room temperature for 2 hours. The reaction solution was concentrated to obtain the crude product (R)-N-((S)-4,6-dihydrospiro[cyclopenta[d]thiazole-5,4'-piperidin]-6-yl)-2-methylpropan e-2-sulfinamide (67 mg, yield: 76 %) as a yellow liquid.

MS m/z (ESI): 314.0 [M+H]⁺.

### Step 8: Preparation of (R)-N-((S)-1-(6-amino-5-((3-chloro-2-cyclopropylpyridin-4-yl)thio)pyrazin-2-yl)-4,6-d ihydrospiro[cyclopenta[d]thiazole-5,4'-piperidin]-6-yl)-2-methylpropane-2-sulfinamide

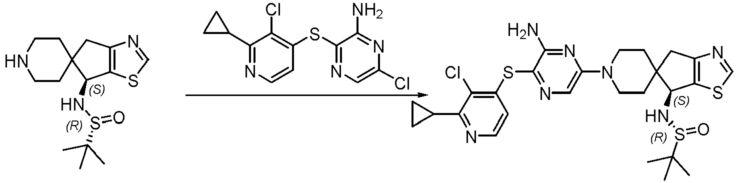

(R)-N-((S)-4,6-Dihydrospiro[cyclopenta[d]thiazole-5,4'-piperidin]-6-yl)-2-methylp ropane-2-sulfinamide (67 mg, 0.21 mmol), 6-chloro-3-((3-chloro-2-cyclopropylpyridin-4-yl)thio)pyrazin-2-amine (78 mg, 0.25 mmol) and DIPEA (0.4 mL) were dissolved in DMF (2.0 mL). The reaction solution was heated to 110°C and stirred for 10 hours. The reaction solution was dissolved in ethyl acetate (100 mL), washed with water (50 mL) twice, and washed with saturated brine (50 mL) three times. The organic layers were combined, dried over anhydrous sodium sulfate, and concentrated to obtain a crude product, which was purified by column chromatography (EtOAc) to obtain the target product (R)-N-((S)-1'-(6-amino-5-((3-chloro-2-cyclopropylpyridin-4-yl)thio)pyrazin-2-yl)-4,6-d ihydrospiro[cyclopenta[d]thiazole-5,4'-piperidin]-6-yl)-2-methylpropane-2-sulfinamide (30 mg, yield: 19 %) as a yellow liquid.

MS m/z (ESI): 590.0 [M+H]⁺.

### Step 9: Preparation of (S)-1'-(6-amino-5-((3-chloro-2-cyclopropylpyridin-4-yl)thio)pyrazin-2-yl)-4,6-dihydros piro[cyclopenta[d]thiazole-5,4'-piperidin]-6-amine

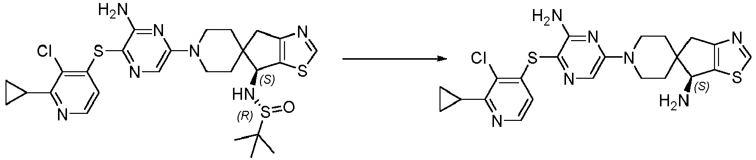

(R)-N-((S)-1'-(6-Amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfina mide (30 mg, 0.05 mmol) was dissolved in anhydrous methanol (3.0 mL). Hydrochloric acid/1,4-dioxane (1.0 mL) was added, and the reaction solution was stirred at room temperature for 0.5 hour. The reaction solution was concentrated to obtain a crude product, which was dissolved in water. Saturated sodium bicarbonate solution was added to adjust the pH to 10, and the reaction solution was extracted with dichloromethane (50 mL) twice. The organic layers were combined, dried over anhydrous sodium sulfate, and concentrated to obtain a crude product, which was purified by preparative HPLC to obtain the target product (S)-1'-(6-amino-5-((3-chloro-2-cyclopropylpyridin-4-yl)thio)pyrazin-2-yl)-4,6-dihydros piro[cyclopenta[d]thiazole-5,4'-piperidin]-6-amine (3.4 mg, yield: 49 %) as a yellow solid.

¹H NMR (500 MHz, CDCl₃) δ 8.77 (s, 1H), 8.48 (s, 1H), 7.45 (d, *J* = 9.7 Hz, 2H), 4.45 (s, 1H), 3.38 (d, *J* = 61.4 Hz, 4H), 2.91 (s, 2H), 2.22 (s, 1H), 1.92 (s, 1H), 1.71 (s, 1H), 1.52 (s, 2H), 1.33-0.92 (m, 8H).

MS m/z (ESI): 486 [M+H]⁺.

### Example 99

### Preparation of

### (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine

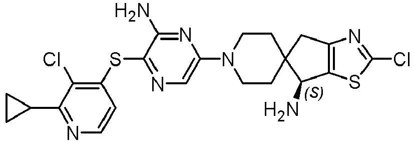

### Step 1: Preparation of (R)-N-((S)-2-chloro-4,6-dihydrospiro[cyclopenta[d]thiazole-5,4'-piperidin]-6-yl)-2-met hylpropane-2-sulfinamide

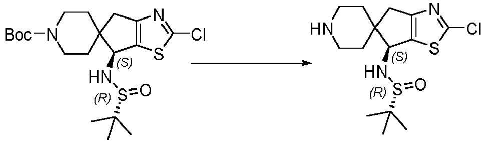

*Tert-butyl* (S)-6-(((R)-*tert*-butylsulfinyl<sulfenyl>)amino)-2-chloro-4,6-dihydrospiro[cyclopenta[d ]thiazole-5,4'-piperidine]-1'-carboxylate (56 mg, 0.13 mmol) was dissolved in anhydrous CH₂Cl₂ (5 mL). Trifluoroacetic acid (1.0 mL) was added, and the reaction solution was stirred at room temperature for 2 hours. The reaction solution was concentrated to obtain the crude product (R)-N-((S)-2-chloro-4,6-dihydrospiro[cyclopenta[d]thiazole-5,4'-piperidin]-6-yl)-2-met hylpropane-2-sulfinamide (40 mg, yield: 100%) as a yellow liquid.

MS m/z (ESI): 348.0 [M+H]⁺.

### Step 2: Preparation of (R)-N-((S)-1'-(6-amino-5-((3-chloro-2-cyclopropylpyridin-4-yl)thio)pyrazin-2-yl)-2-chl oro-4,6-dihydrospiro[cyclopenta[d]thiazole-5,4'-piperidin]-6-yl)-2-methylpropane-2-sul finamide

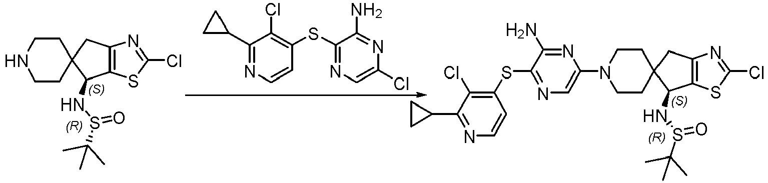

(R)-N-((S)-2-Chloro-4,6-dihydrospiro[cyclopenta[d]thiazole-5,4'-piperidin]-6-yl)-2 -methylpropane-2-sulfinamide (40 mg, 0.1 mmol), 6-chloro-3-((3-chloro-2-cyclopropylpyridin-4-yl)thio)pyrazin-2-amine (15 mg, 0.05 mmol) and DIPEA (0.1 mL) were dissolved in DMF (0.8 mL). The reaction solution was heated to 110°C and stirred for 10 hours. The reaction solution was dissolved in ethyl acetate (100 mL), washed with water (50 mL) twice, and washed with saturated brine (50 mL) three times. The organic layers were combined, dried over anhydrous sodium sulfate, and concentrated to obtain a crude product, which was purified by column chromatography (EtOAc) to obtain the target product (R)-N-((S)-1'-(6-amino-5-((3-chloro-2-cyclopropylpyridin-4-yl)thio)pyrazin-2-yl)-4,6-d ihydrospiro[cyclopenta[d]thiazole-5,4'-piperidin]-6-yl)-2-methylpropane-2-sulfinamide (8.2 mg, yield: 26 %) as a yellow liquid.

MS m/z (ESI): 624.1 [M+H]⁺.

### Step 3: Preparation of (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine

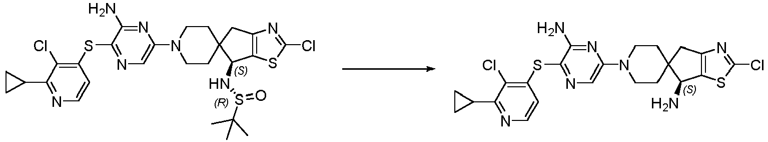

(R)-N-((S)-1'-(6-Amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfina mide (8.2 mg, 0.01 mmol) was dissolved in anhydrous methanol (2.0 mL). Hydrochloric acid/1,4-dioxane (0.5 mL) was added, and the reaction solution was stirred at room temperature for 0.5 hour. The reaction solution was concentrated to obtain a crude product, which was dissolved in water. Saturated sodium bicarbonate solution was added to adjust the pH to 10, and the reaction solution was extracted with dichloromethane (50 mL) twice. The organic layers were combined, dried over anhydrous sodium sulfate, and concentrated to obtain a crude product, which was purified by preparative HPLC to obtain the target product (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine (1.5 mg, yield: 22 %) as a yellow solid.

¹H NMR (500 MHz, CDCl₃) δ 8.48 (s, 1H), 7.45 (d, *J* = 10.3 Hz, 2H), 4.71 (s, 1H), 3.41 (d, *J* = 74.5 Hz, 4H), 2.92 (s, 2H), 2.16 (d, *J* = 56.0 Hz, 2H), 1.74 (s, 1H),

1.52 (s, 2H), 1.26 (d, *J* = 15.5 Hz, 4H), 1.03 (d, *J* = 39.0 Hz, 4H).

MS m/z (ESI): 520.1 [M+H]⁺.

### Example 100

### Preparation of

### (S)-1'-(6-amino-5-((2-(azetidin-1-yl)-3-chloropyridin-4-yl)thio)pyrazin-2-yl)-1,3-dihydr ospiro[indene-2,4'-piperidin]-1-amine

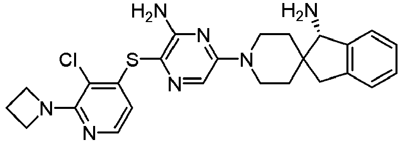

### Step 1: Preparation of 2-ethylhexyl 3-((2,3-dichloropyridin-4-yl)thio)propanoate

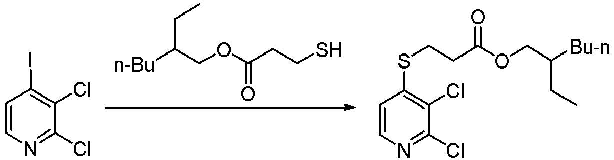

2,3-Dichloro-4-iodopyridine (2.0 g, 7.31 mmol), 2-ethylhexyl 3-mercaptopropanoate (1.8 g, 8.24 mmol), palladium acetate (82 mg, 0.37 mmol), Xantphos (254 mg, 0.44 mmol) and diisopropylethylamine (1.9 g, 14.70 mmol) were stirred in 15 mL of dioxane under a nitrogen atmosphere at 100°C for 5 hours. The reaction solution was cooled to room temperature, and then ethyl acetate was added. The reaction solution was filtered, and the filtrate was concentrated to dryness. The resulting residue was purified by column chromatography (petroleum ether/ethyl acetate=3:1) to obtain the target product 2-ethylhexyl 3-((2,3-dichloropyridin-4-yl)thio)propanoate (910 mg, yield: 34%).

MS m/z (ESI): 364.1 [M+H]⁺.

### Step 2: Preparation of 2-ethylhexyl 3-((2-(azetidin-1-yl)-3-chloropyridin-4-yl)thio)propanoate

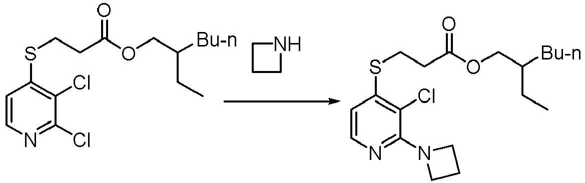

2-Ethylhexyl 3-((2,3-dichloropyridin-4-yl)thio)propanoate (200 mg, 0.55 mmol), azetidine (63 mg, 1.10 mmol) and 1,4-dioxane (5 mL) were stirred at 120°C under microwave for 3 hours. The reaction solution was concentrated to dryness, and then water was added. The solution was extracted with dichloromethane, and the organic phase was concentrated to dryness. The resulting residue was purified by column chromatography (petroleum ether/ethyl acetate=3:1) to obtain the product (130 mg, yield: 62%).

MS m/z (ESI): 385.1 [M+H]⁺.

### Step 3: Preparation of 2-(azetidin-1-yl)-3-chloropyridine-4-thiol

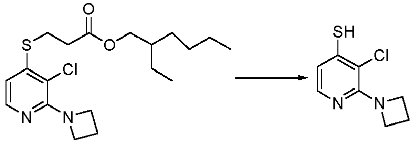

2-Ethylhexyl 3-((2-(azetidin-1-yl)-3-chloropyridin-4-yl)thio)propanoate (130 mg, 0.34 mmol) and potassium *tert*-butoxide (38 mg, 0.34 mmol) were stirred in anhydrous ethanol (5 mL) at room temperature for 1 hour. The reaction solution was concentrated to dryness, and the resulting residue was used directly in the next step.

MS m/z (ESI): 201.0 [M+H]⁺.

### Step 4: Preparation of 3-((2-(azetidin-1-yl)-3-chloropyridin-4-yl)thio)-6-chloropyrazin-2-amine

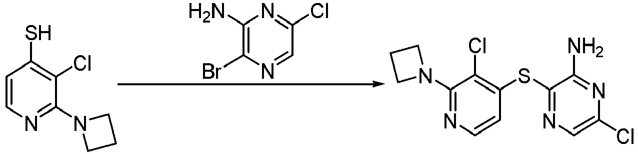

2-(Azetidin-1-yl)-3-chloropyridine-4-thiol (obtained in the previous step), 3-bromo-6-chloropyrazin-2-amine (84 mg, 0.40 mmol), Pd₂(dba)₃ (15 mg, 0.016 mmol), Xantphos (20 mg, 0.035 mmol) and diisopropylethylamine (87 mg, 0.67 mmol) were added to 5 mL of 1,4-dioxane, and the reaction solution was stirred under a nitrogen atmosphere at 100°C overnight. The reaction solution was cooled to room temperature and concentrated to dryness, and the resulting residue was purified by column chromatography (petroleum ether/ethyl acetate= 1:1) to obtain a light yellow solid (71 mg, yield: 64%).

MS m/z (ESI): 328.0 [M+H]⁺.

### Step 5: Preparation of (R)-N-((S)-1,3-dihydrospiro[indene-2,4'-piperidin]-1-yl)-2-methylpropane-2-sulfinamid e

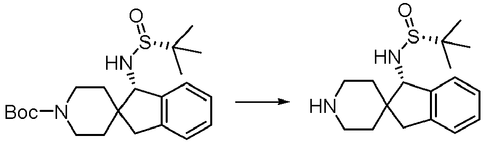

*Tert*-butyl (S)-1-(((R)-*tert*-butylsulfinyl<sulfenyl>)amino)-1,3-dihydrospiro[indene-2,4'-piperidine ]-1'-carboxylate (100 mg, 0.25 mmol) was dissolved in dichloromethane (1 mL). 1 mL of trifluoroacetic acid was added, and the reaction solution was stirred at room temperature for 1 hour. The reaction solution was concentrated to dryness to obtain an oil, which was used directly in the next step.

MS m/z (ESI): 307.1 [M+H]⁺.
[α]²⁰_{D}= 1.773.

### Step 6: Preparation of (R)-N-((S)-1'-(6-amino-5-((2-(azetidin-1-yl)-3-chloropyridin-4-yl)thio)pyrazin-2-yl)-1,3 -dihydrospiro[indene-2,4'-piperidin]-1-yl)-2-methylpropane-2-sulfinamide

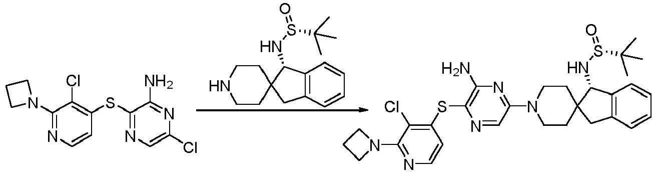

3-((2-(Azetidin-1-yl)-3-chloropyridin-4-yl)thio)-6-chloropyrazin-2-amine (71 mg, 0.22 mmol), (R)-N-((S)-1,3-dihydrospiro[indene-2,4'-piperidin]-1-yl)-2-methylpropane-2-sulfinamid e (obtained in the previous step) and potassium carbonate (299 mg, 2.16 mmol) were stirred in DMF (5 mL) under a nitrogen atmosphere at 100°C under microwave for 2 hours. Water was added, and the solution was extract with ethyl acetate. The organic phase was dried over anhydrous sodium sulfate, and concentrated to dryness to obtain an oil, which was used directly in the next step.

MS m/z (ESI): 598.2[M+H]⁺.

### Step 7: Preparation of (S)-1'-(6-amino-5-((2-(azetidin-1-yl)-3-chloropyridin-4-yl)thio)pyrazin-2-yl)-1,3-dihydr ospiro[indene-2,4'-piperidin]-1-amine

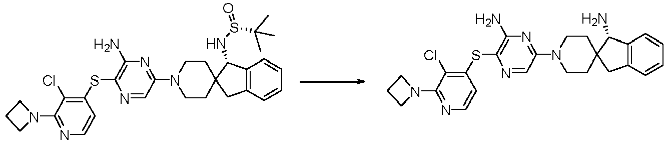

(R)-N-((S)-1'-(6-Amino-5-((2-(azetidin-1-yl)-3-chloropyridin-4-yl)thio)pyrazin-2-yl)-1,3-dihydrospiro[indene-2,4'-piperidin]-1-yl)-2-methylpropane-2-sulfinamide (obtained in the previous step) was dissolved in methanol (5 mL). 4M HCl/1,4-dioxane (5 mL) was added, and the reaction solution was stirred at room temperature for 1 hour. The reaction solution was concentrated to dryness, and the resulting residue was purified by column chromatography to obtain the target product (S)-1'-(6-amino-5-((2-(azetidin-1-yl)-3-chloropyridin-4-yl)thio)pyrazin-2-yl)-1,3-dihydr ospiro[indene-2,4'-piperidin]-1-amine (8.7 mg, yield: 8%).

¹H NMR (400 MHz, Methanol-d4) δ 7.69 (d, *J* = 5.4 Hz, 1H), 7.58 (s, 1H), 7.42 - 7.32 (m, 1H), 7.28 - 7.14 (m, 3H), 5.97 (d, *J* = 5.5 Hz, 1H), 4.35 - 4.16 (m, 6H), 3.96 (s, 1H), 3.28 - 3.19 (m, 2H), 3.15 (d, *J* = 15.7 Hz, 1H), 2.81 (d, *J* = 15.7 Hz, 1H), 2.32 (q, *J* = 7.5 Hz, 2H), 1.80 (dtd, *J* = 32.3, 12.6, 4.3 Hz, 2H), 1.58 (d, *J* = 13.4 Hz, 1H), 1.42 (d, *J* = 13.4 Hz, 1H), 1.31 (d, *J* = 16.2 Hz, 1H).

MS m/z (ESI): 494.1 [M+H]⁺.

### Example 101

### Preparation of

### (S)-1'-(6-amino-5-((3-chloro-2-morpholinopyridin-4-yl)thio)pyrazin-2-yl)-1,3-dihydros piro[indene-2,4'-piperidin]-1-amine

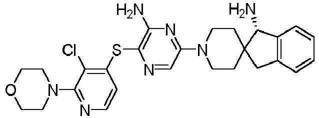

### Step 1: Preparation of 2-ethylhexyl 3-((3-chloro-2-morpholinopyridin-4-yl)thio)propanoate

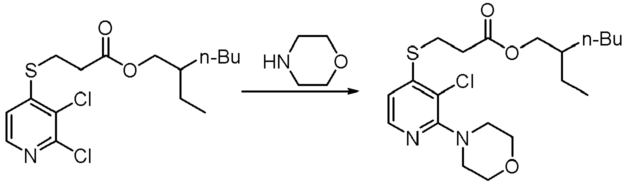

2-Ethylhexyl 3-((2,3-dichloropyridin-4-yl)thio)propanoate (200 mg, 0.55 mmol), morpholine (96 mg, 1.10 mmol) and 1,4-dioxane (5 mL) were stirred at 120°C under microwave for 8 hours. The reaction solution was concentrated to dryness, and then water was added. The solution was extracted with dichloromethane, and the organic phase was concentrated to dryness. The resulting residue was purified by column chromatography (petroleum ether/ethyl acetate=3:1) to obtain the product (152 mg, yield: 67%).

MS m/z (ESI): 415.2 [M+H]⁺.

### Step 2: Preparation of potassium 3-chloro-2-morpholinopyridine-4-thiolate

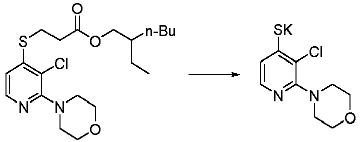

2-Ethylhexyl 3-((3-chloro-2-morpholinopyridin-4-yl)thio)propanoate (100 mg, 0.24 mmol) and potassium *tert*-butoxide (27 mg, 0.24 mmol) were stirred in anhydrous ethanol (5 mL) at room temperature for 1 hour. The reaction solution was concentrated to dryness to obtain the product, which was used directly in the next step.

MS m/z (ESI): 231.0 [M+H]⁺.

### Step 3: Preparation of 6-chloro-3-((3-chloro-2-morpholinopyridin-4-yl)thio)pyrazin-2-amine

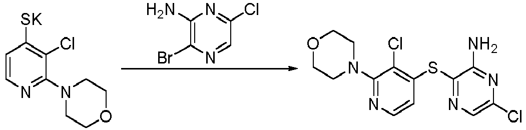

Potassium 3-chloro-2-morpholinopyridine-4-thiolate (obtained in the previous step), 3-bromo-6-chloropyrazin-2-amine (60 mg, 0.29 mmol), Pd₂(dba)₃ (11 mg, 0.012 mmol), Xantphos (14 mg, 0.024 mmol) and diisopropylethylamine (62 mg, 0.48 mmol) were stirred in 5 mL of dioxane under a nitrogen atmosphere at 100°C overnight. The reaction solution was cooled to room temperature and concentrated to dryness, and the resulting residue was purified by column chromatography (petroleum ether/ethyl acetate= 1:1) to obtain a light yellow solid (60 mg, yield: 70%).

MS m/z (ESI): 358.0 [M+H]⁺.

### Step 4: Preparation of (R)-N-((S)-1'-(6-amino-5-((3-chloro-2-morpholinopyridin-4-yl)thio)pyrazin-2-yl)-1,3-di hydrospiro[indene-2,4'-piperidin]-1-yl)-2-methylpropane-2-sulfinamide

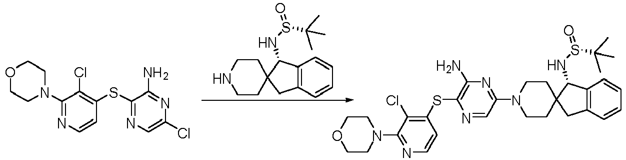

6-Chloro-3-((3-chloro-2-morpholinopyridin-4-yl)thio)pyrazin-2-amine (60 mg, 0.22 mmol), (R)-N-((S)-1,3-dihydrospiro[indene-2,4'-piperidin]-1-yl)-2-methylpropane-2-sulfinamid e (76 mg, 0.25 mmol) and potassium carbonate (231 mg, 1.67 mmol) were dissolved in DMF (5 mL), and the reaction solution was stirred under a nitrogen atmosphere at 100°C under microwave for 2 hours. Water was added, and the solution was extract with ethyl acetate. The organic phase was dried over anhydrous sodium sulfate, and concentrated to dryness to obtain an oil, which was used directly in the next step.

MS m/z (ESI): 628.2[M+H]⁺.

### Step 5: Preparation of (S)-1'-(6-amino-5-((3-chloro-2-morpholinopyridin-4-yl)thio)pyrazin-2-yl)-1,3-dihydros piro[indene-2,4'-piperidin]-1-amine

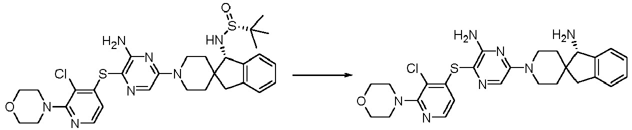

(R)-N-((S)-1'-(6-amino-5-((3-chloro-2-morpholinopyridin-4-yl)thio)pyrazin-2-yl)-1,3-dihydrospiro[indene-2,4'-piperidin]-1-yl)-2-methylpropane-2-sulfinamide (obtained in the previous step) was dissolved in methanol (5 mL). 4M HCl/1,4-dioxane (5 mL) was added, and the reaction solution was stirred at room temperature for 1 hour. The reaction solution was concentrated to dryness, and the resulting residue was purified by column chromatography to obtain the target product (S)-1'-(6-amino-5-((3-chloro-2-morpholinopyridin-4-yl)thio)pyrazin-2-yl)-1,3-dihydros piro[indene-2,4'-piperidin]-1-amine (6 mg, yield of two steps: 5%).

MS m/z (ESI): 524.1[M+H]⁺, 526.1[M+H]⁺.

The compounds of Examples 102 to 103, 108 to 110 and 120 to 130 were prepared by referring to the experimental scheme of Example 101.

### Example 102

### Preparation of

### (S)-1'-(6-amino-5-((3-chloro-2-(3,3-difluoroazetidin-1-yl)pyridin-4-yl)thio)pyrazin-2-yl )-1,3-dihydrospiro[indene-2,4'-piperidin]-1-amine

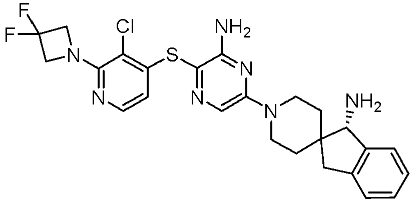

The compound of Example 102 was prepared by referring to the experimental scheme of Example 101.

MS m/z (ESI): 530.1 [M+H]⁺, 532.1 [M+2+H]⁺.

### Example 103

### Preparation of

### (S)-1'-(6-amino-5-((3-chloro-2-(pyrrolidin-1-yl)pyridin-4-yl)thio)pyrazin-2-yl)-1,3-dihy drospiro[indene-2,4'-piperidin]-1-amine

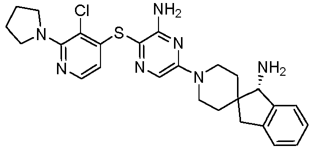

The compound of Example 103 was prepared by referring to the experimental scheme of Example 101.

MS m/z (ESI): 508.1 [M+H]⁺, 510.1[M+2+H]⁺.

### Example 104

### Preparation of

### 6-(4-amino-4-methylpiperidin-1-yl)-3-(2,3-dichlorophenyl)-5-(oxetan-3-yl)pyrazin-2-a mine

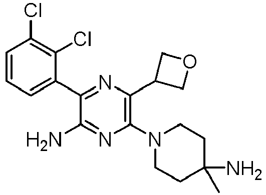

The compound of Example 104 was prepared by referring to the experimental scheme of Example 31.

MS m/z (ESI): 408.1 [M+H]⁺, 410.1 [M+2+H]⁺.

### Example 105

### Preparation of

### (S)-(3-(1-amino-1,3-dihydrospiro[indene-2,4'-piperidin]-1'-yl)-6-(2,3-dichlorophenyl)-5 -methylpyrazin-2-yl)methanol

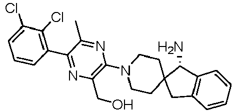

### Step 1: Preparation of (S)-1,3-dihydrospiro[indene-2,4'-piperidin]-1-amine

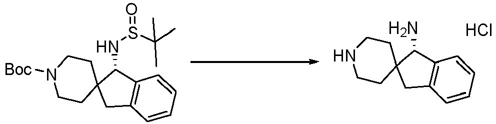

Hydrochloric acid in ethyl acetate (4.0 M, 3 mL) was added to a solution of *tert*-butyl (1S)-1-((*tert*-butylsulfinyl<sulfenyl>)amino)-1,3-dihydrospiro[indene-2,4'-piperidine]-1' -carboxylate (100 mg, 0.25 mmol) in methanol (3 mL), and the reaction solution was stirred at room temperature for 3 hours. The reaction solution was concentrated to obtain (S)-1,3-dihydrospiro[indene-2,4'-piperidin]-1-amine hydrochloride (100 mg, yield: 100%) as a white solid.

MS m/z (ESI): 203.1 [M+H]⁺,
[α]²⁰_{D}=1.773.

### Step 2: Preparation of ethyl (S)-3-(1-amino-1,3-dihydrospiro[indene-2,4'-piperidin]-1'-yl)-6-(2,3-dichlorophenyl)-5-methylpyrazine-2-carboxylate

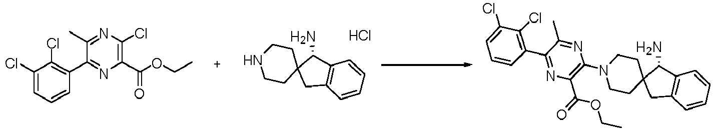

Ethyl 3-chloro-6-(2,3-dichlorophenyl)-5-methylpyrazine-2-carboxylate (50 mg, 0.15 mmol), (S)-1,3-dihydrospiro[indene-2,4'-piperidin]-1-amine hydrochloride (100 mg, 0.25 mmol) and potassium carbonate (20 mg, 0.73 mmol) were stirred in N,N-dimethylformamide (3 mL) at 100°C for 18 hours. The reaction solution was concentrated, and purified by column chromatography [eluent: dichloromethane ∼ dichloromethane/methanol (97:3)] to obtain ethyl (S)-3-(1-amino-1,3-dihydrospiro[indene-2,4'-piperidin]-1'-yl)-6-(2,3-dichlorophenyl)-5-methylpyrazine-2-carboxylate (60 mg, yield: 81%) as a yellow oil.

MS m/z (ESI): 511.1[M+H]⁺, 513.1 [M+2+H]⁺.

### Step 3: Preparation of (S)-(3-(1-amino-1,3-dihydrospiro[indene-2,4'-piperidin]-1'-yl)-6-(2,3-dichlorophenyl)-5 -methylpyrazin-2-yl)methanol

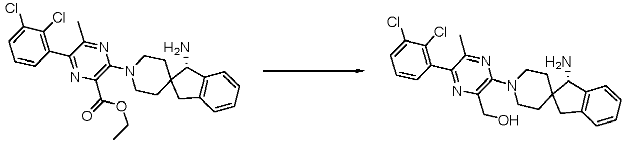

Diisobutyl aluminum hydride (0.47 mL, 1.0 M) was added dropwise to a solution of ethyl (S)-3-(1-amino-1,3-dihydrospiro[indene-2,4'-piperidin]-1'-yl)-6-(2,3-dichlorophenyl)-5-methylpyrazine-2-carboxylate (60 mg, 0.12 mmol) in dichloromethane (10 mL) at -78°C. The reaction solution was stirred at -78°C for 30 minutes, at 0°C for 30 minutes, and at room temperature for 2 hours. Sodium potassium tartrate was added to quench the reaction. The reaction solution was stirred overnight and extracted with dichloromethane (50 mL). The organic phase was concentrated, and purified by thin layer chromatography (dichloromethane/methanol= 10/1) to obtain (S)-(3-(1-amino-1,3-dihydrospiro[indene-2,4'-piperidin]-1'-yl)-6-(2,3-dichlorophenyl)-5 -methylpyrazin-2-yl)methanol (14 mg, 25%) as a white solid.

¹H NMR (400 MHz, DMSO) δ 7.77-7.69 (m, 1H), 7.53-7.39 (m, 2H), 7.33-7.32 (m, 1H), 7.23 - 7.11 (m, 3H), 5.27 (t, *J* = 8 Hz, 1H), 4.52 (d, *J* = 8 Hz, 2H), 3.88 (s, 1H), 3.76-3.69 (m, 2H), 3.22 - 2.94 (m, 4H), 2.70 - 2.55 (m, 2H), 2.19 (s, 3H), 1.97 - 1.78 (m, 2H), 1.56 (d, *J* = 12 Hz, 1H), 1.17 (d, *J* = 12 Hz, 1H).

MS m/z (ESI): 469.1 [M+H]⁺, 471.1 [M+2+H]⁺.

### Example 106

### Preparation of

### (3-((S)-1-amino-1,3-dihydrospiro[indene-2,4'-piperidin]-1'-yl)-6-(2,3-dichlorophenyl)-5 -methylpyrazin-2-yl)(cyclopropyl)methanol

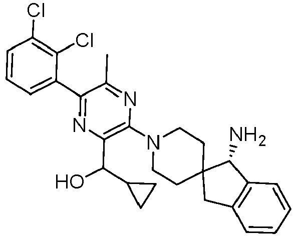

### Step 1: Preparation of (S)-3-(1-amino-1,3-dihydrospiro[indene-2,4'-piperidin]-1'-yl)-6-(2,3-dichlorophenyl)-5-methylpyrazine-2-carbaldehyde

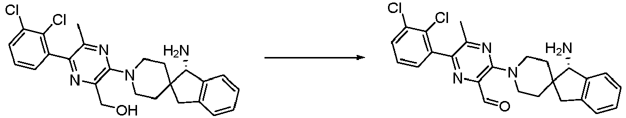

(S)-(3-(1-amino-1,3-dihydrospiro[indene-2,4'-piperidin]-1'-yl)-6-(2,3-dichlorophen yl)-5-methylpyrazin-2-yl)methanol (100 mg, 0.21 mmol) was dissolved in dichloromethane (20 mL). Dess-Martin reagent (130 mg, 0.31 mmol) was added, and the reaction solution was stirred at room temperature for 30 minutes. Water was added to quench the reaction, and the solution was extracted with dichloromethane (50 mL). The organic phase was washed with saturated NaHCO₃ aqueous solution, dried over anhydrous sodium sulfate and filtered. The filtrate was concentrated to obtain the crude product (S)-3-(1-amino-1,3-dihydrospiro[indene-2,4'-piperidin]-1'-yl)-6-(2,3-dichlorophenyl)-5-methylpyrazine-2-carbaldehyde (120 mg), which was used directly in the next step.

MS m/z (ESI): 447.1 [M+H]⁺, 449.1 [M+2+H]⁺.

### Step 2: Preparation of (3-((S)-1-amino-1,3-dihydrospiro[indene-2,4'-piperidin]-1'-yl)-6-(2,3-dichlorophenyl)-5 -methylpyrazin-2-yl)(cyclopropyl)methanol

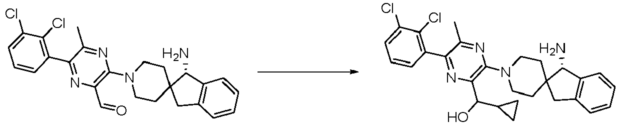

Isopropyl magnesium bromide (0.3 mL, 1.0 M) was added dropwise to a solution of (S)-3-(1-amino-1,3-dihydrospiro[indene-2,4'-piperidin]-1'-yl)-6-(2,3-dichlorophenyl)-5-methylpyrazine-2-carbaldehyde (crude, 120 mg, 0.21 mmol) in THF (20 mL) at -20°C. After completion of the addition, the reaction solution was stirred for 30 minutes, then warmed up to room temperature and stirred for 1 hour. Water was added to quench the reaction, and the solution was extracted with dichloromethane (3*20 mL). The organic phase was dried over anhydrous sodium sulfate and filtered. The filtrate was concentrated and purified by preparative HPLC to obtain (3-((S)-1-amino-1,3-dihydrospiro[indene-2,4'-piperidin]-l'-yl)-6-(2,3-dichlorophenyl)-5 -methylpyrazin-2-yl)(cyclopropyl)methanol (6 mg, yield of two steps: 5%) as a white solid.

¹H NMR (500 MHz, CDCl₃) δ 8.15 (s, 8H), 7.88 (s, 8H), 7.37 (s, 4H), 7.26 (s, 10H), 7.22 (s, 8H), 7.13 (s, 11H), 7.06 (s, 4H), 5.48 (s, 8H), 3.73 (s, 8H), 3.47 (s, 9H), 3.32 (s, 6H), 3.18 (s, 8H), 2.92 (s, 16H), 2.80 (s, 24H), 2.04 (s, 8H), 1.78 (d, *J* = 18.1 Hz, 18H), 1.51 (s, 5H), 0.88 (s, 3H), 0.49 (s, 8H), 0.25 (s, 8H).

MS m/z (ESI): 509.1 [M+H]⁺, 511.1 [M+2+H]⁺.

### Example 107

### Preparation of

### (S)-1-(3-(1-amino-1,3-dihydrospiro[indene-2,4'-piperidin]-1'-yl)-6-(2,3-dichlorophenyl) -5-methylpyrazin-2-yl)cyclopropan-1-ol

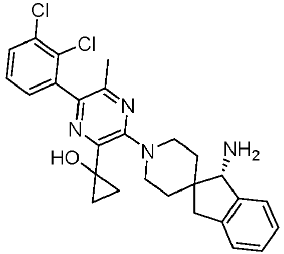

### Step 1: Preparation of (S)-1-(3-(1-amino-1,3-dihydrospiro[indene-2,4'-piperidin]-1'-yl)-6-(2,3-dichlorophenyl) -5-methylpyrazin-2-yl)cyclopropan-1-ol

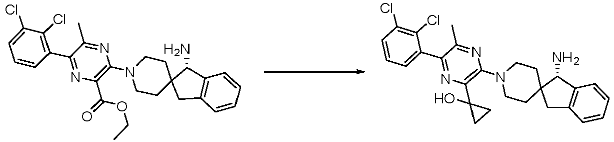

Ethylmagnesium bromide (0.1 mL, 0.30 mmol, 3M) and tetraisopropyl titanate (43 mg, 0.15 mmol) were added dropwise to a solution of ethyl (S)-3-(1-amino-1,3-dihydrospiro[indene-2,4'-piperidin]-1'-yl)-6-(2,3-dichlorophenyl)-5-methylpyrazine-2-carboxylate (50 mg, 0.10 mmol) in THF (10 mL) at 0°C. The reaction solution was warmed up to room temperature and stirred for 5 hours. Saturated NH₄Cl aqueous solution was added to quench the reaction, and the reaction solution was extracted with dichloromethane (3*20 mL). The organic phase was dried over anhydrous sodium sulfate, concentrated and purified by preparative HPLC to obtain (S)-1-(3-(1-amino-1,3-dihydrospiro[indene-2,4'-piperidin]-1'-yl)-6-(2,3-dichlorophenyl) -5-methylpyrazin-2-yl)cyclopropan-1-ol (5 mg, yield: 10%) as a white solid.

¹H NMR (500 MHz, CDCl₃) δ 8.15 (s, 22H), 7.88 (s, 22H), 7.40 (s, 6H), 7.40 (s, 5H), 7.40 - 7.20 (m, 59H), 7.13 (s, 27H), 7.06 (s, 11H), 3.72 (s, 21H), 3.54 (s, 32H), 3.31 (s, 29H), 3.07 (s, 42H), 2.80 (s, 63H), 2.21 (s, 21H), 2.05 (s, 21H), 1.95 (s, 21H), 1.76 (s, 31H), 1.51 (s, 31H), 0.96 (s, 37H), 0.72 (s, 28H).

MS m/z (ESI): 495.1 [M+H]⁺, 497.1 [M+2+H]⁺.

### Example 108

### Preparation of

### (S)-1'-(6-amino-5-((2-(azetidin-1-yl)-3-chloropyridin-4-yl)thio)pyrazin-2-yl)-4,6-dihydr ospiro[cyclopenta[d]oxazole-5,4'-piperidin]-6-amine

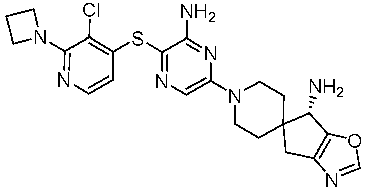

The compound of Example 108 was prepared by referring to the experimental scheme of Example 101.

MS m/z (ESI): 508.1 [M+H]⁺, 510.1 [M+2+H]⁺.

### Example 109

### Preparation of

### (S)-1'-(6-amino-5-((2-(azetidin-1-yl)-3-chloropyridin-4-yl)thio)pyrazin-2-yl)-1-methyl-4,6-dihydro-1H-spiro[cyclopenta[d]imidazole-5,4'-piperidin]-6-amine

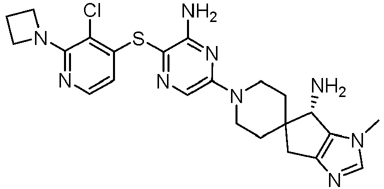

The compound of Example 109 was prepared by referring to the experimental scheme of Example 101.

MS m/z (ESI): 498.1 [M+H]⁺, 500.1 [M+2+H]⁺.

### Example 110

### Preparation of

### (S)-1'-(6-amino-5-((2-(azetidin-1-yl)-3-chloropyridin-4-yl)thio)pyrazin-2-yl)-2-methyl-2,6-dihydro-4H-spiro[cyclopenta[c]pyrazole-5,4'-piperidin]-4-amine

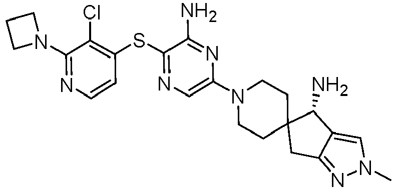

The compound of Example 110 was prepared by referring to the experimental scheme of Example 101.

MS m/z (ESI): 498.1 [M+H]⁺, 500.1 [M+2+H]⁺.

### Example 111

### Preparation of

### (S)-1'-(6-amino-5-((3-chloro-2-morpholinopyridin-4-yl)thio)-3-methylpyrazin-2-yl)-1,3 -dihydrospiro[indene-2,4'-piperidin]-1-amine

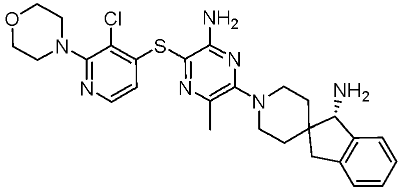

The compound of Example 111 was prepared by referring to the experimental scheme of Example 96.

MS m/z (ESI): 538.1 [M+H]⁺, 540.1 [M+2+H]⁺.

### Example 112

### Preparation of

### (S)-1'-(6-amino-5-((3-chloro-2-(pyrrolidin-1-yl)pyridin-4-yl)thio)-3-methylpyrazin-2-yl )-1,3-dihydrospiro[indene-2,4'-piperidin]-1-amine

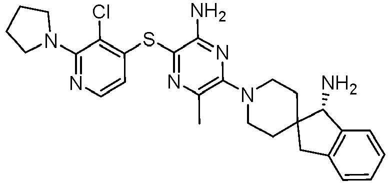

The compound of Example 112 was prepared by referring to the experimental scheme of Example 96.

MS m/z (ESI): 522.1 [M+H]⁺, 524.1 [M+2+H]⁺.

### Example 113

### Preparation of

### (S)-1'-(6-amino-5-((3-chloro-2-(pyrrolidin-1-yl)pyridin-4-yl)thio)-3-methylpyrazin-2-yl )-4,6-dihydrospiro[cyclopenta[d]thiazole-5,4'-piperidin]-6-amine

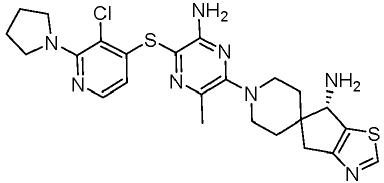

The compound of Example 113 was prepared by referring to the experimental scheme of Example 96.

MS m/z (ESI): 529.1 [M+H]⁺, 531.1 [M+2+H]⁺.

### Example 114

### Preparation of

### (S)-1'-(6-amino-5-((3-chloro-2-(pyrrolidin-1-yl)pyridin-4-yl)thio)-3-methylpyrazin-2-yl )-4,6-dihydrospiro[cyclopenta[d]oxazole-5,4'-piperidin]-6-amine

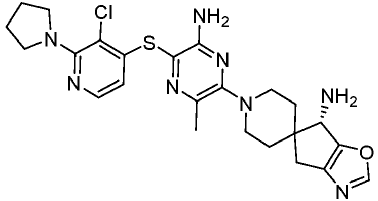

The compound of Example 114 was prepared by referring to the experimental scheme of Example 96.

MS m/z (ESI): 513.1 [M+H]⁺, 515.1[M+2+H]⁺.

### Example 115

### Preparation of

### (S)-1'-(6-amino-5-((3-chloro-2-(pyrrolidin-1-yl)pyridin-4-yl)thio)-3-methylpyrazin-2-yl )-1-methyl-4,6-dihydro-1H-spiro[cyclopenta[d]imidazole-5,4'-piperidin]-6-amine

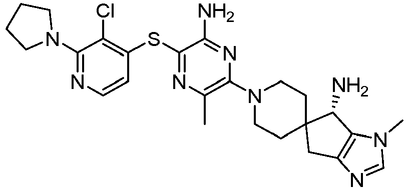

The compound of Example 115 was prepared by referring to the experimental scheme of Example 96.

MS m/z (ESI): 526.1 [M+H]⁺, 528.1 [M+2+H]⁺.

### Example 116

### Preparation of

### (S)-1'-(6-amino-5-((3-chloro-2-(pyrrolidin-1-yl)pyridin-4-yl)thio)-3-methylpyrazin-2-yl )-2-methyl-2,6-dihydro-4H-spiro[cyclopenta[c]pyrazole-5,4'-piperidin]-4-amine

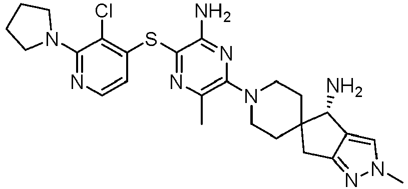

The compound of Example 116 was prepared by referring to the experimental scheme of Example 96.

MS m/z (ESI): 526.1 [M+H]⁺, 528.1 [M+2+H]⁺.

### Example 117

### Preparation of

### (S)-1'-(6-amino-5-((3-chloro-2-morpholinopyridin-4-yl)thio)-3-methylpyrazin-2-yl)-4,6 -dihydrospiro[cyclopenta[d]oxazole-5,4'-piperidin]-6-amine

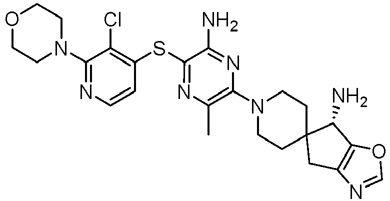

The compound of Example 117 was prepared by referring to the experimental scheme of Example 96.

MS m/z (ESI): 529.1 [M+H]⁺, 531.1 [M+2+H]⁺.

### Example 118

### Preparation of

### (S)-1'-(6-amino-5-((3-chloro-2-morpholinopyridin-4-yl)thio)-3-methylpyrazin-2-yl)-2-methyl-2,6-dihydro-4H-spiro[cyclopenta[c]pyrazole-5,4'-piperidin]-4-amine

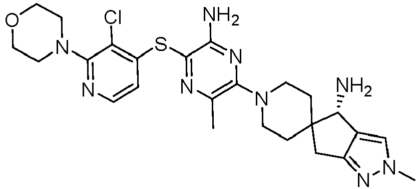

The compound of Example 118 was prepared by referring to the experimental scheme of Example 96.

MS m/z (ESI): 542.1 [M+H]⁺, 544.1 [M+2+H]⁺.

### Example 119

### Preparation of

### (S)-1'-(6-amino-5-((3-chloro-2-cyclopropylpyridin-4-yl)thio)-3-methylpyrazin-2-yl)-4,6 -dihydrospiro [cyclopenta[d]oxazole-5,4'-piperidin]-6-amine

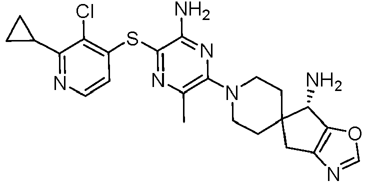

The compound of Example 119 was prepared by referring to the experimental scheme of Example 96.

MS m/z (ESI): 484.1 [M+H]⁺, 486.1 [M+2+H]⁺.

### Example 120

### Preparation of

### (S)-1'-(6-amino-5-((3-chloro-2-cyclopropylpyridin-4-yl)thio)pyrazin-2-yl)-4,6-dihydros piro[cyclopenta[d]oxazole-5,4'-piperidin]-6-amine

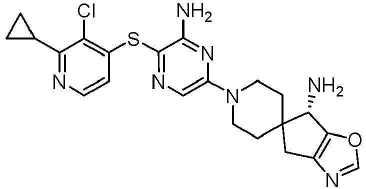

The compound of Example 120 was prepared by referring to the experimental scheme of Example 91.

MS m/z (ESI): 470.1 [M+H]⁺, 472.1 [M+2+H]⁺.

### Example 121

### Preparation of

### (S)-1'-(6-amino-5-((2-cyclopropylpyridin-4-yl)thio)pyrazin-2-yl)-4,6-dihydrospiro[cycl openta[d]oxazole-5,4'-piperidin]-6-amine

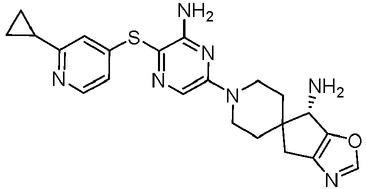

The compound of Example 121 was prepared by referring to the experimental scheme of Example 91.

MS m/z (ESI): 436.1 [M+H]⁺.

### Example 122

### Preparation of

### (S)-1'-(6-amino-5-((2-cyclopropyl-3-methylpyridin-4-yl)thio)pyrazin-2-yl)-4,6-dihydros piro[cyclopenta[d]oxazole-5,4'-piperidin]-6-amine

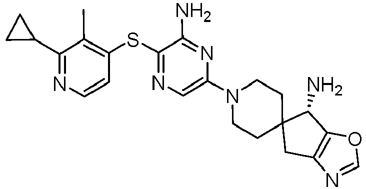

The compound of Example 122 was prepared by referring to the experimental scheme of Example 91.

MS m/z (ESI): 450.1 [M+H]⁺.

### Example 123

### Preparation of

### (S)-1'-(6-amino-5-((2-cyclopropyl-3-fluoropyridin-4-yl)thio)pyrazin-2-yl)-4,6-dihydros piro[cyclopenta[d]oxazole-5,4'-piperidin]-6-amine

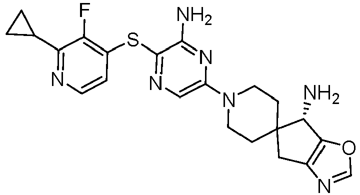

The compound of Example 123 was prepared by referring to the experimental scheme of Example 91.

MS m/z (ESI): 454.1 [M+H]⁺.

### Example 124

### Preparation of

### (S)-1'-(6-amino-5-((2-cyclopropylpyrimidin-4-yl)thio)pyrazin-2-yl)-4,6-dihydrospiro[cy clopenta[d]oxazole-5,4'-piperidin]-6-amine

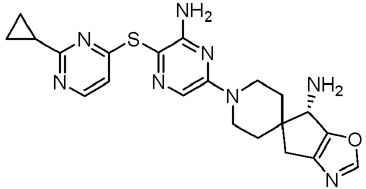

The compound of Example 124 was prepared by referring to the experimental scheme of Example 91.

MS m/z (ESI): 437.1 [M+H]⁺.

### Example 125

### Preparation of

### (S)-1-(4-((3-amino-5-(1-amino-1,3-dihydrospiro[indene-2,4'-piperidin]-1'-yl)pyrazin-2-yl)thio)-3-chloropyridin-2-yl)azetidin-2-one

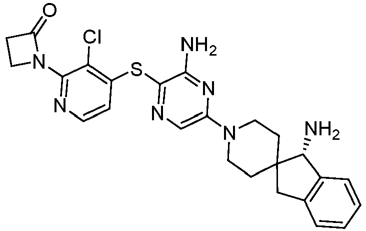

The compound of Example 125 was prepared by referring to the experimental scheme of Example 91.

MS m/z (ESI): 508.1 [M+H]⁺, 510.1 [M+2+H]⁺.

### Example 126

### Preparation of

### (S)-1-(4-((3-amino-5-(1-amino-1,3-dihydrospiro[indene-2,4'-piperidin]-1'-yl)pyrazin-2-yl)thio)-3-chloropyridin-2-yl)pyrrolidin-2-one

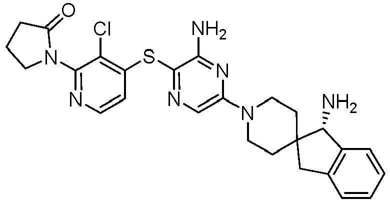

The compound of Example 126 was prepared by referring to the experimental scheme of Example 91.

MS m/z (ESI): 522.1 [M+H]⁺, 524.1 [M+2+H]⁺.

### Example 127

### Preparation of

### (S)-1'-(6-amino-5-((3-cyclopropylphenyl)thio)pyrazin-2-yl)-1,3-dihydrospiro[indene-2, 4'-piperidin]-1-amine

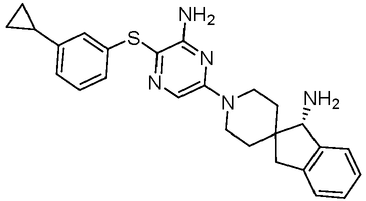

The compound of Example 127 was prepared by referring to the experimental scheme of Example 91.

MS m/z (ESI): 444.1 [M+H]⁺.

### Example 128

### Preparation of

### (S)-1'-(6-amino-5-((3-cyclopropyl-4-fluorophenyl)thio)pyrazin-2-yl)-1,3-dihydrospiro[i ndene-2,4'-piperidin]-1-amine

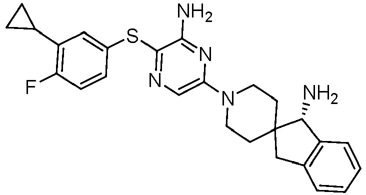

The compound of Example 128 was prepared by referring to the experimental scheme of Example 91.

MS m/z (ESI): 462.1 [M+H]⁺.

### Example 129

### Preparation of

### (S)-1'-(6-amino-5-((3-cyclopropyl-2-fluorophenyl)thio)pyrazin-2-yl)-1,3-dihydrospiro[i ndene-2,4'-piperidin]-1-amine

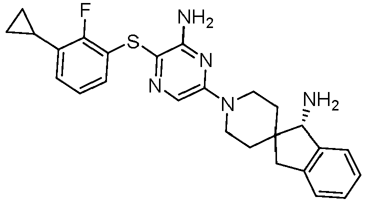

The compound of Example 129 was prepared by referring to the experimental scheme of Example 91.

MS m/z (ESI): 462.1 [M+H]⁺.

### Example 130

### Preparation of

### (S)-1'-(6-amino-5-((4-fluoro-3-morpholinophenyl)thio)pyrazin-2-yl)-1,3-dihydrospiro[i ndene-2,4'-piperidin]-1-amine

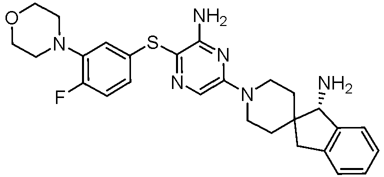

The compound of Example 130 was prepared by referring to the experimental scheme of Example 101.

MS m/z (ESI): 507.1 [M+H]⁺.

### Example 131

### Preparation of

### 2-(3-((S)-1-amino-1,3-dihydrospiro[indene-2,4'-piperidin]-1'-yl)-6-(2,3-dichlorophenyl) -5-methylpyrazin-2-yl)cyclopropan-1-ol

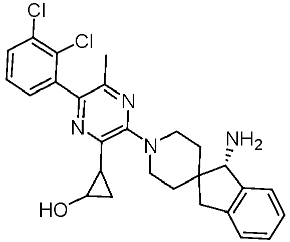

The compound of Example 131 was prepared by referring to the experimental scheme of Example 106.

MS m/z (ESI): 495.1 [M+H]⁺, 497.1 [M+2+H]⁺.

### Example 132

### Preparation of

### (S)-1-(3-(1-amino-1,3-dihydrospiro[indene-2,4'-piperidin]-1'-yl)-6-(2,3-dichlorophenyl) -5-methylpyrazin-2-yl)azetidin-3-ol

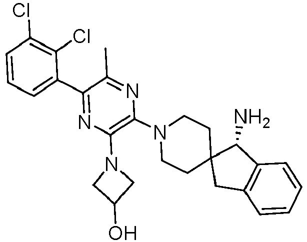

The compound of Example 132 was prepared by referring to the experimental scheme of Example 96.

MS m/z (ESI): 510.1 [M+H]⁺, 512.1 [M+2+H]⁺.

### Example 133

### Preparation of

### (S)-1-(3-(1-amino-1,3-dihydrospiro[indene-2,4'-piperidin]-1'-yl)-6-(2,3-dichlorophenyl) -5-methylpyrazin-2-yl)azetidin-3-one

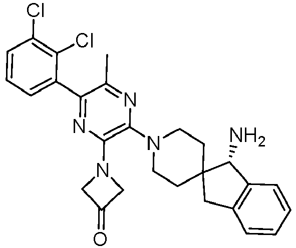

The compound of Example 133 was prepared by referring to the experimental scheme of Example 96.

MS m/z (ESI): 508.1 [M+H]⁺, 510.1 [M+2+H]⁺.

### Example 134

### Preparation of

### (S)-1-(3-(1-amino-1,3-dihydrospiro[indene-2,4'-piperidin]-1'-yl)-6-(2,3-dichlorophenyl) -5-methylpyrazin-2-yl)azetidin-2-one

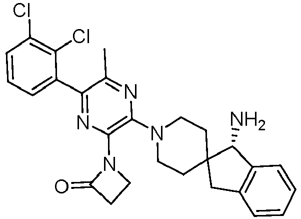

The compound of Example 134 was prepared by referring to the experimental scheme of Example 96.

MS m/z (ESI): 508.1 [M+H]⁺, 510.1 [M+2+H]⁺.

### Example 135

### Preparation of

### (S)-1-(3-(5-amino-5,7-dihydrospiro[cyclopenta[b]pyridine-6,4'-piperidin]-1'-yl)-6-(2,3-dichlorophenyl)-5-methylpyrazin-2-yl)cyclopropan-1-ol

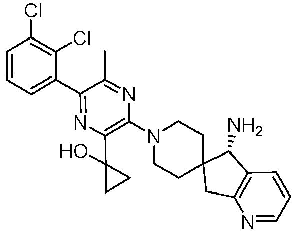

The compound of Example 135 was prepared by referring to the experimental scheme of Example 107.

MS m/z (ESI): 496.1 [M+H]⁺, 498.1 [M+2+H]⁺.

### Example 136

### Preparation of

### (S)-1-(3-(6-amino-4,6-dihydrospiro[cyclopenta[d]thiazole-5,4'-piperidin]-1'-yl)-6-(2,3-d ichlorophenyl)-5-methylpyrazin-2-yl)cyclopropan-1-ol

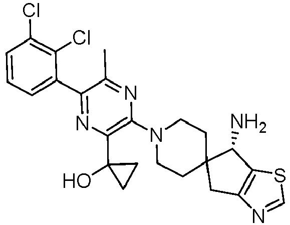

The compound of Example 136 was prepared by referring to the experimental scheme of Example 107.

MS m/z (ESI): 502.1 [M+H]⁺, 504.1 [M+2+H]⁺.

### Example 137

### Preparation of

### (S)-1-(3-(6-amino-4,6-dihydrospiro[cyclopenta[d]oxazole-5,4'-piperidin]-1'-yl)-6-(2,3-d ichlorophenyl)-5-methylpyrazin-2-yl)cyclopropan-1-ol

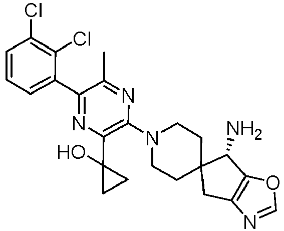

The compound of Example 137 was prepared by referring to the experimental scheme of Example 107.

MS m/z (ESI): 486.1 [M+H]⁺, 488.1 [M+2+H]⁺.

### Example 138

### Preparation of

### (S)-1-(3-(6-amino-1-methyl-4,6-dihydro-1H-spiro[cyclopenta[d]imidazole-5,4'-piperidi n]-1'-yl)-6-(2,3-dichlorophenyl)-5-methylpyrazin-2-yl)cyclopropan-1-ol

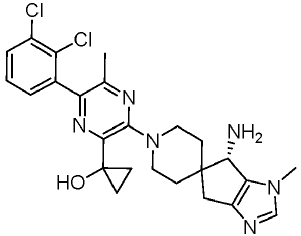

The compound of Example 138 was prepared by referring to the experimental scheme of Example 107.

MS m/z (ESI): 499.1 [M+H]⁺, 501.1 [M+2+H]⁺.

### Example 139

### Preparation of

### (S)-1-(3-(4-amino-2-methyl-2,6-dihydro-4H-spiro[cyclopenta[c]pyrazole-5,4'-piperidin] -1-yl)-6-(2,3-dichlorophenyl)-5-methylpyrazin-2-yl)cyclopropan-1-ol

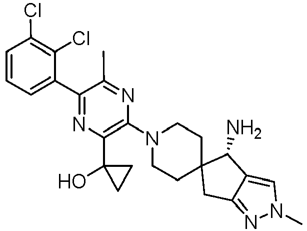

The compound of Example 139 was prepared by referring to the experimental scheme of Example 107.

MS m/z (ESI): 499.1 [M+H]⁺, 501.1 [M+2+H]⁺.

### Example 140

### Preparation of

### (S)-1-(3-(1-amino-1,3-dihydrospiro[indene-2,4'-piperidin]-1'-yl)-6-((2-cyclopropylpyrid in-4-yl)thio)-5-methylpyrazin-2-yl)cyclopropan-1-ol

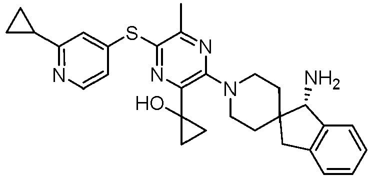

The compound of Example 140 was prepared by referring to the experimental scheme of Example 107.

MS m/z (ESI): 500.1 [M+H]⁺.

### Example 141

### Preparation of

### (S)-1-(3-(1-amino-1,3-dihydrospiro[indene-2,4'-piperidin]-1'-yl)-6-((3-chloro-2-cyclopr opylpyridin-4-yl)thio)-5-methylpyrazin-2-yl)cyclopropan-1-ol

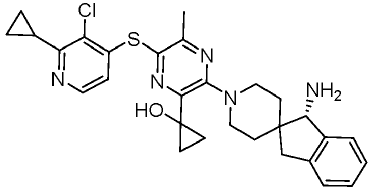

The compound of Example 141 was prepared by referring to the experimental scheme of Example 107.

MS m/z (ESI): 534.1 [M+H]⁺, 536.1 [M+2+H]⁺.

### Example 142

### Preparation of

### (S)-1-(3-(1-amino-1,3-dihydrospiro[indene-2,4'-piperidin]-1'-yl)-6-((2-amino-3-chlorop yridin-4-yl)thio)-5-methylpyrazin-2-yl)cyclopropan-1-ol

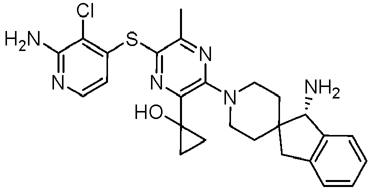

The compound of Example 142 was prepared by referring to the experimental scheme of Example 107.

MS m/z (ESI): 509.1 [M+H]⁺, 511.1 [M+2+H]⁺.

### Example 143

### Preparation of

### (S)-8-(6-amino-5-((2-amino-3-chloropyridin-4-yl)thio)pyrazin-2-yl)-2-cyclopropyl-8-az aspiro[4.5]dec-2-en-1-amine

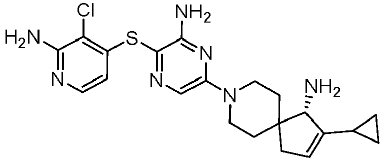

### Step 1: Preparation of tert-butyl 4-allyl-4-formylpiperidine-1-carboxylate

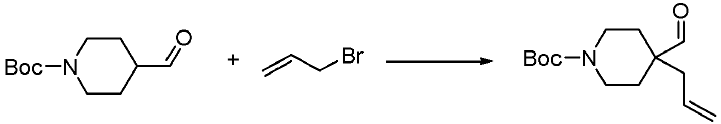

Lithium *tert*-butoxide (13.5 g, 16.9 mmol) was added to a solution of *tert*-butyl 4-formylpiperidine-1-carboxylate (30 g, 14.1 mmol) in N,N-dimethylformamide (300 mL) at 0°C, and the reaction solution was stirred for 30 minutes after completion of the addition. Allyl bromide (19 g, 16.2 mmol) was added, and the reaction solution was stirred at 0°C for 2 hours after completion of the addition. The reaction solution was poured into aqueous ammonium chloride solution (1 L), and extracted with ethyl acetate (1 L×2). The ethyl acetate layer was washed with saturated sodium chloride aqueous solution (500 mL), dried over anhydrous sodium sulfate, and purified by column chromatography [eluent: petroleum ether ∼ ethyl acetate/petroleum ether from 0% to 2%] to obtain the product *tert*-butyl 4-allyl-4-formylpiperidine-1-carboxylate (15 g, yield: 42%) as a colorless oil.

### Step 2: Preparation of tert-butyl 4-allyl-4-(1-hydroxyallyl)piperidine-1-carboxylate

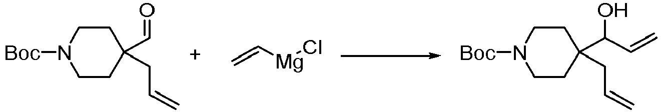

Vinyl magnesium chloride (65 mL, 123.5 mmol) was added dropwise to a solution of *tert*-butyl 4-allyl-4-formylpiperidine-l-carboxylate (25 g, 99 mmol) in tetrahydrofuran (300 mL) at -78°C. After completion of the addition, the reaction solution was slowly warmed up to room temperature and stirred for 30 minutes. The reaction solution was poured into aqueous ammonium chloride solution (1 L), and extracted with ethyl acetate (1 L×2). The ethyl acetate layer was washed with saturated sodium chloride aqueous solution (500 mL), dried over anhydrous sodium sulfate, and purified by column chromatography [eluent: petroleum ether ∼ ethyl acetate/petroleum ether from 0% to 5%] to obtain the product *tert*-butyl 4-allyl-4-(1-hydroxyallyl)piperidine-1-carboxylate (25 g, yield: 90%) as a colorless oil.

### Step 3: Preparation of tert-butyl 4-acryloyl-4-allylpiperidine-1-carboxylate

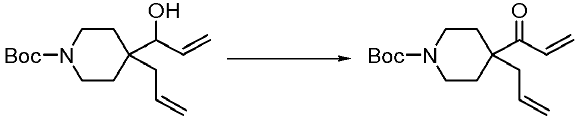

Dess-Martin reagent (41.5 g, 98 mmol) was added to a solution of *tert*-butyl 4-allyl-4-(1-hydroxyallyl)piperidine-1-carboxylate (25 g, 89 mmol) in dichloromethane (400 mL) at 0°C. After completion of the addition, the reaction solution was stirred at 40°C for 1 hour. The reaction solution was slowly poured into sodium bicarbonate/sodium sulfite aqueous solution (1/1, 1 L), and extracted with dichloromethane (1 L×2). The dichloromethane layer was washed with saturated sodium chloride aqueous solution (500 mL), dried over anhydrous sodium sulfate, and concentrated to dryness by rotary evaporation. *N*-heptane (200 mL) was added, and the solution was stirred for 5 minutes. The solution was filtered to remove insoluble substance, and the filtrate was concentrated to obtain the product *tert*-butyl 4-acryloyl-4-allylpiperidine-1-carboxylate (24.8 g, yield: 100%) as a colorless oil, which was rapidly used in the next step.

### Step 4: Preparation of tert-butyl 1-oxo-8-azaspiro[4.5]dec-2-ene-8-carboxylate

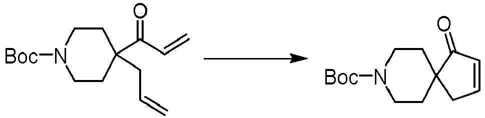

*Tert*-butyl 4-acryloyl-4-allylpiperidine-1-carboxylate (24.8 g, 89 mmol) and dichloro(2-isopropoxyphenylmethylene)(tricyclohexylphosphine)ruthenium(II) (1.2 g, 1.4 mmol) were stirred in toluene (700 mL) at 90°C for 2 hours. The reaction solution was cooled, concentrated and purified by column chromatography [eluent: petroleum ether ∼ ethyl acetate/petroleum ether from 0% to 20%] to obtain the product *tert*-butyl 1-oxo-8-azaspiro[4.5]dec-2-ene-8-carboxylate (13 g, yield: 58%) as a reddish black solid.

MS m/z (ESI): 252.1 [M+H]⁺.

### Step 5: Preparation of tert-butyl 2-bromo-1-oxo-8-azaspiro[4.5]dec-2-ene-8-carboxylate

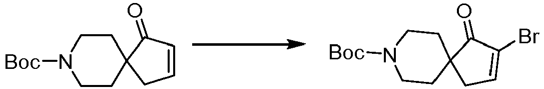

Pyridine N-oxide (11 g, 116 mmol) was added to a solution of *tert*-butyl 1-oxo-8-azaspiro[4.5]dec-2-ene-8-carboxylate (13 g, 51.8 mmol) in acetonitrile (300 mL), followed by the addition of N-bromosuccinimide (28 g, 157 mmol). After completion of the addition, the reaction solution was stirred at 80°C for 18 hours. The reaction solution was cooled, concentrated and purified by column chromatography [eluent: petroleum ether ∼ ethyl acetate/petroleum ether from 0% to 20%] to obtain the product *tert*-butyl 2-bromo-1-oxo-8-azaspiro[4.5]dec-2-ene-8-carboxylate (4.2 g, yield: 25%) as a yellow solid.

MS m/z (ESI): 330.1 [M+H]⁺, 332.1 [M+2+H]⁺.

### Step 6: Preparation of tert-butyl 2-cyclopropyl-1-oxo-8-azaspiro[4.5]dec-2-ene-8-carboxylate

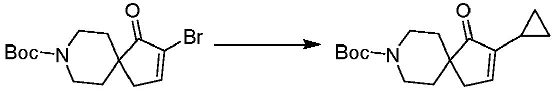

*Tert*-butyl 2-bromo-1-oxo-8-azaspiro[4.5]dec-2-ene-8-carboxylate (4.2 g, 12.8 mmol), potassium cyclopropyl trifluoroborate (3.79 g, 25.6 mmol), cesium carbonate (12.5 g, 38.4 mmol) and [1,1'-bis(diphenylphosphino)ferrocene]dichloropalladium(II) dichloromethane complex (731 mg, 0.9 mmol) were stirred in dioxane (100 mL) and water (10 mL) at 100°C for 18 hours. Water (150 mL) was added to quench the reaction, and the solution was extracted with ethyl acetate (150 mL×2). The ethyl acetate layer was washed with saturated sodium chloride aqueous solution (50 mL), dried over anhydrous sodium sulfate, and purified by column chromatography [eluent: petroleum ether ∼ ethyl acetate/petroleum ether from 0% to 12%] to obtain the product *tert*-butyl 2-cyclopropyl-1-oxo-8-azaspiro[4.5]dec-2-ene-8-carboxylate (1 g, yield: 27%) as a yellow oil.

MS m/z (ESI): 292.2 [M+H]⁺.

### Step 7: Preparation of tert-butyl (R,Z)-I-((tert-butylsulfinyl<sulfenyl>)imino)-2-cyclopropyl-8-azaspiro[4.5]dec-2-ene-8 -carboxylate

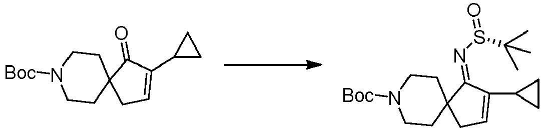

*Tert*-butyl 2-cyclopropyl-1-oxo-8-azaspiro[4.5]dec-2-ene-8-carboxylate (1 g, 3.4 mmol) and (R)-(+)-*tert*-butylsulfinamide (2 g, 17.2 mmol) were stirred in tetraethyl titanate (30 mL) at 100°C for 18 hours. The reaction solution was cooled, and then ethyl acetate (100 mL) was added. The reaction solution was poured into water (150 mL) to precipitate a large amount of white solid. The reaction solution was filtered through diatomaceous earth, and the filtrate was separated into two phases. The organic phase was concentrated to dryness by rotary evaporation, and the resulting residue was purified by column chromatography [eluent: petroleum ether ∼ ethyl acetate/petroleum ether from 0% to 50%] to obtain the product *tert*-butyl (R,Z)-1-((*tert*-butylsulfinyl<sulfenyl>)imino)-2-cyclopropyl-8-azaspiro[4.5]dec-2-ene-8 -carboxylate (190 mg, yield: 14%) as a yellow oil.

MS m/z (ESI): 395.2 [M+H]⁺.

### Step 8: Preparation of tert-butyl (S)-1-(((R)-tert-butylsulfinyl<sulfenyl>)amino)-2-cyclopropyl-8-azaspiro[4.5]dec-2-ene -8-carboxylate

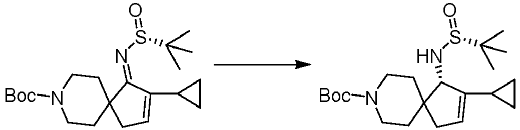

Diisobutyl aluminum hydride (0.9 mL, 1 mmol) was added dropwise to a solution of *tert*-butyl (R,Z)-1-((*tert*-butylsulfinyl<sulfenyl>)imino)-2-cyclopropyl-8-azaspiro[4.5]dec-2-ene-8 -carboxylate (210 mg, 0.53 mmol) in tetrahydrofuran (10 mL) at -78°C. After completion of the addition, the reaction solution was stirred for 15 minutes. Sodium sulfate decahydrate was added at -78°C to quench the reaction, and the reaction solution was stirred for 10 minutes. The reaction solution was filtered, and the filtrate was concentrated and purified by column chromatography [eluent: petroleum ether ∼ ethyl acetate/petroleum ether from 0% to 5%] to obtain the product *tert*-butyl (S)-1-(((R)-*tert*-butylsulfinyl<sulfenyl>)amino)-2-cyclopropyl-8-azaspiro[4.5]dec-2-ene -8-carboxylate (105 mg, yield: 50%) as a colorless oil.

MS m/z (ESI): 397.2 [M+H]⁺.

### Step 9: Preparation of (S)-2-cyclopropyl-8-azaspiro[4.5]dec-2-en-1-amine

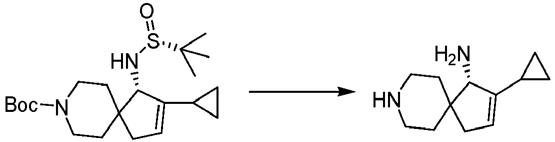

Hydrochloric acid in dioxane (15 mL, 60 mmol) was added to a solution of *tert*-butyl (S)-1-(((R)-*tert*-butylsulfinyl<sulfenyl>)amino)-2-cyclopropyl-8-azaspiro[4.5]dec-2-ene -8-carboxylate (105 mg, 0.27 mmol) in methanol (3 mL). After completion of the addition, the reaction solution was stirred at room temperature for 1 hour. The reaction solution was concentrated to dryness by rotary evaporation to obtain the product (S)-2-cyclopropyl-8-azaspiro[4.5]dec-2-en-1-amine (105 mg, yield: 100%) as a white solid.

MS m/z (ESI): 193.2 [M+H]⁺.

### Step 10: Preparation of (S)-8-(6-amino-5-((2-amino-3-chloropyridin-4-yl)thio)pyrazin-2-yl)-2-cyclopropyl-8-az aspiro[4.5]dec-2-en-1-amine

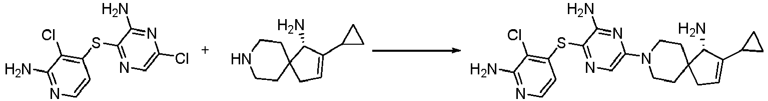

3-((2-Amino-3-chloropyridin-4-yl)thio)-6-chloropyrazin-2-amine (76 mg, 0.26 mmol), (S)-2-cyclopropyl-8-azaspiro[4.5]dec-2-en-1-amine (80 mg, 0.2 mmol) and potassium carbonate (167 mg, 1.2 mmol) were stirred in N,N-dimethylformamide (8 mL) at 100°C for 7 hours. Water (60 mL) was added, and the solution was extracted with ethyl acetate (50 mL×2). The ethyl acetate layer was washed with saturated sodium chloride aqueous solution (20 mL), dried over anhydrous sodium sulfate, and purified by thin layer chromatography [eluent: dichloromethane/methanol= 7/1] to obtain the product (S)-8-(6-amino-5-((2-amino-3-chloropyridin-4-yl)thio)pyrazin-2-yl)-2-cyclopropyl-8-az aspiro[4.5]dec-2-en-1-amine (37 mg, yield: 41%) as a yellow oil.

¹H NMR (400 MHz, MeOD) δ 7.67 - 7.51 (m, 2H), 5.92 (d, *J* = 8 Hz, 1H), 5.27 (s, 1H), 4.26 - 4.04 (m, 2H), 3.33 (s, 1H), 3.27 - 3.08 (m, 2H), 2.44 - 2.18 (m, 2H), 1.81 - 1.69 (m, 1H), 1.68 - 1.47 (m, 2H), 1.42 - 1.24 (m, 2H), 0.81 - 0.60 (m, 2H), 0.57 - 0.28 (m, 2H).

MS m/z (ESI): 444.1 [M+H]⁺, 446.1 [M+2+H]⁺.

### Example 144

### Preparation of

### (S)-8-(5-((2-amino-3-chloropyridin-4-yl)thio)pyrazin-2-yl)-2-cyclopropyl-8-azaspiro[4. 5]dec-2-en-1-amine

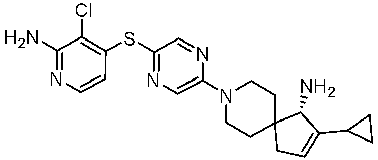

### Step 1: Preparation of 3-chloro-4-((5-chloropyrazin-2-yl)thio)pyridin-2-amine

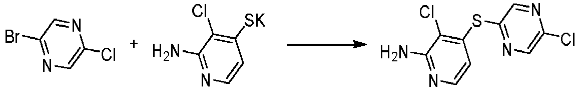

Potassium 2-amino-3-chloropyridine-4-thiolate (1 g, 2.64 mmol), 2-bromo-5-chloropyrazine (597 mg, 3.11 mmol), tris(dibenzylideneacetone)dipalladium (284 mg, 0.311 mmol), chloro[(4,5-bis(diphenylphosphino)-9,9-dimethylxanthene)-2-(2'-amino-1,1'-biphenyl)] palladium(II) (360 mg, 0.622 mmol) and N,N-diisopropylethylamine (1.2 g, 9.33 mmol) were stirred in N,N-dimethylformamide (10 mL) at 90°C under microwave for 1 hour. The reaction solution was concentrated to dryness by rotary evaporation, and the resulting residue was purified by column chromatography [eluent: petroleum ether ∼ ethyl acetate/petroleum ether from 0% to 30%] to obtain the product 3-chloro-4-((5-chloropyrazin-2-yl)thio)pyridin-2-amine (250 mg, yield: 35%) as a white solid.

MS m/z (ESI): 273.0 [M+H]⁺, 275.0 [M+2+H]⁺.

### Step 2: Preparation of (S)-8-(5-((2-amino-3-chloropyridin-4-yl)thio)pyrazin-2-yl)-2-cyclopropyl-8-azaspiro[4. 5]dec-2-en-1-amine

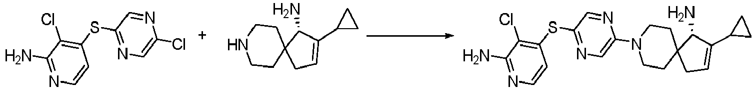

3-Chloro-4-((5-chloropyrazin-2-yl)thio)pyridin-2-amine (100 mg, 0.368 mmol), (S)-2-cyclopropyl-8-azaspiro[4.5]dec-2-en-1-amine hydrochloride (105 mg, 0.265 mmol) and potassium carbonate (254 mg, 1.84 mmol) were stirred in N,N-dimethylformamide (8 mL) at 100°C for 6 hours. Water (40 mL) was added, and the reaction solution was extracted with ethyl acetate (40 mL*2). The organic phase was concentrated, and purified by thin layer chromatography (dichloromethane/methanol= 10/1) to obtain the product (S)-8-(5-((2-amino-3-chloropyridin-4-yl)thio)pyrazin-2-yl)-2-cyclopropyl-8-azaspiro[4. 5]dec-2-en-1-amine (40 mg, yield: 35%) as a yellow solid.

¹H NMR (400 MHz, MeOD) δ 8.29 (d, *J* = 16 Hz, 2H), 7.60 (d, *J* = 4 Hz, 1H), 5.93 (d, *J* = 4 Hz, 1H), 5.31 (s, 1H), 4.34 - 4.14 (m, 2H), 3.43 - 3.35 (m, 2H), 3.28 - 3.21 (m, 1H), 2.45 - 2.21 (m, 2H), 1.86 - 1.49 (m, 3H), 1.44 - 1.22 (m, 2H), 0.82 - 0.61 (m, 2H), 0.58 - 0.30 (m, 2H).

MS m/z (ESI): 429.1 [M+H]⁺, 431.1 [M+2+H]⁺.

### Example 145

### Preparation of

### (S)-8-(6-amino-5-((2-amino-3-chloropyridin-4-yl)thio)-3-methylpyrazin-2-yl)-2-methyl -8-azaspiro[4.5]dec-2-en-1-amine

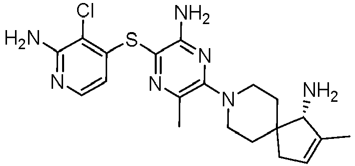

The compound of Example 145 was prepared by referring to the experimental scheme of Example 96.

MS m/z (ESI): 432.1 [M+H]⁺, 434.1 [M+2+H]⁺.

### Example 146

### Preparation of

### (S)-8-(6-amino-5-((2-amino-3-chloropyridin-4-yl)thio)-3-methylpyrazin-2-yl)-2-ethyl-8 -azaspiro[4.5]dec-2-en-1-amine

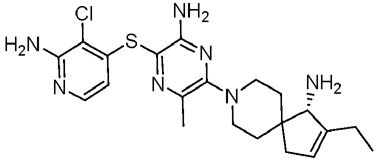

The compound of Example 146 was prepared by referring to the experimental scheme of Example 96.

MS m/z (ESI): 446.1 [M+H]⁺, 448.1 [M+2+H]⁺.

### Example 147

### Preparation of

### (S)-8-(6-amino-5-((2-amino-3-chloropyridin-4-yl)thio)-3-methylpyrazin-2-yl)-2-isoprop yl-8-azaspiro[4.5]dec-2-en-1-amine

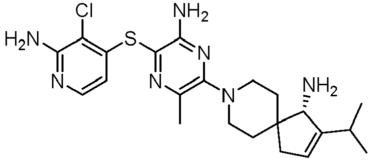

The compound of Example 147 was prepared by referring to the experimental scheme of Example 96.

MS m/z (ESI): 460.1 [M+H]⁺, 462.1 [M+2+H]⁺.

### Example 148

### Preparation of

### (S)-8-(6-amino-5-((2-amino-3-chloropyridin-4-yl)thio)-3-methylpyrazin-2-yl)-2-cyclopr opyl-8-azaspiro[4.5]dec-2-en-1-amine

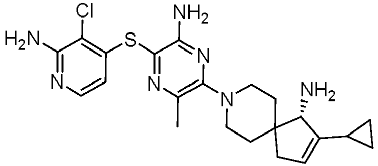

The compound of Example 148 was prepared by referring to the experimental scheme of Example 96.

MS m/z (ESI): 458.1 [M+H]⁺, 460.1 [M+2+H]⁺.

### Example 149

### Preparation of

### (R)-8-(6-amino-5-((2-amino-3-chloropyridin-4-yl)thio)-3-methylpyrazin-2-yl)-8-azadis piro[2.1.55.23]dodecan-4-amine

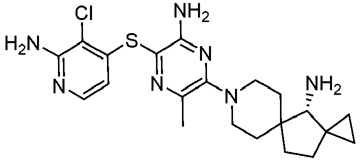

The compound of Example 149 was prepared by referring to the experimental scheme of Example 96.

MS m/z (ESI): 446.1 [M+H]⁺, 448.1 [M+2+H]⁺.

### Example 150

### Preparation of

### (R)-8-(6-amino-5-((2-amino-3-chloropyridin-4-yl)thio)-3-methylpyrazin-2-yl)-11-oxa-8 -azadispiro[2.1.55.23]dodecan-4-amine

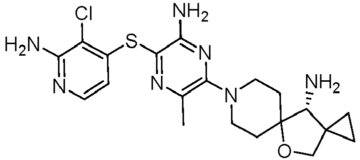

The compound of Example 150 was prepared by referring to the experimental scheme of Example 96.

MS m/z (ESI): 448.1 [M+H]⁺, 450.1 [M+2+H]⁺.

### Example 151

### Preparation of

### (R)-8-(6-amino-5-((2-amino-3-chloropyridin-4-yl)thio)pyrazin-2-yl)-8-azadispiro[2.1.5 5.23]dodecan-4-amine

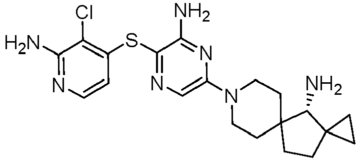

The compound of Example 151 was prepared by referring to the experimental scheme of Example 91.

MS m/z (ESI): 432.1 [M+H]⁺, 434.1 [M+2+H]⁺.

### Example 154

### Preparation of

### (S)-8-(6-amino-3-methyl-5-((2-(trifluoromethyl)pyridin-3-yl)thio)pyrazin-2-yl)-2-methy 1-8-azaspiro[4.5]dec-2-en-1-amine

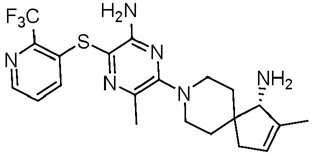

The compound of Example 154 was prepared by referring to the experimental scheme of Example 96.

MS m/z (ESI): 451.1 [M+H]⁺.

### Example 155

### Preparation of

### (S)-8-(6-amino-3-methyl-5-((2-(trifluoromethyl)pyridin-3-yl)thio)pyrazin-2-yl)-2-cyclo propyl-8-azaspiro[4.5]dec-2-en-1-amine

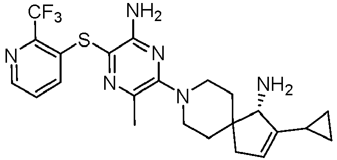

The compound of Example 155 was prepared by referring to the experimental scheme of Example 96.

MS m/z (ESI): 477.1 [M+H]⁺.

### Example 157

### Preparation of

### (R)-8-(6-amino-3-methyl-5-((2-(trifluoromethyl)pyridin-3-yl)thio)pyrazin-2-yl)-11-oxa-8-azadispiro[2.1.55.23]dodecan-4-amine

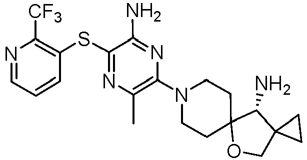

The compound of Example 157 was prepared by referring to the experimental scheme of Example 96.

MS m/z (ESI): 467.1 [M+H]⁺.

### Example 159

### Preparation of

### (S)-1-(6-((2-amino-3-chloropyridin-4-yl)thio)-3-(6-amino-4,6-dihydrospiro[cyclopenta[ d]oxazole-5,4'-piperidin]-1'-yl)-5-methylpyrazin-2-yl)cyclopropan-1-ol

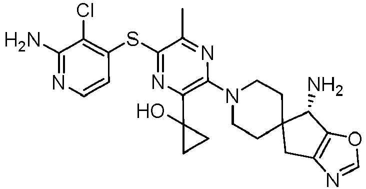

The compound of Example 159 was prepared by referring to the experimental scheme of Example 107.

MS m/z (ESI): 500.1 [M+H]⁺, 502.1 [M+2+H]⁺.

### Example 160

### Preparation of

### (R)-1-(6-((2-amino-3-chloropyridin-4-yl)thio)-3-(1-amino-8-azaspiro[4.5]dec-2-en-8-yl )-5-methylpyrazin-2-yl)cyclopropan-1-ol

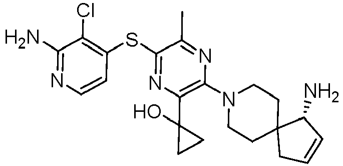

The compound of Example 160 was prepared by referring to the experimental scheme of Example 107.

MS m/z (ESI): 459.1 [M+H]⁺, 461.1 [M+2+H]⁺.

### Example 161

### Preparation of

### (S)-1-(3-(1-amino-2-methyl-8-azaspiro[4.5]dec-2-en-8-yl)-6-((2-amino-3-chloropyridin -4-yl)thio)-5-methylpyrazin-2-yl)cyclopropan-1-ol

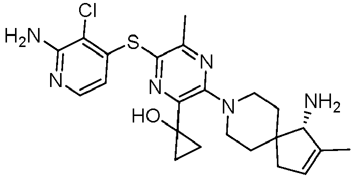

The compound of Example 161 was prepared by referring to the experimental scheme of Example 107.

MS m/z (ESI): 473.1 [M+H]⁺, 475.1 [M+2+H]⁺.

### Example 162

### Preparation of

### (S)-1-(3-(1-amino-2-cyclopropyl-8-azaspiro[4.5]dec-2-en-8-yl)-6-((2-amino-3-chloropy ridin-4-yl)thio)-5-methylpyrazin-2-yl)cyclopropan-1-ol

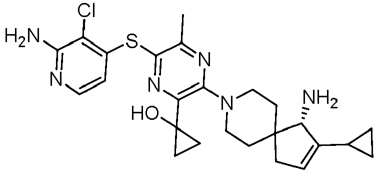

The compound of Example 162 was prepared by referring to the experimental scheme of Example 107.

MS m/z (ESI): 499.1 [M+H]⁺, 501.1 [M+2+H]⁺.

### Example 163

### Preparation of

### (R)-1-(6-((2-amino-3-chloropyridin-4-yl)thio)-3-(4-amino-8-azadispiro[2.1.55.23]dodec an-8-yl)-5-methylpyrazin-2-yl)cyclopropan-1-ol

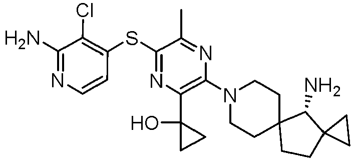

The compound of Example 163 was prepared by referring to the experimental scheme of Example 107.

MS m/z (ESI): 487.1 [M+H]⁺, 489.1 [M+2+H]⁺.

### Example 164

### Preparation of

### (S)-1'-(6-amino-5-((2-aminopyrimidin-4-yl)thio)-3-methylpyrazin-2-yl)-4,6-dihydrospir o[cyclopenta[d]oxazole-5,4'-piperidin]-6-amine

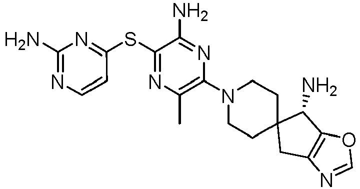

The compound of Example 164 was prepared by referring to the experimental scheme of Example 96.

MS m/z (ESI): 426.1 [M+H]⁺.

### Example 165

### Preparation of

### (S)-1'-(6-amino-5-((2-aminopyrimidin-4-yl)thio)-3-methylpyrazin-2-yl)-1,3-dihydrospir o[indene-2,4'-piperidin]-1-amine

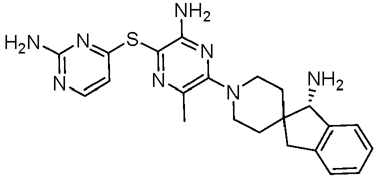

The compound of Example 165 was prepared by referring to the experimental scheme of Example 96.

MS m/z (ESI): 435.1 [M+H]⁺.

### Example 166

### Preparation of

### (S)-8-(6-amino-5-((2-aminopyrimidin-4-yl)thio)-3-methylpyrazin-2-yl)-2-methyl-8-azas piro[4.5]dec-2-en-1-amine

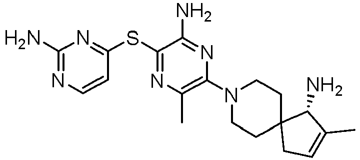

The compound of Example 166 was prepared by referring to the experimental scheme of Example 96.

MS m/z (ESI): 399.1 [M+H]⁺.

### Example 167

### Preparation of

### (S)-8-(6-amino-5-((2-aminopyrimidin-4-yl)thio)-3-methylpyrazin-2-yl)-2-cyclopropyl-8 -azaspiro[4.5]dec-2-en-1-amine

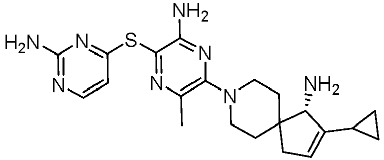

The compound of Example 167 was prepared by referring to the experimental scheme of Example 96.

MS m/z (ESI): 425.1 [M+H]⁺.

### Example 168

### Preparation of

### (S)-1'-(6-amino-3-methyl-5-((2-(methylamino)pyrimidin-4-yl)thio)pyrazin-2-yl)-1,3-dih ydrospiro[indene-2,4'-piperidin]-1-amine

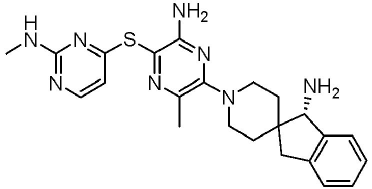

The compound of Example 168 was prepared by referring to the experimental scheme of Example 96.

MS m/z (ESI): 449.1 [M+H]⁺.

### Example 169

### Preparation of

### (S)-1'-(6-amino-5-((2-(cyclopropylamino)pyrimidin-4-yl)thio)-3-methylpyrazin-2-yl)-1, 3-dihydrospiro[indene-2,4'-piperidin]-1-amine

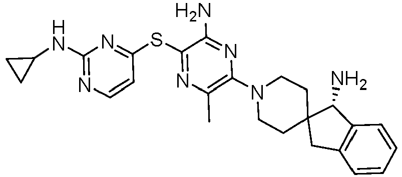

The compound of Example 169 was prepared by referring to the experimental scheme of Example 96.

MS m/z (ESI): 475.1 [M+H]⁺.

### Example 170

### Preparation of

### (S)-(3-(1-amino-1,3-dihydrospiro[indene-2,4'-piperidin]-1'-yl)-6-((2-amino-3-chloropyri din-4-yl)thio)-5-methylpyrazin-2-yl)methanol

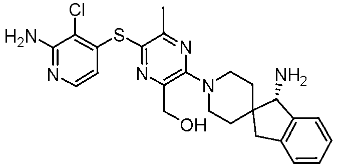

### Step 1: Preparation of 2-ethylhexyl 3-((2-amino-3-chloropyridin-4-yl)thio)propanoate

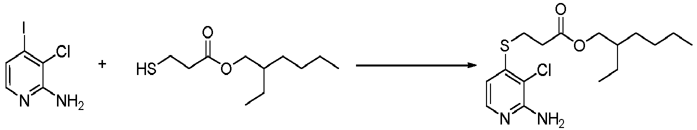

3-Chloro-4-iodopyridin-2-amine (3 g, 11.8 mmol), 2-ethylhexyl 3-mercaptopropanoate (2.9 g, 14.2 mmol), palladium acetate (132 mg, 0.59 mmol), 4,5-bis(diphenylphosphino)-9,9-dimethylxanthene (683 mg, 1.18 mmol) and N,N-diisopropylethylamine (561 mg, 4.3 mmol) were stirred in dioxane (80 mL) at 100°C for 18 hours. The reaction solution was filtered. The filtrate was concentrated, and purified by column chromatography [eluent: petroleum ether ∼ petroleum ether/ethyl acetate (60:40)] to obtain 2-ethylhexyl 3-((2-amino-3-chloropyridin-4-yl)thio)propanoate (3.8 g, yield: 94%) as a light yellow solid.

MS m/z (ESI): 345.1 [M+H]⁺, 347.0 [M+2+H]⁺.

### Step 2: Preparation of potassium 2-amino-3-chloropyridine-4-thiolate

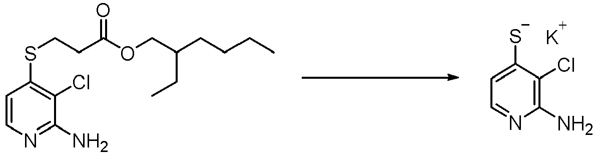

Potassium *tert*-butoxide (174 mg, 1.53 mmol) was added to a solution of 2-ethylhexyl 3-((2-amino-3-chloropyridin-4-yl)thio)propanoate (500 mg, 1.5 mmol) in ethanol (30 mL). After completion of the addition, the reaction solution was stirred at 40°C for 2 hours. The reaction solution was concentrated to obtain the crude product potassium 2-amino-3-chloropyridine-4-thiolate (500 mg) as a yellow solid, which was used directly in the next step.

MS m/z (ESI): 161.0 [M+H]⁺, 163.0 [M+2+H]⁺.

### Step 3: Preparation of ethyl 6-bromo-3-((S)-1-(((R)-tert-butylsulfinyl<sulfenyl>)amino)-1,3-dihydrospiro[indene-2, 4'-piperidin]-1'-yl)-5-methylpyrazine-2-carboxylate

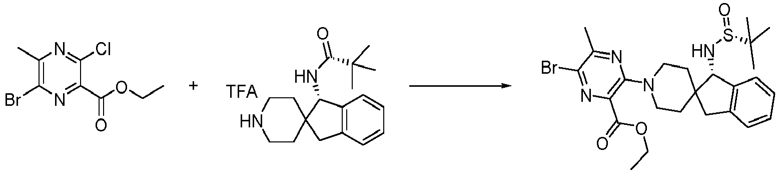

Ethyl 6-bromo-3-chloro-5-methylpyrazine-2-carboxylate (840 mg, 3 mmol), (S)-N-(1,3-dihydrospiro[indene-2,4'-piperidin]-1-yl)pivalamide trifluoroacetate (1.5 g, 3.6 mmol) and potassium carbonate (1.7 g, 12 mmol) were stirred in acetonitrile (40 mL) at 55°C for 18 hours. The reaction solution was filtered. The filtrate was concentrated, and purified by column chromatography [eluent: petroleum ether ∼ petroleum ether/ethyl acetate (60:40)] to obtain ethyl 6-bromo-3-((S)-1-(((R)-*tert*-butylsulfinylsulfenyl>)amino)-1,3-dihydrospiro[indene-2, 4'-piperidin]-1'-yl)-5-methylpyrazine-2-carboxylate (1.5 g, yield: 91%) as a yellow solid.

MS m/z (ESI): 549.2 [M+H]⁺, 551.2 [M+2+H]⁺.

### Step 4: Preparation of ethyl 6-((2-amino-3-chloropyridin-4-yl)thio)-3-((S)-1-(((R)-tert-butylsulfinyl<sulfenyl>)amin o)-1,3-dihydrospiro[indene-2,4'-piperidin]-1'-yl)-5-methylpyrazine-2-carboxylate

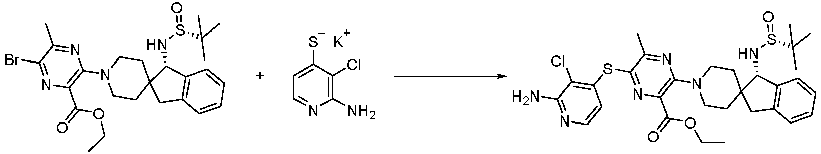

Ethyl 6-bromo-3-((S)-1-(((R)-*tert*-butylsulfinyl<sulfenyl>)amino)-1,3-dihydrospiro[indene-2, 4'-piperidin]-1'-yl)-5-methylpyrazine-2-carboxylate (798 mg, 1.45 mmol), potassium 2-amino-3-chloropyridine-4-thiolate (500 mg, 1.45 mmol, crude), tris(dibenzylideneacetone)dipalladium (66 mg, 0.073 mmol), 4,5-bis(diphenylphosphino)-9,9-dimethylxanthene (84 mg, 0.145 mmol) and N,N-diisopropylethylamine (561 mg, 4.35 mmol) were stirred in N,N-dimethylformamide (15 mL) at 100°C under microwave for 1.5 hours. The reaction solution was filtered. The filtrate was concentrated, and purified by column chromatography [eluent: dichloromethane ∼ dichloromethane/methanol (98.5:1.5)] to obtain ethyl 6-((2-amino-3-chloropyridin-4-yl)thio)-3-((S)-1-(((R)-*tert*-butylsulfinyl<sulfenyl>)amin o)-1,3-dihydrospiro[indene-2,4'-piperidin]-1'-yl)-5-methylpyrazine-2-carboxylate (500 mg, yield: 55%) as a yellow solid.

MS m/z (ESI): 629.1 [M+H]⁺, 631.1 [M+2+H]⁺.

### Step 5: Preparation of (R)-N-((S)-1'-(5-((2-amino-3-chloropyridin-4-yl)thio)-3-(hydroxymethyl)-6-methylpyra zin-2-yl)-1,3-dihydrospiro[indene-2,4'-piperidin]-1-yl)-2-methylpropane-2-sulfinamide

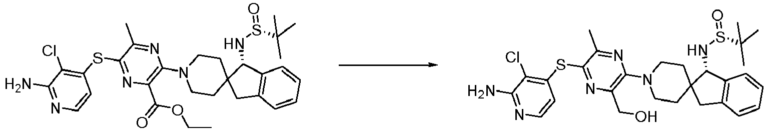

Lithium aluminum hydride (0.7 mL, 2.5 M) was added dropwise to a solution of ethyl 6-((2-amino-3-chloropyridin-4-yl)thio)-3-((S)-1-(((R)-*tert*-butylsulfinyl<sulfenyl>)amin o)-1,3-dihydrospiro[indene-2,4'-piperidin]-1'-yl)-5-methylpyrazine-2-carboxylate (500 mg, 0.795 mmol) in tetrahydrofuran (30 mL) at 0°C. The reaction solution was stirred at 0°C for 30 minutes. Sodium sulfate decahydrate was added to quench the reaction, and the reaction solution was stirred for 10 minutes. The reaction solution was filtered through diatomaceous earth, and the filter cake was washed with tetrahydrofuran (50 mL) twice. The filtrate was concentrated, and purified by column chromatography [eluent: dichloromethane ∼ dichloromethane/methanol (95:5)] to obtain (R)-N-((S)-1'-(5-((2-amino-3-chloropyridin-4-yl)thio)-3-(hydroxymethyl)-6-methylpyra zin-2-yl)-1,3-dihydrospiro[indene-2,4'-piperidin]-1-yl)-2-methylpropane-2-sulfinamide (220 mg, yield: 47%) as a yellow solid.

MS m/z (ESI): 587.1 [M+H]⁺, 589.1 [M+2+H]⁺.

### Step 6: Preparation of (S)-(3-(1-amino-1,3-dihydrospiro[indene-2,4'-piperidin]-1'-yl)-6-((2-amino-3-chloropyri din-4-yl)thio)-5-methylpyrazin-2-yl)methanol

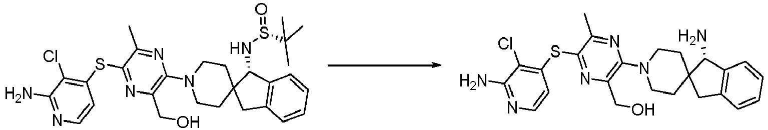

Hydrochloric acid in dioxane (20 mL, 4.0 M) was added to a solution of (R)-N-((S)-1'-(5-((2-amino-3-chloropyridin-4-yl)thio)-3-(hydroxymethyl)-6-methylpyra zin-2-yl)-1,3-dihydrospiro[indene-2,4'-piperidin]-1-yl)-2-methylpropane-2-sulfinamide (220 mg, 0.375 mmol) in methanol (3 mL), and the reaction solution was stirred at room temperature for 30 minutes. The reaction solution was concentrated, and dissolved in methanol (10 mL). The reaction solution was adjusted to alkaline with ammonia, and concentrated to dryness by rotary evaporation. The resulting residue was purified by column chromatography [eluent: dichloromethane ∼ dichloromethane/methanol (1%NH3-H2O) (95:5)] to obtain (S)-(3-(1-amino-1,3-dihydrospiro[indene-2,4'-piperidin]-1'-yl)-6-((2-amino-3-chloropyri din-4-yl)thio)-5-methylpyrazin-2-yl)methanol (115 mg, yield: 63%) as a white solid.

¹H NMR (400 MHz, MeOD) δ 7.59 (d, *J* = 4 Hz, 1H), 7.41 - 7.34 (m, 1H), 7.27 - 7.16 (m, 3H), 5.89 (d, *J* = 4 Hz, 1H), 4.64 (s, 2H), 4.00 (s, 1H), 3.88 (d, *J* = 12 Hz, 2H), 3.28 - 3.20 (m, 2H), 3.14 (d, *J* = 16 Hz, 1H), 2.82 (d, *J* = 16 Hz, 1H), 2.48 (s, 3H), 2.01 - 1.85 (m, 2H), 1.67 - 1.58 (m, 1H), 1.52 - 1.43 (m, 1H).

MS m/z (ESI): 483.2 [M+H]⁺, 485.2 [M+2+H]⁺.

### Example 171

### Preparation of

### (S)-(6-((2-amino-3-chloropyridin-4-yl)thio)-3-(6-amino-4,6-dihydrospiro[cyclopenta[d] thiazole-5,4'-piperidin]-1'-yl)-5-methylpyrazin-2-yl)methanol

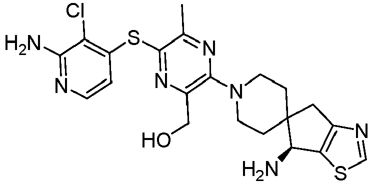

### Step 1: Preparation of ethyl 6-bromo-3-((S)-6-(((R)-tert-butylsulfinyl<sulfenyl>)amino)-4,6-dihydrospiro[cyclopent a[d]thiazole-5,4'-piperidin]-1'-yl)-5-methylpyrazine-2-carboxylate

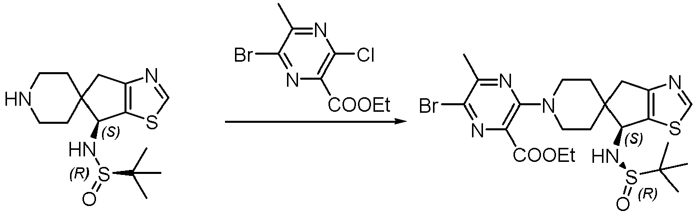

(R)-N-((S)-4,6-Dihydrospiro[cyclopenta[d]thiazole-5,4'-piperidin]-6-yl)-2-methylp ropane-2-sulfinamide (470 mg, 1.5 mmol, 1.0 eq), ethyl 6-bromo-3-chloro-5-methylpyrazine-2-carboxylate (398 mg, 1.43 mmol, 0.95 eq) and potassium carbonate (1.04 g, 7.5 mmol, 5.0 eq) were dissolved in DMF (4.0 mL). The reaction solution was heated to 60°C under a nitrogen atmosphere and stirred for 3 hours. After completion of the reaction, the reaction solution was concentrated to remove DMF. The resulting crude product was purified by column chromatography (100% EtOAc in Petro ether) to obtain the product ethyl 6-bromo-3-((S)-6-(((R)-*tert*-butylsulfinyl<sulfenyl>)amino)-4,6-dihydrospiro[cyclopent a[d]thiazole-5,4'-piperidin]-1'-yl)-5-methylpyrazine-2-carboxylate (600 mg, yield: 72%) as a yellow solid.

MS m/z (ESI): 556.1[M+H]⁺, 558.1[M+H+2]⁺.

### Step 2: Preparation of ethyl 6-((2-amino-3-chloropyridin-4-yl)thio)-3-((S)-6-(((R)-tert-butylsulfinyl<sulfenyl>)amin o)-4,6-dihydrospiro[cyclopenta[d]thiazole-5,4'-piperidin]-1'-yl)-5-methylpyrazine-2-car boxylate

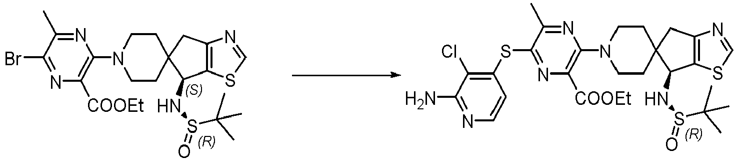

In a microwave reaction tube, ethyl 6-bromo-3-((S)-6-(((R)-*tert*-butylsulfinyl<sulfenyl>)amino)-4,6-dihydrospiro[cyclopent a[d]thiazole-5,4'-piperidin]-1'-yl)-5-methylpyrazine-2-carboxylate (600 mg, 1.08 mmol, 1.0 eq) and potassium 2-amino-3-chloropyridine-4-thiolate (536 mg, 2.70 mmol, 2.5 eq) were dissolved in 10 mL of DMF, followed by the addition of tris(dibenzylideneacetone)dipalladium (50 mg, 0.054 mmol, 0.05 eq), Xantphos (62 mg, 0.11 mmol, 0.1 eq) and DIPEA (419 mg, 3.24 mmol, 3.0 eq). The reaction solution was bubbled with nitrogen for 3 minutes, heated under microwave to 105°C and reacted for 75 minutes. The reaction solution was cooled to room temperature, diluted with 20 mL of ethyl acetate, and filtered through diatomaceous earth. The filtrate was concentrated, and the resulting crude product was purified by column chromatography (10 % MeOH in CH₂Cl₂) to obtain the product ethyl 6-((2-amino-3-chloropyridin-4-yl)thio)-3-((S)-6-(((R)-*tert*-butylsulfinyl<sulfenyl>)amin o)-4,6-dihydrospiro[cyclopenta[d]thiazole-5,4'-piperidin]-1'-yl)-5-methylpyrazine-2-car boxylate (360 mg, yield: 52%) as a yellow solid.

MS m/z (ESI): 636.0 [M+H]⁺, 638.0 [M+H+2]⁺.

### Step 3: Preparation of ethyl 6-((2-amino-3-chloropyridin-4-yl)thio)-3-((S)-6-(((R)-tert-butylsulfinyl<sulfenyl>)amin o)-4,6-dihydrospiro[cyclopenta[d]thiazole-5,4'-piperidin]-1'-yl)-5-methylpyrazine-2-car boxylate

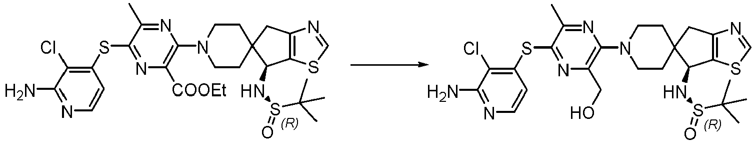

Ethyl 6-((2-amino-3-chloropyridin-4-yl)thio)-3-((S)-6-(((R)-*tert*-butylsulfinyl<sulfenyl>)amin o)-4,6-dihydrospiro[cyclopenta[d]thiazole-5,4'-piperidin]-1'-yl)-5-methylpyrazine-2-car boxylate (360 mg, 0.57 mmol, 1.0 eq) was dissolved in 40 mL of anhydrous THF, and the solution was purged with nitrogen. The solution was cooled to 0°C, and then a solution of LiAlH₄ in tetrahydrofuran (0.6 mL, 1.14 mmol, 2.0 eq., D= 2M) was added dropwise. After completion of the addition, the reaction solution was slowly warmed up to room temperature and stirred for 3 hours. After completion of the reaction, the reaction solution was diluted with ethyl acetate (100 mL), and cooled to 0°C. 2 mL of water was added dropwise, and 4 mL of 15% sodium hydroxide aqueous solution was added. 6 mL of water was added, and the reaction solution was warmed up to room temperature and stirred for 30 minutes. An appropriate amount of anhydrous sodium sulfate was added, and the reaction solution was stirred for 15 minutes. The reaction solution was filtered to remove the solid, and the filtrate was concentrated. The resulting crude product was purified by column chromatography (10 % MeOH in CH₂Cl₂) to obtain the product ethyl 6-((2-amino-3-chloropyridin-4-yl)thio)-3-((S)-6-(((R)-*tert*-butylsulfinyl<sulfenyl>)amin o)-4,6-dihydrospiro[cyclopenta[d]thiazole-5,4'-piperidin]-1'-yl)-5-methylpyrazine-2-car boxylate (150 mg, yield: 44%) as a yellow solid.

MS m/z (ESI): 594.1[M+H]⁺, 596.1[M+H+2]⁺.

### Step 4: Preparation of (S)-(6-((2-amino-3-chloropyridin-4-yl)thio)-3-(6-amino-4,6-dihydrospiro[cyclopenta[d] thiazole-5,4'-piperidin]-1'-yl)-5-methylpyrazin-2-yl)methanol

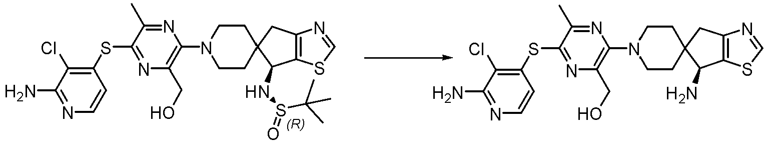

Ethyl 6-((2-amino-3-chloropyridin-4-yl)thio)-3-((S)-6-(((R)-*tert*-butylsulfinyl<sulfenyl>)amin o)-4,6-dihydrospiro[cyclopenta[d]thiazole-5,4'-piperidin]-1'-yl)-5-methylpyrazine-2-car boxylate (150 mg, 0.25 mmol, 1.0 eq) was dissolved in MeOH (5 mL). A solution of hydrochloric acid in dioxane (1.0 mL, 4M) was added, and the reaction solution was reacted at room temperature for 1 hour. The reaction solution was concentrated to dryness and dissolved in 5 mL of methanol, and the pH was adjusted to alkaline with 7M NH₃ in methanol. The reaction solution was concentrated, and purified by normal-phase column chromatography (10 % MeOH in CH₂Cl₂) and reversed-phase column chromatography (30 % MeCN in H₂O (0.1 % NH₃.H₂O)) successively to obtain the target compound (S)-(6-((2-amino-3-chloropyridin-4-yl)thio)-3-(6-amino-4,6-dihydrospiro[cyclopenta[d] thiazole-5,4'-piperidin]-1'-yl)-5-methylpyrazin-2-yl)methanol (52 mg, yield: 42%) as a grey solid.

¹H NMR (400 MHz, DMSO) δ 8.93 (s, 1H), 7.62 (s, 1H), 6.31 (s, 1H), 5.72 (s, 1H), 5.41 (s, 1H), 4.45 (s, 2H), 3.98 (s, 1H), 3.84 (s, 2H), 2.76 (dd, *J* = 47.2, 16.9 Hz, 4H), 2.37 (s, 3H), 1.60 (dd, *J* = 165.2, 116.4 Hz, 7H).

MS m/z (ESI): 490.1 [M+H]⁺, 492.1 [M+H+2]⁺.

### Example 172

### Preparation of

### (S)-(6-((2-amino-3-chloropyridin-4-yl)thio)-3-(6-amino-4,6-dihydrospiro[cyclopenta[d] oxazole-5,4'-piperidin]-1'-yl)-5-methylpyrazin-2-yl)methanol

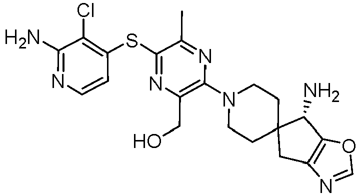

The compound of Example 172 was prepared by referring to the experimental scheme of Example 171.

MS m/z (ESI): 474.1 [M+H]⁺, 476.1 [M+2+H]⁺.

### Example 173

### Preparation of

### (R)-8-(6-amino-5-((2-amino-3-chloropyridin-4-yl)thio)pyrazin-2-yl)-2-methylene-8-aza spiro[4.5]decan-1-amine

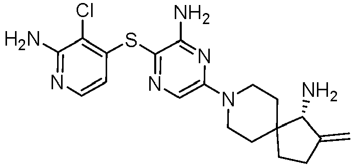

The compound of Example 173 was prepared by referring to the experimental scheme of Example 91.

MS m/z (ESI): 418.1 [M+H]⁺, 420.1 [M+2+H]⁺.

### Example 174

### Preparation of

### (S,E)-8-(6-amino-5-((2-amino-3-chloropyridin-4-yl)thio)pyrazin-2-yl)-2-ethylidene-8-a zaspiro[4.5]decan-1-amine

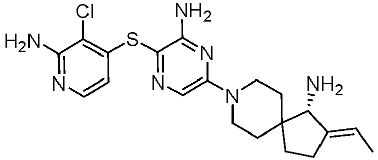

The compound of Example 174 was prepared by referring to the experimental scheme of Example 91.

MS m/z (ESI): 432.1 [M+H]⁺, 434.1 [M+2+H]⁺.

### Example 175

### Preparation of

### (S)-8-(6-amino-5-((2-amino-3-chloropyridin-4-yl)thio)pyrazin-2-yl)-2-(propan-2-yliden e)-8-azaspiro[4.5]decan-1-amine

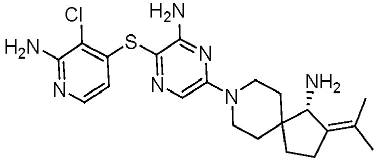

The compound of Example 175 was prepared by referring to the experimental scheme of Example 91.

MS m/z (ESI): 446.1 [M+H]⁺, 448.1 [M+2+H]⁺.

### Example 176

### Preparation of

### (R)-8-(6-amino-5-((2-aminopyrimidin-4-yl)thio)pyrazin-2-yl)-2-methylene-8-azaspiro[4 .5]decan-1-amine

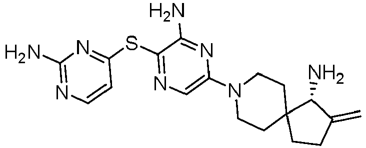

The compound of Example 176 was prepared by referring to the experimental scheme of Example 91.

MS m/z (ESI): 385.1 [M+H]⁺.

### Example 177

### Preparation of

### (S,E)-8-(6-amino-5-((2-amino-3-chloropyridin-4-yl)thio)-3-methylpyrazin-2-yl)-2-ethyl idene-8-azaspiro[4.5]decan-1-amine

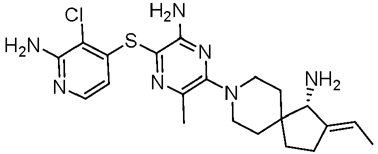

The compound of Example 177 was prepared by referring to the experimental scheme of Example 96.

MS m/z (ESI): 446.1 [M+H]⁺, 448.1 [M+2+H]⁺.

### Example 178

### Preparation of

### (R)-8-(6-amino-5-((2-amino-3-chloropyridin-4-yl)thio)-3-methylpyrazin-2-yl)-2-methyl ene-8-azaspiro[4.5]decan-1-amine

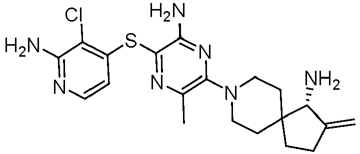

The compound of Example 178 was prepared by referring to the experimental scheme of Example 96.

MS m/z (ESI): 432.1 [M+H]⁺, 434.1 [M+2+H]⁺.

### Example 179

### Preparation of

### (S)-1'-(6-amino-5-((2-amino-3-chloropyridin-4-yl)thio)-3-fluoropyrazin-2-yl)-1,3-dihyd rospiro[indene-2,4'-piperidin]-1-amine

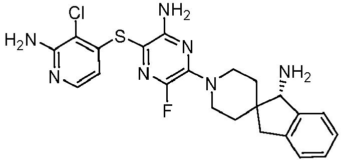

### Step 1: Preparation of (R)-N-((S)-1'-(6-amino-5-((2-amino-3-chloropyridin-4-yl)thio)pyrazin-2-yl)-1,3-dihydr ospiro[indene-2,4'-piperidin]-1-yl)-2-methylpropane-2-sulfinamide

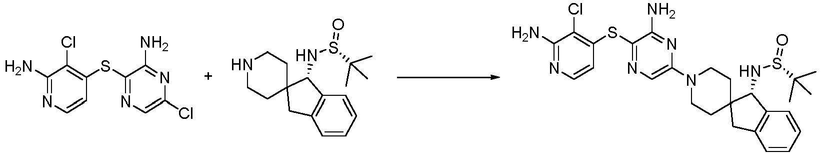

3-((2-Amino-3-chloropyridin-4-yl)thio)-6-chloropyrazin-2-amine (200 mg, 0.694 mmol), (R)-N-((S)-1,3-dihydrospiro[indene-2,4'-piperidin]-1-yl)-2-methylpropane-2-sulfinamid e (350 mg, 0.833 mmol, [α]²⁰_{D}= 1.773) and potassium carbonate (479 mg, 3.47 mmol) were stirred in N,N-dimethylformamide (7 mL) at 100°C for 8 hours. After cooling, 40 mL of water was added, and the reaction solution was extracted with ethyl acetate (40 mL×2). The ethyl acetate layer was washed with saturated sodium chloride aqueous solution (20 mL), dried over anhydrous sodium sulfate, and purified by column chromatography [eluent: dichloromethane ∼ dichloromethane/methanol from 0% to 3%] to obtain the product (R)-N-((S)-1'-(6-amino-5-((2-amino-3-chloropyridin-4-yl)thio)pyrazin-2-yl)-1,3-dihydr ospiro[indene-2,4'-piperidin]-1-yl)-2-methylpropane-2-sulfinamide (200 mg, yield: 52%) as an orange solid.

MS m/z (ESI): 558.2 [M+H]⁺.

### Step 2: Preparation of (S)-1'-(6-amino-5-((2-amino-3-chloropyridin-4-yl)thio)pyrazin-2-yl)-1,3-dihydrospiro[i ndene-2,4'-piperidin]-1-amine

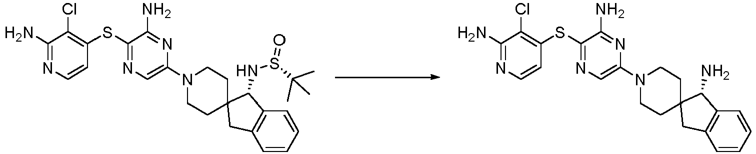

Hydrochloric acid in dioxane (20 mL, 80 mmol) was added to a solution of (R)-N-((S)-1'-(6-amino-5-((2-amino-3-chloropyridin-4-yl)thio)pyrazin-2-yl)-1,3-dihydr ospiro[indene-2,4'-piperidin]-1-yl)-2-methylpropane-2-sulfinamide (188 mg, 0.337 mmol) in methanol (5 mL), and the reaction solution was stirred at room temperature for 1 hour. The reaction solution was concentrated to dryness, and dissolved in 5 mL of methanol. The pH was adjusted to alkaline with ammonia. The reaction solution was concentrated and purified by column chromatography [eluent: dichloromethane ∼ dichloromethane/methanol from 0% to 7.5%] to obtain the product (S)-1'-(6-amino-5-((2-amino-3-chloropyridin-4-yl)thio)pyrazin-2-yl)-1,3-dihydrospiro[i ndene-2,4'-piperidin]-1-amine (130 mg, yield: 85%) as a white solid.

MS m/z (ESI): 454.2 [M+H]⁺.

### Step 3: Preparation of (S)-1'-(6-amino-5-((2-amino-3-chloropyridin-4-yl)thio)-3-fluoropyrazin-2-yl)-1,3-dihyd rospiro[indene-2,4'-piperidin]-1-amine

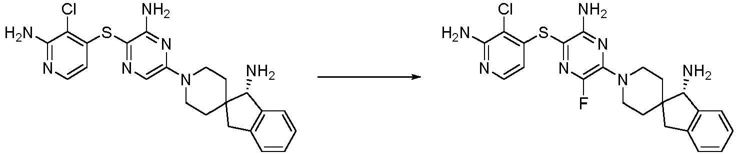

N-Fluorobisbenzenesulfonamide (69 mg, 0.22 mmol) was added to a solution of (S)-1'-(6-amino-5-((2-amino-3-chloropyridin-4-yl)thio)pyrazin-2-yl)-1,3-dihydrospiro[i ndene-2,4'-piperidin]-1-amine (100 mg, 0.22 mmol) in acetonitrile (10 mL) at -10°C. After completion of the addition, the reaction solution was slowly warmed up to room temperature and stirred for 7 hours. The reaction solution was concentrated to dryness by rotary evaporation, and the resulting residue was purified by thin layer chromatography (dichloromethane/methanol = 10/1) to obtain the product (S)-1'-(6-amino-5-((2-amino-3-chloropyridin-4-yl)thio)-3-fluoropyrazin-2-yl)-1,3-dihyd rospiro[indene-2,4'-piperidin]-1-amine (28 mg, yield: 27%) as a khaki solid.

¹H NMR (400 MHz, MeOD) δ 7.62 (d, *J* = 4 Hz, 1H), 7.42 - 7.33 (m, 1H), 7.26 - 7.15 (m, 3H), 6.00 (d, *J* = 4 Hz, 1H), 4.67 - 4.54 (m, 2H), 4.30 (d, *J* = 16 Hz, 2H), 3.97 (s, 1H), 3.16 (d, *J* = 16 Hz, 1H), 2.82 (d, *J* = 16 Hz, 1H), 1.95 - 1.78 (m, 2H), 1.65 - 1.53 (m, 1H), 1.48 - 1.38 (m, 1H).

MS m/z (ESI): 472.1 [M+H]⁺, 474.1 [M+2+H]⁺.
[α]²⁰_{D}= -36.893.

### Example 180

### (S)-1'-(6-Amino-5-((2-amino-3-chloropyridin-4-yl)thio)-3-fluoropyrazin-2-yl)-5,7-dihy drospiro[cyclopenta[b]pyridine-6,4'-piperidin]-5-amine

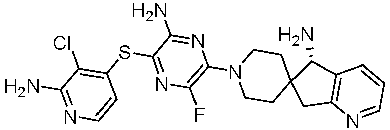

### Step 1: Preparation of 3-bromo-2-(chloromethyl)pyridine

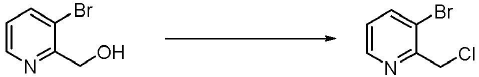

(3-Bromopyridin-2-yl)methanol (7.5 g, 40 mmol) was dissolved in dichloromethane (20 mL) and mixed well. SOCl₂ (9.5 g, 80 mmol) was added dropwise in an ice water bath, and the reaction solution was stirred for 1 hour. The reaction solution was concentrated under reduced pressure, and the resulting crude product was purified by rapid silica gel column chromatography to obtain the target compound 3-bromo-2-(chloromethyl)pyridine (5 g, yield: 61%) as a colorless liquid.

MS *m*/*z* (ESI): 205.9 [M+H]⁺.

### Step 2: Preparation of 1-(tert-butyl) 4-methyl 4-((3-bromopyridin-2-yl)methyl)piperidine-1,4-dicarboxylate

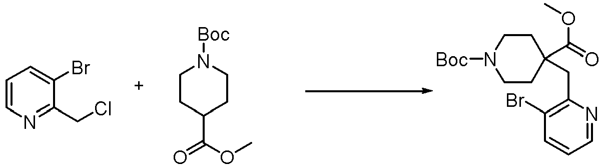

1-(*Tert*-butyl) 4-methyl piperidine-1,4-dicarboxylate (7.7 g, 31.7 mmol) and LDA (18.3 mL, 36.6 mmol) were added to THF (20 mL), and the reaction solution was reacted at -60°C for 1 hour. 3-Bromo-2-(chloromethyl)pyridine (5 g, 24.4 mmol) was added, and the reaction solution was reacted at -60°C for 2 hours. The reaction solution was concentrated under reduced pressure, and the resulting crude product was purified by rapid silica gel column chromatography to obtain the target compound 1-(*tert*-butyl) 4-methyl 4-((3-bromopyridin-2-yl)methyl)piperidine-1,4-dicarboxylate (9 g, yield: 89%) as a light yellow solid.

MS *m*/*z* (ESI):413.0 [M+H]⁺.

### Step 3: Preparation of tert-butyl 5-oxo-5,7-dihydrospiro[cyclopenta[b]pyridine-6,4'-piperidine]-1'-carboxylate

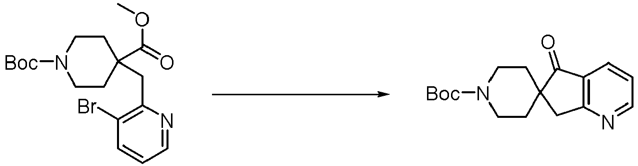

THF (100 mL) was added to 1-(*tert*-butyl) 4-methyl 4-((3-bromopyridin-2-yl)methyl)piperidine-1,4-dicarboxylate (9 g, 21.84 mmol), and the solution was cooled to -78°C. *N*-butyl lithium solution (1.6 N, 17 mL) was slowly added dropwise, and the reaction solution was reacted at -78°C for 1 hour. Ammonium chloride aqueous solution was added to quench the reaction, and the reaction solution was extracted with ethyl acetate. The organic phase was dried over anhydrous sodium sulfate and filtered, and the filtrate was concentrated under reduced pressure. The resulting crude product was purified by rapid silica gel column chromatography to obtain the target compound *tert*-butyl 5-oxo-5,7-dihydrospiro[cyclopenta[b]pyridine-6,4'-piperidine]-1'-carboxylate (3 g, yield: 45 %) as a light yellow solid.

MS *m*/*z* (ESI):303.1 [M+H]⁺.

### Step 4: Preparation of tert-butyl (R,Z)-5-((tert-butylsulfinyl<sulfenyl>)imino)-5,7-dihydrospiro[cyclopenta[b]pyridine-6 ,4'-piperidine]-1'-carboxylate

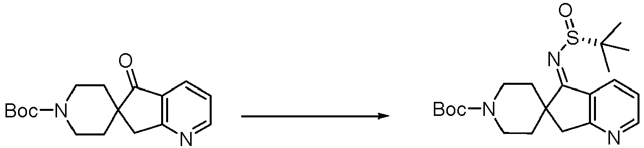

*Tert*-butyl 5-oxo-5,7-dihydrospiro[cyclopenta[b]pyridine-6,4'-piperidine]-1'-carboxylate (3 g, 9.93 mmol) and (R)-2-methylpropane-2-sulfinamide (3.6 g, 29.8 mmol) were added to ethyl titanate (50 mL). The reaction solution was stirred well, and reacted at 110°C for 13 hours. The reaction solution was cooled to room temperature, and water (100 mL) was slowly added dropwise to precipitate a solid. Ethyl acetate (200 mL) was added, and the reaction solution was filtered. The organic phase was concentrated under reduced pressure to obtain the target compound *tert*-butyl (R,Z)-5-((*tert*-butylsulfinyl<sulfenyl>)imino)-5,7-dihydrospiro[cyclopenta[b]pyridine-6 ,4'-piperidine]-1'-carboxylate (3 g, yield: 75%), which was used directly in the next step.

MS *m*/*z* (ESI): 406.1 [M+H]⁺.

### Step 5: Preparation of tert-butyl (S)-5-(((R)-tert-butylsulfinyl<sulfenyl>)amino)-5,7-dihydrospiro[cyclopenta[b]pyridine -6,4'-piperidine]-1'-carboxylate

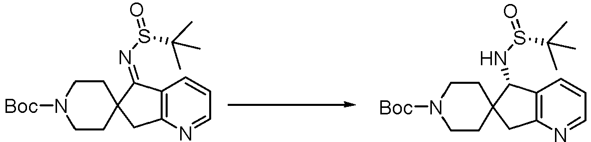

*Tert*-butyl (R,Z)-5-((*tert*-butylsulfinyl<sulfenyl>)imino)-5,7-dihydrospiro[cyclopenta[b]pyridine-6 ,4'-piperidine]-1'-carboxylate (3 g, 7.41 mmol) was added to THF (40 mL). The solution was cooled to 0°C, and BH₃ (7.5 mL, 2N in THF) was slowly added dropwise. After completion of the addition, the reaction solution was kept at 0°C and reacted for 2 hours. Methanol (10 mL) was added dropwise, and the reaction solution was concentrated under reduced pressure. The resulting crude product was purified by rapid silica gel column chromatography to obtain the target compound *tert*-butyl (S)-5-(((R)-*tert*-butylsulfinyl<sulfenyl>)amino)-5,7-dihydrospiro[cyclopenta[b]pyridine -6,4'-piperidine]-1'-carboxylate (2.5 g, yield: 83%) as a light yellow solid.

MS *m*/*z* (ESI): 408.1 [M+H]⁺.

### Step 6: Preparation of (S)-5,7-dihydrospiro[cyclopenta[b]pyridine-6,4'-piperidin]-5-amine

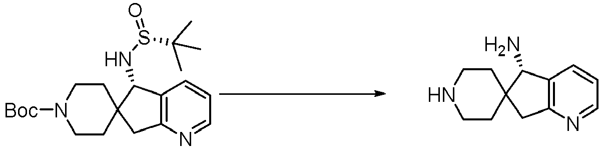

*Tert*-butyl (S)-5-(((R)-*tert*-butylsulfinyl<sulfenyl>)amino)-5,7-dihydrospiro[cyclopenta[b]pyridine -6,4'-piperidine]-1'-carboxylate (2.5 g, 6.14 mmol) and hydrochloric acid in dioxane (10 mL, 4N) were added to CH₂Cl₂ (40 mL). The reaction solution was stirred at room temperature for 3 hours, and then concentrated under reduced pressure to obtain the target compound (S)-5,7-dihydrospiro[cyclopenta[b]pyridine-6,4'-piperidin]-5-amine (1.5 g), which was used directly in the next step.

MS *m*/*z* (ESI):204.1 [M+H]⁺.

### Step 7: Preparation of (S)-1'-(6-amino-5-((2-amino-3-chloropyridin-4-yl)thio)pyrazin-2-yl)-5,7-dihydrospiro[c yclopenta[b]pyridine-6,4'-piperidin]-5-amine

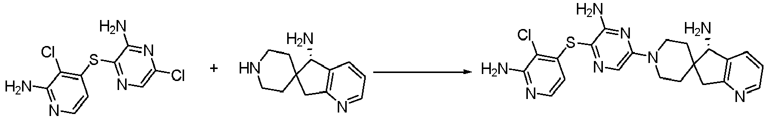

(S)-5,7-Dihydrospiro[cyclopenta[b]pyridine-6,4'-piperidin]-5-amine (1.5 g, 7.39 mmol), 3-((2-amino-3-chloropyridin-4-yl)thio)-6-bromopyrazine-2-amine (3.7 g, 11.09 mmol) and cesium carbonate (7.2 g, 22.17 mmol) were added to DMF (50 mL). The reaction solution was reacted at 90°C for 13 hours. The reaction solution was concentrated under reduced pressure, and the resulting crude product was purified by rapid silica gel column chromatography to obtain the target compound (S)-1'-(6-amino-5-((2-amino-3-chloropyridin-4-yl)thio)pyrazin-2-yl)-5,7-dihydrospiro[c yclopenta[b]pyridine-6,4'-piperidin]-5-amine (1 g, yield: 29%) as a light yellow solid.

MS *m*/*z* (ESI): 455.1 [M+H]⁺, 457.1 [M+H+2]⁺.

### Step 8: Preparation of (S)-1'-(6-amino-5-((2-amino-3-chloropyridin-4-yl)thio)-3-fluoropyrazin-2-yl)-5,7-dihyd rospiro[cyclopenta[b]pyridine-6,4'-piperidin]-5-amine

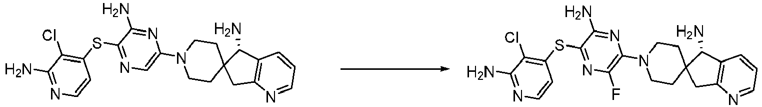

(S)-1'-(6-Amino-5-((2-amino-3-chloropyridin-4-yl)thio)pyrazin-2-yl)-5,7-dihydros piro[cyclopenta[b]pyridine-6,4'-piperidin]-5-amine (600 mg, 1.32 mmol) and NFSI (830 mg, 2.64 mmol) were added to THF (10 mL). The reaction solution was reacted at room temperature for 3 hours. The reaction solution was concentrated under reduced pressure, and the resulting crude product was purified by rapid silica gel column chromatography to obtain the target compound (S)-1'-(6-amino-5-((2-amino-3-chloropyridin-4-yl)thio)-3-fluoropyrazin-2-yl)-5,7-dihyd rospiro[cyclopenta[b]pyridine-6,4'-piperidin]-5-amine (90 mg, yield: 14%) as a white solid.

MS *m*/*z* (ESI): 473.0 [M+H]⁺, 475.1 [M+H+2]⁺.

¹H NMR (400 MHz, MeOH-*d*₄) 8 8.33 (d, *J*=4.8 Hz, 1H), 7.82 (d, *J*=7.2 Hz, 1H), 7.62 (d, *J*=5.6 Hz, 1H), 7.28-7.25 (m, 1 H), 6.01 (d, *J*=5.6 Hz, 1H), 4.35-4.31(m,2 H), 4.03 (s, 1 H), 3.36-3.31(m, 2 H),3.25-3.21 (m,1H), 2.92 (d, *J*=16.8 Hz, 1H), 1.98-1.84 (m, 2 H),1.64-1.60 (m,l H), 1.42-1.39(m, 1 H).

### Example 181

### Preparation of

### (S)-1'-(6-amino-5-((2-amino-3-chloropyridin-4-yl)thio)-3-fluoropyrazin-2-yl)-4,6-dihyd rospiro[cyclopenta[d]thiazole-5,4'-piperidin]-6-amine

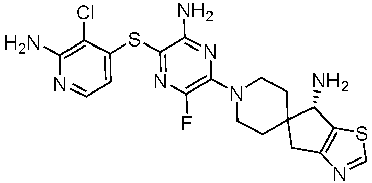

### Step 1: Preparation of (R)-N-((S)-1'-(6-amino-5-((3-chloro-2-cyclopropylpyridin-4-yl)thio)pyrazin-2-yl)-1,3-d ihydrospiro[indene-2,4'-piperidin]-1-yl)-2-methylpropane-2-sulfinamide

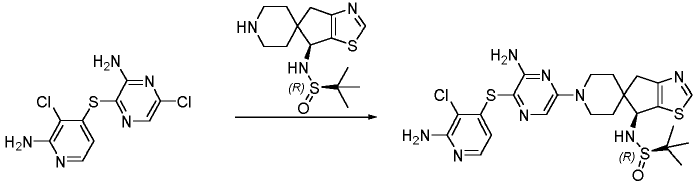

(R)-N-((S)-4,6-Dihydrospiro[cyclopenta[d]thiazole-5,4'-piperidin]-6-yl)-2-methylp ropane-2-sulfinamide (260 mg, 0.83 mmol, 1.0 eq), 3-((2-amino-3-chloropyridin-4-yl)thio)-6-chloropyrazin-2-amine (227 mg, 0.79 mmol, 0.95 eq) and potassium carbonate (574 mg, 4.15 mmol, 5.0 eq) were dissolved in DMF (2 mL). The reaction solution was heated to 105°C under a nitrogen atmosphere and stirred for 10 hours. After completion of the reaction, the reaction solution was concentrated to remove DMF. The resulting crude product was purified by column chromatography (8% MeOH in CH₂Cl₂) to obtain the product (R)-N-((S)-1'-(6-amino-5-((3-chloro-2-cyclopropylpyridin-4-yl)thio)pyrazin-2-yl)-1,3-d ihydrospiro[indene-2,4'-piperidin]-1-yl)-2-methylpropane-2-sulfinamide (340 mg, yield: 76%) as a yellow solid.

MS m/z (ESI): 565.1[M+H]⁺, 567.1[M+H+2]⁺.

### Step 2: Preparation of (S)-1'-(6-amino-5-((2-amino-3-chloropyridin-4-yl)thio)pyrazin-2-yl)-4,6-dihydrospiro[c yclopenta[d]thiazole-5,4'-piperidin]-6-amine

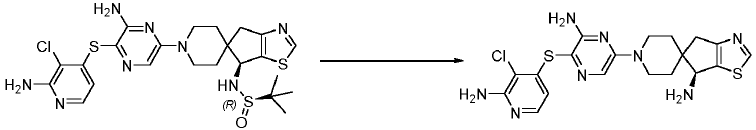

(R)-N-((S)-1'-(6-Amino-5-((3-chloro-2-cyclopropylpyridin-4-yl)thio)pyrazin-2-yl)-1,3-dihydrospiro[indene-2,4'-piperidin]-1-yl)-2-methylpropane-2-sulfinamide (340 mg, 0.60 mmol, 1.0 eq) was dissolved in MeOH (5 mL). A solution of HCl in dioxane (1.0 mL, 4M) was added, and the reaction solution was reacted at room temperature for 1 hour. The reaction solution was concentrated to dryness and dissolved in 5 mL of methanol, and the pH was adjusted to alkaline with 7M NH₃ in methanol. The reaction solution was concentrated, and purified by normal-phase column chromatography (10 % MeOH in CH₂Cl₂) and reversed-phase column chromatography (40 % MeCN in H₂O (0.1 % NH₃.H₂O)) successively to obtain the target compound (S)-1'-(6-amino-5-((2-amino-3-chloropyridin-4-yl)thio)pyrazin-2-yl)-4,6-dihydrospiro[c yclopenta[d]thiazole-5,4'-piperidin]-6-amine (220 mg, yield: 80 %) as a grey solid.

MS m/z (ESI): 461.1 [M+H]⁺, 463.1 [M+H+2]⁺.

### Step 3: Preparation of (S)-1'-(6-amino-5-((2-amino-3-chloropyridin-4-yl)thio)-3-fluoropyrazin-2-yl)-4,6-dihyd rospiro[cyclopenta[d]thiazole-5,4'-piperidin]-6-amine

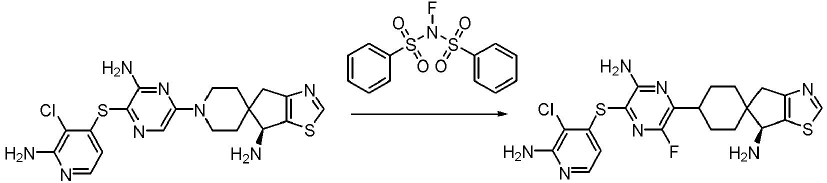

(S)-1'-(6-amino-5-((2-amino-3-chloropyridin-4-yl)thio)pyrazin-2-yl)-4,6-dihydrosp iro[cyclopenta[d]thiazole-5,4'-piperidin]-6-amine (220 mg, 0.48 mmol, 1.0 eq) was dissolved in anhydrous DMF (2 mL) and anhydrous MeCN (10 mL). The reaction solution was purged with nitrogen three times, and the reagent N-fluoro-N-(benzenesulfonyl)benzenesulfonamide (159 mg, 0.5 mmol, 1.05 eq.) was added in batches under a nitrogen atmosphere. After completion of the addition, the reaction solution was stirred at room temperature for 3 hours. The reaction solution was added dropwise to MeOH (50 mL) under stirring, and 1 mL of water was added. The reaction solution was concentrated to obtain a crude product, which was purified by preparative HPLC to obtain the product (S)-1'-(6-amino-5-((2-amino-3-chloropyridin-4-yl)thio)-3-fluoropyrazin-2-yl)-4,6-dihyd rospiro[cyclopenta[d]thiazole-5,4'-piperidin]-6-amine (40 mg, yield: 17%) as a grey solid.

¹H NMR (400 MHz, DMSO) δ 8.96 (s, 1H), 7.67 (d, *J* = 5.3 Hz, 1H), 6.28 (s, 2H), 6.17 (s, 2H), 5.86 (d, *J* = 5.4 Hz, 1H), 4.10 (s, 2H), 3.98 (s, 1H), 2.81 (dd, *J* = 52.7, 15.2 Hz, 4H), 1.88 (s, 2H), 1.75 - 1.52 (m, 4H).

¹⁹F NMR (376 MHz, DMSO) δ -88.17 (s).

MS m/z (ESI): 479.0 [M+H]⁺, 481.0 [M+H+2]⁺.

### Example 182

### Preparation of

### (S)-8-(6-amino-5-((2-amino-3-chloropyridin-4-yl)thio)-3-fluoropyrazin-2-yl)-2-cyclopr opyl-8-azaspiro[4.5]dec-2-en-1-amine

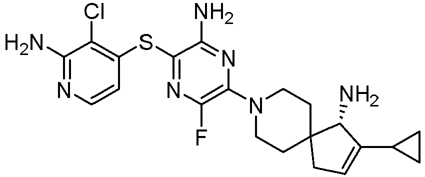

### Step 1: Preparation of (S)-8-(6-amino-5-((2-amino-3-chloropyridin-4-yl)thio)-3-fluoropyrazin-2-yl)-2-cyclopr opyl-8-azaspiro[4.5]dec-2-en-1-amine

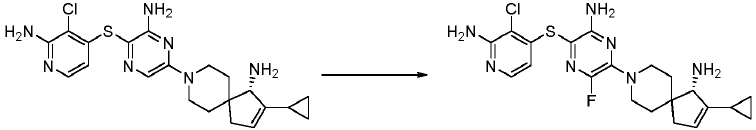

N-fluorobisbenzenesulfonamide (40 mg, 0.128 mmol) was added to a solution of (S)-8-(6-amino-5-((2-amino-3-chloropyridin-4-yl)thio)pyrazin-2-yl)-2-cyclopropyl-8-az aspiro[4.5]dec-2-en-1-amine (35 mg, 0.085 mmol) in tetrahydrofuran (18 mL) at 25°C. After completion of the addition, the reaction solution was stirred for 1 hour. TLC showed that the reaction was not complete, additional N-fluorobisbenzenesulfonamide (27 mg, 0.085 mmol) was added, and the reaction solution was stirred for another 1 hour. TLC showed that the reaction was not complete, additional N-fluorobisbenzenesulfonamide (27 mg, 0.085 mmol) was added, and the reaction solution was stirred for another 1 hour. TLC showed the presence of impurities. The reaction solution was quenched with hydrochloric acid (40 mL, 0.5 N), and washed with ethyl acetate (20 mL*2). The aqueous phase was adjusted to the pH about 8 with sodium carbonate, and extracted with ethyl acetate (30 mL*2). The organic phase was concentrated to dryness by rotary evaporation, and the resulting residue was purified by preparative chromatography (dichloromethane/methanol= 10/1) to obtain (S)-8-(6-amino-5-((2-amino-3-chloropyridin-4-yl)thio)-3-fluoropyrazin-2-yl)-2-cyclopr opyl-8-azaspiro[4.5]dec-2-en-1-amine (28 mg, yield: 27%) as a light yellow solid.

¹H NMR (400 MHz, MeOD) δ 7.61 (d, *J* = 8 Hz, 1H), 5.99 (d, *J* = 8 Hz, 1H), 5.36 (s, 1H), 4.31 - 4.13 (m, 2H), 3.45 (s, 1H), 3.30 - 3.15 (m, 2H), 2.43 - 2.24 (m, 2H), 1.89 - 1.48 (m, 5H), 0.84 - 0.65 (m, 2H), 0.57 - 0.36 (m, 2H).

MS m/z (ESI): 462.1 [M+H]⁺, 474.1 [M+2+H]⁺.

### Example 183

### Preparation of

### (S)-(3-(1-amino-1,3-dihydrospiro[indene-2,4'-piperidin]-1'-yl)-6-((2-aminopyrimidin-4-yl)thio)-5-methylpyrazin-2-yl)methanol

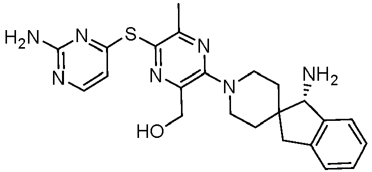

### Step 1: Preparation of 2-ethylhexyl 3-((2-((2,4-dimethoxybenzyl)amino)pyrimidin-4-yl)thio)propanoate

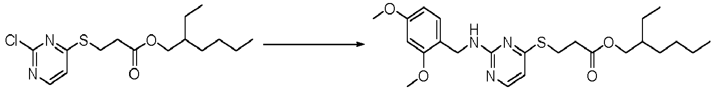

2-Ethylhexyl 3-((2-chloropyrimidin-4-yl)thio)propanoate (2.0 g, 6.06 mmol, 1.0 eq) and (2,4-dimethoxyphenyl)methylamine (1.02 g, 6.06 mmol, 1.0 eq) were dissolved in 20 mL of acetonitrile. Potassium carbonate (1.0 g, 7.24 mmol, 1.2 eq) was added, and the reaction solution was heated to 100°C and reacted for 18 hours. The reaction solution was cooled, diluted with 20 mL of ethyl acetate, and filtered through diatomaceous earth to remove insoluble substance. The filtrate was concentrated to dryness by rotary evaporation, and the resulting residue was purified by column chromatography (10∼15% ethyl acetate/petroleum ether) to obtain 2-ethylhexyl 3-((2-((2,4-dimethoxybenzyl)amino)pyrimidin-4-yl)thio)propanoate (1.5 g, yield: 54%) as a colorless oil.

MS m/z (ESI): 462.1 [M+H]⁺.

### Step 2: Preparation of potassium 2-((2,4-dimethoxybenzyl)amino)pyrimidine-4-thiolate

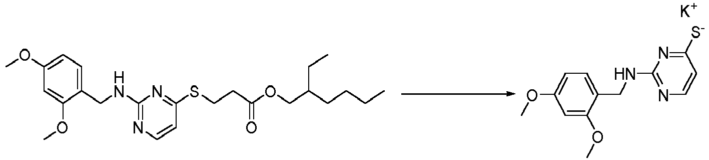

2-Ethylhexyl 3-((2-((2,4-dimethoxybenzyl)amino)pyrimidin-4-yl)thio)propanoate (1.0 g, 2.17 mmol, 1.0 eq) was dissolved in 30 mL of ethanol. Potassium *tert*-butoxide (272 mg, 2.39 mmol, 1.1 eq) was added, and the reaction solution was stirred at 40°C for 1 hour. The reaction solution was concentrated to dryness, and the resulting residue was used directly in the next step.

MS m/z (ESI): 278.1 [M+H]⁺.

### Step 3: Preparation of ethyl 3-((S)-1-(((S)-tert-butylsulfinyksulfenyl>)amino)-1,3-dihydrospiro[indene-2,4'-piperidi n]-1'-yl)-6-((2-((2,4-dimethoxybenzyl)amino)pyrimidin-4-yl)thio)-5-methylpyrazine-2-c arboxylate

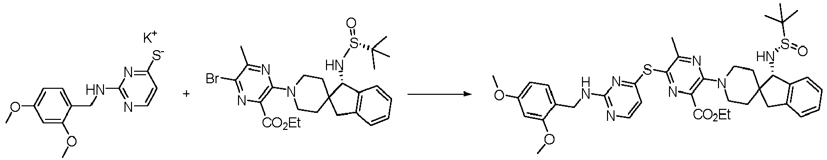

In a microwave reaction tube, potassium 2-((2,4-dimethoxybenzyl)amino)pyrimidine-4-thiolate (260 mg, 0.82 mmol, 1.0 eq) and ethyl 6-bromo-3-((S)-1-(((R)-*tert*-butylsulfinyl<sulfenyl>)amino)-1,3-dihydrospiro[indene-2, 4'-piperidin]-1'-yl)-5-methylpyrazine-2-carboxylate (450 mg, 0.82 mmol, 1.0 eq) were dissolved in 5 mL of DMF, followed by the addition of tris(dibenzylideneacetone)dipalladium (73 mg, 0.08 mmol, 0.1 eq), Xantphos (100 mg, 0.16 mmol, 0.2 eq) and DIPEA (320 mg, 2.5 mmol, 3.0 eq). The reaction solution was bubbled with nitrogen for 3 minutes, heated under microwave to 90°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (10∼80% ethyl acetate/petroleum ether) to obtain the product ethyl 3-((S)-1-(((S)-*tert-*butylsulfinyksulfenyl>)amino)-1,3-dihydrospiro[indene-2,4'-piperidi n]-1'-yl)-6-((2-((2,4-dimethoxybenzyl)amino)pyrimidin-4-yl)thio)-5-methylpyrazine-2-c arboxylate (270 mg, yield: 44%) as a yellow solid.

MS m/z (ESI): 746.1 [M+H]⁺.

### Step 4: Preparation of (S)-N-((S)-1'-(5-((2-((2,4-dimethoxybenzyl)amino)pyrimidin-4-yl)thio)-3-(hydroxymet hyl)-6-methylpyrazin-2-yl)-1,3-dihydrospiro[indene-2,4'-piperidin]-1-yl)-2-methylpropa ne-2-sulfinamide

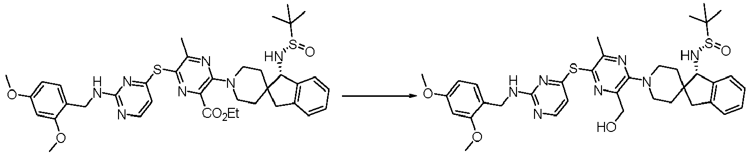

Ethyl 3-((S)-1-(((S)-*tert*-butylsulfinyksulfenyl>)amino)-1,3-dihydrospiro[indene-2,4'-piperidi n]-1'-yl)-6-((2-((2,4-dimethoxybenzyl)amino)pyrimidin-4-yl)thio)-5-methylpyrazine-2-c arboxylate (270 mg, 0.36 mmol, 1.0 eq) was dissolved in 20 mL of THF. A solution of lithium aluminum hydride in THF (0.34 mL, 0.8 mmol, 2.2 eq) was added dropwise in an ice bath. The reaction solution was stirred at 0°C under a nitrogen atmosphere for 1 hour. Na₂SO₄.10H₂O was added to quench the reaction, and the reaction solution was stirred for 10 minutes. The reaction solution was diluted with 20 mL of ethyl acetate, and filtered through diatomaceous earth. The filtrate was concentrated to dryness by rotary evaporation, and the resulting residue was purified by column chromatography (1 10% MeOH in CH₂Cl₂) to obtain (S)-N-((S)-1'-(5-((2-((2,4-dimethoxybenzyl)amino)pyrimidin-4-yl)thio)-3-(hydroxymet hyl)-6-methylpyrazin-2-yl)-1,3-dihydrospiro[indene-2,4'-piperidin]-1-yl)-2-methylpropa ne-2-sulfinamide (160 mg, yield: 64%) as a yellow solid.

MS m/z (ESI): 704.1 [M+H]⁺.

### Step 5: Preparation of (S)-(3-(1-amino-1,3-dihydrospiro[indene-2,4'-piperidin]-1'-yl)-6-((2-aminopyrimidin-4-yl)thio)-5-methylpyrazin-2-yl)methanol

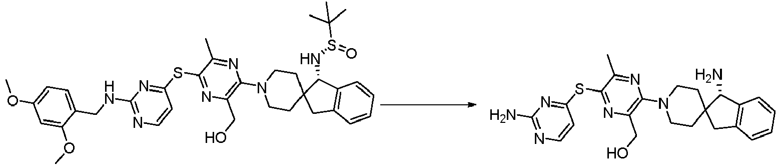

(S)-N-((S)-1'-(5-((2-((2,4-Dimethoxybenzyl)amino)pyrimidin-4-yl)thio)-3-(hydrox ymethyl)-6-methylpyrazin-2-yl)-1,3-dihydrospiro[indene-2,4'-piperidin]-1-yl)-2-methyl propane-2-sulfinamide (100 mg, 0.15 mmol, 1.0 eq) was dissolved in 5 mL of trifluoroacetic acid. The reaction solution was stirred at 60°C for 6 hours. The reaction solution was concentrated to dryness and dissolved in 5 mL of methanol, and the pH was adjusted to alkaline with 7M NH₃ in methanol. The reaction solution was concentrated to dryness by rotary evaporation, and the resulting residue was purified by column chromatography (5-10 % MeOH in CH₂Cl₂) to obtain the target product (S)-(3-(1-amino-1,3-dihydrospiro[indene-2,4'-piperidin]-1'-yl)-6-((2-aminopyrimidin-4-yl)thio)-5-methylpyrazin-2-yl)methanol (3 mg, yield: 5%) as a white solid.

MS m/z (ESI): 450.1 [M+H]⁺.

### Example 184

### Preparation of

### (S)-8-(6-amino-5-((2-amino-3-chloropyridin-4-yl)thio)-3-fluoropyrazin-2-yl)-2-methyl-8-azaspiro[4.5]dec-2-en-1-amine

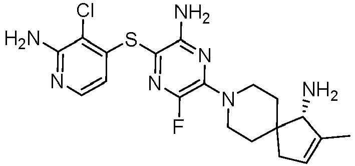

The compound of Example 184 was prepared by referring to the experimental scheme of Example 179.

MS m/z (ESI): 436.1 [M+H]⁺, 438.1 [M+2+H]⁺.

### Example 185

### Preparation of

### (S)-(3-(1-amino-2-cyclopropyl-8-azaspiro[4.5]dec-2-en-8-yl)-6-((2-amino-3-chloropyri din-4-yl)thio)-5-methylpyrazin-2-yl)methanol

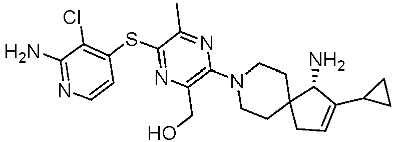

The compound of Example 184 was prepared by referring to the experimental scheme of Example 183.

MS m/z (ESI): 473.1 [M+H]⁺, 475.1 [M+2+H]⁺.

### Example 186

### Preparation of

### (S)-1'-(6-amino-5-((2-amino-3-chloropyridin-4-yl)thio)pyrazin-2-yl)-1,3-dihydrospiro[i ndene-2,4'-piperidin]-1-amine

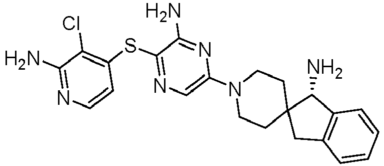

The compound of Example 186 was prepared by referring to the experimental scheme of Example 179.

¹H NMR (400 MHz, MeOD) δ 7.66 - 7.54 (m, 2H), 7.42 - 7.33 (m, 1H), 7.29 - 7.16 (m, 3H), 5.93 (d, *J* = 5.5 Hz, 1H), 4.37 - 4.19 (m, 2H), 3.98 (s, 1H), 3.29 - 3.20 (m, 2H), 3.15 (d, *J* = 16 Hz, 1H), 2.83 (d, *J* = 16 Hz, 1H), 1.91 - 1.68 (m, 2H), 1.65 - 1.53 (m, 1H), 1.50 - 1.39 (m, 1H).

MS m/z (ESI): 454.1 [M+H]⁺, 456.1 [M+2+H]⁺.

### Biological Assay and Evaluation

The present invention is further described below in combination with the following test examples, which are not intended to limit the scope of the present invention.

### Test Example 1. Determination of the inhibitory effect of the compounds of the present invention on SHP-2 kinase activity

### Experimental objective:

The objective of this test is to determine the inhibitory effect of the compounds on SHP-2 full-length protein allosteric activity.

Experimental instruments: Centrifuge (5810R, purchased from Eppendorf), pipette (purchased from Eppendorf or Rainin), and microplate reader (Model: SynergyH1 full-function microplate reader, purchased from BioTek, USA).

Experimental method: Homogeneous Full Length SHP-2 Assay Kit (BPS Bioscience, #79330) was used to determine the *in vitro* SHP-2 activity. First, 18 µL of Master Mix was added to a 96-well low adsorption microplate (NUNC, #267342), *i.e.,* the reaction buffer with a final concentration of 1× comprised 0.5 µM SHP-2 activating Peptide and 5 mM DTT. After centrifugation, 5 µL of test compound/DMSO was added to each well (the final DMSO content was 1%, V/V, the test compound was dissolved in DMSO to 1 mM, a three-fold serial dilution was carried out to obatin ten concentrations, the final concentration of the reaction system ranged from 1 µM to 0.05 nM). SHP-2 was diluted in the 1×reaction buffer to a final concentration of 0.06 nM, and the resulting solution was added to the reaction microplate (2 µL per well). Full activity control (compound only with DMSO) and full inhibition control (no SHP-2) were set up on the reaction plate. After centrifugation, the reaction mixture was incubated at room temperature for 60 minutes.

After completion of the incubation, 25 µL of Substrate solution, which comprised Substrate with a final concentration of 10 µM and 5 mM DTT, was added to each well. After centrifugation, the reaction mixture was incubated at room temperature for 30 minutes. After completion of the reaction, readings were mearsured on the Synergy HI full-function microplate reader (BioTek) (excitation wavelength: 340 nm, emission wavelength: 455 nM, gain value: 75).

### Experimental data processing method:

Based on the full activity control and full inhibition control used as the Max and Min values, the percentage inhibition rate data of the wells treated by the compounds was calculated through the positive control well (DMSO control well) and negative control well (no kinase) on the plate {% inhibition rate = 100-[(test compound value - Min average) / (Max average - Min average)] × 100}. The IC₅₀ value of the test compounds was calculated by fitting the percentage inhibition rate and data of ten concentrations to a four-parameter nonlinear logic formula with GraphPad prism.

### Experimental conclusion:

According to the above scheme, the compounds of the examples of the present invention showed the following biological activities in Table 1 in the SHP-2 kinase activity test.

**Table 1: Relative IC₅₀ value of the compounds on inhibiting SHP-2 kinase activity**

| Example No. | SHP-2 IC₅₀ (nM) |
|---|---|
| Example 91 | 17.8 |
| Example 92 | 4.17 |
| Example 93 | 4.33 |
| Example 94 | 11.7 |
| Example 95 | 6.27 |
| Example 96 | 9.28 |
| Example 97 | 13.2 |
| Example 98 | 3.93 |
| Example 99 | 29.6 |
| Example 100 | 0.98 |
| Example 101 | 5.22 |
| Example 102 | 3.75 |
| Example 103 | 0.66 |
| Example 105 | 1.56 |
| Example 143 | 3.80 |
| Example 144 | 1.48 |
| Example 151 | 7.71 |
| Example 170 | 6.81 |
| Example 171 | 1.73 |
| Example 179 | 1.75 |
| Example 180 | 9.29 |
| Example 181 | 3.07 |
| Example 182 | 3.78 |
| Example 183 | 6.70 |
| Example 184 | 4.25 |

The above data show that the compounds of the examples of the present invention have a good activity in inhibiting SHP-2 kinase activity.

### Test Example 2. Determination of the proliferation inhibitory activity of the compounds of the present invention on KYSE520 cells

The objective of this test example is to determine the proliferation inhibitory effect of the compounds of the present invention on tumor cells. The proliferation inhibitory activity of the compounds on tumor cells was determined by the CellTiter-Glo method, and the half inhibitory concentration IC₅₀ of the compounds on inhibiting cell proliferation was obtained. 2000 cells (90 µL of cell suspension) were inoculated to each well of a 96-well cell culture plate (Corning, #3610). The culture plate was incubated in an incubator overnight (37°C, 5% CO₂). On the next day, 10 µL of gradient dilutions of the test compound solution (the final DMSO content was 0.1%, V/V, the final concentration of the reaction system ranged from 100 µM to 45 nM) was added to each well of the culture plate. At the same time, full activity control (compound only with DMSO) and full inhibition control (no cells) were set up on the reaction plate. The culture plate was incubated in the incubator for 3 days (37°C, 5% CO₂).

After completion of the incubation, 50 µL of CellTiter-Glo reagent was added to each well of the cell culture plate, which was left to stand at room temperature for 10 minutes. The chemiluminescence signal value was mearsured on the Synergy HI full-function microplate reader (BioTek). Based on the full activity control and full inhibition control used as the Max and Min values, the percentage inhibition rate data of the wells treated by the compounds was calculated {% inhibition rate = 100-[(test compound value - Min average) / (Max average - Min average)] × 100}. The IC₅₀ value of the test compounds was calculated by fitting the percentage inhibition rate and data of eight concentrations to a four-parameter nonlinear logic formula with GraphPad prism.

According to the above scheme, the compounds of the present invention showed a biological activity of about 0.1 nM to 200 nM (IC₅₀) in the proliferation inhibitory activity test on KYSE520 cells.

In some embodiments, the IC₅₀ of the compounds of the present invention on inhibiting the proliferation of KYSE520 cells was less than about 200 nM, preferably less than about 100 nM, further preferably less than about 10 nM, more preferably less than about 1 nM, and most preferably less than 1 nM.

### Test Example 3. Determination of the proliferation inhibitory activity of the compounds of the present invention on NCI-H358 cells

Experimental objective: The objective of this test example is to determine the proliferation inhibitory effect of the compounds of the present invention on tumor cells.

Experimental instruments: Centrifuge (5810R, purchased from Eppendorf), carbon dioxide incubator (purchased from Thermo), biological safety cabinet (purchased from Shanghai Boxun Company), pipette (purchased from Eppendorf or Rainin), and microplate reader (Model: SynergyH1 full-function microplate reader, purchased from BioTek, USA).

Experimental method: 3000 cells (90 µL of cell suspension) were inoculated to each well of a 96-well cell culture plate (Corning, #3610). The culture plate was incubated in an incubator overnight (37°C, 5% CO₂). On the next day, 10 µL of gradient dilutions of the test compound solution (the final DMSO content was 0.2%, V/V, the final concentration of the reaction system ranged from 10 µM to 4.5 nM) was added to each well of the culture plate. At the same time, full activity control (compound only with DMSO) and full inhibition control (no cells) were set up on the reaction plate. The culture plate was incubated in the incubator for 3 days (37°C, 5% CO₂).

After completion of the incubation, 50 µL of CellTiter-Glo reagent was added to each well of the cell culture plate, which was left to stand at room temperature for 10 minutes. The chemiluminescence signal value was mearsured in the Synergy HI full-function microplate reader (BioTek). Based on the full activity control and full inhibition control used as the Max and Min values, the percentage inhibition rate data of the wells treated by the compounds was calculated {% inhibition rate = 100-[(test compound value - Min average) / (Max average - Min average)] × 100}. The IC₅₀ value of the test compounds was calculated by fitting the percentage inhibition rate and data of eight concentrations to a four-parameter nonlinear logic formula with GraphPad prism.

### Experimental data processing method:

The percentage inhibition data of the wells treated by the compounds of the examples was calculated through the vehicle control well on the plate {% inhibition rate = 100 -(test compound value / vehicle control value) × 100}. The IC₅₀ value was calculated by fitting the data of different concentrations and corresponding percentage inhibition rates to a four-parameter nonlinear logic formula with GraphPad prism.

### Experimental conclusion:

According to the above scheme, the compounds of the examples of the present invention showed the following biological activities in Table 2 in the NCI-H358 cell proliferation inhibition test.

**Table 2: IC₅₀ value of the compounds on inhibiting the proliferation of NCI-H358 cells**

| Example No. | NCI-H358 IC₅₀ (nM) |
|---|---|
| Example 91 | 37.0 |
| Example 92 | 10.5 |
| Example 93 | 31.1 |
| Example 94 | 184 |
| Example 95 | 6.39 |
| Example 96 | 13.4 |
| Example 97 | 41.87 |
| Example 98 | 18.5 |
| Example 99 | 32.8 |
| Example 100 | 24.5 |
| Example 101 | 37.7 |
| Example 102 | 42.6 |
| Example 103 | 47.0 |
| Example 105 | 10.9 |
| Example 143 | 23.3 |
| Example 144 | 9.19 |
| Example 151 | 136.7 |
| Example 170 | 25.9 |
| Example 171 | 11.9 |
| Example 179 | 20.9 |
| Example 180 | 79.9 |
| Example 181 | 46.5 |
| Example 182 | 78.5 |
| Example 183 | 105.2 |
| Example 184 | 56.5 |

The above data show that the compounds of the examples of the present invention have a good activity in inhibiting the proliferation of NCI-H358 cells.

### Test Example 4. Pharmacokinetics assay in mice

### 4.1. Study objective:

Balb/c mice were used as test animals. The pharmacokinetic behavior of the compounds of Examples 144, 171, 179, 180 and 181 after oral administration in mouse body (plasma)was studied.

### 4.2. Test protocol

### 4.2.1 Test compounds:

Compounds of Examples 144, 171, 179, 180 and 181, self-prepared;

### 4.2.2 Test animals:

Male Balb/c mice were purchased from Shanghai Jiesijie Laboratory Animal Co., LTD, with Certificate No.: SCXK (Shanghai) 2013-0006 N0.311620400001794.

### 4.2.3 Preparation of the test compounds:

5 g of hydroxyethyl cellulose (HEC, CMC-Na, viscosity: 800-1200 Cps) was dissolved in 1000 mL of purified water, and 10 g of Tween80 was added. The solution was mixed well to form a clear solution.

1.2 mg of the compounds of Examples 144, 171, 179, 180 and 181 were respectively added to a 4-mL glass bottle, followed by the addition of 2.4 mL of the above solution. The solution was subjected to ultrasound for 10 minutes to obtain a colorless and clear solution with a concentration of 0.5 mg/mL.

### 4.2.4 Administration:

After an overnight fast, male Balb/c mice were administrated orally with the test compounds at an administration dose of 5 mg/kg and an administration volume of 10 mL/kg.

### 4.2.5 Sample collection:

Blood was taken before administration and at 0.25 hour, 0.5 hour, 1 hour, 2 hours, 4 hours, 6 hours, 8 hours and 24 hours after administration. The blood samples were stored in EDTA-2K tubes, and centrifuged for 6 minutes at 6000 rpm, at 4°C to separate the blood plasma. The plasma samples were stored at -80°C. The mice were fed four hours after administration.

### 4.2.6 Test results:

The test results were determined by LCMS/MS method as shown in Table 3.

**Table 3: Pharmacokinetic parameters of the compounds in mice**

| Example No. | Tmax (hr) | Cmax (ng/mL) | AUC_{0-∞} (ng/mL*hr) | T_{1/2} (hr) | MRT (hr) |
|---|---|---|---|---|---|
| Example 144 | 1.0 | 436.0 | 5390.0 | NA | 9.08 |
| Example 171 | 0.5 | 474.7 | 2214.7 | 3.07 | 4.34 |
| Example 179 | 0.5 | 179.0 | 2441.8 | 5.23 | 7.92 |
| Example 180 | 0.5 | 996.3 | 5976.9 | 3.92 | 5.15 |
| Example 181 | 0.5 | 1383.3 | 8156.7 | 4.63 | 5.07 |

The above data show that the compounds of the examples of the present invention have a good metabolic activity at a dose of 5 mg/kg.

### Test Example 5. Pharmacokinetics assay in rats

### 5.1. Study objective:

SD rats were used as test animals. The pharmacokinetic behavior of the compounds of Examples 144, 171, 180, 179, 181 and 186 after oral administration in rat body (plasma) was studied.

### 5.2. Test protocol

### 5.2.1 Test compounds:

Compounds of Examples 144, 171, 179, 180, 181 and 186, self-prepared;

### 5.2.2 Test animals:

Male SD rats (3 rats per group) were purchased from Shanghai Jiesijie Laboratory Animal Co., LTD, with Certificate No.: SCXK (Shanghai) 2013-0006 N0.311620400001794.

### 5.2.3 Preparation of the test compounds:

5 g of hydroxyethyl cellulose (HEC, CMC-Na, viscosity: 800-1200 Cps) was dissolved in 1000 mL of purified water, and 10 g of Tween80 was added. The solution was mixed well to form a clear solution.

3.9 mg of the compounds of Examples 144, 171, 179, 180, 181 and 186 were respectively dissolved in the above solution. The solution was shaked well and subjected to ultrasound for 15 minutes to obtain a colorless and clear solution with a concentration of 0.5 mg/mL.

### 5.2.4 Administration:

After an overnight fast, male SD rats (3 rats per group) were administrated orally with the test compounds at an administration dose of 5 mg/kg and an administration volume of 10 mL/kg.

### 5.2.5 Sample collection:

0.2 mL of blood was taken from jugular vein before administration and at 0.5 hour, 1.0 hour, 2.0 hours, 4.0 hours, 6.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The rats were fed four hours after administration.

### 5.3 Test results: The test results were determined by LCMS/MS method as shown in Table 4.

**Table 4: Pharmacokinetic parameters of the compounds of the present invention in rats**

| Example No. | Tmax (hr) | Cmax (ng/mL) | A UC_{0-∞} (ng/mL*hr) | T_{1/2} (hr) | MRT (hr) |
|---|---|---|---|---|---|
| Example 144 | 1.0 | 247.3 | 3305.3 | 6.49 | 9.24 |
| Example 171 | 1.0 | 189.0 | 1783.0 | 3.69 | 5.78 |
| Example 179 | 2.0 | 201.0 | 3842.0 | 10.51 | 15.15 |
| Example 180 | 1.0 | 666.3 | 6252.6 | 4.12 | 5.67 |
| Example 181 | 2.0 | 614.3 | 5891.6 | 3.55 | 5.84 |
| Example 186 | 2.0 | 113.8 | 1537.4 | 5.11 | 7.52 |

**Experimental conclusion:** The data in the table show that the compounds of the examples of the present invention reached a relatively high exposure in rat plasma after oral administration at a dose of 5 mg/kg. In addtion, Perianal pollution was observed in rats administered with the compound of Example 186, indicating that the compound of Example 186 has a gastrointestinal toxicity.

### Test Example 6. Tumor inhibition test on MiaPaca 2 xenograft model

### 6.1 Experimental objective:

Balb/c nude mice were used as test animals. *In vivo* pharmacodynamic test was carried out on human pancreatic cancer cell MiaPaca 2 xenograft (CDX) model to evaluate the anti-tumor effect of the test compounds.

### 6.2 Experimental instruments and reagents:

### 6.2.1 Instruments:

Ultra-clean workbench (BSC-1300II A2, Shanghai Boxun Medical Biological Instrument Corp.)
CO₂ incubator (Thermo-311, Thermo)
Centrifuge (Centrifuge 5720R, Eppendorf)
Automatic cell counter (Countess II, Life Technologies)
Pipette (10-20 µL, Eppendorf)
Microscope (Ts 2, Nikon)
Vernier caliper (CD-6"AX, Mitutoyo, Japan)
Cell culture flask (T25/T75/T225, Corning)
Constant temperature water tank (HWS12, Shanghai YIHENG Technical Co., Ltd.)

### 6.2.2 Reagents:

DMEM (11995-065, Gibco)
Fetal Bovine Serum (FBS) (10091-148, Gibco)
0.25% Trypsin (25200-056, Gibco)
Penicillin-streptomycin double antibiotics (P/S) (SV30010, GE) Phosphate buffered saline (PBS) (10010-023, Gibco)
Matrigel (356234, Corning)
Gln (25030-081, Gibco)

### 6.3 Experimental process:

MiaPaca 2 cells were taken from the cell bank and thawed. The cells were added to the DMEM medium (containing 10% FBS, 1% Glu and 1% P/S), and incubated in the CO₂ incubator (the temperature of the incubator was 37°C, and the CO₂ concentration was 5%). After the cells covered 80-90% of the bottom of the culture flask, the cells were passaged. After passage, the cells continued to be incubated in the CO₂ incubator. The process was repeated until the number of cells met the inoculation requirement of the *in vivo* pharmacodynamic test. The cells in logarithmic growth phase were collected, and counted by the automatic cell counter. According to the counting results, the cells were resuspended with PBS and Matrigel (volume ratio: 1:1) to obtain a cell suspension (density: 8×10⁷/ml), which was placed in an ice box for later use.

Female Balb/c nude mice (6-8 weeks old, body weight: about 18-22 grams) were used as test animals. The mice were maintained in an environment free of special pathogens and in a single ventilated cage (five mice per cage). All cages, bedding and water were disinfected before use. All animals had free access to standard certified commercial laboratory animal diets. The nude mice were marked with a disposable universal ear tag for rats and mice before the test. The skin of the inoculation site was disinfected with 75% medical alcohol before the inoculation. Each mouse was inoculated with 0.1 ml of MiaPaca 2 tumor cells (containing 8*10⁶ cells) subcutaneously on the right back. When the average tumor volume reached 100 to 200 mm³, administration was carried out. The test compounds were administered intragastrically daily. The dose, frequency of administration and the efficacy of each group at the end of the test are shown in Table 5. The tumor volume (mm³) was measured with the vernier caliper twice a week. The tumor volume calculation formula: V=0.5*D*d*d, wherein D and d represent the long diameter and short diameter of the tumor, respectively. The anti-tumor efficacy was determined by dividing the average tumor increase volume of the compound-treated animals by the average tumor increase volume of the untreated animals. Tumor growth inhibition rate calculation formula: TGI(%)=1-[(Vt-V0) administration group /(Vt-V0) vehicle control group]*100%. All animals were euthanized after the experiment.

### 6.4 Experimental results:

**Table 5: Pharmacodynamic parameters of the compounds in mice with xenograft**

| **Group** | **Tumor volume (mm³, Mean ± SD)** | | **ΔT/ΔC (%)** | **TGI (%)** |
|---|---|---|---|---|
| | **Day 0** | **Day 21** | **Day 21** | **Day 21** |
| Example 96 6 mg/kg QD x 3w | 170±28 (171±25) | 183±70 (780±248) | 2.24 | 97.76 |
| Example 143 6 mg/kg QD x 3w | 166±27 (168±32) | 196±62 (847±287) | 4.36 | 95.64 |
| Example 144 3 mg/kg QD x 3w | 172±26 (173±28) | 216±66 (1064±236) | 4.93 | 95.07 |
| Example 171 6 mg/kg QD x 3w | 167±26 (168±32) | 198±70 (847±287) | 4.57 | 95.43 |
| Example 179 6 mg/kg QD x 3w | 166±28 (168±32) | 227±37 (847±287) | 9.00 | 91.00 |
| Example 181 10 mg/kg QD x 3w | 176±36 (176±39) | 240±86 (1078±395) | 7.06 | 92.94 |

| | | | | |
|---|---|---|---|---|
| Note: The data in parentheses refers to the tumor volume of the Vehicle QD x 3w group (*i.e.* the control group) corresponding to this Example at the corresponding time (*i.e.* Day 0 and Day 21, respectively). | | | | |

Experimental conclusion: The above data show that after 21 days of continuous oral administration, the compounds of the examples of the present invention can significantly inhibit the growth of MiaPaca 2 xenograft in nude mice under the administration condition of 3-10 mg/kg QD.

### Test Example 7. Pharmacokinetics assay in tumor-bearing mice

### 7.1. Study objective:

MiaPaca 2 tumor-bearing mice were used as test animals. The pharmacokinetic behavior of the compound of Example 179 after oral administration at a dose of 6 mg/kg in mouse body (plasma, tumor tissue and intestine) was studied.

### 7.2. Test protocol

### 7.2.1 Test compound:

Compound of Example 179, self-prepared.

### 7.2.2 Test animals:

Twenty-four female MiaPaca 2 tumor-bearing mice were used. Three mice were used for each time point (0 hour, 1 hour, 2 hours, 4 hours, 6 hours, 8 hours, 16 hours and 24 hours). The mice were purchased from Shanghai Sippr-BK laboratory animal Co. Ltd., with Certificate No.: SCXK (Shanghai) 2018-0006.

### 7.2.3 Preparation of the test compound:

5 g of hydroxymethyl cellulose was dissolved in 1000 mL of purified water, and 10 g of Tween80 was added. The solution was mixed well to form a clear solution.

4.57 mg of the compound of Example 179, 22.6 mg was dissolved in the above solution. The solution was shaked well, and subjected to ultrasound for 15 minutes to obtain a uniform suspension with a concentration of 0.6 mg/mL.

### 7.2.4 Administration:

After fast, MiaPaca 2 tumor-bearing mice were administered orally (p.o.) with the test compound according to body weight at an administration dose of 6 mg/kg and an administration volume of 10 mL/kg (the animals were not administered with the test compound on 0 hour).

### 7.2.5 Sample collection:

The mice were euthanized by CO₂ before administration and after administration, and 0.5 mL of blood was taken from heart. The blood samples were stored in EDTA-2K tubes, and centrifuged for 6 minutes at 6000 rpm, at 4°C to separate the blood plasma. The plasma samples were stored at -80°C. The tumor tissue was weighed, placed in a 2 mL centrifuge tube, and stored at -80°C. An appropriate length of duodenum, ileum and colon tissues were taken and cut open with scissors, and the contents were removed. The above tissues were washed with PBS twice, and the moisture was absorbed with absorbent paper. The tissues were weighed, placed in a 2 mL centrifuge tube, and stored at -80°C.

### 7.3 Test results: The test results were determined by LCMS/MS method as shown in Table 6.

**Table 6: Pharmacokinetic parameters of the compound of the present invention in mice**

| Compound No. | | Tmax (hr) | Cmax (ng/mL) | A UC_{0-∞} (ng/mL*hr) | T_{1/2} (hr) | MRT (hr) |
|---|---|---|---|---|---|---|
| Example 179 | Plasma | 1.0 | 385.7 | 2857.0 | 4.8 | 7.5 |
| | Tumor | 8.0 | 2388.3 | 51774.2 | 11.5 | 19.1 |
| | Duodenum | 1.0 | 5416.7 | 16252.0 | 4.8 | 4.8 |
| | Ileum | 2.0 | 1928.3 | 10008.3 | 5.3 | 6.1 |
| | Colon | 4.0 | 1310.0 | 9182.5 | 2.8 | 5.8 |

Experimental conclusion: The data in the table show that at a dose of 6 mg/kg, the exposure of the compound of the example of the present invention in mice tumor reached a very high level, and the exposure in tumor was significantly higher than that in blood. It can be seen from Tmax and MRT that the concentration of the compound in tumor gradually increased and the T1/2 was longer, indicating that the compound will gradually accumulate in tumor and always maintain a higher concentration in tumor, thereby ensuring a better tumor inhibition effect.

### Test Example 8. Test of inhibitory activity on hERG potassium channel

### 8.1 Cell preparation

8.1.1 CHO-hERG cells were cultured in a 175 cm² culture flask. When the cells were grown to a density of 60 to 80%, the culture solution was removed, and the cells were washed once with 7 mL of PBS, and then 3 mL of Detachin was added for digestion of the cells.
8.1.2 After the digestion was complete, the cells were neutralized by adding 7 mL of the culture solution and then centrifuged. The supernatant was removed, and then 5 mL of the culture solution was added for resuspension to ensure a cell density of 2∼5×10⁶ cells/mL.

### 8.2 Solution preparation

**Table 7: Components of intracellular fluid and extracellular fluid**

| Reagent | Extracellular fluid (mM) | Intracellular fluid (mM) |
|---|---|---|
| CaCl₂ | 2 | 5.374 |
| MgCl₂ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity∼305 mOsm | 7.25 (adjusted with KOH), Osmolarity∼290 mOsm |

### 8.3 Electrophysiological recording process

The single-cell high-impedance sealing and whole-cell pattern formation processes were both completed automatically by the Qpatch instrument. After the whole-cell recording pattern was obtained, the cells were clamped at -80 millivolts. A pre-voltage of -50 millivolts was applied for 50 milliseconds prior to the application of a depolarizing stimulus of +40 millivolts for 5 seconds, and then the cells were repolarized to -50 millivolts and maintained for 5 seconds, and then returned back to -80 millivolts. This voltage stimulation was applied every 15 seconds, and data were recorded for 2 minutes, and thereafter the extracellular fluid was administered and data were recorded for 5 minutes, and then the administration process was started. The compound was administered from the lowest test concentration, and 2.5 minutes were given for each test concentration. After all the concentrations of the compound were administered, 3 M Cisapride as a positive control compound was administered. At least three cells (n≥3) were tested for each concentration.

### 8.4 Compound preparation

8.4.1 20 mM compound mother solution was diluted with the extracellular fluid. 5 µL of the 20 mM compound mother solution was diluted 500 times to 40 µM by adding 2495 µL of the extracellular fluid, and then 3-fold serial dilutions were performed sequentially in the extracellular fluid containing 0.2% DMSO to obtain a final concentration to be tested.
8.4.2 The highest test concentration was 40 µM, and there were six concentrations in total, *i.e.,* 40, 13.33, 4.44, 1.48, 0.49, and 0.16 µM, respectively.
8.4.3 The DMSO content in the final test concentration was not more than 0.2%. This concentration of DMSO had no effect on the hERG potassium channel.

### 8.5 Data analysis

The experimental data were analyzed by XLFit software.

### 8.6 Quality control

Environment: Humidity 20 to 50% and temperature 22 to 25°C.

Reagents: The experimental reagents used were purchased from Sigma, with a purity >98%.

The experimental data in the report must meet the following criteria:
Whole cell sealing impedance > 100 MΩ
Tail current amplitude > 400 pA

### Pharmacological parameters:

The inhibitory effect of multiple concentrations of Cisapride on hERG channel was set as a positive control.

### 8.7 Experimental results

The inhibitory effect of the compounds of the examples at multiple concentrations on hERG current is as follows:

**Table 8: Inhibitory effect of the compounds of the examples at multiple concentrations on hERG current**

| Example No. | hERG (uM) |
|---|---|
| Example 96 | 5.24 |
| Example 143 | 6.17 |
| Example 144 | 2.34 |
| Example 171 | 18.78 |
| Example 179 | 4.51 |
| Example 181 | 21.91 |

Experimental conclusion: The inhibition of a drug on the cardiac hERG potassium channel is the main reason that the drug causes QT prolongation syndrome. It can be seen from the experimental results that the compounds of the examples of the present invention have no obvious inhibitory effect on cardiac hERG potassium channel, thereby avoiding cardiotoxic side effects at high doses.

### Test Example 9. Pharmacokinetics assay in mice

### 9.1. Study objective:

Balb/c mice were used as test animals. The pharmacokinetic behavior of the compounds of Examples 179 and 186 after single intravenous injection in mouse body (plasma) was studied.

### 9.2. Test protocol

### 9.2.1 Test compounds:

Compounds of Examples 179 and 186, self-prepared;

### 9.2.2 Test animals:

Male Balb/c mice were purchased from Shanghai Jiesijie Laboratory Animal Co., LTD, with Certificate No.: SCXK (Shanghai) 2013-0006 N0.311620400001794.

### 9.2.3 Preparation of the test compounds:

Aqueous solution containing 10% Solutol HS15: 10 g of Solutol HS15 was added to purified water for injection, and dissolved in it under stirring. The solution was diluted to 100 mL with purified water for injection and shaked well to obtain the desired solution.

Aqueous solution containing 20% HP-β-CD: 20 g of HP-β-CD was added to purified water for injection, and dissolved in it under stirring. The solution was diluted to 100 mL with purified water for injection and shaked well to obtain the desired solution.

0.5 ml of DMSO, 8.5 ml of 20% HP-β-CD aqueous solution and 1 ml of 10% Solutol HS15 aqueous solution were mixed and shaked well to obtain a clear solution.

0.234 mg of the compounds of Examples 179 and 186 were weighed and added to 1.17 mL of the above solution. The solution was subjected to ultrasound to obtain a clear solution. The solution was filtered with a 0.22 µm water filter, and the filtrate was the formulation for intravenous administration (i.v.). The concentration was 0.2 mg/mL.

### 9.2.4 Administration:

After an overnight fast, male Balb/c mice were administered intravenously with the test compounds at an administration dose of 1 mg/kg and an administration volume of 5 mL/kg.

### 9.2.5 Sample collection:

Blood was taken before administration and at 0 minute, 5 minutes, 15 minutes, 0.5 hour, 1.0 hour, 2.0 hours, 4.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The mice were fed four hours after administration.

### 9.3 Test results: The test results were determined by LCMS/MS method as shown in Table 9.

**Table 9: Pharmacokinetic parameters of the compounds in mice**

| Example No. | C₀ (ng/mL) | A UC_{0-∞} (ng/mL*hr) | T_{1/2} (hr) | MRT (hr) | CL (mL/min/kg) | Vss (L/kg) | F (%) |
|---|---|---|---|---|---|---|---|
| Example 179 | 264.5 | 736.0 | 7.74 | 9.04 | 22.6 | 12.3 | 71 |
| Example 186 | 257.4 | 404.0 | 5.81 | 5.96 | 41.3 | 14.7 | 150 |

Experimental conclusion: the compounds of the examples of the present invention have good metabolic characteristics in mouse body at a dose of 1 mg/kg.

### Test Example 10. Pharmacokinetics assay in rats

### 10.1. Study objective:

SD rats were used as test animals. The pharmacokinetic behavior of the compounds of Examples 179 and 186 after single intravenous injection in rat body (plasma) was studied.

### 10.2. Test protocol

### 10.2.1 Test compounds:

Compounds of Examples 179 and 186, self-prepared.

### 10.2.2 Test animals:

Male SD rats (3 rats per group) were purchased from Shanghai Jiesijie Laboratory Animal Co., LTD, with Certificate No.: SCXK (Shanghai) 2013-0006 N0.311620400001794.

### 10.2.3 Preparation of the test compounds:

Aqueous solution containing 10% Solutol HS15: 10 g of Solutol HS15 was added to purified water for injection, and dissolved in it under stirring. The solution was diluted to 100 mL with purified water for injection and shaked well to obtain the desired solution.

Aqueous solution containing 20% HP-β-CD: 20 g of HP-β-CD was added to purified water for injection, and dissolved in it under stirring. The solution was diluted to 100 mL with purified water for injection and shaked well to obtain the desired solution.

0.5 ml of DMSO, 8.5 ml of 20% HP-β-CD aqueous solution and 1 ml of 10% Solutol HS15 aqueous solution were mixed and shaked well to obtain a clear solution.

0.78 mg of the compounds of Examples 179 and 186 were weighed and added to 3.9 mL of the above solution. The solution was subjected to ultrasound to obtain a clear solution. The solution was filtered with a 0.22 µm water filter, and the filtrate was the formulation for intravenous administration (i.v.). The concentration was 0.2 mg/mL.

### 10.2.4 Administration:

After an overnight fast, male SD rats (3 rats per group) were administered intravenously with the test compounds at an administration dose of 1 mg/kg and an administration volume of 5 mL/kg.

### 10.2.5 Sample collection:

0.2 mL of blood was taken from jugular vein before administration and at 0 minute, 5 minutes, 15 minutes, 0.5 hour, 1.0 hour, 2.0 hours, 4.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The rats were fed four hours after administration.

### 10.3 Test results: The test results were determined by LCMS/MS method as shown in Table 10.

**Table 10: Pharmacokinetic parameters of the compounds of the present invention in rats**

| Example No. | C₀ (ng/mL) | AUC_{0-∞} (ng/mL*hr) | T_{1/2} (hr) | MRT (hr) | CL (mL/min/kg) | Vss (L/kg) | F (%) |
|---|---|---|---|---|---|---|---|
| Example 179 | 311.1 | 612.6 | 3.35 | 4.26 | 27.2 | 7.0 | 118 |
| Example 186 | 516.5 | 427.6 | 1.83 | 1.79 | 39.0 | 4.2 | 72 |

Experimental conclusion: the compounds of the examples of the present invention have good metabolic characteristics in rat body at a dose of 1 mg/kg.

### Test Example 11. Tolerance assay in mice

### 11.1. Study objective:

Balb/c mice were used as test animals. The tolerance of the compounds of Examples 179 and 186 after oral administration in female and male mice was studied.

### 11.2. Test protocol

### 11.2.1 Test compounds:

Compounds of Examples 179 and 186, self-prepared.

### 11.2.2 Test animals:

Balb/c mice (9 male and 9 female) were purchased from Shanghai Jiesijie Laboratory Animal Co., LTD, with Certificate No.: SCXK (Shanghai) 2013-0006 N0.311620400001794.

### 11.2.3 Preparation of the test compounds:

5 g of hydroxyethyl cellulose (HEC, CMC-Na, viscosity: 800-1200 Cps) was dissolved in 1000 mL of purified water, and 10 g of Tween80 was added. The solution was mixed well to form a clear solution.

41.0 mg of the compound of Example 179 and 25.9 mg of the compound of Example 186 were respectively added to a 100-mL glass bottle, followed by the addition of 41.0 mL and 25.9 mL of the above solution. The solution was subjected to ultrasound for 10 minutes to obtain a colorless and clear solution with a concentration of 1 mg/mL.

### 11.2.4 Administration:

After an overnight fast, Balb/c mice (3 male and 3 female) were administrated orally with the test compound at an administration dose of 10 mg/kg and an administration volume of 10 mL/kg.

### 11.2.5 Data collection:

The mice were weighed daily before and after administration, and abnormal responses were observed. The mice were fed four hours after administration.

### 11.2.6 Test results:

The body weight was weighed with a balance, and the test results were shown in Table 11.

**Table 11: Comparison of mouse body weight changes**

| **Group** | **Body weight (g, Mean)** | | **Body weight changes ΔW/W1 (%, Mean)** |
|---|---|---|---|
| | **Day 1** | **Day 16** | **Day 16** |
| Control group QD male | 22.99 | 27.34 | 18.98 |
| Example 179 10 mg/kg QD male | 23.01 | 28.30 | 23.25 |
| Example 186 10 mg/kg QD male | 23.03 | 21.57 | -8.02 |
| Control group QD female | 22.00 | 23.77 | 7.99 |
| Example 179 10 mg/kg QD female | 22.07 | 25.09 | 13.80 |
| Example 186 10 mg/kg QD female | 22.10 | 18.74 | -15.32 |

Experimental conclusion: The above data show that the compounds of the examples of the present invention have a good tolerance in mice at a dose of 10 mg/kg. However, the mice administered with the compound of Example 186 showed loose stools, blood in the stool, significant weight loss and the like, indicating that this compound has a poor tolerance.

In summary, the present invention provides a series of highly active and selective SHP2 kinase inhibitors with novel structures. These inhibitors show good pharmacokinetic characteristics in both rats and mice, and also show good efficacy on Miapaca2 tumor-bearing mouse model. These inhibitors have great potential to be developed into single drugs or combination drugs for treating a tumor disease.

## Claims

1. A compound of formula (I), a stereoisomer thereof or a pharmaceutically acceptable salt thereof: 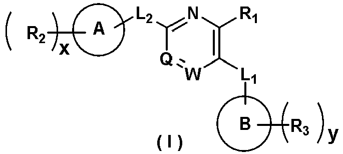 wherein:
W is selected from the group consisting of CR₄ and N;
Q is selected from the group consisting of CR₅ and N;
L₁ is selected from the group consisting of a bond, oxygen, sulfur, alkylene, alkenyl, alkynyl, cycloalkyl, heterocyclyl and -NRₐₐ-;
L₂ is selected from the group consisting of a bond, oxygen and sulfur;
ring A is selected from the group consisting of cycloalkyl, heterocyclyl, aryl and heteroaryl, wherein the cycloalkyl, heterocyclyl, aryl and heteroaryl are each optionally further substituted by one or more substituent(s) selected from the group consisting of deuterium, alkyl, haloalkyl, halogen, amino, oxo, nitro, cyano, hydroxy, alkenyl, alkynyl, alkoxy, haloalkoxy, hydroxyalkyl, cycloalkyl, heterocyclyl, aryl, heteroaryl, -(CH₂)ₙ₁-, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, -(CH₂)ₙ₁NRₐₐC(O)R_{bb} and -(CH₂)ₙ₁NRₐₐS(O)ₘ₁R_{bb};
ring B is selected from the group consisting of cycloalkyl, heterocyclyl, aryl and heteroaryl, wherein the cycloalkyl, heterocyclyl, aryl and heteroaryl are each optionally further substituted by one or more substituent(s) selected from the group consisting of deuterium, alkyl, haloalkyl, halogen, amino, oxo, nitro, cyano, hydroxy, alkenyl, alkynyl, alkoxy, haloalkoxy, hydroxyalkyl, cycloalkyl, heterocyclyl, aryl, heteroaryl, -(CH₂)ₙ₁-, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, -(CH₂)ₙ₁NRₐₐC(O)R_{bb} and -(CH₂)ₙ₁NRₐₐS(O)ₘ₁R_{bb};
R₁ is selected from the group consisting of hydrogen, deuterium, alkyl, deuterated alkyl, haloalkyl, alkoxy, haloalkoxy, hydroxyalkyl, halogen, amino, nitro, hydroxy, cyano, alkenyl, alkynyl, cycloalkyl, heterocyclyl, oxoheterocyclyl, thioxoheterocyclyl, aryl, heteroaryl, -(CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, -N=S=O(RₐₐR_{bb}), -P(O)RₐₐR_{bb}, -(CH₂)ₙ₁NRₐₐC(O)R_{bb} and -(CH₂)ₙ₁NRₐₐS(O)ₘ₁R_{bb}, wherein the alkyl, deuterated alkyl, haloalkyl, hydroxyalkyl, alkoxy, haloalkoxy, amino, alkenyl, alkynyl, cycloalkyl, heterocyclyl, aryl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
R₂ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb};
or, two R₂ on the same carbon atom or different carbon atoms are bonded to form a cycloalkyl, heterocyclyl, aryl or heteroaryl, wherein the cycloalkyl, heterocyclyl, aryl and heteroaryl are each optionally further substituted by one or more substituent(s) selected from the group consisting of deuterium, substituted or unsubstituted alkyl, substituted or unsubstituted cycloalkyl, substituted or unsubstituted haloalkyl, halogen, substituted or unsubstituted amino, oxo, thioxo, nitro, cyano, hydroxy, alkoxycarbonyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted heterocyclyl, substituted or unsubstituted aryl, substituted or unsubstituted heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb};
R₃ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb}, wherein the alkyl, deuterated alkyl, haloalkyl, alkoxy, hydroxyalkyl, haloalkoxy, amino, alkenyl, alkynyl, cycloalkyl, heterocyclyl, aryl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
or, two R₃ on the same carbon atom or different carbon atoms are bonded to form a cycloalkyl, heterocyclyl, aryl or heteroaryl, wherein the cycloalkyl, heterocyclyl, aryl and heteroaryl are each optionally further substituted by one or more substituent(s) selected from the group consisting of deuterium, substituted or unsubstituted alkyl, substituted or unsubstituted cycloalkyl, substituted or unsubstituted haloalkyl, halogen, substituted or unsubstituted amino, oxo, thioxo, nitro, cyano, hydroxy, alkoxycarbonyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted heterocyclyl, substituted or unsubstituted aryl, substituted or unsubstituted heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb};
R₄ and R₅ are each independently selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, -(CH₂)ₙ₁C(O)NHRₐₐ, -(CH₂)ₙ₁NRₐₐC(O)R_{bb} and -(CH₂)ₙ₁NRₐₐS(O)ₘ₁R_{bb};
Rₐₐ, R_{bb}, R_{cc} and R_{dd} are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are each optionally further substituted by one or more substituent(s) selected from the group consisting of deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

2. The compound, the stereoisomer thereof or the pharmaceutically acceptable salt thereof according to claim 1, **characterized in that** the formula (I) is further as shown in formula (II): 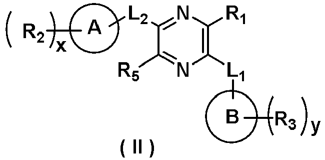 wherein:
L₁ is selected from the group consisting of a bond, oxygen, alkenyl, alkynyl, heterocyclyl and -NRₐₐ-;
L₂ is selected from the group consisting of a bond and sulfur;
ring A is selected from the group consisting of aryl and heteroaryl;
ring B is selected from the group consisting of cycloalkyl, heterocyclyl, aryl and heteroaryl;
R₁ is selected from the group consisting of hydrogen, halogen, alkyl, hydroxyalkyl, heterocyclyl, heteroaryl, hydroxyalkyl and -(CH₂)ₙ₁ORₐₐ, wherein the alkyl, hydroxyalkyl, heterocyclyl and heteroaryl are each optionally further substituted by one or more substituent(s) selected from the group consisting of hydrogen, deuterium, alkyl, deuterated alkyl, haloalkyl, alkoxy, hydroxyalkyl, haloalkoxy, oxo, halogen, amino, nitro, hydroxy, cyano, alkenyl, alkynyl, cycloalkyl, heterocyclyl, aryl and heteroaryl;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₅ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

3. The compound, the stereoisomer thereof or the pharmaceutically acceptable salt thereof according to claim 1, **characterized in that** the formula (I) is further as shown in formula (III): 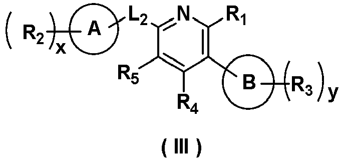 wherein:
L₂ is selected from the group consisting of a bond and sulfur;
ring A is selected from the group consisting of aryl and heteroaryl;
ring B is selected from the group consisting of heterocyclyl, aryl and heteroaryl;
R₁ is selected from the group consisting of hydrogen, alkyl, hydroxyalkyl, heterocyclyl, heteroaryl and -(CH₂)ₙ₁ORₐₐ;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₄ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
R₅ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

4. The compound, the stereoisomer thereof or the pharmaceutically acceptable salt thereof according to claim 1, **characterized in that** the formula (I) is further as shown in formula (IV): 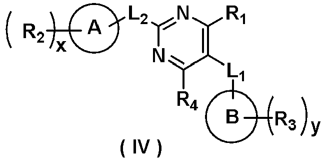 wherein:
L₁ is selected from the group consisting of a bond, oxygen, alkenyl, alkynyl, heterocyclyl and -NRₐₐ-;
L₂ is selected from the group consisting of a bond and sulfur;
ring A is selected from the group consisting of aryl and heteroaryl;
ring B is selected from the group consisting of cycloalkyl, heterocyclyl, aryl and heteroaryl;
R₁ is selected from the group consisting of hydrogen, alkyl, hydroxyalkyl, heterocyclyl, heteroaryl and -(CH₂)ₙ₁ORₐₐ;
R₂ is selected from the group consisting of hydrogen, halogen, hydroxyalkyl, amino, cyano, alkyl, cycloalkyl, heterocyclyl, -(CH₂)ₙ₁C(O)ORₐₐ and -(CH₂)ₙ₁C(O)NRₐₐR_{bb};
R₃ is selected from the group consisting of hydrogen, amino, alkyl, cycloalkyl, heterocyclyl, aryl, heteroaryl, -(CH₂)ₙ₁NRₐₐR_{bb} and -(CRₐₐR_{bb})ₙ₁NR_{cc}R_{dd}, wherein the amino, alkyl, cycloalkyl, heterocyclyl, aryl and heteroaryl are each optionally further substituted by one or more substituent(s) selected from the group consisting of hydrogen, halogen, hydroxy, alkyl and amino;
R₄ is selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl and heteroaryl;
Rₐₐ, R_{bb}, R_{cc} and R_{dd} are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl;
x is 0, 1, 2, 3, 4 or 5;
y is 0, 1, 2, 3, 4 or 5;
m₁ is 0, 1 or 2; and
n₁ is 0, 1, 2, 3, 4 or 5.

5. The compound, the stereoisomer thereof or the pharmaceutically acceptable salt thereof according to claim 1, **characterized in that** the formula (I) is further as shown in formula (V): 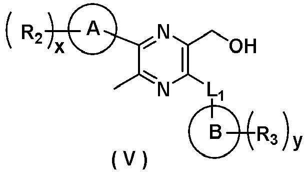 wherein:
ring A, ring B, L₁, R₂, R₃, x and y are as defined in claim 2.

6. The compound, the stereoisomer thereof or the pharmaceutically acceptable salt thereof according to claim 1, **characterized in that** the formula (I) is further as shown in formula (VI): 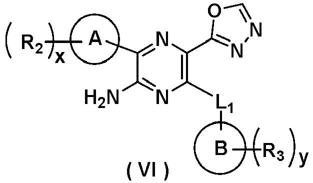 wherein:
ring A, ring B, L₁, R₂, R₃, x and y are as defined in claim 2.

7. The compound, the stereoisomer thereof or the pharmaceutically acceptable salt thereof according to claim 1, **characterized in that** the formula (I) is further as shown in formula (VII): 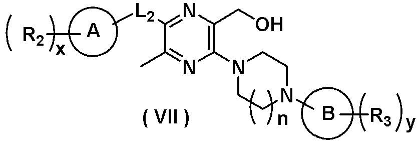 wherein:
n is 0,1,2 or 3; and
ring A, ring B, L₂, R₂, R₃, x and y are as defined in claim 2.

8. The compound, the stereoisomer thereof or the pharmaceutically acceptable salt thereof according to claim 1, **characterized in that** the formula (I) is further as shown in formula (IIA) or (IIB): 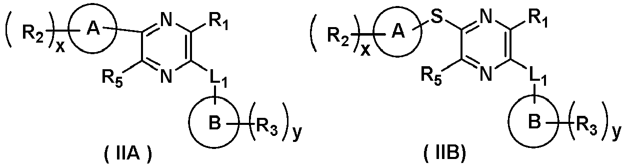 wherein:
ring A, ring B, L₁, R₁ to R₃, R₅, x and y are as defined in claim 2.

9. The compound, the stereoisomer thereof or the pharmaceutically acceptable salt thereof according to claim 1, **characterized in that** the formula (I) is further as shown in formula (IIIA) or (IIIB): 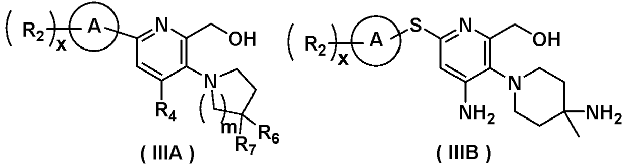 wherein:
m is 0, 1, 2 or 3;
R₆ and R₇ are identical or different and are each independently selected from the group consisting of hydrogen, deuterium, alkyl, deuterated alkyl, haloalkyl, alkoxy, hydroxyalkyl, haloalkoxy, halogen, amino, nitro, hydroxy, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁NRₐₐR_{bb}, -(CRₐₐR_{bb})ₙ₁NR_{cc}R_{dd}, -(CH₂)ₙ₁C(O)NRₐₐR_{bb} and -(CH₂)ₙ₁NRₐₐC(O)R_{bb};
or, R₆ and R₇ are bonded to form a cycloalkyl, heterocyclyl, aryl or heteroaryl, wherein the cycloalkyl, heterocyclyl, aryl and heteroaryl are each further substituted by one or more substituent(s) selected from the group consisting of deuterium, alkyl, cycloalkyl, haloalkyl, halogen, amino, oxo, thioxo, nitro, cyano, hydroxy, alkenyl, alkynyl, alkoxy, haloalkoxy, hydroxyalkyl, heterocyclyl, aryl and heteroaryl; and
ring A, R₂, R₄, Rₐₐ, R_{bb}, R_{cc}, R_{dd} and x are as defined in claim 3.

10. The compound, the stereoisomer thereof or the pharmaceutically acceptable salt thereof according to claim 1, **characterized in that** the formula (I) is further as shown in formula (IVA) or (IVB): 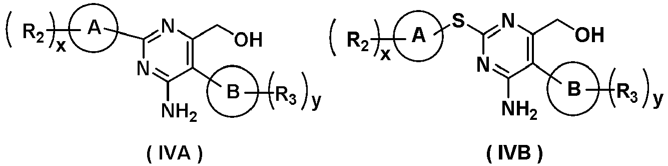 wherein:
ring A, ring B, R₂, R₃, x and y are as defined in claim 4.

11. The compound, the stereoisomer thereof or the pharmaceutically acceptable salt thereof according to claim 1, **characterized in that** the formula (I) is further as shown in formula (VA): 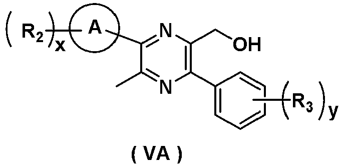 wherein:
ring A, R₂, R₃, x and y are as defined in claim 2.

12. The compound, the stereoisomer thereof or the pharmaceutically acceptable salt thereof according to claim 1, **characterized in that** the formula (I) is further as shown in formula (VIII): 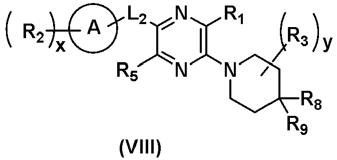 wherein:
L₂ is selected from the group consisting of a bond and sulfur;
ring A is selected from the group consisting of C₆₋₁₂ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₃ is selected from the group consisting of hydrogen, amino, halogen, oxo, hydroxy, C₁₋₆ alkyl and C₃₋₈ cycloalkyl;
R₅ is selected from the group consisting of hydrogen, deuterium, halogen, amino, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl and C₃₋₈ cycloalkyl;
R₈ and R₉ are identical or different and are each independently selected from the group consisting of hydrogen, deuterium, halogen, amino, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl and 5 to 12 membered heteroaryl;
or, R₈ and R₉ are bonded to form a C₃₋₁₂ cycloalkyl or 3 to 12 membered heterocyclyl, wherein the C₃₋₁₂ cycloalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
n₁ is 0, 1, 2, 3, 4 or 5;
x is 0, 1,2,3,4 or 5; and
y is 0, 1, 2, 3, 4 or 5.

13. The compound, the stereoisomer thereof or the pharmaceutically acceptable salt thereof according to claim 1, **characterized in that** the formula (I) is further as shown in formula (IX): 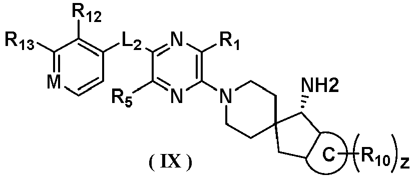 wherein:
M is selected from the group consisting of CR₁₁ and N;
L₂ is selected from the group consisting of a bond and sulfur;
ring C is selected from the group consisting of C₃₋₈ cycloalkyl, 3 to 12 membered heterocyclyl, C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of hydrogen, amino and C₁₋₆ alkyl;
R₁₀ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₁ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₃ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and
z is 0, 1, 2 or 3.

14. The compound, the stereoisomer thereof or the pharmaceutically acceptable salt thereof according to claim 1, **characterized in that** the formula (I) is further as shown in formula (X): 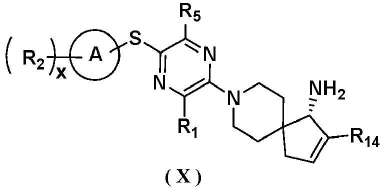 wherein:
ring A is selected from the group consisting of C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of hydrogen, amino and C₁₋₆ alkyl;
R₁₄ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and x is 0, 1, 2, 3, 4 or 5.

15. The compound, the stereoisomer thereof or the pharmaceutically acceptable salt thereof according to claim 1, **characterized in that** the formula (I) is further as shown in formula (XI): 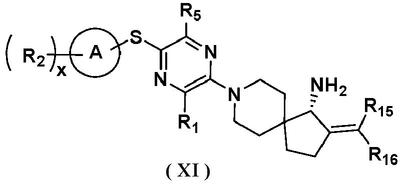 wherein:
ring A is selected from the group consisting of C₆₋₁₀ aryl and 5 to 12 membered heteroaryl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, C₁₋₆ alkoxy, C₁₋₆ hydroxyalkyl, 3 to 12 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₅ is selected from the group consisting of amino and C₁₋₆ alkyl;
R₁₅ and R₁₆ are identical or different and are each independently selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl; and
x is 0, 1, 2, 3, 4 or 5.

16. The compound, the stereoisomer thereof or the pharmaceutically acceptable salt thereof according to claim 14, **characterized in that** the formula (I) is further as shown in formula (X-A): 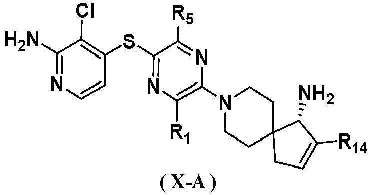 wherein:
R₁, R₅ and R₁₄ are as defined in claim 14.

17. The compound, the stereoisomer thereof or the pharmaceutically acceptable salt thereof according to claim 1, **characterized in that** the formula (I) is further as shown in formula (XII): 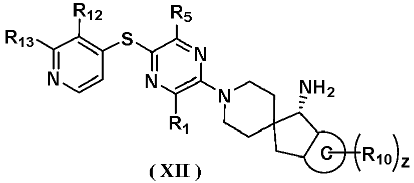 wherein:
ring C is selected from the group consisting of C₃₋₈ cycloalkyl, 3 to 12 membered heterocyclyl, C₆₋₁₀ aryl and 5 to 12 membered heteroaryl, and preferably cyclopropyl, phenyl, pyridyl, pyrazolyl, imidazolyl, oxazolyl or thiazolyl;
R₁ is selected from the group consisting of hydrogen, halogen, C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl, wherein the C₁₋₆ alkyl, C₃₋₈ cycloalkyl, C₁₋₆ alkoxy, C₁₋₆ hydroxyalkyl, 3 to 12 membered heterocyclyl and 5 to 12 membered heteroaryl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁ is preferably hydrogen, halogen, C₁₋₃ alkyl, C₁₋₃ hydroxyalkyl or C₃₋₆ cycloalkyl, wherein the C₁₋₃ alkyl, C₁₋₃ hydroxyalkyl or C₃₋₆ cycloalkyl is optionally further substituted by one or more substituent(s) selected from the group consisting of hydrogen, halogen and hydroxy;
R₁ is more preferably hydrogen, fluorine, chlorine, bromine, methyl, hydroxyethyl or cyclopropyl substituted by hydroxy;
R₅ is selected from the group consisting of hydrogen, amino and C₁₋₆ alkyl, and preferably hydrogen, amino or methyl;
R₁₀ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₀ is preferably hydrogen, halogen, C₁₋₃ alkyl or C₃₋₆ cycloalkyl;
R₁₀ is more preferably hydrogen, chlorine or methyl;
R₁₂ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₂ is preferably halogen, C₁₋₃ alkyl or C₃₋₆ cycloalkyl;
R₁₂ is more preferably fluorine, chlorine, cyclopropyl or methyl;
R₁₃ is selected from the group consisting of hydrogen, halogen, amino, cyano, C₁₋₆ alkyl, C₃₋₈ cycloalkyl, C₁₋₆ alkoxy, C₁₋₆ hydroxyalkyl and 3 to 12 membered heterocyclyl, wherein the C₁₋₆ alkyl, C₃₋₈ cycloalkyl, C₁₋₆ alkoxy, C₁₋₆ hydroxyalkyl and 3 to 12 membered heterocyclyl are each optionally further substituted by one or more substituent(s) selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₁₃ is more preferably hydrogen, fluorine, chlorine, amino, methyl, cyclopropyl, pyrrolidinyl, azetidinyl, fluorine-substituted azetidinyl, azetidinonyl, morpholinyl or pyrrolidonyl;
z is 0, 1, 2 or 3; and
when R₁₂ is chlorine and R₁₃ is amino, then R₁ is not hydrogen.

18. The compound, the stereoisomer thereof or the pharmaceutically acceptable salt thereof according to claim 17, **characterized in that** the formula (I) is further as shown in formula (XII-A): 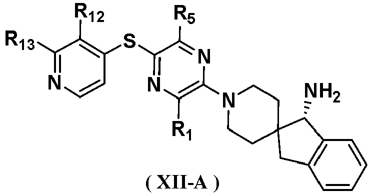 wherein:
R₁, R₅ and R₁₂ to R₁₃ are as defined in claim 17.

19. The compound, the stereoisomer thereof or the pharmaceutically acceptable salt thereof according to claim 17, **characterized in that** the formula (I) is further as shown in formula (XII-B): 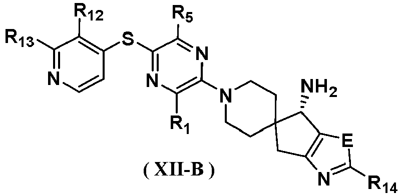 wherein:
E is selected from the group consisting of O, S and NR₁₅;
R₁₄ is selected from the group consisting of hydrogen, halogen and C₁₋₆ alkyl;
R₁₅ is selected from the group consisting of hydrogen and C₁₋₆ alkyl;
R₁, R₅ and R₁₂ to R₁₃ are as defined in claim 17.

20. The compound, the stereoisomer thereof or the pharmaceutically acceptable salt thereof according to any one of claims 1 to 14, **characterized in that**:
ring A is selected from the group consisting of:

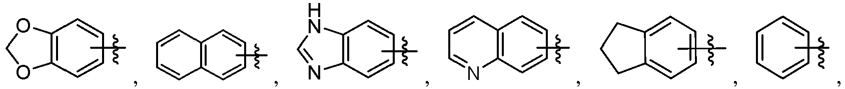

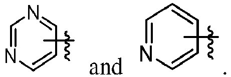

21. The compound, the stereoisomer thereof or the pharmaceutically acceptable salt thereof according to any one of claims 1 to 10, **characterized in that**,
ring B is selected from the group consisting of:

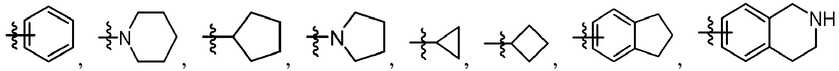

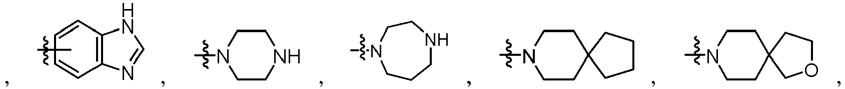

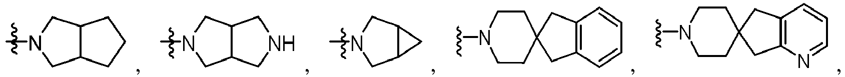

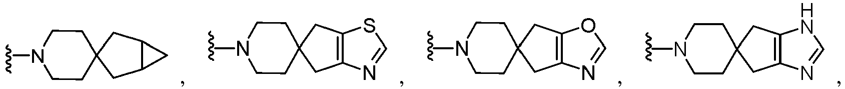

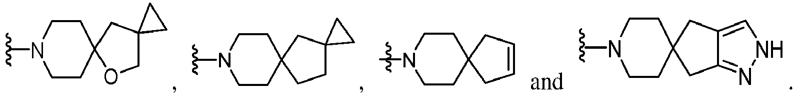

22. The compound, the stereoisomer thereof or the pharmaceutically acceptable salt thereof according to claim 13 or 17, **characterized in that**:
ring C is selected from the group consisting of:

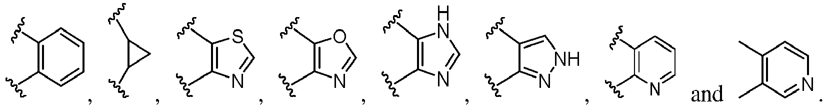

23. The compound, the stereoisomer thereof or the pharmaceutically acceptable salt thereof according to any one of claims 1 to 22, **characterized in that**,
R₁ is selected from the group consisting of hydrogen, halogen, C₁₋₆ alkyl, C₃₋₈ cycloalkyl, C₁₋₆ hydroxyalkyl, 3 to 8 membered heterocyclyl, 5 to 8 membered heteroaryl and -(CH₂)ₙ₁ORₐₐ, wherein the C₁₋₆ alkyl, C₃₋₈ cycloalkyl, C₁₋₆ hydroxyalkyl, 3 to 8 membered heterocyclyl and 5 to 8 membered heteroaryl are each optionally further substituted by one or more substituent(s) selected from the group consisting of hydrogen, deuterium, halogen, amino, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₂ is selected from the group consisting of hydrogen, halogen, amino, cyano, C₁₋₆ alkyl, C₁₋₆ haloalkyl, C₃₋₈ cycloalkyl, 3 to 8 membered heterocyclyl, -(CH₂)ₙ₁C(O)ORₐₐ and -(CH₂)ₙ₁C(O)NRₐₐR_{bb}, wherein the C₁₋₆ alkyl, C₃₋₈ cycloalkyl and 3 to 8 membered heterocyclyl are each optionally further substituted by one or more substituent(s) selected from the group consisting of hydrogen, deuterium, halogen, amino, hydroxy, cyano, oxo, C₁₋₆ alkyl, C₁₋₆ deuterated alkyl, C₁₋₆ haloalkyl, C₁₋₆ alkoxy, C₁₋₆ hydroxyalkyl, C₁₋₆ haloalkoxy, C₂₋₆ alkenyl, C₂₋₆ alkynyl, C₃₋₈ cycloalkyl, 3 to 12 membered heterocyclyl, C₆₋₁₂ aryl and 5 to 12 membered heteroaryl;
R₃ is selected from the group consisting of hydrogen, amino, C₁₋₆ alkyl, C₃₋₈ cycloalkyl, 3 to 8 membered heterocyclyl, 5 to 12 membered heteroaryl, -(CH₂)ₙ₁NRₐₐR_{bb} and -(CRₐₐR_{bb})ₙ₁NR_{cc}R_{dd};
R₄ is selected from the group consisting of hydrogen, halogen, amino, C₁₋₆ alkyl and C₁₋₆ hydroxyalkyl;
R₈ is selected from the group consisting of hydrogen, C₁₋₆ alkyl and amino;
R₁₄ is selected from the group consisting of hydrogen, C₁₋₆ alkyl, C₃₋₈ cycloalkyl, 3 to 8 membered heterocyclyl and 5 to 12 membered heteroaryl;
R₁₅ and R₁₆ are each independently selected from the group consisting of hydrogen and C₁₋₆ alkyl;
Rₐₐ and R_{bb} are each independently selected from the group consisting of hydrogen, C₁₋₆ alkyl, C₃₋₆ cycloalkyl, C₁₋₆ haloalkyl and C₁₋₆ alkoxy; and
R_{cc} and R_{dd} are each independently selected from the group consisting of hydrogen, C₁₋₆ alkyl, C₃₋₆ cycloalkyl, C₁₋₆ haloalkyl and C₁₋₆ alkoxy.

24. The compound, the stereoisomer thereof or the pharmaceutically acceptable salt thereof according to claim 13, **characterized in that**,
R₁ is selected from the group consisting of hydrogen, halogen, C₁₋₃ alkyl, C₃₋₆ cycloalkyl, C₁₋₃ hydroxyalkyl, C₁₋₃ alkoxy, 3 to 6 membered heterocyclyl and 5 to 6 membered heteroaryl, wherein the C₁₋₃ alkyl, C₁₋₃ hydroxyalkyl, C₁₋₃ alkoxy, C₃₋₆ cycloalkyl, 3 to 6 membered heterocyclyl and 5 to 6 membered heteroaryl are each optionally further substituted by one or more substituent(s) selected from the group consisting of hydrogen, deuterium, halogen, amino, hydroxy, cyano, oxo, C₁₋₃ alkyl, C₁₋₃ deuterated alkyl, C₁₋₃ haloalkyl, C₁₋₃ alkoxy, C₁₋₃ hydroxyalkyl, C₁₋₃ haloalkoxy, C₂₋₄ alkenyl, C₂₋₄ alkynyl, C₃₋₆ cycloalkyl, 3 to 16 membered heterocyclyl, C₆₋₁₂ aryl and 5 to 12 membered heteroaryl;
R₅ is selected from the group consisting of hydrogen, amino and C₁₋₃ alkyl;
R₁₂ and R₁₃ are each independently selected from the group consisting of hydrogen, halogen, amino, C₃₋₆ cycloalkylamino, cyano, C₁₋₃ alkyl, C₁₋₃ haloalkyl, C₃₋₆ cycloalkyl and 3 to 6 membered heterocyclyl containing 1 to 2 nitrogen or oxygen atom(s), optionally further substituted by one or more substituent(s) selected from the group consisting of deuterium, halogen, amino, hydroxy, cyano, oxo, C₁₋₃ alkyl, C₁₋₃ deuterated alkyl, C₁₋₃ haloalkyl, C₁₋₃ alkoxy, C₁₋₃ hydroxyalkyl, C₁₋₃ haloalkoxy, C₂₋₄ alkenyl, C₂₋₄ alkynyl, C₃₋₆ cycloalkyl, 3 to 6 membered heterocyclyl, C₆₋₁₂ aryl and 5 to 12 membered heteroaryl; preferably, the heterocyclyl is selected from the group consisting of

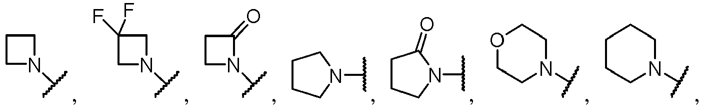

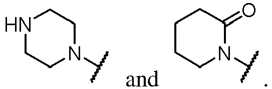

25. The compound, the stereoisomer thereof or the pharmaceutically acceptable salt thereof according to any one of claims 1 to 24, **characterized by** being selected from the group consisting of: 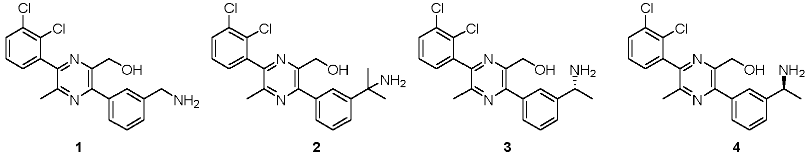 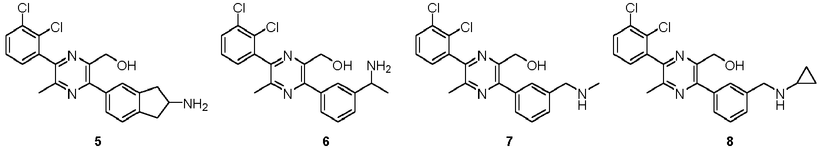 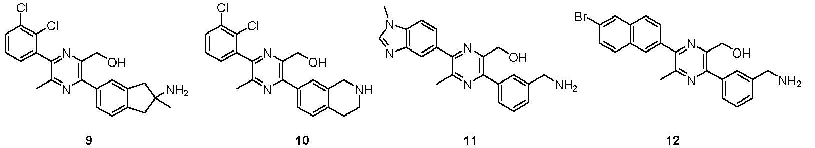 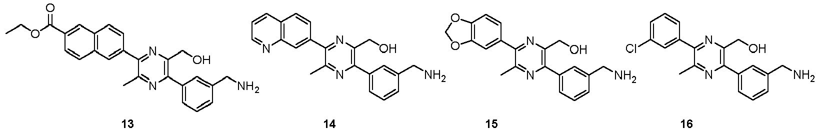 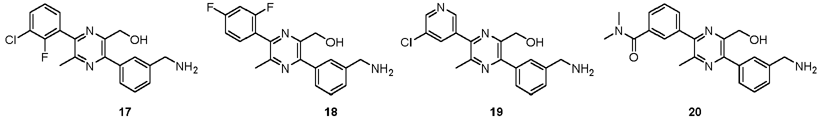 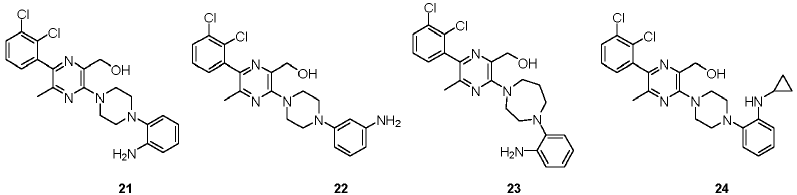 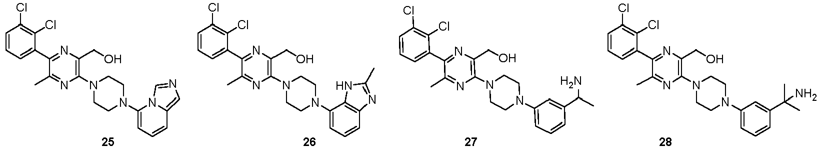 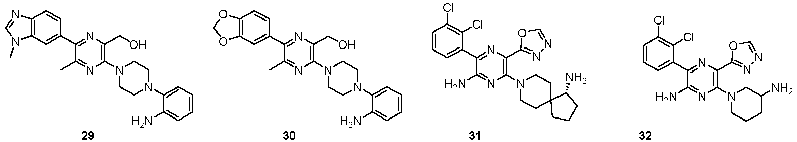 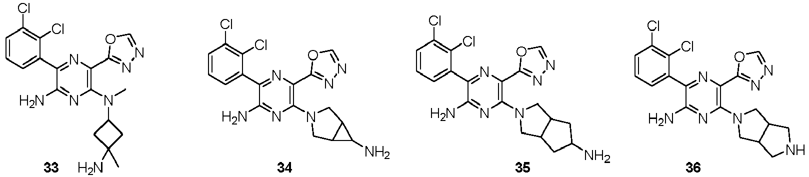 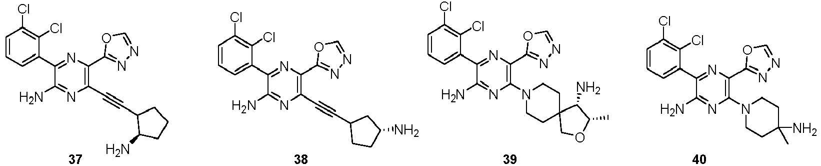 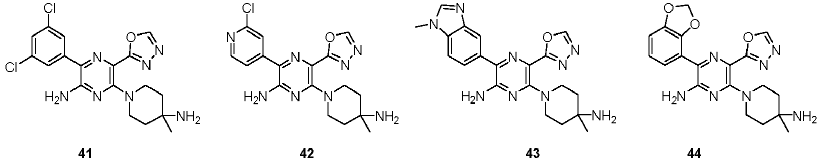 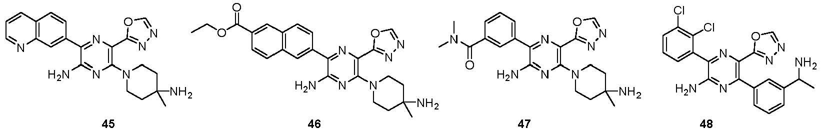 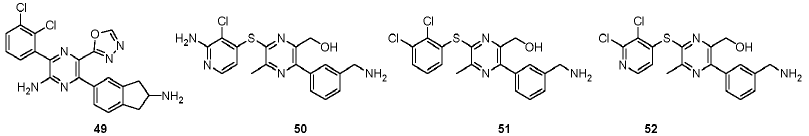 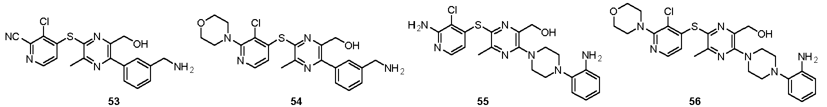 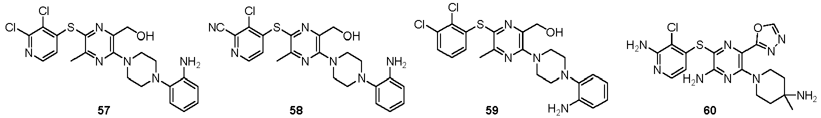 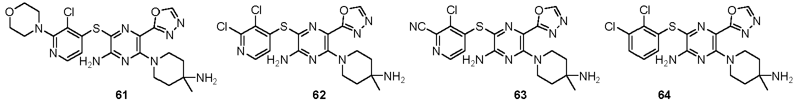 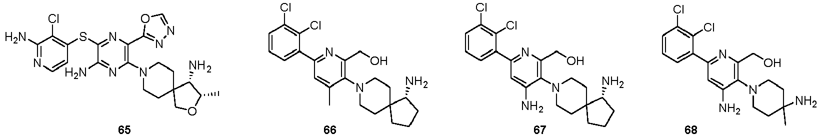 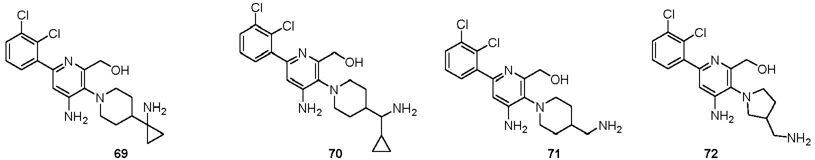 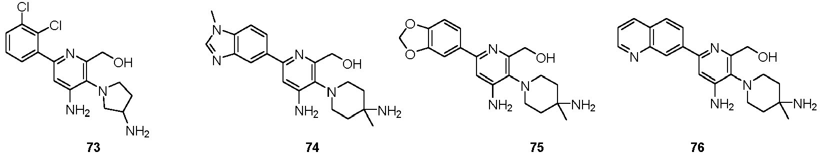 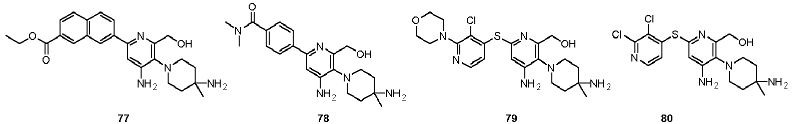 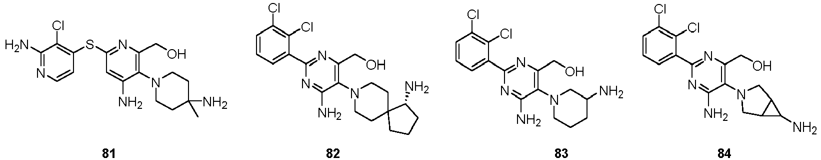 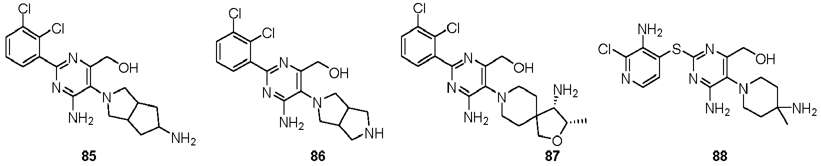 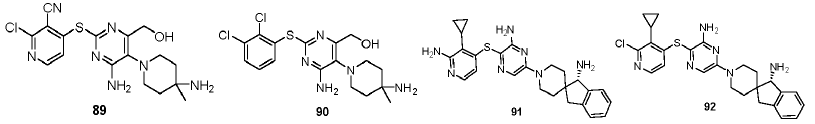 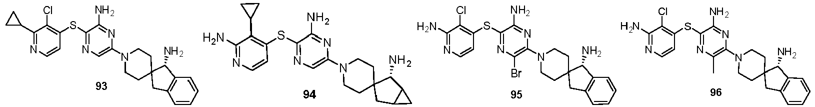 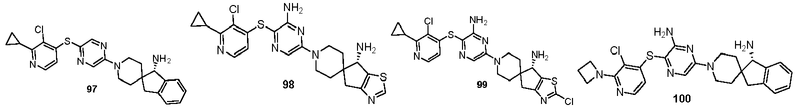 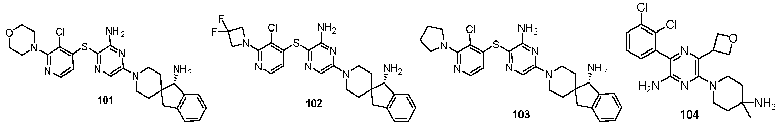 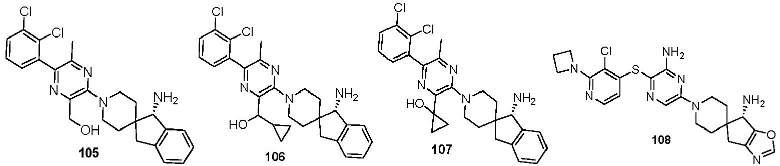 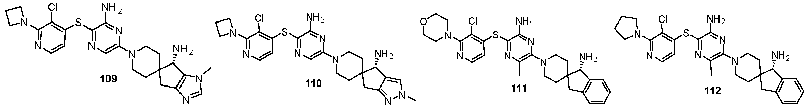 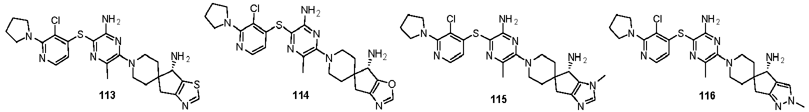 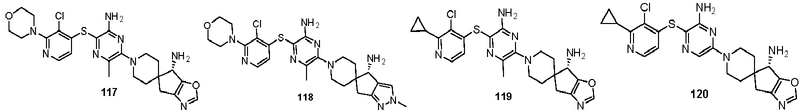 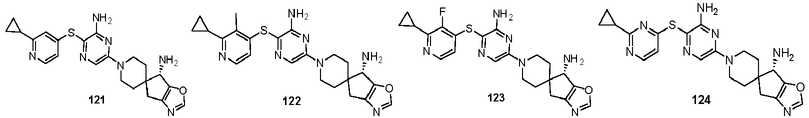 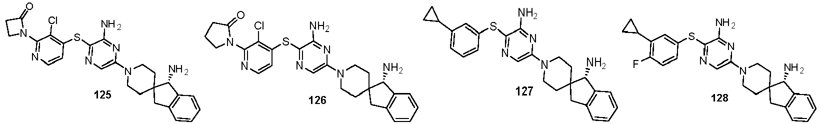 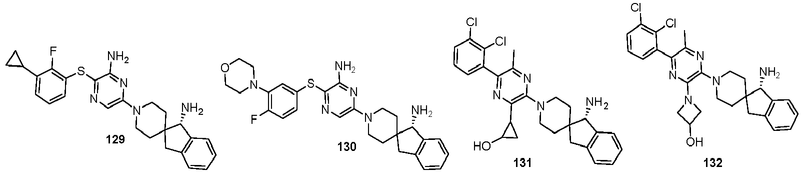 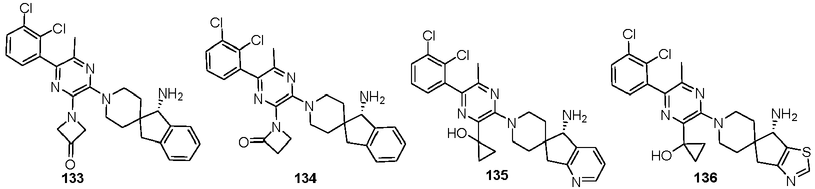 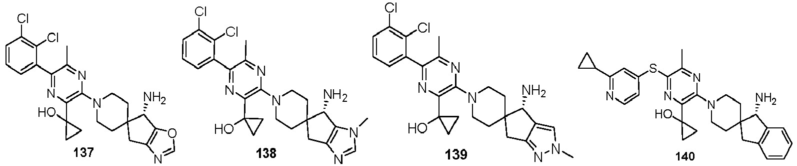 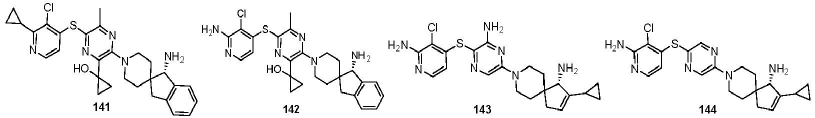 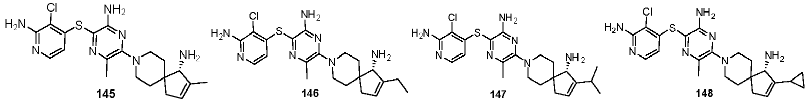 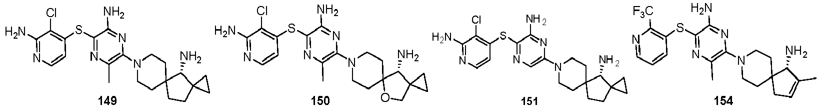 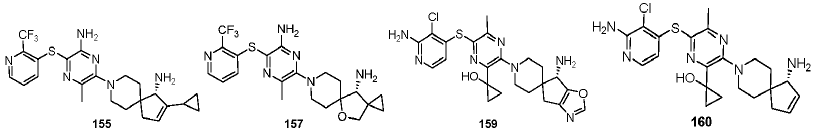 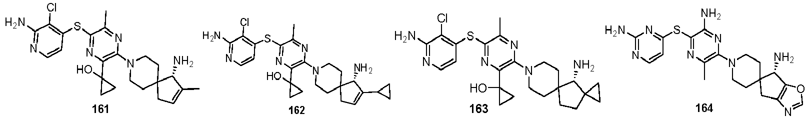 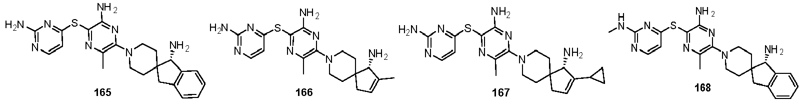 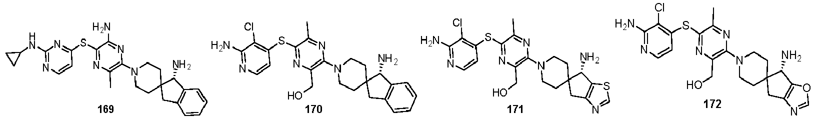 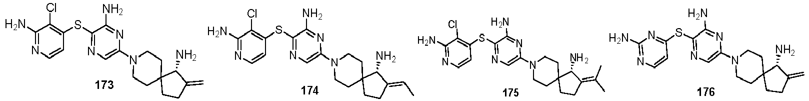 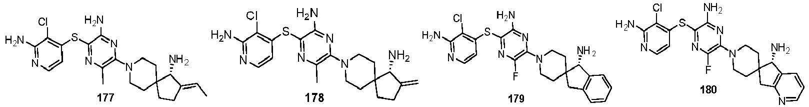 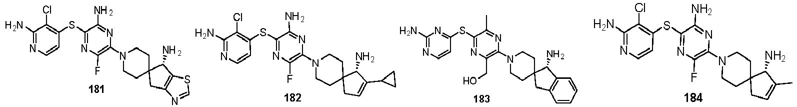 and

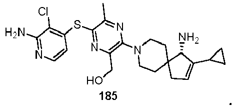

26. A method for preparing the compound, the stereoisomer thereof or the pharmaceutically acceptable salt thereof according to claim 17, **characterized by** comprising the following step of: 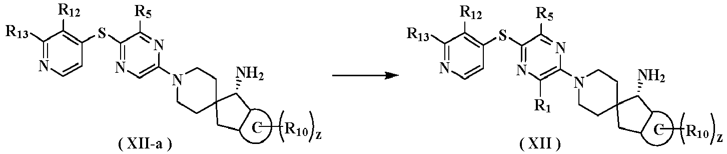 subjecting a compound of formula (XII-a) to a substitution reaction to obtain the compound of formula (XII), the stereoisomer thereof or the pharmaceutically acceptable salt thereof;
wherein:
ring C, R₁, R₅, R₁₀, R₁₂, R₁₃ and z as defined in claim 17.

27. The method according to claim 26, **characterized by** further comprising the following step of: 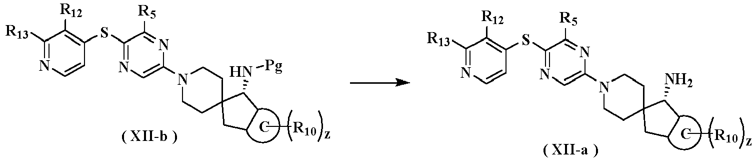 deprotecting a compound of formula (XII-b) to obtain the compound of formula (XII-a), the stereoisomer thereof or the pharmaceutically acceptable salt thereof;
wherein:
Pg is an amino protecting group selected from the group consisting of *tert*-butylsulfinyl, benzyloxycarbonyl, *tert*-butoxycarbonyl, 9-fluorenylmethoxycarbonyl, benzyl, *p*-methoxybenzyl, allyloxycarbonyl, trityl and phthaloyl, and preferably *tert*-butylsulfinyl.

28. The method according to claim 27, **characterized by** further comprising the following step of: 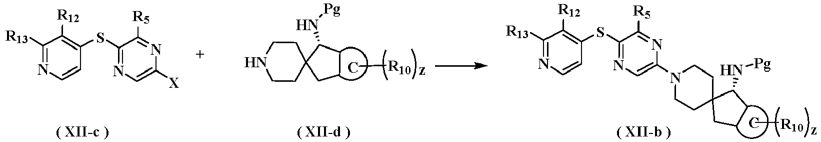 reacting a compound of formula (XII-c) with a compound of formula (XII-d) to obtain the compound of formula (XII-b), the stereoisomer thereof or the pharmaceutically acceptable salt thereof;
wherein:
X is a halogen, preferably fluorine, chlorine, bromine or iodine, and more preferably chlorine.

29. A pharmaceutical composition, comprising a therapeutically effective amount of the compound, the stereoisomer thereof or the pharmaceutically acceptable salt thereof according to any one of claims 1 to 25, and one or more pharmaceutically acceptable carrier(s), diluent(s) or excipient(s).

30. Use of the compound, the stereoisomer thereof or the pharmaceutically acceptable salt thereof according to any one of claims 1 to 25, or the pharmaceutical composition according to claim 29 in the preparation of a SHP-2 inhibitor medicament.

31. Use of the compound, the stereoisomer thereof or the pharmaceutically acceptable salt thereof according to any one of claims 1 to 25, or the pharmaceutical composition according to claim 29 in the preparation of a medicament for treating a disease or condition such as Noonan syndrome, leopard syndrome, leukemia, neuroblastoma, melanoma, esophageal cancer, head and neck tumor, breast cancer, lung cancer and colon cancer, and preferably non-small cell lung cancer, esophageal cancer or head and neck tumor.
